# EUROPEAN PATENT APPLICATION

(11) **EP 4 671 247 A1**
(43) Date of publication of application: **31.12.2025**
(21) Application number: 24760425.9
(22) Date of filing: 22.02.2024
(51) Int. Cl.: C07D 487/04, A61K 31/519, A61P 1/04, A61P 1/16, A61P 9/00, A61P 9/10, A61P 17/00, A61P 19/02, A61P 21/00, A61P 25/00, A61P 25/14, A61P 25/16, A61P 25/28, A61P 29/00

(54) **SUBSTITUTED PYRAZOLOPYRIMIDINE COMPOUND AND PHARMACEUTICAL USE THEREOF**

(30) Priority: 24.02.2023 JP 2023027432
(71) Applicant: Japan Tobacco Inc., Tokyo 105-6927 (JP)
(72) Inventor: OHBA, Yusuke, Takatsuki-shi, Osaka 569-1125 (JP); ADACHI, Kaoru, Takatsuki-shi, Osaka 569-1125 (JP); SAKURAI, Kentaro, Takatsuki-shi, Osaka 569-1125 (JP); SATO, Shimpei, Takatsuki-shi, Osaka 569-1125 (JP)
(74) Representative: Vossius & Partner Patentanwälte Rechtsanwälte mbB
(86) International application number: PCT/JP2024/006433
(87) International publication number: WO 2024/177127

(57) **Abstract**

Provided are substituted pyrazolopyrimidine compounds, or pharmaceutically acceptable salts thereof, having NLRP3 inflammasome inhibitory activity, pharmaceutical compositions comprising the same, and their medical use, etc. A compound of Formula [I], or a pharmaceutically acceptable salt thereof, wherein each symbol is as defined in the specification.

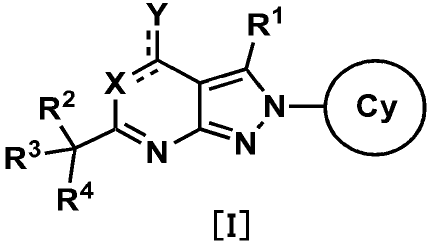

## Description

### TECHNICAL FIELD

The present invention relates to substituted pyrazolopyrimidine compounds, or pharmaceutically acceptable salts thereof, having NLRP3 inflammasome inhibitory activity, pharmaceutical compositions comprising the same, and medical use thereof.

### BACKGROUND ART

NLRP3 (NOD-, LRR-, and pyrin domain-containing protein 3) is a pattern recognition receptor that belongs to an NLR (NOD-like receptors) family, and is also expressed in non-immune cells such as glomerular epithelial cells and tubular epithelial cells as well as phagocytes such as macrophage and microglia.

NLRP3 recognizes DAMPs (Danger Associated Molecular Patterns) which are a molecular pattern specific to cellular damage factors, such as ATP, HMGB1, S100, urate crystals, and silica, and PAMPs (Pathogen Associated Molecular Patterns) which are a molecular pattern specific to pathogenic microorganisms, such as viruses, bacteria, and fungi, and binds to these molecules to be activated.

Activated NLRP3 associates with an adaptor protein, ASC (Apoptosis-associated speck-like protein containing a caspase recruitment domain), and a cysteine protease, caspase 1, by protein-protein interaction to form an NLRP3 inflammasome, which is a cellular protein complex. The formation of an NLRP3 inflammasome converts caspase 1 in the complex into its activated form, and the activated caspase 1 converts proIL-1β, which is a precursor of a proinflammatory cytokine, IL-1β, into an activated form of IL-1β, while it also converts proIL-18, which is a precursor of IL-18, into an activated form of IL-18. The activated IL-1β secreted outside the cell induces proinflammatory cytokine-chemokine production by surrounding cells, and activates immune cells such as T cells, which causes inflammatory reactions.

In multiple sclerosis patients, the increase of the amount of DAMPs was observed in the brain and cerebral spinal fluid (Non Patent Literature 1), and the increase of the expression level of caspase 1 in involved sites and the increase of the amount of IL-1β in cerebral spinal fluid were also observed (Non Patent Literature 2). It has been reported that activated microglia was present in involved sites during the chronic progressive phase of this disease (Non Patent Literature 3), and the activated microglia stimulated by DAMPs produced proinflammatory cytokine such as IL-1β, which induced nerve inflammation and nerve disorder (Non Patent Literature 4). Thus, an NLRP3 inflammasome is considered to get involved in the expression of disease states of multiple sclerosis.

MOG₃₅₋₅₅EAE model mice prepared by sensitization of Myelin Oligodendrocyte Glycoprotein (MOG) expressed impairment of motor function as seen in multiple sclerosis. The onset of the impairment of motor function was inhibited in NLRP3-knockout mice in the MOG₃₅₋₅₅EAE model (Non Patent Literature 5). Demyelination of central nerve as seen in multiple sclerosis was expressed in cuprizone-model mice prepared by administration of a copper-chelate compound, cuprizone, to mice, while the progress of demyelination was delayed in NLRP3-knockout mice in the cuprizone model (Non Patent Literature 6). Administration of an NLPR3 inflammasome inhibitor, JC-171, after the onset inhibited the impairment of motor function in the MOG₃₅₋₅₅EAE model (Non Patent Literature 7). Thus, an NLRP3 inflammasome inhibitor is considered to become a drug for treating multiple sclerosis.

The increase of the expression of NLRP3 inflammasome-related genes has been reported in the kidney of patients suffering from chronic kidney disease (Non Patent Literatures 8, 9). Further, the inhibitory activity of proteinuria and tubulointerstitial fibrosis by NLRP3-knockout has been reported in a non-clinical chronic kidney disease model, i.e., a 5/6 kidney-enucleated model (Non Patent Literature 10). Accordingly, an NLRP3 inflammasome inhibitor is considered to become a drug for treating chronic kidney disease.

The increase of the expression of NLRP3 inflammasome-related genes has been reported in the intestine of patients suffering from inflammatory bowel disease (for example, ulcerative colitis and Crohn's disease) (Non Patent Literature 11). It has been reported that IL-1β produced by the activation of NLRP 3 was increased in the intestinal mucosa of IBD patients, and that the increased IL-1β secretion from the colonic region was positively correlated with the deterioration of the disease state (Non Patent Literature 11). It has also been reported that the dysfunction of CARD8, which negatively regulates inflammasome activity, increases susceptibility to Crohn's disease, and that the activation of NLRP3 inflammasome enhances IL-1β production from monocytes (Non Patent Literature 12). The suppression of intestinal pathology by NLRP3 deficiency has been reported in TNBS-induced colitis model, a colitis model (Non Patent Literature 13). Accordingly, an NLRP3 inflammasome inhibitor is considered to become a drug for treating inflammatory bowel disease.

The increase of the expression of NLRP3 inflammasome-related genes has been reported in the arteriosclerotic region of coronary arteries of patients suffering from myocardial infarction (Non Patent Literature 14). In addition, the suppressed lesion formation by NLRP3-knockout has been reported in low-density lipoprotein receptor (LDL) receptor-deficient mice fed high-fat diet, an arteriosclerosis model (Non Patent Literature 15). Accordingly, an NLRP3 inflammasome inhibitor is considered to become a drug for treating arteriosclerosis.

Cryopyrin-associated periodic syndrome (CAPS), a generic name of autoinflammatory diseases caused by activating mutation of NLRP3 gene, is classified into 3 disease types as follows: a mild disease type of familial cold autoinflammatory syndrome (FCAS), a moderate disease type of Muckle-Wells syndrome (MWS), a severe disease type of chronic infantile neurologic cutaneous and articular syndrome (CINCA) or Neonatal onset multisystem inflammatory disease (NOMID) (Non Patent Literature 16). More than 200 mutations in NLRP3 gene have been reported in CAPS (Non Patent Literature 17). These NLRP3 gene mutations cause the formation and activation of NLRP3 inflammasome even in the absence of an activation signal. Mice expressing CAPS-related NLRP3 mutations exhibit systemic lethal inflammation dependent on IL-1β and IL-18 which are NLRP3 inflammasome and a downstream signal transduction molecule (Non Patent Literature 18). In a mouse strain expressing CAPS-related NLRP3 mutations, CY-09, an NLRP3 inflammasome inhibitor, suppressed systemic lethal inflammation and improved the survival (Non Patent Literature 19). Accordingly, an NLRP3 inflammasome inhibitor is considered to become a drug for treating CAPS.

The increase of the expression of NLRP3 inflammasome-related genes has been reported in liver tissues of patients suffering from nonalcoholic steato-hepatitis (NASH) (Non Patent Literature 20). In addition, the suppressed hepatic fibrogenesis by NLRP3-knockout has been reported in a choline deficient amino acid defined diet fed model, an NASH model (Non Patent Literature 20). Accordingly, an NLRP3 inflammasome inhibitor is considered to become a drug for treating NASH.

In gout and gouty arthritis, urate crystals deposited in the joint and periarticular tissues induce inflammation (Non Patent Literature 21). Urate crystals activate macrophage NLRP3 to produce IL-1β and IL-18 (Non Patent Literature 22). OLT1177, an NLRP3 inflammasome inhibitor, suppressed arthritis in an intra-articular urate-injected arthritis model (Non Patent Literature 23). Accordingly, an NLRP3 inflammasome inhibitor is considered to become a drug for treating gout and gouty arthritis.

The increase of the expression of NLRP3 inflammasome-related genes has been reported in joint synovium, peripheral-blood mononuclear cells of patients suffering from rheumatoid arthritis (Non Patent Literature 24). In addition, the increase of the expression of NLRP3 inflammasome-related genes in synovium has been reported in collagen-induced arthritis, a model of rheumatoid arthritis (Non Patent Literature 25). Accordingly, an NLRP3 inflammasome inhibitor is considered to become a drug for treating rheumatoid arthritis.

It has been reported that trinitrochlorobenzene, which induces contact dermatitis, increased IL-1β production from human skin keratinocytes via NLRP3 activation, and that NLRP3 knockout inhibits development of dermatitis in a trinitrochlorobenzene-induced dermatitis model, a model of contact dermatitis (Non Patent Literature 26). Accordingly, an NLRP3 inflammasome inhibitor is considered to become a drug for treating contact dermatitis.

The increase of the expression of NLRP3 inflammasome-related genes has been reported in the tear fluid and ocular surface of patients suffering from dry eye (Non Patent Literatures 27 and 28). In addition, it has been reported that increased expression of NLRP3 inflammasome-related genes and increased IL-1β production were observed when hypertonic stress was applied to cultured human corneal epithelial cells to induce a dry eye condition, and that IL-1β production was suppressed by knockdown of NLRP3 gene (Non Patent Literature 28). Accordingly, an NLRP3 inflammasome inhibitor is considered to become a drug for treating dry eye.

The increase of the expression of ASC domain of NLRP3 inflammasome has been reported in macrophages and neutrophils infiltrated into myocardial tissue of patients suffering from acute myocardial infarction (Non Patent Literature 29). In addition, it has been reported that the increased expression of NLRP3 inflammasome-related genes were observed in the infarct site in an ischemia-reperfusion model, a model of myocardial infarction, and that knockdown of NLRP3 gene decreased the infarct area and suppressed the reduction of myocardial contractility (Non Patent Literature 30). Accordingly, an NLRP3 inflammasome inhibitor is considered to become a drug for treating ischemic heart disease such as acute myocardial infarction.

It has been reported that the expression of IL-1β or IL-18 was increased in sera and glomeruli of patients with systemic lupus erythematosus (SLE) (Non Patent Literature 31, 32), and that the expression of NLRP3 gene and the production of IL-1β were increased in the macrophages (Non Patent Literature 33). In Nlrp3-R258W mice, which have an activating mutation of NLRP3 gene, lupus nephritis-like symptoms caused by pristane administration were exacerbated (Non Patent Literature 34). Accordingly, an NLRP3 inflammasome inhibitor is considered to become a drug for treating SLE.

In addition to the above diseases, diseases for which an NLRP3 inflammasome inhibitor is expected to be effective include systemic juvenile idiopathic arthritis (Non Patent Literature 35), recurrent pericarditis (Non Patent Literature 36), adult onset Still's disease (for example, hemophagocytic lymphohistiocytosis and macrophage activation syndrome) (Non Patent Literature 37), Schnitzler syndrome (Non Patent Literature 38), deficiency of the IL-1 receptor antagonist (Non Patent Literature 39), familial Mediterranean fever (Non Patent Literature 40), mevalonate kinase deficiency (Non Patent Literature 40), hyper IgD syndrome (Non Patent Literature 40), TNF receptor-associated periodic syndrome (Non Patent Literature 40), Behcet's disease (Non Patent Literature 41), lung cancer (Non Patent Literature 42) and the like. It has been reported that anti-IL-1β antibody such as canakinumab and IL-1 inhibitor such as rilonacept are effective for the treatment of these diseases. Since NLRP3 inflammasome is involved in the production of proinflammatory cytokines such as IL-1β, an NLRP3 inflammasome inhibitor is considered to become a drug for treating these diseases.

It is has been reported that the NLRP3 rs10733113 genotype is significantly increased in patients with psoriasis and increases psoriasis susceptibility (Non Patent Literature 43). In addition, NLRP3 deficiency has been reported to suppress psoriatic symptoms in an IL-23 induced psoriasis model, a psoriasis model (Non-patent document 44). Accordingly, an NLRP3 inflammasome inhibitor is considered to become a drug for treating psoriasis.

Gout, atherosclerosis (arteriosclerosis), and chronic kidney disease, which are associated with NLRP3 inflammasome activation, involve hypertension. NLRP3 deficiency has been reported to suppress hypertension in a mouse model of left renal artery stenosis (Non Patent Literature 45). In addition, MCC950, an NLRP3 inflammasome inhibitor, has been reported to suppress hypertension in a mouse model of deoxycorticosterone acetate-salt (Non Patent Literature 46). Accordingly, an NLRP3 inflammasome inhibitor is considered to become a drug for treating hypertension.

It has been reported that NLRP3 expression is enhanced in fibrovascular membranes of patients with diabetic retinopathy (Non Patent Literature 47). In addition, NLRP3 expression is increased in a STZ-induced retinopathy model, a model of diabetic retinopathy (Non Patent Literature 48). In this model, it has been reported that decreased NLRP3 expression by NLRP3 shRNA exhibits decreased secretions of IL-1β and VEGF, increased ganglion cell mass, and recovery of retinal damage (Non Patent Literature 49). Thus, an NLRP3 inflammasome inhibitor is considered to become a drug for treating diabetic retinopathy.

NLRP3 inflammasome activation occurs in the brain of Alzheimer's disease patients, MCI (mild cognitive impairment) patients, and APP/PS1 mice, a model mouse of Alzheimer's disease. NLRP3 deficiency in APP/PS1 mice suppresses the development of spatial memory impairment (Non Patent Literature 50). MCC950, an NLRP3 inhibitor, suppresses NLRP3 activation in microglia and improves cognitive dysfunction in APP/PS1 mice (Non Patent Literature 51). Thus, an NLRP3 inflammasome inhibitor is considered to become a drug for treating Alzheimer's disease and MCI.

In the substantia nigra of Parkinson's disease patients and mice injected with α-synuclein PFF (pre-formed fibril), a pathological model of Parkinson's disease, increased expression of NLRP3 inflammasome-related molecules and NLRP3 inflammasome activation occur in microglia (Non Patent Literature 52). In α-synuclein PFF injected mice, MCC950, an NLRP3 inhibitor, inhibits NLRP3 activation in the substantia nigra and suppresses neuronal death of dopamine neurons in the substantia nigra (Non Patent Literature 52). Thus, an NLRP3 inflammasome inhibitor is considered to become a drug for treating Parkinson's disease.

In patients with Huntington's disease, cerebrospinal fluid levels of IL-1β, an NLRP3 inflammasome-associated cytokine, are increased (Non Patent Literature 53). The expression level of NLRP3 inflammasome is increased in the striatum of R6/2 mice, a model of Huntington's disease (Non Patent Literature 54). MCC950, an NLRP3 inhibitor, inhibits NLRP3 inflammasome activation in the striatum of R6/2 mice, suppresses neuronal death in the striatum, and suppresses symptom progression (Non Patent Literature 55). Thus, an NLRP3 inflammasome inhibitor is considered to become a drug for treating Huntington's disease.

The expressions of the NLRP3 inflammasome, IL-18, and active caspase 1 are increased in the spinal cord of patients with amyotrophic lateral sclerosis (ALS) (Non Patent Literature 56). In the spinal cord of SOD1G93A mice and TDP-43Q331K mice, which are ALS model mice, mRNA expressions of IL-1β, Nlrp3, Pycard, and Casp1 are increased (Non Patent Literature 57). MCC950, an NLRP3 inhibitor, inhibits SOD1G93A and TDP-43 protein-induced NLRP3 activation in microglia and decreases IL-1β production (Non-patent Document 57). In SOD1G93A mice, deficiency of IL-1β or caspase 1 prolongs survival time, and administration of IL-1β receptor antibody suppresses disease progression and prolongs survival time (Non Patent Literature 58). Thus, an NLRP3 inflammasome inhibitor is considered to become a drug for treating ALS.

The expression level of NLRP3 inflammasome is increased in brain tissue and cerebrospinal fluid of patients with traumatic brain injury (TBI) (Non Patent Literatures 59 and 60). In the brain tissue of TBI model rats, the expression level of NLRP3 inflammasome is increased, and the expression levels of IL-1β and IL-18 are also increased (Non Patent Literature 61). MCC950, an NLRP 3 inhibitor, inhibits IL-1β production in TBI model mice and suppresses the development of neurological symptoms after brain injury (Non Patent Literature 62). Thus, an NLRP3 inflammasome inhibitor is considered to become a drug for treating TBI.

In cerebral infarct patients; middle cerebral artery occlusion (MCAO) mice, a model of cerebral infarct; and intracerebral bleeding model rats, the expressions of NLRP3 inflammasome, IL-1β, and IL-18 are increased in the brain tissue (Non Patent Literatures 63 and 64). In addition, MCC950, an NLRP 3 inhibitor, showed neuroprotective effects in the MCAO model and intracerebral bleeding model rats. Thus, an NLRP3 inflammasome inhibitor is considered to become a drug for treating cerebral infarct and intracerebral bleeding.

NLRP inflammasome expression is increased in brain tissue of patients with temporal lobe epilepsy and in Pilocarpine-induced epileptic model mice (Non Patent Literatures 65 and 66). In addition, in the pilocarpine-induced epilepsy model mice, NLRP3 inflammasome deficiency and administration of MCC950, an NLRP3 inhibitor, suppress apoptosis of hippocampal neurons, which causes the development of epilepsy (Non Patent Literature 66). Thus, an NLRP3 inflammasome inhibitor is considered to become a drug for treating epilepsy.

In peripheral blood of depressive illness patients, the expression level of NLRP3 inflammasome, the IL-1β level, and the IL-18 level are increased, and the IL-1β level correlates with the depression symptom score (Non Patent Literature 67). In an LPS-induced model, a chronic stress-induced model, or a social defeat model, which are pathological models of depressive illness, the expression level of NLRP3 inflammasome, IL-1β, or IL-18 in brain tissue is increased, and NLRP3 inflammasome is activated (Non Patent Literatures 68, 69 and 70). In the pathological models, administration of MCC950, an NLRP3 inhibitor, or NLRP3 deficiency improves depressive symptoms (Non Patent Literatures 69 and 70). Thus, an NLRP3 inflammasome inhibitor is considered to become a drug for treating depressive illness.

NLRP3 inflammasome expression and IL-1β and IL-18 levels are increased in peripheral blood of patients with autism spectrum disorder (ASD) (Non Patent Literature 71). In a maternal immune activation (MIA) model, administration of PolyIC to pregnant animals causes ASD symptoms in offspring. The expression of IL-1β is increased in the fetal brain of this model, and administration of MCC950, an NLRP 3 inhibitor, to the mother suppresses ASD symptoms in offspring (Non Patent Literature 72). Thus, an NLRP3 inflammasome inhibitor is considered to become a drug for treating ASD.

In the spinal cord of mice with spinal cord injury, NLRP3 inflammasome or IL-1β expression is increased and NLRP3 activation is observed (Non Patent Literatures 73 and 74). When MCC950, an NLRP3 inhibitor, is administered to mice after spinal cord injury, NLRP3 activation and IL-1β expression in the spinal cord are suppressed, and the recovery of motor function is promoted (Non Patent Literature 73). Thus, an NLRP3 inflammasome inhibitor is considered to become a drug for treating spinal cord injury.

In an intestinal perforation model, an animal model of sepsis, increased expression and activation of NLRP3 inflammasome or IL-1β occur in the brain, resulting in damage to hippocampal neurons and memory impairment, a symptom of septic encephalopathy (Non Patent Literatures 75 and 76). When MCC950, an NLRP3 inhibitor, is administered to the intestinal perforation model, NLRP3 inflammasome activation is suppressed and the memory impairment is improved (Non Patent Literature 76). Thus, an NLRP3 inflammasome inhibitor is considered to become a drug for treating septic encephalopathy.

In a chronic constriction injury (CCI) model, an animal model of neuropathic pain, the expression levels of IL-1β and NLRP3 inflammasome-related molecules are increased in glial cells and neurons in the spinal cord (Non Patent Literature 77). In a paclitaxel-induced pain model, a neuropathic pain model of anticancer drug-induced neuropathy, the expression level of NLRP3 inflammasome-related molecules is increased in the dorsal root ganglion and sciatic nerve (Non Patent Literature 78). In a trigeminal neuralgia model animal, the expression level of NLRP3 inflammasome in the spinal cord dorsal horn is increased, and silencing NLRP3 in the spinal cord inhibits the NLRP3 inflammasome activation in the spinal cord and mechanical allodynia (Non Patent Literature 79). Thus, an NLRP3 inflammasome inhibitor is considered to become a drug for treating neuropathic pain.

Mice infected with SARS-CoV-2 show the increased expression levels of IL-1β and NLRP3 inflammasome-related molecules in lung tissue. NLRP3 knockout mice, on the other hand, do not show an increase in their expression levels and the severe respiratory inflammation caused by SARS-CoV-2 is reduced. In addition, administration of the NLRP3 inhibitor MCC950 to mice infected with SARS-CoV-2 inhibits NLRP3 inflammasome activation in the lung and suppresses the dysregulated immune response (Non Patent Literature 80). Thus, an NLRP3 inflammasome inhibitor is considered to become a drug for treating COVID-19 caused by SARS-CoV-2.

Increases of the ASC domain of NLRP3 inflammasome and matured IL-1β protein have been reported in the cerebral cortex of patients with frontotemporal dementia having mutations in protein tau (Non Patent Literature 81). Increases of the ASC domain of NLRP3 inflammasome and post-truncation caspase 1 have also been reported in the cerebral cortex of a frontotemporal dementia model, Tau22 mice which are human-mutant tau protein-expressed mice, which indicated that knockout of NLRP3 inhibited formation of tau pathologies and cognitive function decline (Non Patent Literature 81). These results suggest that an NLRP3 inflammasome inhibitor is considered to become a drug for treating frontotemporal dementia.

A possible causative substance, drusen, that is considered to develop age-related macular degeneration (AMD) was confirmed to be formed in patients with NLRP3-associated autoinflammatory disease (NLRP3-AID) caused by activation mutation in the NLRP3 gene (Non Patent Literature 82). An NLRP3 inhibitor also suppressed degeneration of retinal pigment epithelial cells in one of models of age-related macular degeneration, a model with Alu RNA-induced degeneration in retinal pigment epithelial cells (Non Patent Literature 83). An NLRP3 inhibitor suppressed neoangiogenesis in another model of age-related macular degeneration, a model with laser-induced choroidal neovascularization (Non Patent Literature 83). These results suggest that an NLRP3 inflammasome inhibitor is considered to become a drug for treating age-related macular degeneration.

Diabetes increases retinal vascular permeability in patients with diabetic macular edema, which results in leak of blood ingredients into retina (Non Patent Literature 84). An NLRP3 inhibitor, MCC950, alleviated increased vascular permeability in STZ-induced diabetic mice (Non Patent Literature 85). Thus, an NLRP3 inflammasome inhibitor is considered to become a drug for treating diabetic macular edema.

Hereditary transient corneal endotheliitis is one of Cryopyrin-associated periodic syndromes that is occurred by activation mutation in the NLRP3 gene (Non Patent Literature 86). Thus, an NLRP3 inflammasome inhibitor is considered to become a drug for treating hereditary transient corneal endotheliitis.

### CITATION LIST

### NON PATENT LITERATURE

[Non Patent Literature 1] Andersson, A et al., Pivotal advance: HMGB1 expression in active lesions of human and experimental multiple sclerosis. J Leukoc Biol., 2008, Vol 84 (5), p.1248-55
[Non Patent Literature 2] Voet, S et al., A20 critically controls microglia activation and inhibits inflammasome-dependent neuroinflammation. Nat Commun., 2018, Vol 9(1), p2036.
[Non Patent Literature 3] Politis, M et al., Increased PK11195 PET binding in the cortex of patients with MS correlates with disability. Neurology, 2012, Vol 79(6), p523-30.
[Non Patent Literature 4] Hernandez-Pedro, N et al., PAMP-DAMPs interactions mediates development and progression of multiple sclerosis. Front Biosci (Schol Ed), 2016, Vol 8, p13-28.
[Non Patent Literature 5] Denis, G et al., NLRP3 Plays a Critical Role in the Development of Experimental Autoimmune Encephalomyelitis by Mediating Th1 and Th17 Responses. J Immunol., 2010, Vol 185 (2) p974-981
[Non Patent Literature 6] Jha, S et al., The inflammasome sensor, NLRP3, regulates CNS inflammation and demyelination via caspase-1 and interleukin-18. J Neurosci., 2010 Vol 30(47), p15811-20
[Non Patent Literature 7] Guo, C et al., Development and Characterization of a Hydroxyl-Sulfonamide Analogue, 5-Chloro-N-[2-(4-hydroxysulfamoyl-phenyl)-ethyl]-2-methoxybenzamide, as a Novel NLRP3 Inflammasome Inhibitor for Potential Treatment of Multiple Sclerosis. ACS Chem Neurosci. , 2017, Vol 8(10), p2194-2201
[Non Patent Literature 8] Akosua Vilaysane et al., The NLRP3 Inflammasome Promotes Renal Inflammation and Contributes to CKD. J Am Soc Nephrol. 2010 Oct; 21(10): 1732-1744.
[Non Patent Literature 9] Shahzad K et al., Nlrp3-inflammasome activation in non-myeloid-derived cells aggravates diabetic nephropathy. Kidney Int. 2015 Jan;87(1) :74-84.
[Non Patent Literature 10] Gong W et al., NLRP3 deletion protects against renal fibrosis and attenuates mitochondrial abnormality in mouse with 5/6 nephrectomy. Am J Physiol Renal Physiol. 2016 May 15;310(10):F1081-8
[Non Patent Literature 11] Ranson N et al., NLRP3-dependent and -independent processing Interleiukin-1β in active Ulcerative colitis. Int J mol Sci 2018: 20pii:E57.
[Non Patent Literature 12] Mao L et al., Loss-of-function CARD8 mutation causes NLRP3 inflammasome activation and Crohn's disease. J Clin Invest 2018: vol 128:1793-1806.
[Non Patent Literature 13] Bauer c. et al., Protective and aggravating effects of NLRP3 inlammasome activation in IBD models: influence of genetic and environmental factors. Dig.Dis 2012 vol 30 suppl 1 82-90.
[Non Patent Literature 14] Paramel V G et al., NLRP3 Inflammasome Expression and Activation in Human Atherosclerosis. J Am Heart Assoc. 2016 May 20;5(5):e003031.
[Non Patent Literature 15] Duewell P et al., NLRP3 inflammasomes are required for atherogenesis and activated by cholesterol crystals. Nature. 2010 Apr 29;464(7293):1357-61.
[Non Patent Literature 16] Broderick L et al., The inflammasomes and autoinflammatory syndromes. Annu Rev Pathol. 2015;10:395-424.
[Non Patent Literature 17] Sarrauste M C et al., INFEVERS: the Registry for FMF and hereditary inflammatory disorders mutations. Nucleic Acids Res. 2003 Jan 1;31(1):282-5.
[Non Patent Literature 18] Brydges SD et al., Divergence of IL-1, IL-18, and cell death in NLRP3 inflammasomopathies. J Clin Invest. 2013 Nov;123(11):4695-705.
[Non Patent Literature 19] Jiang H et al., Identification of a selective and direct NLRP3 inhibitor to treat inflammatory disorders. J Exp Med. 2017 Nov 6;214(11):3219-3238.
[Non Patent Literature 20] Wree A et al., NLRP3 inflammasome activation is required for fibrosis development in NAFLD. J Mol Med (Berl). 2014 Oct;92(10):1069-82.
[Non Patent Literature 21] So AK et al., Inflammation in gout: mechanisms and therapeutic targets. Nat Rev Rheumatol. 2017 Nov;13(11):639-647.
[Non Patent Literature 22] Martinon F et al., Gout-associated uric acid crystals activate the NALP3 inflammasome. Nature. 2006 Mar 9;440(7081):237-41.
[Non Patent Literature 23] Marchetti C et al., NLRP3 inflammasome inhibitor OLT1177 suppresses joint inflammation in murine models of acute arthritis. Arthritis Res Ther. 2018 Aug 3;20(1):169.
[Non Patent Literature 24] Mathews RJ et al., Evidence of NLRP3-inflammasome activation in rheumatoid arthritis (RA); genetic variants within the NLRP3-inflammasome complex in relation to susceptibility to RA and response to anti-TNF treatment. Ann Rheum Dis. 2014 Jun;73(6):1202-10.
[Non Patent Literature 25] Zhang Y et al., NLRP3 Inflammasome Plays an Important Role in the Pathogenesis of Collagen-Induced Arthritis. Mediators Inflamm. 2016;2016:9656270.
[Non Patent Literature 26] Watanabe H et al., Activation of the IL-1beta-processing inflammasome is involved in contact hypersensitivity. J Invest Dermatol. 2007 Aug;127(8):1956-63.
[Non Patent Literature 27] Niu L et al., Upregulation of NLRP3 Inflammasome in the Tears and Ocular Surface of Dry Eye Patients. PLoS One. 2015 May 11;10(5):e0126277.
[Non Patent Literature 28] Zheng Q et al., Reactive oxygen species activated NLRP3 inflammasomes initiate inflammation in hyperosmolarity stressed human corneal epithelial cells and environment-induced dry eye patients. Exp Eye Res. 2015 May;134:133-40.
[Non Patent Literature 29] Kawaguchi M et al., Inflammasome activation of cardiac fibroblasts is essential for myocardial ischemia/reperfusion injury. Circulation. 2011 Feb 15;123(6):594-604.
[Non Patent Literature 30] Sandanger O et al., The NLRP3 inflammasome is up-regulated in cardiac fibroblasts and mediates myocardial ischaemia-reperfusion injury. Cardiovasc Res. 2013 Jul 1;99(1):164-74.
[Non Patent Literature 31] Dellalibera-Joviliano R et al., Kinins and cytokines in plasma and cerebrospinal fluid of patients with neuropsychiatric lupus. J Rheumatol. 2003 Mar;30(3):485-92.
[Non Patent Literature 32] Tucci M et al., Glomerular accumulation of plasmacytoid dendritic cells in active lupus nephritis: role of interleukin-18. Arthritis Rheum. 2008 Jan;58(1):251-62.
[Non Patent Literature 33] Yang CA et al., Sex-dependent differential activation of NLRP3 and AIM2 inflammasomes in SLE macrophages. Rheumatology (Oxford). 2015 Feb;54(2):324-31.
[Non Patent Literature 34] Lu A et al., Hyperactivation of the NLRP3 Inflammasome in Myeloid Cells Leads to Severe Organ Damage in Experimental Lupus. J Immunol. 2017 Feb 1;198(3):1119-1129.
[Non Patent Literature 35] Ruperto N et al., Two randomized trials of canakinumab in systemic juvenile idiopathic arthritis. N Engl J Med. 2012 Dec 20;367(25):2396-406.
[Non Patent Literature 36] Klein AL et al., Phase 3 Trial of Interleukin-1 Trap Rilonacept in Recurrent Pericarditis. N Engl J Med. 2021 Jan 7; 384(1):31-41.
[Non Patent Literature 37] Junge G et al., Adult onset Still's disease-The evidence that anti-interleukin-1 treatment is effective and well-tolerated (a comprehensive literature review). Seminars in Arthritis Rheumatism 2017 Oct; 47(2):295-302.
[Non Patent Literature 38] Krause K et al., Efficacy and safety of canakinumab in Schnitzler syndrome: A multicenter randomized placebo-controlled study. J Allergy Clin Immunol. 2017 Apr;139(4):1311-1320.
[Non Patent Literature 39] Garg M et al., Rilonacept maintains long-term inflammatory remission in patients with deficiency of the IL-1 receptor antagonist. JCI Insight. 2017 Aug 17;2(16).
[Non Patent Literature 40] De Benedetti F et al., Canakinumab for the Treatment of Autoinflammatory Recurrent Fever Syndromes. N Engl J Med. 2018 May 17;378(20):1908-1919.
[Non Patent Literature 41] Emmi G et al., Efficacy and safety profile of anti-interleukin-1 treatment in Behcet's disease: a multicenter retrospective study. Clin. Rheumatol., 35 (2016), pp. 1281-1286.
[Non Patent Literature 42] Ridker P.M. et al., Antiinflammatory Therapy with Canakinumab for Atherosclerotic Disease. Lancet (2017) 390 1833-42.
[Non Patent Literature 43] M Carlstrom et al., Genetic support for the role of the NLRP3 inflammasome in psoriasis susceptibility. Exp Dermatol. (2012) 21:932-7.
[Non Patent Literature 44] J. A Diaz-Perez et al., Extracellular ATP and IL-23 Form a Local Inflammatory Circuit Leading to the Development of a Neutrophil-Dependent Psoriasiform Dermatitis. J Invest Dermatol. (2018) 138:2595-605.
[Non Patent Literature 45] Wang Q et al., Renin-Dependent Hypertension in Mice Requires the NLRP3- Inflammasome. J. Hypertens (2014) 3:187.
[Non Patent Literature 46] Krishnan S M et al., Pharmacological inhibition of the NLRP3 inflammasome reduces blood pressure, renal damage, and dysfunction in salt-sensitive hypertension. Cardiovasc. Res. (2019) 115 4: 776-787.
[Non Patent Literature 47] Zhang Y et al., Protection of Mcc950 against high-glucose-induced human retinal endothelial cell dysfunction. Cell Death Dis. 2017 Jul 20; 8(7):e2941.
[Non Patent Literature 48] Sheng Li et al., Protective effects of sulforaphane on diabetic retinopathy: activation of the Nrf2 pathway and inhibition of NLRP3 inflammasome formation. Exp Anim. 2019 May 8;68(2):221-231.
[Non Patent Literature 49] Guangrui Chai et al., NLRP3 Blockade Suppresses Pro-Inflammatory and Pro-Angiogenic Cytokine Secretion in Diabetic Retinopathy. Diabetes Metab Syndr Obes. 2020 Aug 25;13:3047-3058.
[Non Patent Literature 50] Heneka MT et al., NLRP3 is activated in Alzheimer's disease and contributes to pathology in APP/PS1 mice. Nature. 2013 Jan 31;493(7434):674-8.
[Non Patent Literature 51] Dempsey C et al., Inhibiting the NLRP3 inflammasome with MCC950 promotes non-phlogistic clearance of amyloid-β and cognitive function in APP/PS1 mice. Brain Behav Immun. 2017 Mar; 61:306-316.
[Non Patent Literature 52] Gordon R et al., Inflammasome inhibition prevents α-synuclein pathology and dopaminergic neurodegeneration in mice. Sci Transl Med. 2018 Oct 31;10(465):eaah4066.
[Non Patent Literature 53] Rodrigues FB et al., Cerebrospinal Fluid Inflammatory Biomarkers Reflect Clinical Severity in Huntington's Disease. PLoS One. 2016 Sep 22;11(9):e0163479.
[Non Patent Literature 54] Paldino E et al., Pyroptotic cell death in the R6/2 mouse model of Huntington's disease: new insight on the inflammasome. Cell Death Discov. 2020 Jul 31;6:69.
[Non Patent Literature 55] Chen KP et al., A selective inhibitor of the NLRP3 inflammasome as a potential therapeutic approach for neuroprotection in a transgenic mouse model of Huntington's disease. J Neuroinflammation. 2022 Feb 26;19(1):56.
[Non Patent Literature 56] Johann S et al., NLRP3 inflammasome is expressed by astrocytes in the SOD1 mouse model of ALS and in human sporadic ALS patients. Glia. 2015 Dec;63(12):2260-73.
[Non Patent Literature 57] Deora V et al., The microglial NLRP3 inflammasome is activated by amyotrophic lateral sclerosis proteins. Glia. 2020 Feb;68(2):407-421.
[Non Patent Literature 58] Meissner F et al., A. Mutant superoxide dismutase 1-induced IL-1beta accelerates ALS pathogenesis. Proc Natl Acad Sci U S A. 2010 Jul 20;107(29):13046-50.
[Non Patent Literature 59] Lin C et al., Omega-3 fatty acids regulate NLRP3 inflammasome activation and prevent behavior deficits after traumatic brain injury. Exp Neurol. 2017 Apr;290:115-122.
[Non Patent Literature 60] Wallisch JS et al., Cerebrospinal Fluid NLRP3 is Increased After Severe Traumatic Brain Injury in Infants and Children. Neurocrit Care. 2017 Aug;27(1):44-50.
[Non Patent Literature 61] Liu HD et al., Expression of the NLRP3 inflammasome in cerebral cortex after traumatic brain injury in a rat model. Neurochem Res. 2013 Oct;38(10):2072-83.
[Non Patent Literature 62] Ismael S et al., MCC950, the Selective Inhibitor of Nucleotide Oligomerization Domain-Like Receptor Protein-3 Inflammasome, Protects Mice against Traumatic Brain Injury. J Neurotrauma. 2018 Jun 1;35(11):1294-1303.
[Non Patent Literature 63] Fann DY et al., Intravenous immunoglobulin suppresses NLRP1 and NLRP3 inflammasome-mediated neuronal death in ischemic stroke. Cell Death Dis. 2013 Sep 5;4(9):e790.
[Non Patent Literature 64] Feng L et al., P2X7R blockade prevents NLRP3 inflammasome activation and brain injury in a rat model of intracerebral hemorrhage: involvement of peroxynitrite. J Neuroinflammation. 2015 Oct 17;12:190.
[Non Patent Literature 65] Yue J et al., NLRP3 inflammasome and endoplasmic reticulum stress in the epileptogenic zone in temporal lobe epilepsy: molecular insights into their interdependence. Neuropathol Appl Neurobiol. 2020 Dec;46(7):770-785.
[Non Patent Literature 66] Wu C et al., The Role of NLRP3 and IL-1β in Refractory Epilepsy Brain Injury. Front Neurol. 2020 Feb 7;10:1418.
[Non Patent Literature 67] Alcocer-Gomez E et al., NLRP3 inflammasome is activated in mononuclear blood cells from patients with major depressive disorder. Brain Behav Immun. 2014 Feb;36:111-7.
[Non Patent Literature 68] Zhang Y et al., Involvement of inflammasome activation in lipopolysaccharide-induced mice depressive-like behaviors. CNS Neurosci Ther. 2014 Feb;20(2):119-24.
[Non Patent Literature 69] Iwata M et al., Psychological Stress Activates the Inflammasome via Release of Adenosine Triphosphate and Stimulation of the Purinergic Type 2X7 Receptor. Biol Psychiatry. 2016 Jul 1;80(1):12-22.
[Non Patent Literature 70] Li W et al., Inhibition of the NLRP3 inflammasome with MCC950 prevents chronic social isolation-induced depression-like behavior in male mice. Neurosci Lett. 2021 Nov 20;765:136290.
[Non Patent Literature 71] Saresella M et al., Multiple inflammasome complexes are activated in autistic spectrum disorders. Brain Behav Immun. 2016 Oct;57:125-133.
[Non Patent Literature 72] Szabo D et al., Maternal P2X7 receptor inhibition prevents autism-like phenotype in male mouse offspring through the NLRP3-IL-1β pathway. Brain Behav Immun. 2022 Mar;101:318-332.
[Non Patent Literature 73] Amo-Aparicio J et al., Inhibition of the NLRP3 inflammasome by OLT1177 induces functional protection and myelin preservation after spinal cord injury. Exp Neurol. 2022 Jan;347:113889.
[Non Patent Literature 74] Jiao J et al., MCC950, a Selective Inhibitor of NLRP3 Inflammasome, Reduces the Inflammatory Response and Improves Neurological Outcomes in Mice Model of Spinal Cord Injury. Front Mol Biosci. 2020 Mar 3;7:37.
[Non Patent Literature 75] Ding H et al., Fisetin ameliorates cognitive impairment by activating mitophagy and suppressing neuroinflammation in rats with sepsis-associated encephalopathy. CNS Neurosci Ther. 2022 Feb;28(2):247-258.
[Non Patent Literature 76] Fu Q et al., NLRP3/Caspase-1 Pathway-Induced Pyroptosis Mediated Cognitive Deficits in a Mouse Model of Sepsis-Associated Encephalopathy. Inflammation. 2019 Feb;42(1):306-318.
[Non Patent Literature 77] Xu L et al., MiR-34c Ameliorates Neuropathic Pain by Targeting NLRP3 in a Mouse Model of Chronic Constriction Injury. Neuroscience. 2019 Feb 10;399:125-134.
[Non Patent Literature 78] Jia M et al., Activation of NLRP3 inflammasome in peripheral nerve contributes to paclitaxel-induced neuropathic pain. Mol Pain. 2017 Jan-Dec;13:1744806917719804.
[Non Patent Literature 79] Sun X et al., The NLRP3-related inflammasome modulates pain behavior in a rat model of trigeminal neuropathic pain. Life Sci. 2021 Jul 15;277:119489.
[Non Patent Literature 80] Zeng J et al., Specific inhibition of the NLRP3 inflammasome suppresses immune overactivation and alleviates COVID-19 like pathology in mice. EBioMedicine. 2022 Jan;75:103803.
[Non Patent Literature 81] Ising C, et al., NLRP3 inflammasome activation drives tau pathology. Nature. 2019 Nov; 575(7784): 669-673.
[Non Patent Literature 82] Bing Li, et al., Early onset drusen and RPE dysfunction in a patient with NLRP3-AID, Ocul Immunol Inflamm. 2022 Nov 17;1-4.
[Non Patent Literature 83] Benjamin J Fowler, et al., Nucleoside reverse transcriptase inhibitors possess intrinsic anti-inflammatory activity, Science. 2014 Nov 21;346(6212):1000-3.
[Non Patent Literature 84] Hideharu Funatsu, Development of treatment for macular edema, Diabetes 48(10): 721-723, 2005.
[Non Patent Literature 85] Keke Ge, et al., Down-expression of the NLRP3 inflammasome delays the progression of diabetic retinopathy, Microvasc Res. 2022 Jan;139:104265.
[Non Patent Literature 86] Joni A Turunen, et al., Keratoendotheliitis Fugax Hereditaria: A Novel Cryopyrin-Associated Periodic Syndrome Caused by a Mutation in the Nucleotide-Binding Domain, Leucine-Rich Repeat Family, Pyrin Domain-Containing 3 (NLRP3) Gene, Am J Ophthalmol. 2018 Apr;188:41-50.

### SUMMARY OF INVENTION

The present invention provides substituted pyrazolopyrimidine compounds, or pharmaceutically acceptable salts thereof, having NLRP3 inflammasome inhibitory activity, pharmaceutical compositions comprising the same, and medical use thereof. Specifically, the present invention includes the embodiments illustrated as follows.

### Clause 1

A compound of Formula [I]: 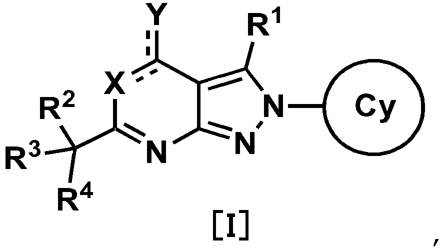 or a pharmaceutically acceptable salt thereof (herein "a compound of Formula [I] or a pharmaceutically acceptable salt thereof" is also referred to as "Compound [I]"), wherein the partial structure: 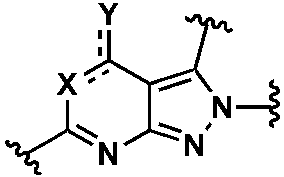 is:
(1) a structure of the formula: 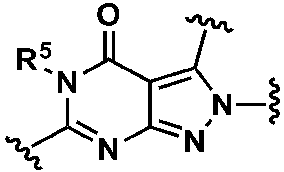 wherein R⁵ is hydrogen or C₁₋₄ alkyl, wherein the alkyl may be optionally substituted with:
   (a) carboxy,
   (b) -CO-C₁₋₄ alkoxy, or
   (c) -CO-NR⁶R⁷, wherein R⁶ and R⁷ are each independently hydrogen or C₁₋₄ alkyl, or
(2) a structure of the formula: 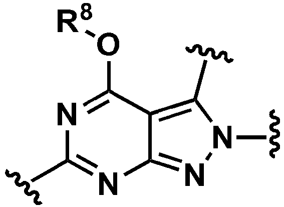 wherein R⁸ is C₁₋₄ alkyl;
   a cyclic group Cy is:
   (1) a group of the formula: 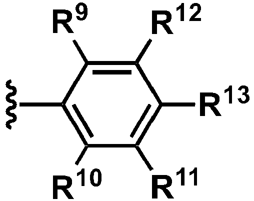 wherein R⁹ and R¹⁰ are each independently
      (a) hydrogen,
      (b) C₁₋₄ alkyl, wherein the alkyl may be optionally substituted with C₁₋₄ alkoxy,
      (c) C₁₋₄ alkoxy,
      (d) halogen,
      (e) C₁₋₄ haloalkyl, or
      (f) -O-C₁₋₄ haloalkyl,
         R¹¹ and R¹² are each independently
            (a) hydrogen,
            (b) C₁₋₄ alkyl, or
            (c) C₁₋₄ haloalkyl,
         R¹³ is
            (a) hydrogen,
            (b) C₁₋₄ alkyl,
            (c) C₁₋₄ alkoxy,
            (d) halogen,
            (e) C₁₋₆ haloalkyl,
            (f) -O-C₁₋₄ haloalkyl, or
            (g) C₃₋₆ cycloalkyl, wherein the cycloalkyl may be optionally substituted with 1 or 2 halogen atoms, or
         R¹³ may be combined together with R¹¹ or R¹² and the carbon atom to which it is attached to form:
            (a) C₅₋₆ cycloalkene, or
            (b) 5- to 7-membered heterocycloalkene comprising 1 or 2 oxygen atoms, or
   (2) a group of the formula: 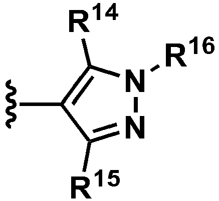 wherein R¹⁴ and R¹⁵ are each independently C₁₋₄ alkyl or C₁₋₄ haloalkyl,
      R¹⁶ is C₁₋₆ alkyl or C₃₋₆ cycloalkyl,
      R¹ is:
         (1) hydrogen,
         (2) cyano,
         (3) C₁₋₄ alkyl, wherein the alkyl may be optionally substituted with hydroxy or C₁₋₄ alkoxy,
         (4) C₁₋₄ haloalkyl, or
         (5) -CO-C₁₋₄ alkyl,
      R², R³, and R⁴ are each independently
         (1) hydrogen,
         (2) hydroxy,
   (3) C₁₋₆ alkyl, wherein the alkyl may be optionally substituted with 1 or 2 substituents independently selected from the group consisting of:
      (a) hydroxy,
      (b) C₁₋₄ alkoxy, and
      (c) -SO₂-C₁₋₄ alkyl,
   (4) C₁₋₆ alkoxy, wherein the alkoxy may be optionally substituted with:
      (a) hydroxy,
      (b) phenyl, or
      (c) 4- to 6-membered heterocycloalkyl comprising 1 or 2 heteroatoms independently selected from the group consisting of nitrogen and oxygen atoms,
   (5) halogen,
   (6) C₁₋₄ haloalkyl, wherein the haloalkyl may be optionally substituted with hydroxy or C₁₋₄ alkoxy,
   (7) -O-C₁₋₄ haloalkyl,
   (8) -OR¹⁷, wherein R¹⁷ is 4- to 6-membered heterocycloalkyl comprising 1 or 2 heteroatoms independently selected from the group consisting of nitrogen and oxygen atoms,
   (9) C₃₋₆ cycloalkyl,
   (10) 4- to 6-membered heterocycloalkyl comprising 1 or 2 heteroatoms independently selected from the group consisting of nitrogen and oxygen atoms, wherein the heterocycloalkyl may be optionally substituted with oxo, or
   (11) a group of the formula: 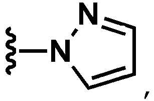 or
      R², R³, and R⁴ may be combined together with the carbon atom to which they are attached and the -CR²R³R⁴ group may form:
      (a) cyano,
      (b) C₃₋₆ cycloalkyl, wherein the cycloalkyl may be optionally substituted with 1 to 3 substituents independently selected from the group consisting of:
         (1) cyano,
         (2) C₁₋₄ alkyl, wherein the alkyl may be optionally substituted with hydroxy or C₁₋₄ alkoxy,
         (3) C₁₋₄ alkoxy,
         (4) halogen, and
         (5) -CO-NR¹⁸R¹⁹, wherein R¹⁸ and R¹⁹ are each independently hydrogen or C₁₋₄ alkyl,
      (c) 4- to 7-membered heterocycloalkyl comprising 1 or 2 heteroatoms independently selected from the group consisting of nitrogen, oxygen, and sulfur atom, wherein the heterocycloalkyl may be optionally substituted with 1 or 2 substituents independently selected from the group consisting of:
         (1) hydroxy,
         (2) C₁₋₄ alkyl,
         (3) C₁₋₄ alkoxy, and
         (4) halogen, or
         any one of the ring-constituting atoms of the heterocycloalkyl may be optionally substituted with 1 or 2 oxo,
      (d) 7- to 9-membered saturated fused heterocyclic group comprising 1 or 2 heteroatoms independently selected from the group consisting of nitrogen and oxygen atoms, wherein the fused heterocyclic group may be optionally substituted with 1 or 2 halogen atoms,
      (e) 5- to 8-membered bridged cycloalkyl, wherein the bridged cycloalkyl may be optionally substituted with halogen,
      (f) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
      (g) C₅₋₆ cycloalkenyl,
      (h) 5- or 6-membered heterocycloalkenyl comprising 1 or 2 heteroatoms independently selected from the group consisting of nitrogen and oxygen atoms, or
      (i) a group of the formula: 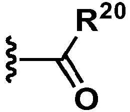 wherein R²⁰ is:
         (1) C₁₋₄ alkyl, or
         (2) -NR²¹R²², wherein R²¹ and R²² are each independently
            (a) hydrogen,
            (b) C₁₋₄ alkyl,
            (c) C₁₋₄ haloalkyl, or
            (d) 4- to 6-membered heterocycloalkyl comprising 1 or 2 heteroatoms independently selected from the group consisting of nitrogen and oxygen atoms.

### Clause 2

The compound according to clause 1, or a pharmaceutically acceptable salt thereof, wherein the partial structure: 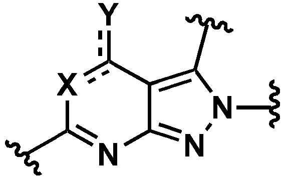 is
(1) a structure of the formula: 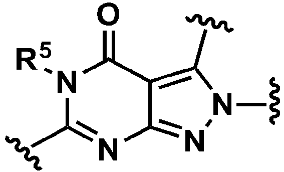 wherein R⁵ is the same as defined in clause 1.

### Clause 3

The compound according to clause 1 or 2, or a pharmaceutically acceptable salt thereof, wherein R¹ is hydrogen.

### Clause 4

The compound according to any one of clauses 1 to 3, or a pharmaceutically acceptable salt thereof, wherein R⁵ is hydrogen.

### Clause 5

The compound according to any one of clauses 1 to 4, or a pharmaceutically acceptable salt thereof, wherein the cyclic group Cy is:
(1) a group of the formula: 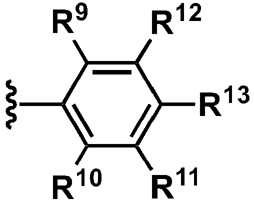 wherein R⁹, R¹⁰, R¹¹, R¹², and R¹³ are the same as defined in clause 1.

### Clause 6

The compound according to any one of clauses 1 to 5, or a pharmaceutically acceptable salt thereof, represented by Formula [II]: 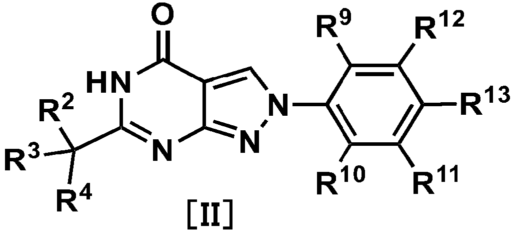 wherein R², R³, R⁴, R⁹, R¹⁰, R¹¹, R¹², and R¹³ are the same as defined in clause 1.

### Clause 7

The compound according to any one of clauses 1 to 6, or a pharmaceutically acceptable salt thereof, wherein R¹¹ and R¹² are hydrogen.

### Clause 8

The compound according to any one of clauses 1 to 7, or a pharmaceutically acceptable salt thereof, represented by Formula [III]: 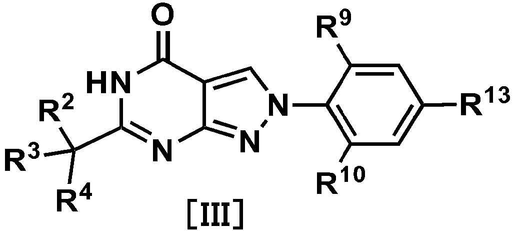 wherein R², R³, R⁴, R⁹, R¹⁰, and R¹³ are the same as defined in clause 1.

### Clause 9

The compound according to any one of clauses 1 to 8, or a pharmaceutically acceptable salt thereof, wherein at least one of R⁹ and R¹⁰ is:
(1) C₁₋₄ alkyl, wherein the alkyl may be optionally substituted with C₁₋₄ alkoxy,
(2) C₁₋₄ alkoxy,
(3) halogen,
(4) C₁₋₄ haloalkyl, or
(5) -O-C₁₋₄ haloalkyl.

### Clause 10

The compound according to any one of clauses 1 to 9, or a pharmaceutically acceptable salt thereof, wherein R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
   (a) hydroxy,
   (b) C₁₋₄ alkoxy, and
   (c) -SO₂-C₁₋₄ alkyl,
(4) C₁₋₆ alkoxy, wherein the alkoxy may be optionally substituted with
   (a) hydroxy,
   (b) phenyl, or
   (c) 4- to 6-membered heterocycloalkyl comprising 1 or 2 heteroatoms independently selected from the group consisting of nitrogen and oxygen atoms,
(5) C₁₋₄ haloalkyl, wherein the haloalkyl may be optionally substituted with hydroxy or C₁₋₄ alkoxy,
(6) -O-C₁₋₄ haloalkyl, or
(7) C₃₋₆ cycloalkyl, or
R², R³, and R⁴ may be combined together with the carbon atom to which they are attached, and the -CR²R³R⁴ group may form:
(a) C₃₋₆ cycloalkyl, wherein the cycloalkyl may be optionally substituted with 1 to 3 substituents independently selected from the group consisting of:
   (1) cyano,
   (2) C₁₋₄ alkyl, wherein the alkyl may be optionally substituted with hydroxy or C₁₋₄ alkoxy,
   (3) C₁₋₄ alkoxy,
   (4) halogen, and
   (5) -CO-NR¹⁸R¹⁹, wherein R¹⁸ and R¹⁹ are each independently hydrogen or C₁₋₄ alkyl,
(b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
   (1) hydroxy,
   (2) C₁₋₄ alkyl,
   (3) C₁₋₄ alkoxy, and
   (4) halogen, or
   any one of the ring-constituting atoms of the heterocycloalkyl may be optionally substituted with 1 or 2 oxo groups,
(c) 5- to 8-membered bridged cycloalkyl, wherein the bridged cycloalkyl may be optionally substituted with halogen,
(d) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
(e) 5- or 6-membered heterocycloalkenyl comprising 1 or 2 heteroatoms independently selected from the group consisting of nitrogen and oxygen atoms, or
(f) a group of the formula: 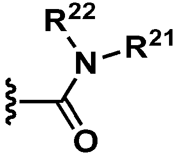 wherein R²¹ and R²² are each independently
   (1) hydrogen,
   (2) C₁₋₄ alkyl,
   (3) C₁₋₄ haloalkyl, or
   (4) 4- to 6-membered heterocycloalkyl comprising 1 or 2 heteroatoms independently selected from the group consisting of nitrogen and oxygen atoms.

### Clause 11

The compound according to any one of clauses 1 to 10, or a pharmaceutically acceptable salt thereof, wherein R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
   (a) hydroxy,
   (b) C₁₋₄ alkoxy, and
   (c) -SO₂-C₁₋₄ alkyl,
(4) C₁₋₆ alkoxy, wherein the alkoxy may be optionally substituted with:
   (a) hydroxy,
   (b) phenyl, or
   (c) 4- to 6-membered heterocycloalkyl comprising 1 or 2 heteroatoms independently selected from the group consisting of nitrogen and oxygen atoms, or
(5) C₁₋₄ haloalkyl, wherein the haloalkyl may be optionally substituted with hydroxy or C₁₋₄ alkoxy, or
R², R³, and R⁴ may be combined together with the carbon atom to which they are attached, and the -CR²R³R⁴ group may form:
(a) C₃₋₆ cycloalkyl, wherein the cycloalkyl may be optionally substituted with 1 to 3 substituents independently selected from the group consisting of:
   (1) cyano,
   (2) C₁₋₄ alkyl, wherein the alkyl may be optionally substituted with hydroxy or C₁₋₄ alkoxy,
   (3) C₁₋₄ alkoxy,
   (4) halogen, and
   (5) -CO-NR¹⁸R¹⁹, wherein R¹⁸ and R¹⁹ are each independently hydrogen or C₁₋₄ alkyl,
(b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
   (1) hydroxy,
   (2) C₁₋₄ alkyl,
   (3) C₁₋₄ alkoxy, and
   (4) halogen, or
   any one of the ring-constituting atoms of the heterocycloalkyl may be optionally substituted with 1 or 2 oxo groups, or
(c) a group of the formula: 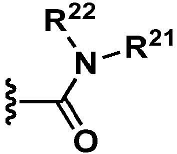 wherein R²¹ and R²² are each independently
   (1) hydrogen,
   (2) C₁₋₄ alkyl,
   (3) C₁₋₄ haloalkyl, or
   (4) 4- to 6-membered heterocycloalkyl comprising 1 or 2 heteroatoms independently selected from the group consisting of nitrogen and oxygen atoms.

### Clause 12

The compound according to clause 1, or a pharmaceutically acceptable salt thereof, selected from the group consisting of the following formulae: 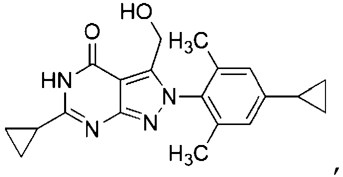 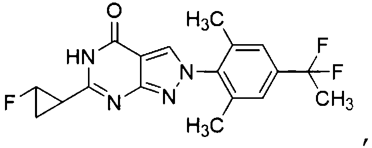 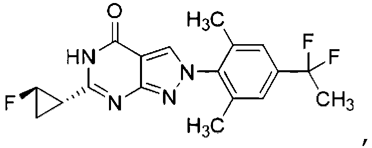 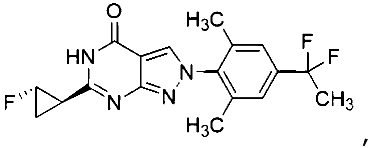 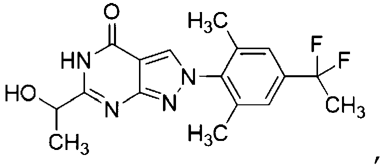 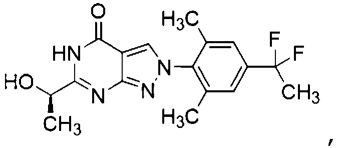 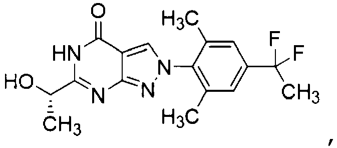 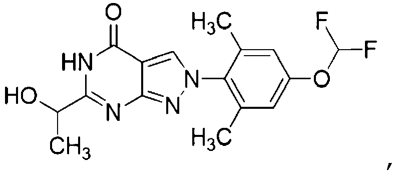 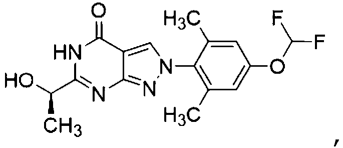 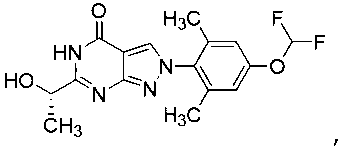 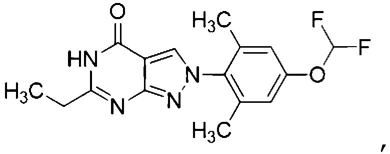 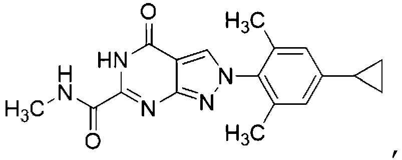 and

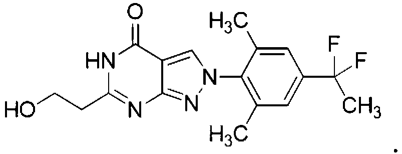

### Clause 13

The compound according to clause 1, or a pharmaceutically acceptable salt thereof, selected from the group consisting of the following formulae: 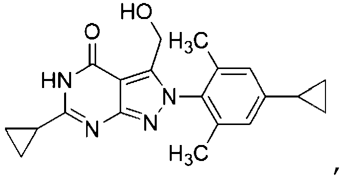 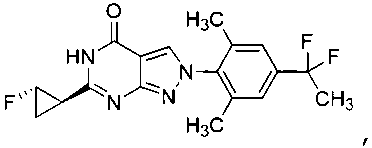 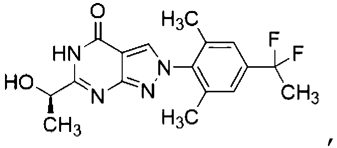 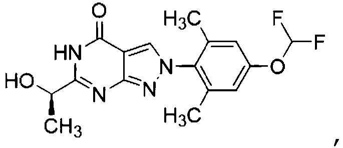 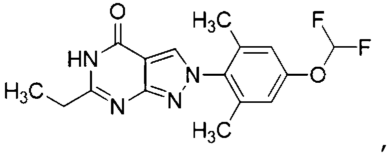 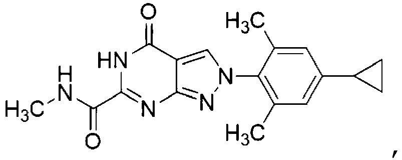 and

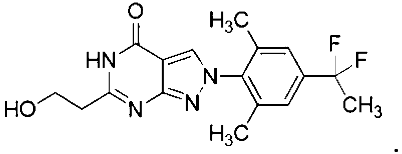

### Clause 14

A pharmaceutical composition comprising a compound according to any one of clauses 1 to 13, or a pharmaceutically acceptable salt thereof, and a pharmaceutically acceptable carrier.

### Clause 15

An NLRP3 inflammasome inhibitor comprising a compound according to any one of clauses 1 to 13, or a pharmaceutically acceptable salt thereof.

### Clause 16

A medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome, comprising a compound according to any one of clauses 1 to 13, or a pharmaceutically acceptable salt thereof.

### Clause 17

The medicament according to clause 16, wherein the inflammatory bowel disease is ulcerative colitis or Crohn's disease.

### Clause 18

The medicament according to clause 16, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 19

A method of inhibiting NLRP3 inflammasome, comprising administering a therapeutically effective amount of a compound according to any one of clauses 1 to 13, or a pharmaceutically acceptable salt thereof, to a mammal.

### Clause 20

A method of treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome, comprising administering a therapeutically effective amount of a compound according to any one of clauses 1 to 13, or a pharmaceutically acceptable salt thereof, to a mammal.

### Clause 21

The method according to clause 20, wherein the inflammatory bowel disease is ulcerative colitis or Crohn's disease.

### Clause 22

The method according to clause 20, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 23

Use of a compound according to any one of clauses 1 to 13, or a pharmaceutically acceptable salt thereof, in the manufacture of an NLRP3 inflammasome inhibitor.

### Clause 24

Use of a compound according to any one of clauses 1 to 13, or a pharmaceutically acceptable salt thereof, in the manufacture of a medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Clause 25

The use according to clause 24, wherein the inflammatory bowel disease is ulcerative colitis or Crohn's disease.

### Clause 26

The use according to clause 24, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 27

A compound according to any one of clauses 1 to 13, or a pharmaceutically acceptable salt thereof, for use in inhibiting NLRP3 inflammasome.

### Clause 28

A compound according to any one of clauses 1 to 13, or a pharmaceutically acceptable salt thereof, for use in treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Clause 29

The compound according to clause 28, or a pharmaceutically acceptable salt thereof, for use, wherein the inflammatory bowel disease is ulcerative colitis or Crohn's disease.

### Clause 30

The compound according to clause 28, or a pharmaceutically acceptable salt thereof, for use, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 31

A commercial package comprising the pharmaceutical composition according to clause 14 and a written matter associated therewith, the written matter indicating that the pharmaceutical composition can be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Clause 32

A commercial kit comprising the pharmaceutical composition according to clause 14 and a written matter associated therewith, the written matter indicating that the pharmaceutical composition can be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Clause 16A

A medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome, comprising a compound according to any one of clauses 1 to 13, or a pharmaceutically acceptable salt thereof.

### Clause 17A

The medicament according to clause 16A, wherein the inflammatory bowel disease is ulcerative colitis or Crohn's disease.

### Clause 18A

The medicament according to clause 16A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 19A

A method for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome, comprising administering a therapeutically effective amount of a compound according to any one of clauses 1 to 13, or a pharmaceutically acceptable salt thereof, to a mammal.

### Clause 20A

The method according to clause 19A, wherein the inflammatory bowel disease is ulcerative colitis or Crohn's disease.

### Clause 21A

The method according to clause 19A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 22A

Use of a compound according to any one of clauses 1 to 13, or a pharmaceutically acceptable salt thereof, in the manufacture of a medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### Clause 23A

The use according to clause 22A, wherein the inflammatory bowel disease is ulcerative colitis or Crohn's disease.

### Clause 24A

The use according to clause 22A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 25A

A compound according to any one of clauses 1 to 13, or a pharmaceutically acceptable salt thereof, for use in treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### Clause 26A

The compound, or a pharmaceutically acceptable salt thereof, for use according to clause 25A, wherein the inflammatory bowel disease is ulcerative colitis or Crohn's disease.

### Clause 27A

The compound, or a pharmaceutically acceptable salt thereof, for use according to clause 25A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Clause 28A

A package for commercial use, comprising the pharmaceutical composition according to clause 14 and a written document associated with the pharmaceutical composition, the document describing that the composition may be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### Clause 29A

A kit for commercial use, comprising the pharmaceutical composition according to clause 14 and a written document associated with the pharmaceutical composition, the document describing that the composition may be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### DESCRIPTION OF EMBODIMENTS

The following are definitions of terms that may be used herein.

A wavy line as follows: 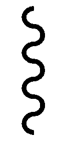 in a chemical formula herein refers to a binding site of the moiety or group represented by the chemical formula.

The term "C₁₋₄ alkyl" refers to a straight- or branched-chain saturated hydrocarbon group having 1 to 4 carbon atoms. "C₁₋₄ alkyl" includes methyl, ethyl, n-propyl, isopropyl, n-butyl, sec-butyl, isobutyl, and tert-butyl. Preferably, methyl and ethyl are included. More preferably, methyl is included.

The term "C₁₋₆ alkyl" refers to a straight- or branched-chain saturated hydrocarbon group having 1 to 6 carbon atoms. "C₁₋₆ alkyl" includes, for example, methyl, ethyl, n-propyl, isopropyl, n-butyl, sec-butyl, isobutyl, tert-butyl, n-pentyl, isopentyl, neopentyl, 2-methylbutyl, 1,1-dimethylpropyl, 1-ethylpropyl, n-hexyl, isohexyl, 1,1-dimethylbutyl, 2,2-dimethylbutyl, 3,3-dimethylbutyl, and 2-ethylbutyl. Preferably, methyl, ethyl, isopropyl, and isopentyl are included. More preferably, methyl is included.

The term "C₁₋₄ alkoxy" refers to a group wherein the above-defined "C₁₋₄ alkyl" binds to an oxygen atom. "C₁₋₄ alkoxy" includes methoxy, ethoxy, n-propoxy, isopropoxy, n-butoxy, sec-butoxy, isobutoxy, and tert-butoxy. Preferably, methoxy and ethoxy are included.

The term "C₁₋₆ alkoxy" refers to a group wherein the above-defined "C₁₋₆ alkyl" binds to an oxygen atom. "C₁₋₆ alkoxy" includes, for example, methoxy, ethoxy, n-propoxy, isopropoxy, n-butoxy, sec-butoxy, isobutoxy, tert-butoxy, pentyloxy, isopentyloxy, neopentyloxy, 2-methylbutoxy, 1,1-dimethylpropoxy, 1-ethylpropoxy, hexyloxy, isohexyloxy, 1,1-dimethylbutoxy, 2,2-dimethylbutoxy, 3,3-dimethylbutoxy, and 2-ethylbutoxy. Preferably, methoxy, ethoxy, and isopentyloxy are included.

The term "halogen" includes, for example, fluorine, chlorine, bromine, and iodine. Preferably, fluorine, chlorine, and bromine are included. More preferably, fluorine is included.

The term "C₁₋₄ haloalkyl" refers to the above-defined "C₁₋₄ alkyl" that is substituted with 1 to 7 halogen atoms independently selected from the group of the above-defined "halogen". "C₁₋₄ haloalkyl" includes, for example, monofluoromethyl, difluoromethyl, trifluoromethyl, chlorofluoromethyl, 2-fluoroethyl, 2-chloroethyl, 2-bromoethyl, 1,1-difluoroethyl, 2,2-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, and 4,4,4-trifluorobutyl. Preferably, monofluoromethyl, difluoromethyl, trifluoromethyl, 2,2-difluoroethyl, and 2,2,2-trifluoroethyl are included. More preferably, difluoromethyl is included.

The term "C₁₋₆ haloalkyl" refers to the above-defined "C₁₋₆ alkyl" that is substituted with 1 to 9 halogen atoms independently selected from the group of the above-defined "halogen". "C₁₋₆ haloalkyl" includes, for example, monofluoromethyl, difluoromethyl, trifluoromethyl, chlorofluoromethyl, 2-fluoroethyl, 2-chloroethyl, 2-bromoethyl, 1,1-difluoroethyl, 2,2-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, 4,4,4-trifluorobutyl, 5,5,5-trifluoropentyl, and 6,6,6-trifluorohexyl. Preferably, trifluoromethyl, 1,1-difluoroethyl, and 1-fluoro-1-methylethyl are included. More preferably, 1,1-difluoroethyl is included.

The term "C₃₋₆ cycloalkyl" refers to a monocyclic saturated hydrocarbon group having 3 to 6 carbon atoms. "C₃₋₆ cycloalkyl" includes, for example, cyclopropyl, cyclobutyl, cyclopentyl, and cyclohexyl.

The term "4- to 6-membered heterocycloalkyl comprising 1 or 2 heteroatoms independently selected from the group consisting of nitrogen and oxygen atom" refers to a 4- to 6-membered monocyclic saturated heterocyclic group comprising one or two heteroatoms independently selected from the group consisting of nitrogen and oxygen atom, besides carbon atoms, as a ring-constituting atom. "4- to 6-membered heterocycloalkyl comprising 1 or 2 heteroatoms independently selected from the group consisting of nitrogen and oxygen atom" includes, for example, azetidinyl, oxetanyl, diazetidinyl, dioxetanyl, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, tetrahydro-1,2-oxazinyl, and dioxanyl. Preferably, oxetanyl, pyrrolidinyl, piperidinyl, tetrahydropyranyl, morpholinyl, and dioxanyl are included.

The term "4- to 7-membered heterocycloalkyl comprising 1 or 2 heteroatoms independently selected from the group consisting of nitrogen, oxygen, and sulfur atoms" refers to a 4- to 7-membered monocyclic saturated heterocyclic group comprising 1 or 2 heteroatoms independently selected from the group consisting of nitrogen, oxygen, and sulfur atoms as well as carbon atoms as the ring-constituting atoms. "4-to 7-membered heterocycloalkyl comprising 1 or 2 heteroatoms independently selected from the group consisting of nitrogen, oxygen, and sulfur atoms" includes, for example, azetidinyl, oxetanyl, thietanyl, diazetidinyl, dioxetanyl, dithietanyl, pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, dithiolanyl, piperidinyl, tetrahydropyranyl, 1,2-diazacyclohexanyl, 1,3-diazacyclohexanyl, piperazinyl, morpholinyl, tetrahydro-1,2-oxadinyl, tetrahydro-1,3-oxadinyl, thiomorpholinyl, dioxanyl, azepanyl, oxepanyl, diazepanyl (for example, 1,4-diazepanyl), oxazepanyl (for example, 1,4-oxazepanyl and 1,2-oxazepanyl), dioxepanyl (for example, 1,4-dioxepanyl), and thiazepanyl. Preferably, oxetanyl, tetrahydrofuranyl, tetrahydrothiophenyl, piperidinyl, tetrahydropyranyl, dioxanyl, and dioxepanyl are included.

The term "C₅₋₆ cycloalkenyl" refers to a monocyclic partially-unsaturated hydrocarbon group having 5 to 6 carbon atoms and comprising at least one double bond. "C₅₋₆ cycloalkenyl" includes, for example, cyclopentenyl, cyclopentadienyl, cyclohexenyl, and cyclohexadienyl. Preferably, cyclopentenyl is included.

The term "C₅₋₆ cycloalkene" refers to a monocyclic partially unsaturated hydrocarbon ring comprising 5 to 6 carbon atoms and at least one double bond. "C₅₋₆ cycloalkene" includes, for example, cyclopentene, cyclopentadiene, cyclohexene, and cyclohexadiene.

The term "5- or 6-membered heterocycloalkenyl comprising 1 or 2 heteroatoms independently selected from the group consisting of nitrogen and oxygen atoms" refers to a 5- or 6-membered monocyclic partially unsaturated heterocyclic group comprising 1 or 2 heteroatoms independently selected from the group consisting of nitrogen and oxygen atoms as well as carbon atoms as the ring-constituting atoms and at least one double bond. "5- or 6-membered heterocycloalkenyl comprising 1 or 2 heteroatoms independently selected from the group consisting of nitrogen and oxygen atoms" includes, for example, pyrrolinyl, pyrazolinyl, imidazolinyl, dihydrofuranyl, dioxolyl, oxazolinyl, isoxazolinyl, tetrahydropyridinyl, tetrahydropyrimidyl, tetrahydropyridazinyl, tetrahydropyrazinyl, dihydropyridinyl, dihydropyranyl, dihydrodioxinyl, pyranyl, and dihydrooxadinyl. Preferably, dihydrofuranyl and dihydropyranyl are included.

The term "5- to 7-membered heterocycloalkene comprising 1 or 2 oxygen atoms" refers to a 5- to 7-membered monocyclic partially unsaturated heterocycle comprising 1 or 2 oxygen atoms as well as carbon atoms as the ring-constituting atoms and at least one double bond. "5- to 7-membered heterocycloalkene comprising 1 or 2 oxygen atoms" includes, for example, dihydrofuran, dioxole, dihydropyrane, dihydrodioxine, pyrane, tetrahydrooxepine, dihydrodioxepine, dihydrooxepine, and dioxepine. Preferably, dihydropyrane is included.

The term "7- to 9-membered saturated fused heterocyclic group comprising 1 or 2 heteroatoms independently selected from the group consisting of nitrogen and oxygen atoms" refers to a 7- to 9-membered saturated fused heterocyclic group comprising 1 or 2 heteroatoms independently selected from the group consisting of nitrogen and oxygen atoms as well as carbon atoms as the ring-constituting atoms. "7- to 9-membered saturated fused heterocyclic group comprising 1 or 2 heteroatoms independently selected from the group consisting of nitrogen and oxygen atoms" includes, for example, the following group:

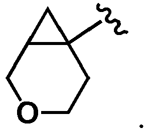

The term "5- to 8-membered bridged cycloalkyl" refers to a 5- to 8-membered bridged cyclic saturated hydrocarbon group. "5- to 8-membered bridged cycloalkyl" includes, for example, bicyclo[1.1.1]pentyl, bicyclo[2.2.1]heptyl, and bicyclo[2.2.2]octyl. Preferably, bicyclo[1.1.1]pentyl is included.

The term "5- to 8-membered bridged heterocycloalkyl comprising 1 or 2 heteroatoms independently selected from the group consisting of nitrogen and oxygen atoms" refers to a 5- to 8-membered bridged saturated heterocyclic group comprising 1 or 2 heteroatoms independently selected from the group consisting of nitrogen and oxygen atoms as well as carbon atoms as the ring-constituting atoms. "5- to 8-membered bridged heterocycloalkyl comprising 1 or 2 heteroatoms independently selected from the group consisting of nitrogen and oxygen atoms" includes, for example, the following groups:

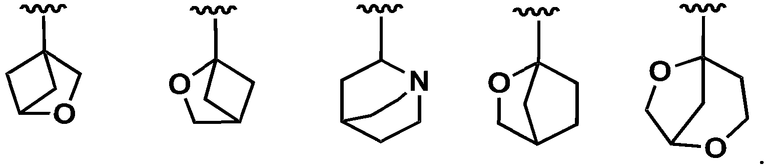

In certain embodiments of the invention, R¹³ combines together with R¹¹ or R¹² and the carbon atoms to which they attach to form a ring structure, and the partial structure: 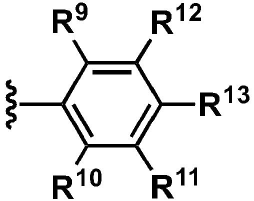 in Formula [I] forms, as a whole, a 9- to 11-membered partially unsaturated fused cyclic group optionally comprising 1 or 2 oxygen atoms, wherein the cyclic group is a fused cyclic group substituted with R⁹ and R¹⁰ and R¹¹ or R¹². The fused cyclic group includes, for example, the following group:

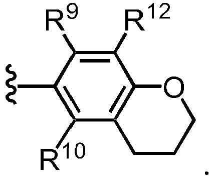

The phrase wherein α may be "optionally substituted with" β means that α is unsubstituted, or any of replaceable hydrogen atoms of α is replaced with β. For example, "C₁₋₆ alkyl optionally substituted with hydroxy" means that C₁₋₆ alkyl is unsubstituted, or any of hydrogen atoms of C₁₋₆ alkyl is replaced with hydroxy.

Embodiments of each substituent of a compound of Formula [I] are illustrated as below. Each substituent of a compound of Formula [I] is, however, not limited to these embodiments, and a compound of Formula [I] also includes any combination of two or more of these embodiments in each substituent.

The partial structure: 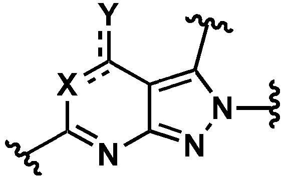 is preferably a structure of the following formula: 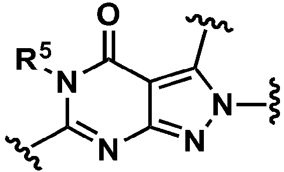 wherein R⁵ is the same as defined above.

R⁵ is preferably hydrogen.

The cyclic group Cy is preferably a group of the following formula: 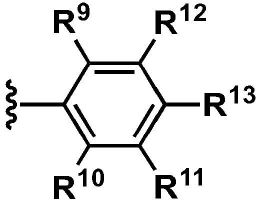 wherein each symbol is the same as defined above.

Preferably, R⁹ and R¹⁰ are each independently
(a) C₁₋₄ alkyl, wherein the alkyl may be optionally substituted with C₁₋₄ alkoxy,
(b) C₁₋₄ alkoxy,
(c) halogen,
(d) C₁₋₄ haloalkyl, or
(e) -O-C₁₋₄ haloalkyl.

More preferably, R⁹ and R¹⁰ are each independently C₁₋₄ alkyl, wherein the alkyl may be optionally substituted with C₁₋₄ alkoxy.

Preferably, R¹¹ and R¹² are each independently hydrogen or C₁₋₄ alkyl.

More preferably, R¹¹ and R¹² are hydrogen.

Preferably, R¹³ is
(a) C₁₋₄ alkyl,
(b) C₁₋₄ alkoxy,
(c) halogen,
(d) C₁₋₆ haloalkyl,
(e) -O-C₁₋₄ haloalkyl, or
(f) C₃₋₆ cycloalkyl, wherein the cycloalkyl may be optionally substituted with 1 or 2 halogen atoms, or
R¹³ combines together with R¹¹ or R¹² and the carbon atoms to which they attach to form:
(a) C₅₋₆ cycloalkene, or
(b) 5- to 7-membered heterocycloalkene comprising 1 or 2 oxygen atoms.

More preferably, R¹³ is C₁₋₆ haloalkyl or -O-C₁₋₄ haloalkyl.

In the partial structure: 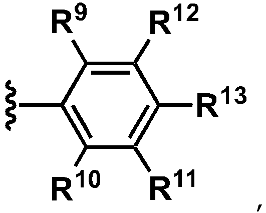 preferably,
R⁹ and R¹⁰ are each independently
   (a) C₁₋₄ alkyl, wherein the alkyl may be optionally substituted with C₁₋₄ alkoxy,
   (b) C₁₋₄ alkoxy,
   (c) halogen,
   (d) C₁₋₄ haloalkyl, or
   (e) -O-C₁₋₄ haloalkyl;
R¹¹ and R¹² are each independently hydrogen or C₁₋₄ alkyl; R¹³ is
   (a) C₁₋₄ alkyl,
   (b) C₁₋₄ alkoxy,
   (c) halogen,
   (d) C₁₋₆ haloalkyl,
   (e) -O-C₁₋₄ haloalkyl, or
   (f) C₃₋₆ cycloalkyl, wherein the cycloalkyl may be optionally substituted with 1 or 2 halogen atoms, or
R¹³ combines together with R¹¹ or R¹² and the carbon atoms to which they attach to form:
(a) C₅₋₆ cycloalkene, or
(b) 5- to 7-membered heterocycloalkene comprising 1 or 2 oxygen atoms.

In the partial structure: 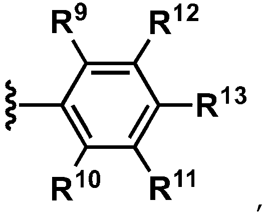 more preferably,
R⁹ and R¹⁰ are each independently C₁₋₄ alkyl, wherein the alkyl may be optionally substituted with C₁₋₄ alkoxy;
R¹¹ and R¹² are hydrogen; and
R¹³ is C₁₋₆ haloalkyl or -O-C₁₋₄ haloalkyl.

In the partial structure: 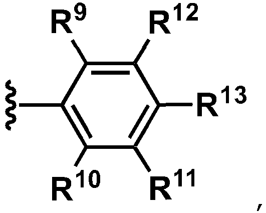 still more preferably,
R⁹ and R¹⁰ are each independently C₁₋₄ alkyl, wherein the alkyl may be optionally substituted with C₁₋₄ alkoxy;
R¹¹ and R¹² are hydrogen; and
R¹³ is C₁₋₆ haloalkyl.

In the partial structure: 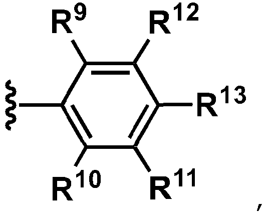 still more preferably,
R⁹ and R¹⁰ are each independently C₁₋₄ alkyl, wherein the alkyl may be optionally substituted with C₁₋₄ alkoxy;
R¹¹ and R¹² are hydrogen; and
R¹³ is -O-C₁₋₄ haloalkyl.

R¹ is preferably hydrogen.

Preferably, R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
   (a) hydroxy,
   (b) C₁₋₄ alkoxy, and
   (c) -SO₂-C₁₋₄ alkyl,
(4) C₁₋₆ alkoxy, wherein the alkoxy may be optionally substituted with:
   (a) hydroxy,
   (b) phenyl, or
   (c) 4- to 6-membered heterocycloalkyl comprising 1 or 2 heteroatoms independently selected from the group consisting of nitrogen and oxygen atoms,
(5) C₁₋₄ haloalkyl, wherein the haloalkyl may be optionally substituted with hydroxy or C₁₋₄ alkoxy,
(6) -O-C₁₋₄ haloalkyl, or
(7) C₃₋₆ cycloalkyl, or

R², R³, and R⁴ are combined together with the carbon atom to which they attach, and the -CR²R³R⁴ group forms:
   (a) C₃₋₆ cycloalkyl, wherein the cycloalkyl may be optionally substituted with 1 to 3 substituents independently selected from the group consisting of:
      (1) cyano,
      (2) C₁₋₄ alkyl, wherein the alkyl may be optionally substituted with hydroxy or C₁₋₄ alkoxy,
      (3) C₁₋₄ alkoxy,
      (4) halogen, and
      (5) -CO-NR¹⁸R¹⁹, wherein R¹⁸ and R¹⁹ are each independently hydrogen or C₁₋₄ alkyl,
   (b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
      (1) hydroxy,
      (2) C₁₋₄ alkyl,
      (3) C₁₋₄ alkoxy, and
      (4) halogen, or
      any one of the ring-constituting atoms of the heterocycloalkyl may be optionally substituted with 1 or 2 oxo groups,
   (c) 5- to 8-membered bridged cycloalkyl, wherein the bridged cycloalkyl may be optionally substituted with halogen,
   (d) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
   (e) 5- or 6-membered heterocycloalkenyl comprising 1 or 2 heteroatoms independently selected from the group consisting of nitrogen and oxygen atoms, or
   (f) a group of the following formula: 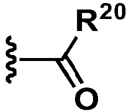
wherein R²⁰ is the same as defined above.

More preferably, R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
   (a) hydroxy,
   (b) C₁₋₄ alkoxy, and
   (c) -SO₂-C₁₋₄ alkyl,
(4) C₁₋₆ alkoxy, wherein the alkoxy may be optionally substituted with:
   (a) hydroxy,
   (b) phenyl, or
   (c) 4- to 6-membered heterocycloalkyl comprising 1 or 2 heteroatoms independently selected from the group consisting of nitrogen and oxygen atoms, or
(5) C₁₋₄ haloalkyl, wherein the haloalkyl may be optionally substituted with hydroxy or C₁₋₄ alkoxy, or

R², R³, and R⁴ are combined together with the carbon atom to which they attach, and the -CR²R³R⁴ group forms:
   (a) C₃₋₆ cycloalkyl, wherein the cycloalkyl may be optionally substituted with 1 to 3 substituents independently selected from the group consisting of:
      (1) cyano,
      (2) C₁₋₄ alkyl, wherein the alkyl may be optionally substituted with hydroxy or C₁₋₄ alkoxy,
      (3) C₁₋₄ alkoxy,
      (4) halogen, and
      (5) -CO-NR¹⁸R¹⁹, wherein R¹⁸ and R¹⁹ are each independently hydrogen or C₁₋₄ alkyl,
   (b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
      (1) hydroxy,
      (2) C₁₋₄ alkyl,
      (3) C₁₋₄ alkoxy, and
      (4) halogen, or
      any one of the ring-constituting atoms of the heterocycloalkyl may be optionally substituted with 1 or 2 oxo groups, or
   (c) a group of the following formula: 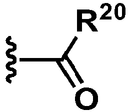
wherein R²⁰ is the same as defined above.

Still more preferably, R², R³, and R⁴ are each independently
(1) hydrogen,
(2) hydroxy, or
(3) C₁₋₆ alkyl, wherein the alkyl may be optionally substituted with 1 or 2 substituents independently selected from the group consisting of:
   (a) hydroxy,
   (b) C₁₋₄ alkoxy, and
   (c) -SO₂-C₁₋₄ alkyl, or

R², R³, and R⁴ are combined together with the carbon atom to which they attach, and the -CR²R³R⁴ group forms:
   (a) C₃₋₆ cycloalkyl, wherein the cycloalkyl may be optionally substituted with 1 to 3 substituents independently selected from the group consisting of:
      (1) cyano,
      (2) C₁₋₄ alkyl, wherein the alkyl may be optionally substituted with hydroxy or C₁₋₄ alkoxy,
      (3) C₁₋₄ alkoxy,
      (4) halogen, and
      (5) -CO-NR¹⁸R¹⁹, wherein R¹⁸ and R¹⁹ are each independently hydrogen or C₁₋₄ alkyl, or
   (b) a group of the following formula: 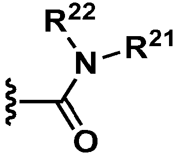
wherein R²¹ and R²² are the same as defined above.

Still more preferably, R², R³, and R⁴ are each independently
(1) hydrogen,
(2) hydroxy, or
(3) C₁₋₆ alkyl, wherein the alkyl may be optionally substituted with 1 or 2 substituents independently selected from the group consisting of:
   (a) hydroxy,
   (b) C₁₋₄ alkoxy, and
   (c) -SO₂-C₁₋₄ alkyl.

Still more preferably, R², R³, and R⁴ are combined together with the carbon atom to which they attach, and the -CR²R³R⁴ group forms C₃₋₆ cycloalkyl, wherein the cycloalkyl may be optionally substituted with 1 to 3 substituents independently selected from the group consisting of:
(1) cyano,
(2) C₁₋₄ alkyl, wherein the alkyl may be optionally substituted with hydroxy or C₁₋₄ alkoxy,
(3) C₁₋₄ alkoxy,
(4) halogen, and
(5) -CO-NR¹⁸R¹⁹, wherein R¹⁸ and R¹⁹ are each independently hydrogen or C₁₋₄ alkyl.

Preferably, R²⁰ is -NR²¹R²², wherein R²¹ and R²² are the same as defined above.

In one preferable embodiment of a compound of Formula [I],
the partial structure: 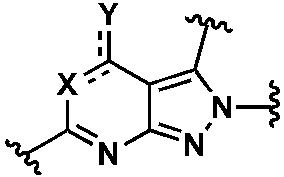 is a structure of the following formula: 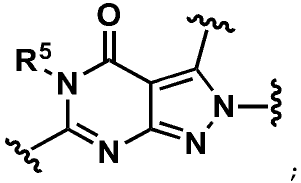
R⁵ is hydrogen;
the cyclic group Cy is a group of the following formula: 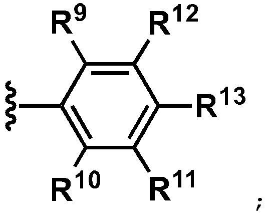
R⁹ and R¹⁰ are each independently
   (a) C₁₋₄ alkyl, wherein the alkyl may be optionally substituted with C₁₋₄ alkoxy,
   (b) C₁₋₄ alkoxy,
   (c) halogen,
   (d) C₁₋₄ haloalkyl, or
   (e) -O-C₁₋₄ haloalkyl;
R¹¹ and R¹² are each independently hydrogen or C₁₋₄ alkyl;
R¹³ is :
   (a) C₁₋₄ alkyl,
   (b) C₁₋₄ alkoxy,
   (c) halogen,
   (d) C₁₋₆ haloalkyl,
   (e) -O-C₁₋₄ haloalkyl, or
   (f) C₃₋₆ cycloalkyl, wherein the cycloalkyl may be optionally substituted with 1 or 2 halogen atoms, or

R¹³ combines together with R¹¹ or R¹² and the carbon atoms to which they attach to form:
   (a) C₅₋₆ cycloalkene, or
   (b) 5- to 7-membered heterocycloalkene comprising 1 or 2 oxygen atoms;
      R¹ is hydrogen; and
      R², R³, and R⁴ are each independently
         (1) hydrogen,
         (2) hydroxy,
         (3) C₁₋₆ alkyl, wherein the alkyl may be optionally substituted with 1 or 2 substituents independently selected from the group consisting of:
            (a) hydroxy,
            (b) C₁₋₄ alkoxy, and
            (c) -SO₂-C₁₋₄ alkyl,
         (4) C₁₋₆ alkoxy, wherein the alkoxy may be optionally substituted with:
            (a) hydroxy,
            (b) phenyl, or
            (c) 4- to 6-membered heterocycloalkyl comprising 1 or 2 heteroatoms independently selected from the group consisting of nitrogen and oxygen atoms,
         (5) C₁₋₄ haloalkyl, wherein the haloalkyl may be optionally substituted with hydroxy or C₁₋₄ alkoxy,
         (6) -O-C₁₋₄ haloalkyl, or
         (7) C₃₋₆ cycloalkyl, or
R², R³, and R⁴ are combined together with the carbon atom to which they attach, and the -CR²R³R⁴ group forms:
   (a) C₃₋₆ cycloalkyl, wherein the cycloalkyl may be optionally substituted with 1 to 3 substituents independently selected from the group consisting of:
      (1) cyano,
      (2) C₁₋₄ alkyl, wherein the alkyl may be optionally substituted with hydroxy or C₁₋₄ alkoxy,
      (3) C₁₋₄ alkoxy,
      (4) halogen, and
      (5) -CO-NR¹⁸R¹⁹, wherein R¹⁸ and R¹⁹ are each independently hydrogen or C₁₋₄ alkyl,
   (b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
      (1) hydroxy,
      (2) C₁₋₄ alkyl,
      (3) C₁₋₄ alkoxy, and
      (4) halogen, or
      any one of the ring-constituting atoms of the heterocycloalkyl may be optionally substituted with 1 or 2 oxo groups,
   (c) 5- to 8-membered bridged cycloalkyl, wherein the bridged cycloalkyl may be optionally substituted with halogen,
   (d) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
   (e) 5- or 6-membered heterocycloalkenyl comprising 1 or 2 heteroatoms independently selected from the group consisting of nitrogen and oxygen atoms, or
   (f) a group of the following formula: 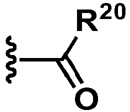
wherein R²⁰ is the same as defined above.

Another preferable embodiment of a compound of Formula [I] is a compound of Formula [I] wherein the partial structure: 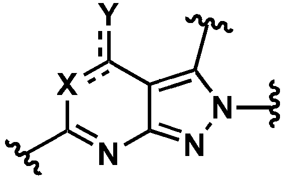 is
(1) a structure of the following formula: 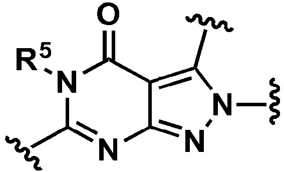 wherein R⁵ is hydrogen or C₁₋₄ alkyl, wherein the alkyl may be optionally substituted with:
   (a) carboxy,
   (b) -CO-C₁₋₄ alkoxy, or
   (c) -CO-NR⁶R⁷, wherein R⁶ and R⁷ are each independently hydrogen or C₁₋₄ alkyl, or
(2) a structure of the following formula: 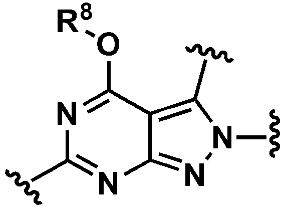 wherein R⁸ is C₁₋₄ alkyl;
   the cyclic group Cy is
   (1) a group of the formula: 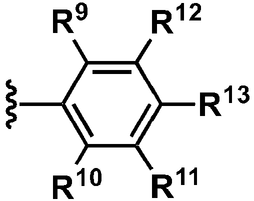
      wherein R⁹ and R¹⁰ are each independently
         (a) hydrogen,
         (b) C₁₋₄ alkyl, wherein the alkyl may be optionally substituted with C₁₋₄ alkoxy,
         (c) C₁₋₄ alkoxy,
         (d) halogen,
         (e) C₁₋₄ haloalkyl, or
         (f) -O-C₁₋₄ haloalkyl,
            R¹¹ and R¹² are each independently
               (a) hydrogen,
               (b) C₁₋₄ alkyl, or
               (c) C₁₋₄ haloalkyl,
            R¹³ is
               (a) hydrogen,
               (b) C₁₋₄ alkyl,
               (c) C₁₋₄ alkoxy,
               (d) C₁₋₆ haloalkyl, or
               (e) -O-C₁₋₄ haloalkyl, or
      R¹³ may combine together with R¹¹ or R¹² and the carbon atoms to which they attach to form:
         (a) C₅₋₆ cycloalkene, or
         (b) 5- to 7-membered heterocycloalkene comprising 1 or 2 oxygen atoms, or
   (2) a group of the formula: 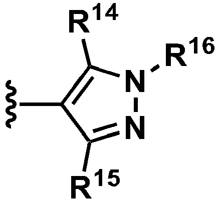 wherein R¹⁴ and R¹⁵ are each independently C₁₋₄ alkyl or C₁₋₄ haloalkyl,
      R¹⁶ is C₁₋₆ alkyl or C₃₋₆ cycloalkyl;
      R¹ is

      (1) hydrogen,
      (2) cyano,
      (3) C₁₋₄ alkyl, wherein the alkyl may be optionally substituted with hydroxy or C₁₋₄ alkoxy,
      (4) C₁₋₄ haloalkyl, or
      (5) -CO-C₁₋₄ alkyl;
         R², R³, and R⁴ are each independently
         (1) hydrogen,
         (2) hydroxy,
         (3) C₁₋₆ alkyl, wherein the alkyl may be optionally substituted with 1 or 2 substituents independently selected from the group consisting of:
            (a) hydroxy,
            (b) C₁₋₄ alkoxy, and
            (c) -SO₂-C₁₋₄ alkyl,
         (4) C₁₋₆ alkoxy, wherein the alkoxy may be optionally substituted with:
            (a) hydroxy,
            (b) phenyl, or
            (c) 4- to 6-membered heterocycloalkyl comprising 1 or 2 heteroatoms independently selected from the group consisting of nitrogen and oxygen atoms,
         (5) halogen,
         (6) C₁₋₄ haloalkyl, wherein the haloalkyl may be optionally substituted with hydroxy or C₁₋₄ alkoxy,
         (7) -O-C₁₋₄ haloalkyl,
         (8) -OR¹⁷, wherein R¹⁷ is 4- to 6-membered heterocycloalkyl comprising 1 or 2 heteroatoms independently selected from the group consisting of nitrogen and oxygen atoms,
         (9) C₃₋₆ cycloalkyl,
         (10) 4- to 6-membered heterocycloalkyl comprising 1 or 2 heteroatoms independently selected from the group consisting of nitrogen and oxygen atoms, wherein the heterocycloalkyl may be optionally substituted with oxo, or
         (11) a group of the formula: 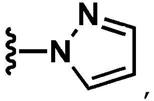 or
            R², R³, and R⁴ are combined together with the carbon atom to which they attach, and the -CR²R³R⁴ group forms:
            (a) cyano,
            (b) C₃₋₆ cycloalkyl, wherein the cycloalkyl may be optionally substituted with 1 to 3 substituents independently selected from the group consisting of:
               (1) cyano,
               (2) C₁₋₄ alkyl, wherein the alkyl may be optionally substituted with hydroxy or C₁₋₄ alkoxy,
               (3) C₁₋₄ alkoxy,
               (4) halogen, and
               (5) -CO-NR¹⁸R¹⁹, wherein R¹⁸ and R¹⁹ are each independently hydrogen or C₁₋₄ alkyl,
            (c) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
               (1) hydroxy,
               (2) C₁₋₄ alkyl,
               (3) C₁₋₄ alkoxy, and
               (4) halogen, or
               any one of the ring-constituting atoms of the heterocycloalkyl may be optionally substituted with 1 or 2 oxo,
            (d) 7- to 9-membered saturated fused heterocyclic group comprising 1 or 2 heteroatoms independently selected from the group consisting of nitrogen and oxygen atoms, wherein the fused heterocyclic group may be optionally substituted with 1 or 2 halogen atoms,
            (e) 5- to 8-membered bridged cycloalkyl, wherein the bridged cycloalkyl may be optionally substituted with halogen,
            (f) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
            (g) C₅₋₆ cycloalkenyl,
            (h) 5- or 6-membered heterocycloalkenyl comprising 1 or 2 heteroatoms independently selected from the group consisting of nitrogen and oxygen atoms, or
            (i) a group of the formula: 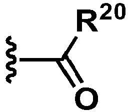 wherein R²⁰ is
               (1) C₁₋₄ alkyl, or
               (2) -NR²¹R²², wherein R²¹ and R²² are each independently
                  (a) hydrogen,
                  (b) C₁₋₄ alkyl,
                  (c) C₁₋₄ haloalkyl, or
                  (d) 4- to 6-membered heterocycloalkyl comprising 1 or 2 heteroatoms independently selected from the group consisting of nitrogen and oxygen atoms.

Still another preferable embodiment of a compound of Formula [I] is a compound of Formula [II]: 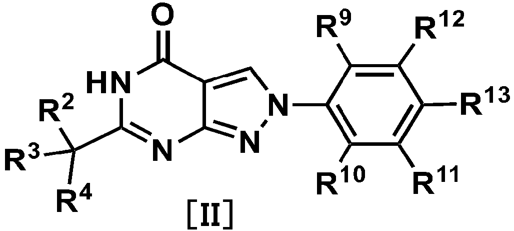
wherein R⁹ and R¹⁰ are each independently
   (a) hydrogen,
   (b) C₁₋₄ alkyl, wherein the alkyl may be optionally substituted with C₁₋₄ alkoxy,
   (c) C₁₋₄ alkoxy,
   (d) halogen,
   (e) C₁₋₄ haloalkyl, or
   (f) -O-C₁₋₄ haloalkyl;

   R¹¹ and R¹² are each independently
      (a) hydrogen,
      (b) C₁₋₄ alkyl, or
      (c) C₁₋₄ haloalkyl;
   R¹³ is
      (a) hydrogen,
      (b) C₁₋₄ alkyl,
      (c) C₁₋₄ alkoxy,
      (d) C₁₋₆ haloalkyl, or
      (e) -O-C₁₋₄ haloalkyl, or
R¹³ combines together with R¹¹ or R¹² and the carbon atoms to which they attach to form:
   (a) C₅₋₆ cycloalkene, or
   (b) 5- to 7-membered heterocycloalkene comprising 1 or 2 oxygen atoms;

   R², R³, and R⁴ are each independently
      (1) hydrogen,
      (2) hydroxy,
      (3) C₁₋₆ alkyl, wherein the alkyl may be optionally substituted with 1 or 2 substituents independently selected from the group consisting of:
         (a) hydroxy,
         (b) C₁₋₄ alkoxy, and
         (c) -SO₂-C₁₋₄ alkyl,
      (4) C₁₋₆ alkoxy, wherein the alkoxy may be optionally substituted with:
         (a) hydroxy,
         (b) phenyl, or
         (c) 4- to 6-membered heterocycloalkyl comprising 1 or 2 heteroatoms independently selected from the group consisting of nitrogen and oxygen atoms,
      (5) halogen,
      (6) C₁₋₄ haloalkyl, wherein the haloalkyl may be optionally substituted with hydroxy or C₁₋₄ alkoxy,
      (7) -O-C₁₋₄ haloalkyl,
      (8) -OR¹⁷, wherein R¹⁷ is 4- to 6-membered heterocycloalkyl comprising 1 or 2 heteroatoms independently selected from the group consisting of nitrogen and oxygen atoms,
      (9) C₃₋₆ cycloalkyl,
      (10) 4- to 6-membered heterocycloalkyl comprising 1 or 2 heteroatoms independently selected from the group consisting of nitrogen and oxygen atoms, wherein the heterocycloalkyl may be optionally substituted with oxo, or
      (11) a group of the formula: 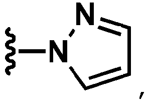 or
   R², R³, and R⁴ are combined together with the carbon atom to which they attach, and the -CR²R³R⁴ group forms:
      (a) cyano,
      (b) C₃₋₆ cycloalkyl, wherein the cycloalkyl may be optionally substituted with 1 to 3 substituents independently selected from the group consisting of:
         (1) cyano,
         (2) C₁₋₄ alkyl, wherein the alkyl may be optionally substituted with hydroxy or C₁₋₄ alkoxy,
         (3) C₁₋₄ alkoxy,
         (4) halogen, and
         (5) -CO-NR¹⁸R¹⁹, wherein R¹⁸ and R¹⁹ are each independently hydrogen or C₁₋₄ alkyl,
      (c) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
         (1) hydroxy,
         (2) C₁₋₄ alkyl,
         (3) C₁₋₄ alkoxy, and
         (4) halogen, or
         any one of the ring-constituting atoms of the heterocycloalkyl may be optionally substituted with 1 or 2 oxo,
      (d) 7- to 9-membered saturated fused heterocyclic group comprising 1 or 2 heteroatoms independently selected from the group consisting of nitrogen and oxygen atoms, wherein the fused heterocyclic group may be optionally substituted with 1 or 2 halogen atoms,
      (e) 5- to 8-membered bridged cycloalkyl, wherein the bridged cycloalkyl may be optionally substituted with halogen,
      (f) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
      (g) C₅₋₆ cycloalkenyl,
      (h) 5- or 6-membered heterocycloalkenyl comprising 1 or 2 heteroatoms independently selected from the group consisting of nitrogen and oxygen atoms, or
      (i) a group of the formula: 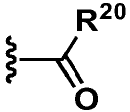
wherein R²⁰ is
   (1) C₁₋₄ alkyl, or
   (2) -NR²¹R²², wherein R²¹ and R²² are each independently
      (a) hydrogen,
      (b) C₁₋₄ alkyl,
      (c) C₁₋₄ haloalkyl, or
      (d) 4- to 6-membered heterocycloalkyl comprising 1 or 2 heteroatoms independently selected from the group consisting of nitrogen and oxygen atoms.

A preferable embodiment of a compound of Formula [II] is a compound of Formula [III]: 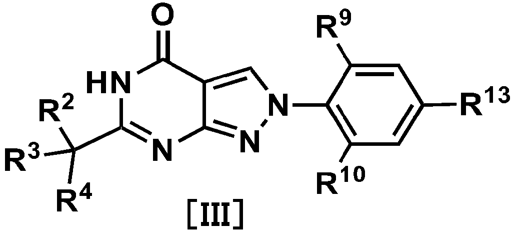
wherein R⁹ and R¹⁰ are each independently
   (a) hydrogen,
   (b) C₁₋₄ alkyl, wherein the alkyl may be optionally substituted with C₁₋₄ alkoxy,
   (c) C₁₋₄ alkoxy,
   (d) halogen,
   (e) C₁₋₄ haloalkyl, or
   (f) -O-C₁₋₄ haloalkyl;
      R¹³ is
      (a) hydrogen,
      (b) C₁₋₄ alkyl,
      (c) C₁₋₄ alkoxy,
      (d) C₁₋₆ haloalkyl, or
      (e) -O-C₁₋₄ haloalkyl, or
R¹³ combines together with R¹¹ or R¹² and the carbon atoms to which they attach to form:
   (a) C₅₋₆ cycloalkene, or
   (b) 5- to 7-membered heterocycloalkene comprising 1 or 2 oxygen atoms;

   R², R³, and R⁴ are each independently
      (1) hydrogen,
      (2) hydroxy,
      (3) C₁₋₆ alkyl, wherein the alkyl may be optionally substituted with 1 or 2 substituents independently selected from the group consisting of:
         (a) hydroxy,
         (b) C₁₋₄ alkoxy, and
         (c) -SO₂-C₁₋₄ alkyl,
      (4) C₁₋₆ alkoxy, wherein the alkoxy may be optionally substituted with:
         (a) hydroxy,
         (b) phenyl, or
         (c) 4- to 6-membered heterocycloalkyl comprising 1 or 2 heteroatoms independently selected from the group consisting of nitrogen and oxygen atoms,
      (5) halogen,
      (6) C₁₋₄ haloalkyl, wherein the haloalkyl may be optionally substituted with hydroxy or C₁₋₄ alkoxy,
      (7) -O-C₁₋₄ haloalkyl,
      (8) -OR¹⁷, wherein R¹⁷ is 4- to 6-membered heterocycloalkyl comprising 1 or 2 heteroatoms independently selected from the group consisting of nitrogen and oxygen atoms,
      (9) C₃₋₆ cycloalkyl,
      (10) 4- to 6-membered heterocycloalkyl comprising 1 or 2 heteroatoms independently selected from the group consisting of nitrogen and oxygen atoms, wherein the heterocycloalkyl may be optionally substituted with oxo, or
      (11) a group of the formula: 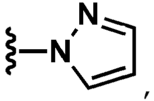 or
   R², R³, and R⁴ are combined together with the carbon atom to which they attach, and the -CR²R³R⁴ group forms:
      (a) cyano,
      (b) C₃₋₆ cycloalkyl, wherein the cycloalkyl may be optionally substituted with 1 to 3 substituents independently selected from the group consisting of:
         (1) cyano,
         (2) C₁₋₄ alkyl, wherein the alkyl may be optionally substituted with hydroxy or C₁₋₄ alkoxy,
         (3) C₁₋₄ alkoxy,
         (4) halogen, and
         (5) -CO-NR¹⁸R¹⁹, wherein R¹⁸ and R¹⁹ are each independently hydrogen or C₁₋₄ alkyl,
      (c) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
         (1) hydroxy,
         (2) C₁₋₄ alkyl,
         (3) C₁₋₄ alkoxy, and
         (4) halogen, or
         any one of the ring-constituting atoms of the heterocycloalkyl may be optionally substituted with 1 or 2 oxo,
      (d) 7- to 9-membered saturated fused heterocyclic group comprising 1 or 2 heteroatoms independently selected from the group consisting of nitrogen and oxygen atoms, wherein the fused heterocyclic group may be optionally substituted with 1 or 2 halogen atoms,
      (e) 5- to 8-membered bridged cycloalkyl, wherein the bridged cycloalkyl may be optionally substituted with halogen,
      (f) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
      (g) C₅₋₆ cycloalkenyl,
      (h) 5- or 6-membered heterocycloalkenyl comprising 1 or 2 heteroatoms independently selected from the group consisting of nitrogen and oxygen atoms, or
      (i) a group of the formula: 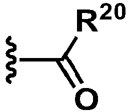
wherein R²⁰ is
   (1) C₁₋₄ alkyl, or
   (2) -NR²¹R²², wherein R²¹ and R²² are each independently
      (a) hydrogen,
      (b) C₁₋₄ alkyl,
      (c) C₁₋₄ haloalkyl, or
      (d) 4- to 6-membered heterocycloalkyl comprising 1 or 2 heteroatoms independently selected from the group consisting of nitrogen and oxygen atoms.

In a preferable embodiment of a compound of Formula [III],
R⁹ and R¹⁰ are each independently
   (a) C₁₋₄ alkyl, wherein the alkyl may be optionally substituted with C₁₋₄ alkoxy,
   (b) C₁₋₄ alkoxy,
   (c) halogen,
   (d) C₁₋₄ haloalkyl, or
   (e) -O-C₁₋₄ haloalkyl;
R¹³ is C₁₋₆ haloalkyl or -O-C₁₋₄ haloalkyl;
R², R³, and R⁴ are each independently
   (1) hydrogen,
   (2) hydroxy,
   (3) C₁₋₆ alkyl, wherein the alkyl may be optionally substituted with 1 or 2 substituents independently selected from the group consisting of:
      (a) hydroxy,
      (b) C₁₋₄ alkoxy, and
      (c) -SO₂-C₁₋₄ alkyl,
   (4) C₁₋₆ alkoxy, wherein the alkoxy may be optionally substituted with:
      (a) hydroxy,
      (b) phenyl, or
      (c) 4- to 6-membered heterocycloalkyl comprising 1 or 2 heteroatoms independently selected from the group consisting of nitrogen and oxygen atoms,
   (5) C₁₋₄ haloalkyl, wherein the haloalkyl may be optionally substituted with hydroxy or C₁₋₄ alkoxy,
   (6) -O-C₁₋₄ haloalkyl, or
   (7) C₃₋₆ cycloalkyl, or
R², R³, and R⁴ are combined together with the carbon atom to which they attach, and the -CR²R³R⁴ group forms:
   (a) C₃₋₆ cycloalkyl, wherein the cycloalkyl may be optionally substituted with 1 to 3 substituents independently selected from the group consisting of:
      (1) cyano,
      (2) C₁₋₄ alkyl, wherein the alkyl may be optionally substituted with hydroxy or C₁₋₄ alkoxy,
      (3) C₁₋₄ alkoxy,
      (4) halogen, and
      (5) -CO-NR¹⁸R¹⁹, wherein R¹⁸ and R¹⁹ are each independently hydrogen or C₁₋₄ alkyl,
   (b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
      (1) hydroxy,
      (2) C₁₋₄ alkyl,
      (3) C₁₋₄ alkoxy, and
      (4) halogen, or
      any one of the ring-constituting atoms of the heterocycloalkyl may be optionally substituted with 1 or 2 oxo,
   (c) 5- to 8-membered bridged cycloalkyl, wherein the bridged cycloalkyl may be optionally substituted with halogen,
   (d) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
   (e) 5- or 6-membered heterocycloalkenyl comprising 1 or 2 heteroatoms independently selected from the group consisting of nitrogen and oxygen atoms, or
   (f) a group of the formula: 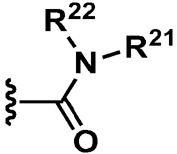 wherein R²¹ and R²² are each independently
      (1) hydrogen,
      (2) C₁₋₄ alkyl,
      (3) C₁₋₄ haloalkyl, or
      (4) 4- to 6-membered heterocycloalkyl comprising 1 or 2 heteroatoms independently selected from the group consisting of nitrogen and oxygen atoms.

In a more preferable embodiment of a compound of Formula [III],
R⁹ and R¹⁰ are each independently C₁₋₄ alkyl, wherein the alkyl may be optionally substituted with C₁₋₄ alkoxy;
R¹³ is C₁₋₆ haloalkyl or -O-C₁₋₄ haloalkyl;
R², R³, and R⁴ are each independently
   (1) hydrogen,
   (2) hydroxy,
   (3) C₁₋₆ alkyl, wherein the alkyl may be optionally substituted with 1 or 2 substituents independently selected from the group consisting of:
      (a) hydroxy,
      (b) C₁₋₄ alkoxy, and
      (c) -SO₂-C₁₋₄ alkyl,
   (4) C₁₋₆ alkoxy, wherein the alkoxy may be optionally substituted with:
      (a) hydroxy,
      (b) phenyl, or
      (c) 4- to 6-membered heterocycloalkyl comprising 1 or 2 heteroatoms independently selected from the group consisting of nitrogen and oxygen atoms, or
   (5) C₁₋₄ haloalkyl, wherein the haloalkyl may be optionally substituted with hydroxy or C₁₋₄ alkoxy, or
R², R³, and R⁴ are combined together with the carbon atom to which they attach, and the -CR²R³R⁴ group forms:
   (a) C₃₋₆ cycloalkyl, wherein the cycloalkyl may be optionally substituted with 1 to 3 substituents independently selected from the group consisting of:
      (1) cyano,
      (2) C₁₋₄ alkyl, wherein the alkyl may be optionally substituted with hydroxy or C₁₋₄ alkoxy,
      (3) C₁₋₄ alkoxy,
      (4) halogen, and
      (5) -CO-NR¹⁸R¹⁹, wherein R¹⁸ and R¹⁹ are each independently hydrogen or C₁₋₄ alkyl,
   (b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
      (1) hydroxy,
      (2) C₁₋₄ alkyl,
      (3) C₁₋₄ alkoxy, and
      (4) halogen, or
      any one of the ring-constituting atoms of the heterocycloalkyl may be optionally substituted with 1 or 2 oxo, or
   (c) a group of the formula: 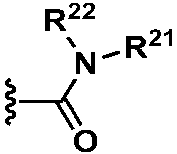 wherein R²¹ and R²² are each independently
      (1) hydrogen,
      (2) C₁₋₄ alkyl,
      (3) C₁₋₄ haloalkyl, or
      (4) 4- to 6-membered heterocycloalkyl comprising 1 or 2 heteroatoms independently selected from the group consisting of nitrogen and oxygen atoms.

In a still more preferable embodiment of a compound of Formula [III],
R⁹ and R¹⁰ are each independently C₁₋₄ alkyl, wherein the alkyl may be optionally substituted with C₁₋₄ alkoxy;
R¹³ is C₁₋₆ haloalkyl or -O-C₁₋₄ haloalkyl;
R², R³, and R⁴ are each independently
   (1) hydrogen,
   (2) hydroxy,
   (3) C₁₋₆ alkyl, wherein the alkyl may be optionally substituted with 1 or 2 substituents independently selected from the group consisting of:
      (a) hydroxy,
      (b) C₁₋₄ alkoxy, and
      (c) -SO₂-C₁₋₄ alkyl, or
R², R³, and R⁴ are combined together with the carbon atom to which they attach, and the -CR²R³R⁴ group forms C₃₋₆ cycloalkyl, wherein the cycloalkyl may be optionally substituted with 1 to 3 substituents independently selected from the group consisting of:
   (1) cyano,
   (2) C₁₋₄ alkyl, wherein the alkyl may be optionally substituted with hydroxy or C₁₋₄ alkoxy,
   (3) C₁₋₄ alkoxy,
   (4) halogen, and
   (5) -CO-NR¹⁸R¹⁹, wherein R¹⁸ and R¹⁹ are each independently hydrogen or C₁₋₄ alkyl.

In a still more preferable embodiment of a compound of Formula [III],
R⁹ and R¹⁰ are each independently C₁₋₄ alkyl, wherein the alkyl may be optionally substituted with C₁₋₄ alkoxy;
R¹³ is C₁₋₆ haloalkyl;
R², R³, and R⁴ are each independently
   (1) hydrogen,
   (2) hydroxy, or
   (3) C₁₋₆ alkyl, wherein the alkyl may be optionally substituted with 1 or 2 substituents independently selected from the group consisting of:
      (a) hydroxy,
      (b) C₁₋₄ alkoxy, and
      (c) -SO₂-C₁₋₄ alkyl, or
R², R³, and R⁴ are combined together with the carbon atom to which they attach, and the -CR²R³R⁴ group forms C₃₋₆ cycloalkyl, wherein the cycloalkyl may be optionally substituted with 1 to 3 substituents independently selected from the group consisting of:
   (1) cyano,
   (2) C₁₋₄ alkyl, wherein the alkyl may be optionally substituted with hydroxy or C₁₋₄ alkoxy,
   (3) C₁₋₄ alkoxy,
   (4) halogen, and
   (5) -CO-NR¹⁸R¹⁹, wherein R¹⁸ and R¹⁹ are each independently hydrogen or C₁₋₄ alkyl.

In a still more preferable embodiment of a compound of Formula [III],
R⁹ and R¹⁰ are each independently C₁₋₄ alkyl, wherein the alkyl may be optionally substituted with C₁₋₄ alkoxy;
R¹³ is -O-C₁₋₄ haloalkyl;
R², R³, and R⁴ are each independently
   (1) hydrogen,
   (2) hydroxy, or
   (3) C₁₋₆ alkyl, wherein the alkyl may be optionally substituted with 1 or 2 substituents independently selected from the group consisting of:
      (a) hydroxy,
      (b) C₁₋₄ alkoxy, and
      (c) -SO₂-C₁₋₄ alkyl, or
R², R³, and R⁴ are combined together with the carbon atom to which they attach, and the -CR²R³R⁴ group forms C₃₋₆ cycloalkyl, wherein the cycloalkyl may be optionally substituted with 1 to 3 substituents independently selected from the group consisting of:
   (1) cyano,
   (2) C₁₋₄ alkyl, wherein the alkyl may be optionally substituted with hydroxy or C₁₋₄ alkoxy,
   (3) C₁₋₄ alkoxy,
   (4) halogen, and
   (5) -CO-NR¹⁸R¹⁹, wherein R¹⁸ and R¹⁹ are each independently hydrogen or C₁₋₄ alkyl.

Another preferable embodiment of a compound of Formula [I] is a compound of Formula [IV], [V], [VI], [VII], [VIII], [IX], [X], or [XI]: 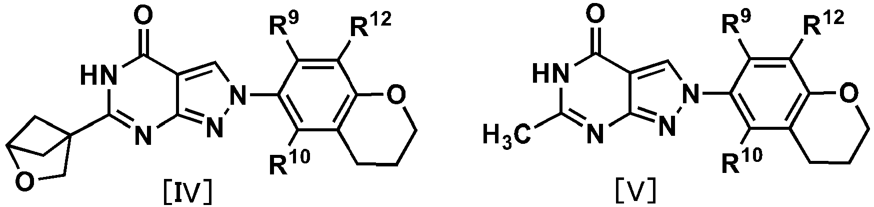 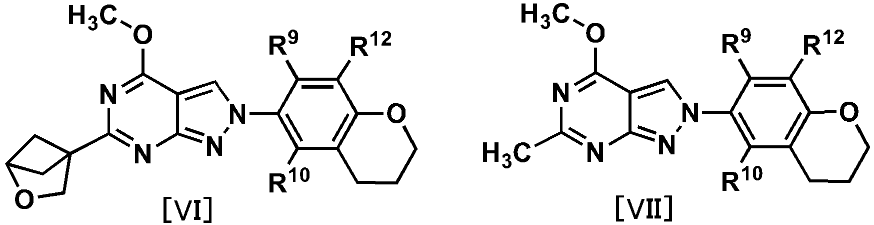 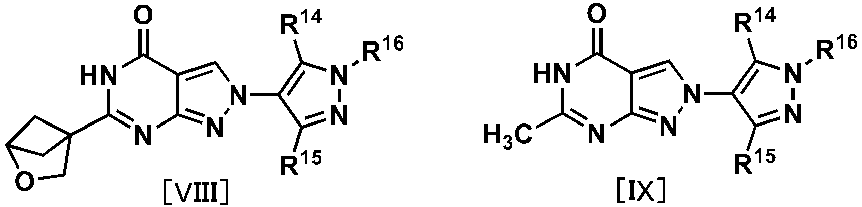 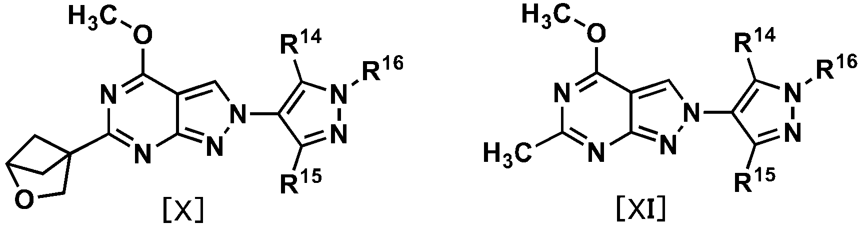 wherein R⁹, R¹⁰, R¹², R¹⁴, R¹⁵, and R¹⁶ are the same as defined above.

The term "pharmaceutically acceptable salt" used herein may be any salts known in the art that are not associated with excessive toxicity. Such a pharmaceutically acceptable salt includes, specifically, salts with inorganic acids, salts with organic acids, salts with inorganic bases, and salts with organic bases. Various forms of pharmaceutically acceptable salts are well known in the art, and are described in, for example, the following references:
(a) Berge et al., J. Pharm. Sci., 66, p1-19 (1977),
(b) Stahl et al., "Handbook of Pharmaceutical Salt: Properties, Selection, and Use" (Wiley-VCH, Weinheim, Germany, 2002),
(c) Paulekuhn et al., J. Med. Chem., 50, p6665-6672 (2007).

A compound of Formula [I] may be reacted with an inorganic acid, organic acid, inorganic base, or organic base according to methods known per se to give a corresponding pharmaceutically acceptable salt thereof.

Such a salt with inorganic acid includes salts with hydrofluoric acid, hydrochloric acid, hydrobromic acid, hydroiodic acid, nitric acid, phosphoric acid, and sulfuric acid. Such a salt preferably includes salts with hydrochloric acid, nitric acid, sulfuric acid, phosphoric acid, and hydrobromic acid.

Such a salt with organic acid includes salts with acetic acid, adipic acid, alginic acid, 4-aminosalicylic acid, anhydromethylenecitric acid, benzoic acid, benzenesulfonic acid, calcium edetate, camphor acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glucoheptonic acid, glycollylarsanilic acid, hexylresorcinol acid, hydroxynaphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid, naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid, and glutamic acid. Such a salt preferably includes salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid, and 2-hydroxy-1-ethanesulfonic acid.

Such a salt with inorganic base includes salts with lithium, sodium, potassium, magnesium, calcium, barium, aluminum, zinc, bismuth, and ammonium. Such a salt preferably includes salts with sodium, potassium, calcium, magnesium, and zinc.

Such a salt with organic base includes salts with arecoline, betaine, choline, clemizole, ethylenediamine, N-methylglucamine, N-benzylphenethylamine, tris(hydroxymethyl)methylamine, arginine, and lysine. Such a salt preferably includes salts with tris(hydroxymethyl)methylamine, N-methylglucamine, and lysine.

Compound [I] may exist in its solvate form. The term "solvate" means a compound where a solvent molecule is coordinated with, for example, Compound [I]. The solvate may be any pharmaceutically acceptable solvates; and includes, for example, a hydrate, an acetic acid solvate, an acetone solvate, an ethanolate, and a dimethyl sulfoxide solvate of Compound [I]. Such a solvate specifically includes a hemihydrate, monohydrate, dihydrate, acetic acid monosolvate, acetone monosolvate, and monoethanolate of a compound of Formula [I]; a monohydrate and acetone monosolvate of a sodium salt of a compound of Formula [I]; and a 2/3 ethanolate of a dihydrochloride salt of a compound of Formula [I]. These solvates may be obtained according to any of known methods.

Compound [I] may exist as a tautomer. In that case, Compound [I] may exist as an individual tautomer or a mixture of tautomers. For example, a structure represented by the following formula: 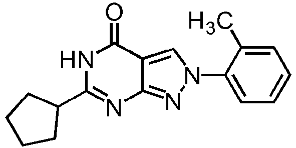 means, unless otherwise specified, that a compound represented by the structure may exist and/or be represented as:
(1) 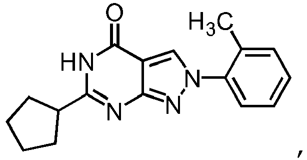
(2) 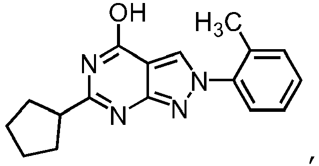
(3) 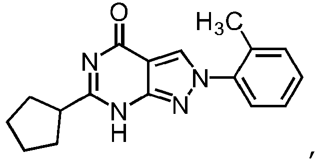
(4) 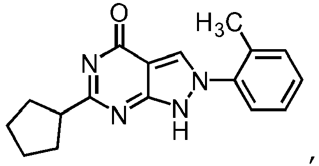 or
(5) a mixture of these structures.

Compound [I] may have a carbon-carbon double bond. In that case, Compound [I] may exist as an E-isomer, a Z-isomer, or a mixture of E- and Z-isomers.

Compound [I] may exist as a stereoisomer which should be recognized as a cis/trans isomer. In that case, Compound [I] may exist as a cis-isomer, a trans-isomer, or a mixture of cis- and trans-isomers.

Compound [I] may have one or more asymmetric carbon atoms. In that case, Compound [I] may exist as a single enantiomer, a single diastereomer, a mixture of enantiomers, or a mixture of diastereomers.

Compound [I] may exist as an atropisomer. In that case, Compound [I] may exist as an individual atropisomer or a mixture of atropisomers.

Compound [I] may simultaneously have multiple structural features that can provide the above isomers. Compound [I] may also contain the above isomers in any ratios.

Formulae, chemical structures, or chemical names without specifying a stereochemistry herein include all the above isomers which may exist, unless otherwise specified.

Diastereomer mixtures may be isolated into each diastereomer by a conventional method such as chromatography and crystallization. Each diastereomer may be also prepared by using a starting material which is a single isomer in terms of stereochemistry or by a synthetic method using a stereoselective reaction.

A mixture of enantiomers may be isolated into each single enantiomer by a well-known method in the art. For example, a mixture of enantiomers may be reacted with a substantially pure enantiomer which is known as a chiral auxiliary to form a mixture of diastereomers, which may be then isolated into a diastereomer with an enhanced isomeric ratio or a substantially pure single diastereomer by a common method such as fractionated crystallization or chromatography. The isolated diastereomer may be converted into a desirable enantiomer by removing the added chiral auxiliary under a cleavage reaction. A mixture of enantiomers may also be directly separated by chromatography well known in the art using a chiral stationary phase. Alternatively, either of enantiomers may also be obtained by using a substantially pure and optically active starting material or a stereoselective synthesis (i.e., asymmetric induction) from a prochiral intermediate with a chiral auxiliary or asymmetric catalyst.

An absolute configuration may be determined by X-ray crystallographic analysis of a crystalline product or intermediate. In that case, the crystalline product or intermediate may be, if needed, induced by an agent having an asymmetric center with a known configuration.

Compound [I] may be labeled with an isotope atom such as ²H(D), ³H, ¹¹C, ¹³C, ¹⁴C, ¹³N, ¹⁵N, ¹⁵O, ¹⁸O, ¹⁸F, ³⁵S, and ¹²³I. For example, in the case where a compound of Formula [I] has a methyl group, the methyl group may be replaced with a -CD₃ group. The compound thus obtained is also encompassed in the present invention. Compound [I] that is labeled with an isotope atom may be useful for medicines, pharmacokinetic studies, *in vitro* and/or *in vivo* assays, and/or diagnostics such as positron emission tomography (PET) and single photon emission computed tomography (SPECT). Compound [I] that is labeled with an isotope atom may be prepared using an isotope-labeled compound, instead of a corresponding non-labeled compound, according to known methods or the methods described herein.

Compound [I] is preferably a substantially purified Compound [I]. A more preferable one is Compound [I] purified in an 80% or more purity.

A pharmaceutical composition in the present invention may be prepared by optionally mixing Compound [I] with at least one or more pharmaceutically acceptable carrier(s) in a certain amount according to known methods in the art of pharmaceutical formulations. The content of Compound [I] in the pharmaceutical composition varies depending on dosage forms and doses, and is for example 0.1 to 100% by weight of the composition.

A dosage form of Compound [I] includes oral preparations such as tablets, capsules, granules, powders, lozenges, syrups, emulsions, and suspensions; and parenteral preparations such as external preparations, suppositories, injections, eye drops, nasal preparations, and pulmonary preparations.

The term "pharmaceutically acceptable carrier" used herein includes various organic or inorganic carrier substances which are conventionally used for a component of a formulation. Such substances include, for example, excipients, disintegrants, binders, fluidizers, and lubricants for solid preparations; solvents, solubilization agents, suspending agents, tonicity agents, buffering agents, and soothing agents for liquid preparations; and bases, emulsifying agents, wetting agents, stabilizers, stabilizing agents, dispersing agents, plasticizing agents, pH adjusters, absorption promoters, gelators, antiseptic agents, bulking agents, solubilizers, solubilization agents, and suspending agents for semisolid preparations. Additives such as preserving agents, antioxidant agents, coloring agents, and sweetening agents may be further used, if needed.

Such excipients include, for example, lactose, white soft sugar, D-mannitol, D-sorbitol, corn starch, dextrin, microcrystalline cellulose, crystalline cellulose, carmellose, carmellose calcium, sodium carboxymethylstarch, low-substituted hydroxypropylcellulose, and gum arabic.

Such disintegrants include, for example, carmellose, carmellose calcium, carmellose sodium, sodium carboxymethylstarch, croscarmellose sodium, crospovidone, low-substituted hydroxypropylcellulose, hydroxypropylmethyl cellulose, and crystalline cellulose.

Such binders include, for example, hydroxypropylcellulose, hydroxypropylmethyl cellulose, povidone, crystalline cellulose, white soft sugar, dextrin, starch, gelatin, carmellose sodium, and gum arabic.

Such fluidizers include, for example, light anhydrous silicic acid and magnesium stearate.

Such lubricants include, for example, magnesium stearate, calcium stearate, and talc.

Such solvents include, for example, purified water, ethanol, propylene glycol, macrogol, sesame oil, corn oil, and olive oil.

Such solubilization agents include, for example, propylene glycol, D-mannitol, benzyl benzoate, ethanol, triethanolamine, sodium carbonate, and sodium citrate.

Such suspending agents include, for example, benzalkonium chloride, carmellose, hydroxypropylcellulose, propylene glycol, povidone, methylcellulose, and glyceryl monostearate.

Such tonicity agents include, for example, glucose, D-sorbitol, sodium chloride, and D-mannitol.

Such buffering agents include, for example, sodium hydrogen phosphate, sodium acetate, sodium carbonate, and sodium citrate.

Such soothing agents include, for example, benzyl alcohol.

Such bases include, for example, water, oils from animals or vegetables such as olive oil, corn oil, arachis oil, sesame oil, and castor oil, lower alcohols such as ethanol, propanol, propylene glycol, 1,3-butylene glycol, and phenol, higher fatty acids and esters thereof, waxes, higher alcohol, polyhydric alcohol, hydrocarbons such as white petrolatum, liquid paraffin, and paraffin, hydrophilic petrolatum, purified lanolin, absorption ointment, hydrous lanolin, hydrophilic ointment, starch, pullulan, gum arabic, tragacanth gum, gelatin, dextran, cellulose derivatives such as methylcellulose, carboxymethyl cellulose, hydroxyethyl cellulose, and hydroxypropyl cellulose, synthetic polymers such as carboxyvinyl polymer, sodium polyacrylate, polyvinylalcohol, and polyvinylpyrrolidone, propylene glycol, macrogol such as Macrogol 200 to 600, and a combination of two or more of them.

Such preserving agents include, for example, ethyl parahydroxybenzoate, chlorobutanol, benzyl alcohol, sodium dehydroacetate, and sorbic acid.

Such anti-oxidant agents include, for example, sodium sulfite and ascorbic acid.

Such coloring agents include, for example, food colors (e.g., Food Red No. 2 or No. 3, Food Yellow No. 4 or No. 5) and β-carotene.

Such sweetening agents include, for example, saccharin sodium, dipotassium glycyrrhizinate, and aspartame.

A pharmaceutical composition in the present invention may be administered to human as well as mammals other than human, such as mice, rats, hamsters, guinea pigs, rabbits, cats, dogs, pigs, cattle, horses, sheep, and monkeys, orally or parenterally such as locally, rectally, intravenously, intramuscularly, and subcutaneously. While a dose, which may be referred to as "a therapeutically effective amount" herein, may vary depending on subjects, diseases, symptoms, dosage forms, routes of administration, and the like, the dose of a compound of Formula [I], or a pharmaceutically acceptable salt thereof, as the active ingredient ranges generally from about 0.01 mg to 1 g per day, for example when administered orally to an adult patient. The dose may be administered once to several times in a divided amount.

Compound [I] has an inhibitory activity of NLRP3 inflammasome and is useful for treating and/or preventing various diseases or conditions that may be expected to be improved by adjusting the NLRP3 inflammasome activity. The various diseases or conditions that may be expected to be improved by adjusting the NLRP3 inflammasome activity include, for example, a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

The expression "inhibiting NLRP3 inflammasome" means inhibiting the function of NLRP3 inflammasome so as to disappear or reduce its activity; and, for example, inhibiting the function of NLRP3 inflammasome on the basis of the condition of Test example 1 as described below. Inhibiting the function of the NLRP3 inflammasome suppresses the production amounts of IL-1β and/or IL-18, and preferably, the production amounts of IL-1β and IL-18. Preferably, "inhibiting NLRP3 inflammasome" means "inhibiting human NLRP3 inflammasome."

Compound [I] has NLRP3 inflammasome inhibitory activity, and Compound [I], or a pharmaceutically acceptable salt thereof, may be used alone for an NLRP3 inflammasome inhibitor or after formulation, if necessary.

The term "treating" used herein includes improving symptoms, preventing aggravation, maintaining remission, preventing exacerbation, and preventing relapse.

The term "preventing" used herein includes suppressing and delaying the onset of symptoms.

As long as an embodiment disclosed herein is compatible with other embodiments disclosed in other portions of the description, any two or more combinations of these embodiments are also intended to be included in the present invention.

### GENERAL METHODS OF PREPARATION

General methods for preparing a compound of Formula [I], or a pharmaceutically acceptable salt thereof, are illustrated as below. A method for preparing a compound of Formula [I], or a pharmaceutically acceptable salt thereof, is however not limited thereto.

Each compound obtained in each step may be isolated and/or purified, if necessary, according to any of known methods such as distillation, recrystallization, and column chromatography, or optionally, a subsequent step can proceed without isolation and/or purification.

Herein, the term "room temperature" refers to a temperature that has not been controlled and includes 1°C to 40°C as one embodiment.

Abbreviations used herein are as follows.
HATU: O-(7-azabenzotriazol-1-yl)-N,N,N',N'-tetramethyluronium hexafluorophosphate
WSC: 1-ethyl-3-(3-dimethylaminopropyl)carbodiimide hydrochloride

### Preparation method A1: Preparation method of Compound [I-A] or a salt thereof

Compound [I-A] or a salt thereof may be prepared by, for example, the following Prepration method A1. 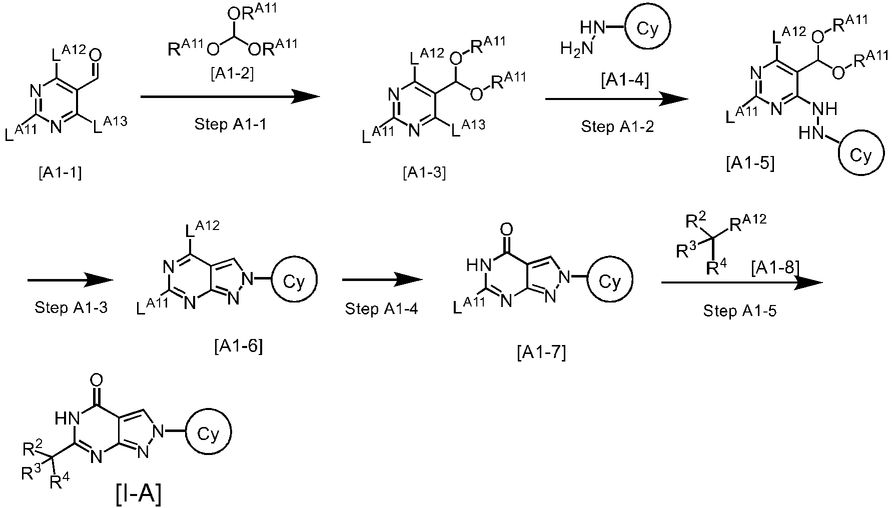 In the scheme, R², R³, R⁴, and the cyclic group Cy are the same as defined above,
R^{A11} is each independently C₁₋₄ alkyl,
R^{A12} is boronic acid, boronic acid ester (for example, boronic acid pinacol ester), trifluoroborate, or tributyltin, and
L^{A11}, L^{A12}, and L^{A13} are each independently a leaving group (for example, halogen, methanesulfonyloxy, and p-toluenesulfonyloxy).

### (Step A1-1)

Compound [A1-3] or a salt thereof may be prepared by reacting Compound [A1-1] or a salt thereof with Compound [A1-2] in a solvent in the presence of an acid.

The acid herein includes, for example, sulfuric acid, hydrochloric acid, formic acid, perchloric acid, methanesulfonic acid, and p-toluenesulfonic acid. The acid is preferably sulfuric acid or p-toluenesulfonic acid.

The solvent herein includes, for example, toluene, methanol, ethanol, isopropanol, tetrahydrofuran, 1,4-dioxane, and a mixed solvent thereof. The solvent is preferably toluene.

The reaction temperature herein ranges, for example, from 0°C to 150°C, preferably from 5°C to 40°C.

Compound [A1-1] or a salt thereof is commercially available, or may be prepared from any commercially available products according to known methods.

Compound [A1-2] is commercially available, or may be prepared from any commercially available products according to known methods.

### (Step A1-2)

Compound [A1-5] or a salt thereof may be prepared by reacting Compound [A1-3] or a salt thereof with Compound [A1-4] or a salt thereof in a solvent in the presence of a base.

The base herein includes, for example, triethylamine, 1,8-diazabicyclo[5,4,0]-7-undecene, and N,N-diisopropylethylamine. The base is preferably triethylamine or N,N-diisopropylethylamine.

The solvent herein includes, for example, toluene, methanol, ethanol, tetrahydrofuran, and a mixed solvent thereof. The solvent is preferably toluene or methanol.

The reaction temperature herein ranges, for example, from -78°C to 100°C, preferably from 0°C to 40°C.

Compound [A1-4] or a salt thereof is commercially available, or may be prepared from any commercially available products according to known methods.

### (Step A1-3)

Compound [A1-6] or a salt thereof may be prepared by reacting Compound [A1-5] or a salt thereof in a solvent in the presence of an acid.

The acid herein includes, for example, trifluoroacetic acid, sulfuric acid, hydrochloric acid, and triethylsilyl trifluoromethanesulfonate. The acid is preferably trifluoroacetic acid.

The solvent herein includes, for example, toluene, tetrahydrofuran, ethyl acetate, cyclopentyl methyl ether, dichloromethane, and a mixed solvent thereof. The solvent is preferably toluene.

The reaction temperature herein ranges, for example, from -78°C to 60°C, preferably from 0°C to 40°C.

### (Step A1-4)

Compound [A1-7] or a salt thereof may be prepared by reacting Compound [A1-6] or a salt thereof in a solvent, in the presence of a base.

The base herein includes, for example, sodium hydroxide and potassium hydroxide. The base is preferably sodium hydroxide.

The solvent herein includes, for example, tetrahydrofuran, 1,2-dimethoxyethane, 1,4-dioxane, chloroform, and a mixed solvent thereof. The solvent is preferably tetrahydrofuran.

The reaction temperature herein ranges, for example, from 0°C to 150°C, preferably from 50°C to 100°C.

### (Step A1-5)

Compound [I-A] or a salt thereof may be prepared by reacting Compound [A1-7] or a salt thereof with Compound [A1-8] or a salt thereof in a solvent in the presence of a catalyst and a base.

The catalyst herein includes, for example, [1,1'-bis(di-phenylphosphino)ferrocene]palladium (II) dichloride dichloromethane adduct, tetrakis(triphenylphosphine)palladium (0), [1,1'-bis(di-tert-butylphosphino)ferrocene]palladium (II) dichloride, bis(triphenylphosphine)palladium (II) dichloride, and bis(di-tert-butyl(4-dimethylaminophenyl)phosphine)dichloropalladium (II). The catalyst is preferably [1,1'-bis(di-phenylphosphino)ferrocene]palladium (II) dichloride dichloromethane adduct.

The base herein includes, for example, tripotassium phosphate, cesium carbonate, potassium carbonate, and lithium chloride. The base is preferably tripotassium phosphate.

When R^{A12} is, for example, boronic acid, boronic acid ester (including boronic acid pinacol ester), or trifluoroborate, the solvent includes, for example, water, toluene, 1,2-dimethoxyethane, 1,4-dioxane, N,N-dimethylacetamide, and a mixed solvent thereof. The solvent is preferably a mixed solvent of toluene and water.

When R^{A12} is, for example, tributyltin, the solvent includes, for example, toluene, N,N-dimethylacetamide, N,N-dimethylformamide, and dimethylsulfoxide. The solvent is preferably N,N-dimethylacetamide.

The reaction temperature herein ranges, for example, from 10°C to 200°C, preferably from 50°C to 150°C.

Compound [A1-8] or a salt thereof is commercially available, or may be prepared from any commercially available products according to known methods.

Any compounds, or salts thereof, having any functional groups or any protected functional groups that can be converted into Compound [A1-8] or a salt thereof by any known reactions may be used in the preparation method herein, instead of Compound [A1-8] or a salt thereof, to give a compound, or a salt thereof, corresponding to Compound [I-A], followed by conversion of the functional groups, resulting in preparation of Compound [I-A] or a salt thereof.

Any compounds, or salts thereof, having any functinal groups or any protected substituents that can be converted into any various substituents on a benzene ring by any known reactions may be used in the preparation method herein, instead of Compound [A1-4] or a salt thereof, to give a compound, or a salt thereof, corresponding to Compound [I-A], followed by conversion of the functional groups or protected substituents into the various substituents, resulting in preparation of Compound [I-A] or a salt thereof. For example, a hydrazine compound, or a salt thereof, substituted with a phenyl group having L^{A41} as described below may be used in the preparation method herein, instead of Compound [A1-4] or a salt thereof, to give a compound corresponding to Compound [I-A], i.e., Compound [I-B], or a salt thereof, followed by conversion of L^{A41} into Ring Cy^{A41} according to Preparation method A4, resulting in Compound [I-C] or a salt thereof.

### Preparation method A2: Preparation method of Compound [I-A] or a salt thereof

Compound [I-A] or a salt thereof may also be prepared by, for example, Preparation method A2 as follows. 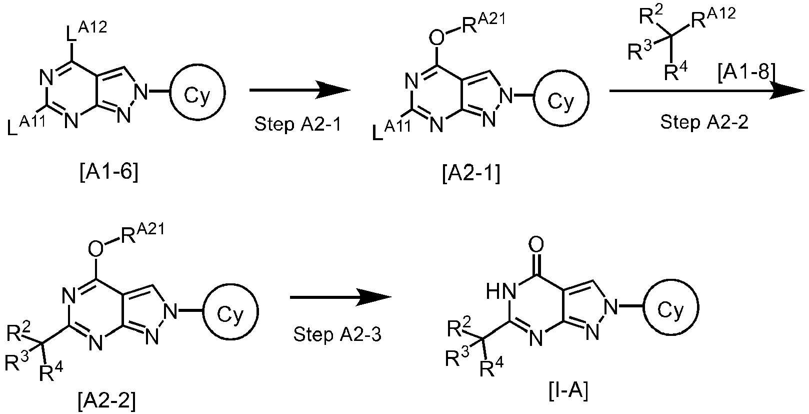 In the scheme, R², R³, R⁴, L^{A11}, L^{A12}, R^{A12}, and the cyclic group Cy are the same as defined above, and
R^{A21} is a protecting group of hydroxy (for example, benzyl, 4-methoxybenzyl, and 2-methoxybenzyl) and R^{A21} is preferably benzyl.

### (Step A2-1)

Compound [A2-1] or a salt thereof may be prepared by reacting Compound [A1-6] or a salt thereof in a solvent, in the presence of an alcohol and a base.

The alcohol herein includes, for example, benzyl alcohol, 4-methoxybenzyl alcohol, and 2-methoxybenzyl alcohol. The alcohol is preferably benzyl alcohol.

The base herein includes, for example, sodium hydride, potassium tert-butoxide, and sodium tert-butoxide. The base is preferably sodium hydride.

The solvent herein includes, for example, tetrahydrofuran, N,N-dimethylformamide, and a mixed solvent thereof. The solvent is preferably tetrahydrofuran.

The reaction temperature herein ranges, for example, from -20°C to 100°C, preferably from 0°C to 50°C.

### (Step A2-2)

Compound [A2-2] or a salt thereof may be prepared by reacting Compound [A2-1] or a salt thereof with Compound [A1-8] or a salt thereof in a solvent in the presence of a catalyst and a base.

The catalyst herein includes, for example, [1,1'-bis(di-phenylphosphino)ferrocene]palladium (II) dichloride dichloromethane adduct, tetrakis(triphenylphosphine)palladium (0), [1,1'-bis(di-tert-butylphosphino)ferrocene]palladium (II) dichloride, bis(triphenylphosphine)palladium (II) dichloride, and bis(di-tert-butyl(4-dimethylaminophenyl)phosphine)dichloropalladium (II). The catalyst is preferably [1,1'-bis(di-phenylphosphino)ferrocene]palladium (II) dichloride dichloromethane adduct.

The base herein includes, for example, tripotassium phosphate, cesium carbonate, potassium carbonate, and lithium chloride. The base is preferably tripotassium phosphate.

When R^{A12} is, for example, boronic acid, boronic acid ester (including boronic acid pinacol ester), or trifluoroborate, the solvent includes, for example, water, toluene, 1,2-dimethoxyethane, 1,4-dioxane, N,N-dimethylacetamide, and a mixed solvent thereof. The solvent is preferably a mixed solvent of toluene and water.

When R^{A12} is, for example, tributyltin, the solvent includes, for example, toluene, N,N-dimethylacetamide, N,N-dimethylformamide, and dimethylsulfoxide. The solvent is preferably N,N-dimethylacetamide.

The reaction temperature herein ranges, for example, from 10°C to 200°C, preferably from 50°C to 150°C.

### (Step A2-3)

Compound [I-A] or a salt thereof may be prepared by removing R^{A21} from Compound [A2-2] or a salt thereof under a deprotection reaction. The deprotection reaction may be carried out under a suitable condition depending on R^{A21}.

For example, when R^{A21} is benzyl, Compound [I-A] or a salt thereof may be prepared by reacting Compound [A2-2] or a salt thereof in the presence of an acid. A solvent may be optionally added in the reaction.

The acid herein includes, for example, formic acid, trifluoroacetic acid, and hydrochloric acid. The acid is preferably formic acid.

The reaction temperature herein ranges, for example, from 0°C to 120°C, preferably from 10°C to 100°C.

The solvent herein includes, for example, toluene, tetrahydrofuran, and 1,4-dioxane.

Any compounds, or salts thereof, having any functinal groups or any protected substituents, that can be converted into Compound [A1-8] or a salt thereof by any known reactions may be used in the preparation method herein, instead of Compound [A1-8] or a salt thereof, to give a compound, or a salt thereof, corresponding to Compound [I-A], followed by conversion of the functional groups, resulting in preparation of Compound [I-A] or a salt thereof.

### Preparation method A3: Preparation method of Compound [I-A] or a salt thereof

Compound [I-A] or a salt thereof may also be prepared by, for example, Preparation method A3 as follows. 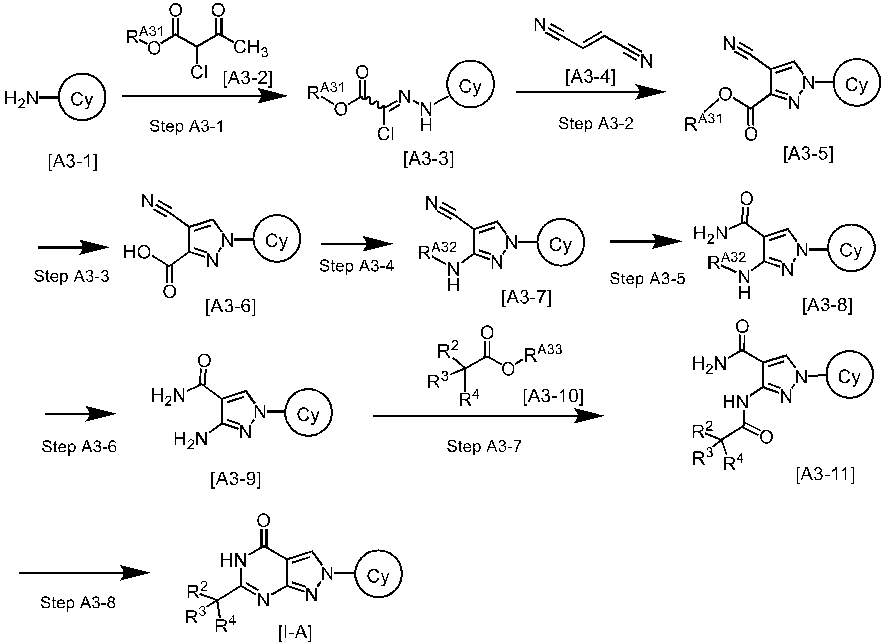 In the scheme, R², R³, R⁴, and a cyclic group Cy are the same as defined above,
R^{A31} is C₁₋₄ alkyl,
R^{A32} is hydrogen or a protecting group of amino (for example, tert-butoxycarbonyl), and
R^{A33} is hydrogen or C₁₋₄ alkyl.

### (Step A3-1)

Compound [A3-3] or a salt thereof may be prepared by reacting Compound [A3-1] or a salt thereof with Compound [A3-2] or a salt thereof in a solvent in the presence of an oxidizing agent, acid, and additive.

The oxidizing agent herein includes, for example, sodium nitrite, butyl nitrite, and isoamyl nitrite. The oxidizing agent is preferably sodium nitrite.

The acid herein includes, for example, concentrated hydrochloric acid, concentrated sulfuric acid, and nitric acid. The acid is preferably concentrated hydrochloric acid.

The additive herein includes, for example, sodium acetate and potassium acetate. The additive is preferably sodium acetate.

The solvent herein includes, for example, ethanol, methanol, butanol, water, and a mixed solvent thereof. The solvent is preferably a mixed solvent of ethanol and water.

The reaction temperature herein ranges, for example, - from 40°C to 50°C, preferably from -10°C to 40°C.

Compound [A3-1] or a salt thereof is commercially available, or may be prepared from any commercially available products according to known methods.

Compound [A3-2] or a salt thereof is commercially available, or may be prepared from any commercially available products according to known methods.

### (Step A3-2)

Compound [A3-5] or a salt thereof may be prepared by reacting Compound [A3-3] or a salt thereof with Compound [A3-4] in a solvent in the presence of a base.

The base herein includes, for example, triethylamine, N,N-diisopropylethylamine and 1,8-diazabicyclo[5,4,0]-7-undecene. The base is preferably triethylamine.

The solvent herein includes, for example, chloroform, 1,2-dichloroethane, dichloromethane, and a mixed solvent thereof. The solvent is preferably chloroform.

The reaction temperature herein ranges, for example, from 20°C to 120°C, preferably from 50°C to 100°C.

Compound [A3-4] is a marketed product.

### (Step A3-3)

Compound [A3-6] or a salt thereof may be prepared by Compound [A3-5] or a salt thereof in a solvent in the presence of a base.

The base herein includes, for example, sodium hydroxide, potassium hydroxide, lithium hydroxide, and calcium hydroxide. The base is preferably sodium hydroxide.

The solvent herein includes, for example, ethanol, methanol, butanol, tetrahydrofuran, water, and a mixed solvent thereof. The solvent is preferably a mixed solvent of ethanol and water.

The reaction temperature herein ranges, for example, from 0°C to 100°C, preferably from 5°C to 50°C.

### (Step A3-4)

Compound [A3-7] or a salt thereof may be prepared by reacting Compound [A3-6] or a salt thereof in a solvent in the presence of a reactant and a base.

The reactant herein includes, for example, sodium azide and diphenylphosphoryl azide. The reactant is preferably diphenylphosphoryl azide.

The base herein includes, for example, triethylamine, N,N-diisopropylethylamine, and 1,8-diazabicyclo[5,4,0]-7-undecene. The base is preferably triethylamine.

The solvent herein includes, for example, tert-butanol, benzyl alcohol, tetrahydrofuran, and a mixed solvent thereof. The solvent is preferably tert-butanol.

The reaction temperature herein ranges, for example, from 0°C to 150°C, preferably from 50°C to 120°C.

### (Step A3-5)

Compound [A3-8] or a salt thereof may be prepared by reacting Compound [A3-7] or a salt thereof in a solvent in the presence of an oxidizing agent and a base.

The oxidizing agent herein includes, for example, hydrogen peroxide solution, triiron tetraoxide, and manganese dioxide. The oxidizing agent is preferably hydrogen peroxide solution.

The base herein includes, for example, sodium hydroxide, potassium hydroxide, and barium hydroxide. The base is preferably sodium hydroxide.

The solvent herein includes, for example, ethanol, dimethylsulfoxide, water, and a mixed solvent thereof. The solvent is preferably ethanol, a mixed solvent of dimethylsulfoxide and water.

The reaction temperature herein ranges, for example, from -20°C to 50°C, preferably from 10°C to 40°C.

### (Step A3-6)

Compound [A3-9] or a salt thereof may be prepared by removing R^{A32} from Compound [A3-8] or a salt thereof under a deprotection reaction. The deprotection reaction may be carried out under a suitable condition depending on R^{A32}

For example, when R^{A32} is tert-butoxycarbonyl, Compound [A3-9] or a salt thereof may be prepared by reacting Compound [A3-8] or a salt thereof in a solvent in the presence of an acid.

The acid herein includes, for example, hydrogen chloride, trifluoroacetic acid, and sulfuric acid. The acid is preferably hydrogen chloride.

The solvent herein includes, for example, ethyl acetate, cyclopentyl methyl ether, and a mixed solvent thereof. The solvent is preferably ethyl acetate.

The reaction temperature herein ranges, for example, from 0°C to 80°C, preferably from 10°C to 50°C.

Compound [A3-9] or a salt thereof may also be prepared by reversing the order of Steps A3-5 and A3-6.

### (Step A3-7)

Compound [A3-11] or a salt thereof may be prepared by reacting Compound [A3-9] or a salt thereof with Compound [A3-10] or a salt thereof in a solvent in the presence of a condensing agent and a base.

The condensing agent herein includes, for example, HATU, WSC, and propylphosphonic acid anhydride. The condensing agent is preferably HATU.

The base herein includes, for example, sodium methoxide, triethylamine, N,N-diisopropylethylamine, potassium tert-butoxide, and 1,8-diazabicyclo[5,4,0]-7-undecene. The base is preferably N,N-diisopropylethylamine.

The solvent herein includes, for example, methanol, N-methylpyrrolidone, N,N-dimethylformamide, N,N-dimethylacetamide, and a mixed solvent thereof. The solvent is preferably N-methylpyrrolidone.

The reaction temperature herein ranges, for example, from 0°C to 120°C, preferably from 50°C to 100°C.

Compound [A3-10] or a salt thereof is commercially available, or may be prepared from any commercially available products according to known methods.

### (Step A3-8)

Compound [I-A] or a salt thereof may be prepared by reacting Compound [A3-11] or a salt thereof in a solvent in the presence of a base.

The base herein includes, for example, sodium methoxide, sodium-tert-butoxide, and 1,8-diazabicyclo[5,4,0]-7-undecene. The base is preferably 1,8-diazabicyclo[5,4,0]-7-undecene.

The solvent herein includes, for example, ethanol, methanol, butanol, water, and a mixed solvent thereof. The solvent is preferably a mixed solvent of ethanol and water.

The reaction temperature herein ranges, for example, from 0°C to 180°C, preferably from 50°C to 150°C.

Any compounds, or salts thereof, having any functinal groups or any protected functional groups, that can be converted into Compound [A3-10] or a salt thereof by any known reactions may be used in the preparation method herein, instead of Compound [A3-10] or a salt thereof, to give a compound, or a salt thereof, corresponding to Compound [I-A], followed by conversion of the functional groups, resulting in preparation of Compound [I-A] or a salt thereof.

Any compounds, or salts thereof, having any functinal groups or any protected substituents that can be converted into any various substituents on a benzene ring by any known reactions may be used in the preparation method herein, instead of Compound [A3-1] or a salt thereof, to give a compound, or a salt thereof, corresponding to Compound [I-A], followed by conversion of the functional groups or protected substituents into the various substituents, resulting in preparation of Compound [I-A] or a salt thereof. For example, a compound, or a salt thereof, having an amino group and L^{A41} as described below on the benzene ring may be used in the preparation method herein, instead of Compound [A3-1] or a salt thereof, to give a compound corresponding to Compound [I-A], i.e., Compound [I-B], or a salt thereof, followed by conversion of L^{A41} into Ring Cy^{A41} according to Preparation method A4, resulting in Compound [I-C] or a salt thereof.

Any compounds, or salts thereof, having any functinal groups or any protected substituents that can be converted into any various substituents on a benzene ring by any known reactions may be used in the preparation method herein, instead of Compound [A3-1] or a salt thereof, to give a compound, or a salt thereof, corresponding to Compound [A3-8], followed by conversion of the functional groups or protected substituents into the various substituents, resulting in preparation of Compound [A3-8] or a salt thereof. For example, a compound, or a salt thereof, having an amino group and L^{A41} as described below on the benzene ring may be used in the preparation method herein, instead of Compound [A3-1] or a salt thereof, to give a compound corresponding to Compound [A3-8], i.e., Compound [A3-8-A], or a salt thereof, followed by conversion of L^{A41} into Ring Cy^{A41} according to Preparation method A5, resulting in Compound [A3-8-B] or a salt thereof.

### Preparation method A4: Preparation method of Compound [I-C] or a salt thereof

Compound [I-C] or a salt thereof may be prepared by, for example, Preparation method A4 as follows.

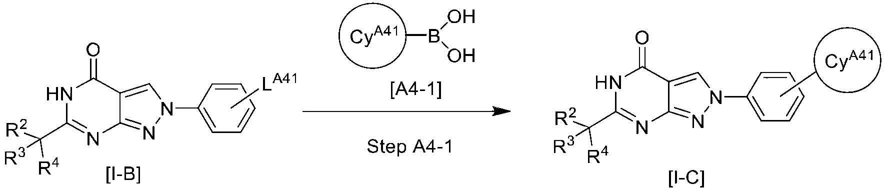

In the scheme, R², R³, and R⁴ are the same as defined above,
Cy^{A41} is C₃₋₆ cycloalkyl, wherein the cycloalkyl may be optionally substituted with 1 or 2 halogen, and
L^{A41} is a leaving group (for example, halogen, methanesulfonyloxy, and trifluoromethanesulfonyloxy), provided that the leaving group is attached to ortho- or para-position of the benzene ring.

### (Step A4-1)

Compound [I-C] or a salt thereof may be prepared by reacting Compound [I-B] or a salt thereof with Compound [A4-1] or a derivative thereof (for example, cyclopropylboronic acid pinacol ester and potassium cyclopropyltrifluoroborate) in a solvent in the presence of a catalyst and a base.

The catalyst herein includes, for example, [1,1'-bis(di-phenylphosphino)ferrocene]palladium (II) dichloride dichloromethane adduct, tetrakis(triphenylphosphine)palladium (0), and [1,1'-bis(di-tert-butylphosphino)ferrocene]palladium (II) dichloride. The catalyst is preferably [1,1'-bis(di-tert-butylphosphino)ferrocene]palladium (II) dichloride.

The base herein includes, for example, tripotassium phosphate, cesium carbonate, and potassium carbonate. The base is preferably tripotassium phosphate.

The solvent herein includes, for example, water, toluene, 1,2-dimethoxyethane, 1,4-dioxane, and a mixed solvent thereof. The solvent is preferably a mixed solvent of toluene and water.

The reaction temperature herein ranges, for example, from 10°C to 200°C, preferably from 50°C to 150°C.

Compound [I-B] or a salt thereof may be prepared from any commercially available products according to known methods. Compound [I-B] or a salt thereof may be prepared by, for example, the above-mentioned preparation methods.

Compound [A4-1] or a derivative thereof is commercially available, or may be prepared from any commercially available products according to known methods.

### Preparation method A5: Preparation method of Compound [A3-8-B] or a salt thereof

Compound [A3-8-B] or a salt thereof may be prepared by, for example, Preparation method A5 as follows.

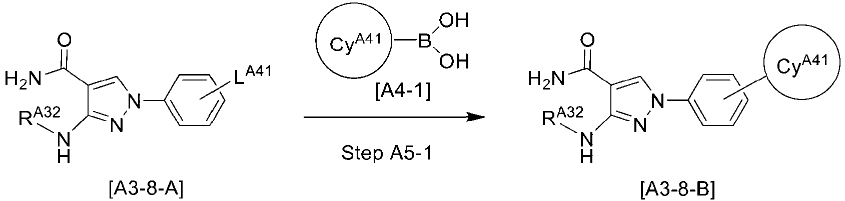

In the scheme, each symbol is the same as defined above.

### (Step A5-1)

Compound [A3-8-B] or a salt thereof may be prepared by reacting Compound [A3-8-A] or a salt thereof with Compound [A4-1] or a derivative thereof (for example, cyclopropylboronic acid pinacol ester and potassium cyclopropyltrifluoroborate) in a solvent in the presence of a catalyst and a base.

The catalyst herein includes, for example, [1,1'-bis(di-phenylphosphino)ferrocene]palladium (II) dichloride dichloromethane adduct, tetrakis(triphenylphosphine)palladium (0), and [1,1'-bis(di-tert-butylphosphino)ferrocene]palladium (II) dichloride. The catalyst is preferably [1,1'-bis(di-tert-butylphosphino)ferrocene]palladium (II) dichloride.

The base herein includes, for example, tripotassium phosphate, cesium carbonate, and potassium carbonate. The base is preferably tripotassium phosphate.

The solvent herein includes, for example, water, toluene, 1,2-dimethoxyethane, 1,4-dioxane, and a mixed solvent thereof. The solvent is preferably a mixed solvent of toluene and water.

The reaction temperature herein ranges, for example, from 10°C to 200°C, preferably from 50°C to 150°C.

Compound [A3-8-A] or a salt thereof may be prepared from any commercially available products according to known methods. Compound [A3-8-A] or a salt thereof may be prepared by, for example, the above-mentioned preparation methods.

### Preparation method A6: Preparation method of Compound [I-D], or a salt thereof, or Compound [I-E], or a salt thereof

Compound [I-D], or a salt thereof, or Compound [I-E], or a salt thereof, may also be prepared by, for example, Preparation method A6 as follows.

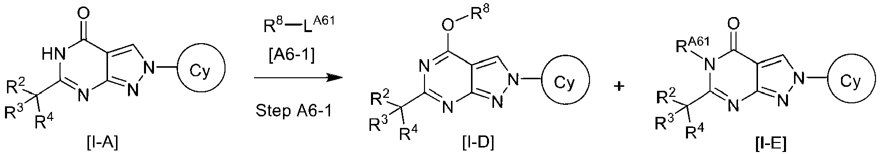

In the scheme, R², R³, R⁴, R⁸, and a cyclic group Cy are the same as defined above,
R^{A61} is C₁₋₄ alkyl, and
L^{A61} is a leaving group (for example, halogen, methanesulfonyloxy, and p-toluenesulfonyloxy).

### (Step A6-1)

Compound [I-D], or a salt thereof, or Compound [I-E], or a salt thereof, may be prepared by reacting Compound [I-A], or a salt thereof, with Compound [A6-1] in a solvent in the presence of a base.

The base herein includes, for example, cesium carbonate, sodium methoxide, sodium hydride, and potassium carbonate. The base is preferably sodium hydride.

The solvent herein includes, for example, methanol, N,N-dimethylformamide and tetrahydrofuran. The solvent is preferably N,N-dimethylformamide.

The reaction temperature herein ranges, for example, from 0°C to 100°C, preferably from 10°C to 50°C.

Compound [A6-1] is commercially available, or may be prepared from any commercially available products according to known methods.

### EXAMPLES

Preparation methods of a compound of Formula [I], or a pharmaceutically acceptable salt thereof, are described specifically in the following Preparation examples. However, preparation methods of a compound of Formula [I], or a pharmaceutically acceptable salt thereof, are not intended to be limited thereto.

% refers to weight %, unless otherwise specified. The ratio recited in a mixed solvent refers to a volume ratio, unless otherwise specified.

NMRs were determined at 400 MHz.

### [Preparation example 1]: Synthesis of 2-(2,4-dimethylphenyl)-5,6-dimethyl-2,5-dihydro-4H-pyrazolo[3,4-d]pyrimidin-4-one (Example 13)

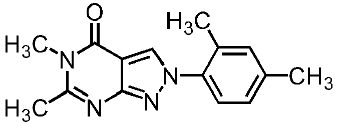

### Step 1-1: 4-chloro-2-(2,4-dimethylphenyl)-6-methyl-2H-pyrazolo[3,4-d]pyrimidine

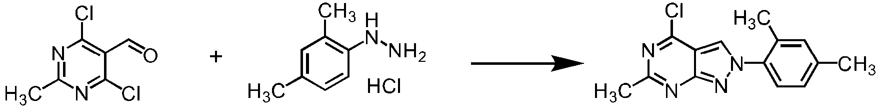

To a mixture of (2,4-dimethylphenyl)hydrazine hydrochloride (2.0 g), triethylamine (3.2 mL), and ethanol (40 mL) was added 4,6-dichloro-2-methylpyrimidine-5-carbaldehyde (2.0 g) under an argon atmosphere at 0°C, and the mixture was stirred at 100°C for 3 hours. Then, solvents were removed under reduced pressure. The residue was purified by column chromatography (eluent solvent(s): 10 vol% to 70 vol% ethyl acetate/hexane) to give the title compound (1.6 g).
¹H-NMR (CDCl₃) δ: 8.19 (1H, s), 7.29 (1H, d, *J* = 8.1 Hz), 7.19 (1H, t, J = 0.6 Hz), 7.15-7.13 (1H, m), 2.81 (3H, s), 2.41 (3H, s), 2.25 (3H, s).

### Step 1-2: 2-(2,4-dimethylphenyl)-6-methyl-2,5-dihydro-4H-pyrazolo[3,4-d]pyrimidin-4-one

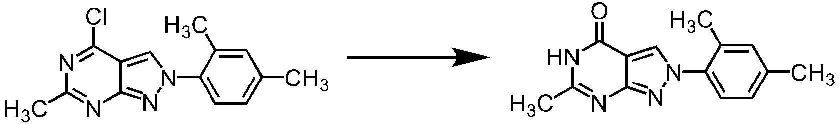

To a mixture of 4-chloro-2-(2,4-dimethylphenyl)-6-methyl-2H-pyrazolo[3,4-d]pyrimidine (1.6 g) and 1,2-dimethoxyethane (13 mL) was added 4M aqueous sodium hydroxide solution (6.3 mL), and the mixture was stirred at 110°C for 6 hours. Then, 2M hydrochloric acid was added to the mixture for neutralization. The residue was purified by column chromatography (eluent solvent(s): 10 vol% to 100 vol% ethyl acetate/hexane) to give the title compound (1.2 g).
¹H-NMR (DMSO-D₆) δ: 11.71 (1H, br s), 8.68 (1H, s), 7.31 (1H, d, *J* = 8.1 Hz), 7.23 (1H, s), 7.17 (1H, d, *J* = 8.1 Hz), 2.34 (3H, s), 2.31 (3H, s), 2.15 (3H, s).
LC-MS (MH+): 255.

### Step 1-3: 2-(2,4-dimethylphenyl)-5,6-dimethyl-2,5-dihydro-4H-pyrazolo[3,4-d]pyrimidin-4-one

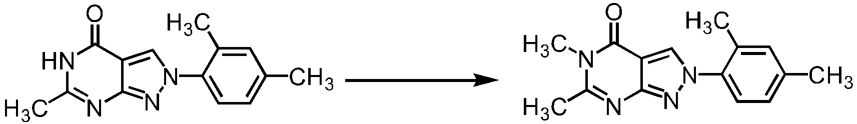

To a mixture of 2-(2,4-dimethylphenyl)-6-methyl-2,5-dihydro-4H-pyrazolo[3,4-d]pyrimidin-4-one (50 mg) and N,N-dimethylformamide (0.5 mL) were added cesium carbonate (130 mg) and iodomethane (0.024 mL), and the mixture was stirred at room temperature for 2 hours. Water was added to the reaction mixture which was then extracted with ethyl acetate. The obtained organic layer was dried over anhydrous sodium sulfate, and then solvents were removed under reduced pressure. The residue was purified by column chromatography to give the title compound (32 mg).
¹H-NMR (DMSO-d₆) δ: 8.74 (1H, s), 7.33 (1H, d, J = 7.9 Hz), 7.25-7.25 (1H, m), 7.19-7.17 (1H, m), 3.48 (3H, s), 2.56 (3H, s), 2.36 (3H, s), 2.16 (3H, s).
LC-MS (MH+) : 269.

### [Preparation example 2]: Synthesis of 6-cyclopropyl-2-(4-cyclopropyl-2,6-dimethylphenyl)-3-(hydroxymethyl)-2,5-dihydro-4H-pyrazolo[3,4-d]pyrimidin-4-one (Example 83) and 6-cyclopropyl-2-(4-cyclopropyl-2,6-dimethylphenyl)-3-(methoxymethyl)-2,5-dihydro-4H-pyrazolo[3,4-d]pyrimidin-4-one (Example 88)

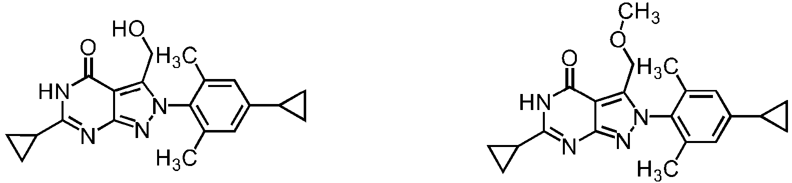

### Step 2-1: 2,4,6-trichloro-5-(dimethoxymethyl)pyrimidine

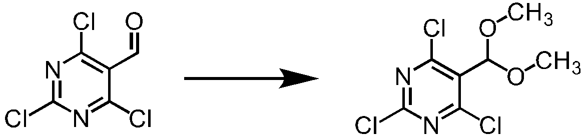

To a mixture of 2,4,6-trichloropyrimidine-5-carbaldehyde (170 g) and toluene (1.0 L) were added trimethyl orthoformate (500 mL) and sulfuric acid (1.1 mL) under a nitrogen atmosphere, and the mixture was stirred at room temperature for 2.5 hours. To the reaction mixture was added basic silica gel (Fuji Silysia Chemical Ltd., 330 g), and the mixture was stirred for 1.5 hours, and then the added silica gel was collected by filtration. The silica gel was washed with ethyl acetate, and then solvent was removed under reduced pressure to give the title compound (175 g).
¹H-NMR (CDCl₃) δ: 5.68 (1H, s), 3.49 (6H, s).

### Step 2-2: 4-(2-(4-bromo-2,6-dimethylphenyl)hydrazinyl)-2,6-dichloro-5-(dimethoxymethyl)pyrimidine

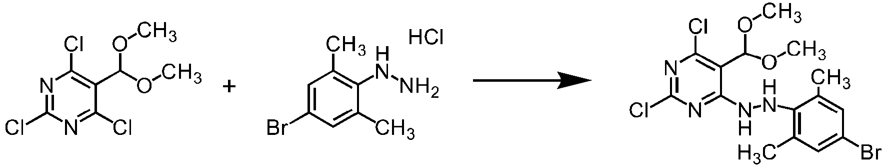

To a mixture of (4-bromo-2,6-dimethylphenyl)hydrazine hydrochloride (80 g) and methanol (560 mL) was added 2,4,6-trichloro-5-(dimethoxymethyl)pyrimidine (82 g) under a nitrogen atmosphere, and then the mixture was cooled under ice-bath cooling. To the reaction mixture was slowly added triethylamine (133 mL), and then the mixture was stirred for 1.5 hours at the same temperature. The resulted solid was collected by filtration, and washed sequentially with methanol (150 mL) and hexane (100 mL) to give the title compound (120 g).
¹H-NMR (CDCl₃) δ: 8.28 (1H, d, *J* = 4.4 Hz), 7.10 (2H, s), 6.14 (1H, d, J = 4.9 Hz), 5.56 (1H, s), 3.45 (6H, s), 2.43 (6H, s).
LC-MS (MH+): 436.

### Step 2-3: 2-(4-bromo-2,6-dimethylphenyl)-4,6-dichloro-2H-pyrazolo[3,4-d]pyrimidine

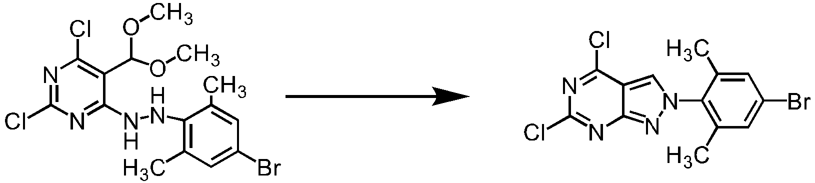

To a mixture of 4-(2-(4-bromo-2,6-dimethylphenyl)hydrazinyl)-2,6-dichloro-5-(dimethoxymethyl)pyrimidine (121 g) and toluene (970 mL) was added dropwise and slowly trifluoroacetic acid (43 mL) over 10 minutes under a nitrogen atmosphere at 0°C. The reaction mixture was added dropwise and slowly to a mixture of ice-cooled tripotassium phosphate (120 g) and water (400 mL) and tetrahydrofuran (640 mL). The organic layer was separated, and then the aqueous layer was extracted with ethyl acetate. All organic layers were combined, washed with saturated brine, and dried over anhydrous sodium sulfate, and then solvent was removed under reduced pressure to give a crude product of the title compound (109 g).
LC-MS (MH+): 372.

### Step 2-4: 2-(4-bromo-2,6-dimethylphenyl)-6-chloro-2,5-dihydro-4H-pyrazolo[3,4-d]pyrimidin-4-one

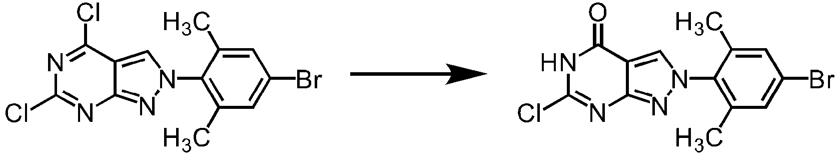

To a mixture of the crude product of 2-(4-bromo-2,6-dimethylphenyl)-4,6-dichloro-2H-pyrazolo[3,4-d]pyrimidine (109 g) and tetrahydrofuran (830 mL) was added 4 M aqueous sodium hydroxide solution (210 mL) at room temperature, and the mixture was stirred at 65°C for 5 hours. The reaction mixture was cooled under ice-bath cooling, and then thereto was added dropwise and slowly 2 M hydrochloric acid (280 mL). The reaction mixture was extracted with ethyl acetate, and then the aqueous layere was extracted again with a mixed solution of ethyl acetate/tetrahydrofuran (v/v = 1/4). All organic layers were combined, washed with saturated brine, and dried over anhydrous sodium sulfate, and then solvent was removed under reduced pressure. To the resulted crude product were added diisopropylether (550 mL) and ethyl acetate (150 mL), and the mixture was stirred for 1 hour, and then solid was collected by filtration to give the title compound (79 g).
¹H-NMR (DMSO-D₆) δ: 12.88 (1H, br s), 8.81 (1H, s), 7.53 (2H, s), 1.95 (6H, s).
LC-MS (MH+): 354.

### Step 2-5: 6-cyclopropyl-2-(4-cyclopropyl-2,6-dimethylphenyl)-2,5-dihydro-4H-pyrazolo[3,4-d]pyrimidin-4-one

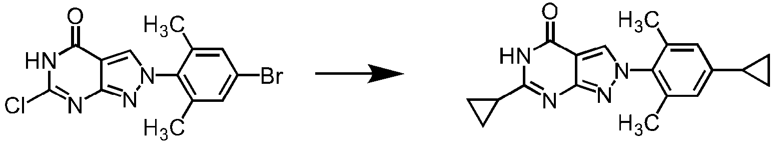

To a mixture of 2-(4-bromo-2,6-dimethylphenyl)-6-chloro-2,5-dihydro-4H-pyrazolo[3,4-d]pyrimidin-4-one (1.0 g), cyclopropylboronic acid (1.2 g), toluene (15 mL), and 2 M aqueous tripotassium phosphate solution (7.1 mL) was added 1,1'-bis(di-tert-butylphosphino) ferrocene]dichloropalladium (II) (0.37 g) under an argon atmosphere, and the mixture was stirred at 100°C for 5 hours. The reaction mixture was let cool to room temperature, and then extracted with ethyl acetate. The obtained organic layer was dried over anhydrous magnesium sulfate, and then solvent was removed under reduced pressure. The residue was purified by column chromatography (eluent solvent(s): 30 vol% to 80 vol% ethyl acetate/hexane) to give the title compound (740 mg).
¹H-NMR (DMSO-D₆) δ: 11.98 (1H, s), 8.57 (1H, s), 6.94 (2H, s), 1.99-1.89 (8H, m), 1.08 (2H, t, J = 3.7 Hz), 1.02-0.95 (4H, m), 0.72 (2H, dt, J = 8.6, 3.2 Hz).
LC-MS(MH+): 321.

### Step 2-6: 6-cyclopropyl-2-(4-cyclopropyl-2,6-dimethylphenyl)-4-oxo-4,5-dihydro-2H-pyrazolo[3,4-d]pyrimidine-3-carbaldehyde

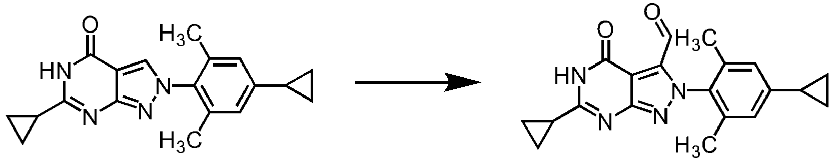

To a mixture of 6-cyclopropyl-2-(4-cyclopropyl-2,6-dimethylphenyl)-2,5-dihydro-4H-pyrazolo[3,4-d]pyrimidin-4-one (100 mg) and tetrahydrofuran (1.0 mL) was added lithium bis(trimethylsilyl)amide (1.1 M tetrahydrofuran solution, 1.1 mL) under an argon atmosphere at 0°C, and the mixture was stirred at the same temperature for 10 minutes. To the reaction mixture was added N,N-dimethylformamide (0.12 mL), and then the mixture was stirred at the same temperature for 1 hour. To the reaction mixture was added saturated aqueous ammonium chloride solution, and then the mixture was extracted with ethyl acetate. The obtained organic layer was dried over anhydrous magnesium sulfate, and then solvent was removed under reduced pressure to give a crude product of the title compound (110 mg).
LC-MS (MH+): 349.

### Step 2-7: 6-cyclopropyl-2-(4-cyclopropyl-2,6-dimethylphenyl)-3-(hydroxymethyl)-2,5-dihydro-4H-pyrazolo[3,4-d]pyrimidin-4-one (Example 83)

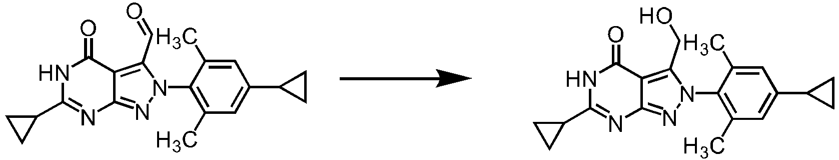

To a mixture of the crude product of 6-cyclopropyl-2-(4-cyclopropyl-2,6-dimethylphenyl)-4-oxo-4,5-dihydro-2H-pyrazolo[3,4-d]pyrimidine-3-carbaldehyde (110 mg), methanol (1.1 mL), and tetrahydrofuran (1.1 mL) was added sodium borohydride (35 mg) under water-bath cooling, and the mixture was stirred at the same temperature for 2 hours. To the reaction mixture was added saturated aqueous ammonium chloride solution, and then the mixture was extracted with ethyl acetate. The obtained organic layer was dried over anhydrous magnesium sulfate, and then solvent was removed under reduced pressure. The residue was purified by column chromatography (eluent solvent(s): 20 vol% to 80 vol% ethyl acetate/hexane) to give the title compound (60 mg).
¹H-NMR (DMSO-D₆) δ: 12.04 (1H, s), 6.92 (2H, s), 4.46 (2H, d, J = 4.9 Hz), 1.98-1.90 (2H, m), 1.84 (6H, s), 1.09-1.05 (2H, m), 1.01-0.97 (4H, m), 0.75-0.72 (2H, m). (1 peak lost (OH))
LC-MS (MH+): 351.

### Step 2-8: 3-(bromomethyl)-6-cyclopropyl-2-(4-cyclopropyl-2,6-dimethylphenyl)-2,5-dihydro-4H-pyrazolo[3,4-d]pyrimidin-4-one

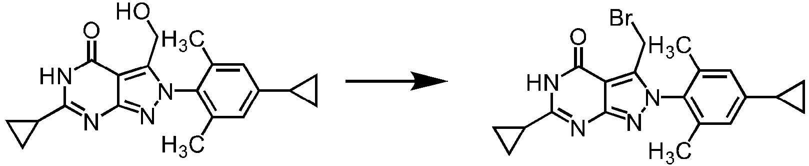

To a mixture of 6-cyclopropyl-2-(4-cyclopropyl-2,6-dimethylphenyl)-3-(hydroxymethyl)-2,5-dihydro-4H-pyrazolo[3,4-d]pyrimidin-4-one (30 mg) and deuterated chloroform (1.5 mL) was added phosphorus tribromide (8.1 µL) under an argon atmosphere at 0°C, and the mixture was stirred at the same temperature for 1.5 hours. To the reaction mixture was added saturated aqueous sodium hydrogen carbonate solution, and then the mixture was extracted with ethyl acetate. The obtained organic layer was dried over anhydrous magnesium sulfate, and then solvent was removed under reduced pressure to give a crude product of the title compound (36 mg).
LC-MS (MH+): 413.

### Step 2-9: 6-cyclopropyl-2-(4-cyclopropyl-2,6-dimethylphenyl)-3-(methoxymethyl)-2,5-dihydro-4H-pyrazolo[3,4-d]pyrimidin-4-one (Example 88)

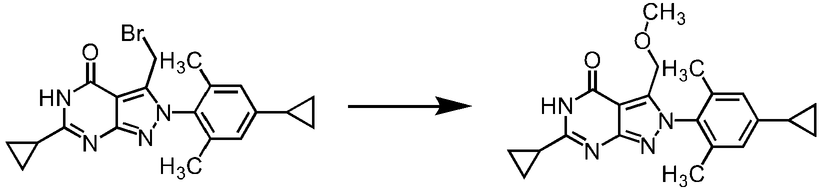

To a mixture of the crude product of 3-(bromomethyl)-6-cyclopropyl-2-(4-cyclopropyl-2,6-dimethylphenyl)-2,5-dihydro-4H-pyrazolo[3,4-d]pyrimidin-4-one (36 mg) and tetrahydrofuran (1.1 mL) was added 5 M solution of sodium methoxide in methanol (0.086 mL) at 0°C, and the mixture was stirred at the same temperature for 2 hours. To the reaction mixture was added saturated aqueous ammonium chloride solution, and then the mixture was extracted with ethyl acetate. The obtained organic layer was dried over anhydrous magnesium sulfate, and then solvent was removed under reduced pressure. The residue was purified by column chromatography (eluent solvent (s): 20 vol% to 60 vol% ethyl acetate/hexane), followed by reverse-phase column chromatography (eluent solvent(s): 10 vol% to 100 vol% acetonitrile/waer) to give the title compound (5.7 mg).
¹H-NMR (DMSO-D₆) δ: 12.04 (1H, s), 6.94 (2H, s), 4.39 (2H, s), 3.15 (3H, s), 1.99-1.90 (2H, m), 1.82 (6H, s), 1.09-1.07 (2H, m), 1.02-0.96 (4H, m), 0.76-0.72 (2H, m).
LC-MS (MH+): 365.

### [Preparation example 3]: Synthesis of 2-[4-(1,1-difluoroethyl)-2,6-dimethylphenyl]-6-[(1R,2S)-2-fluorocyclopropyl]-2,5-dihydro-4H-pyrazolo[3,4-d]pyrimidin-4-one (Example 147), and 2-[4-(1,1-difluoroethyl)-2,6-dimethylphenyl]-6-[(1S,2R)-2-fluorocyclopropyl]-2,5-dihydro-4H-pyrazolo[3,4-d]pyrimidin-4-one (Example 146)

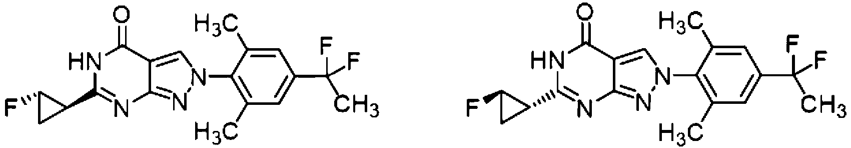

### Step 3-1: methyl 2-{2-(4-bromo-2,6-dimethylphenyl)hydrazinylidene}-2-chloroacetate

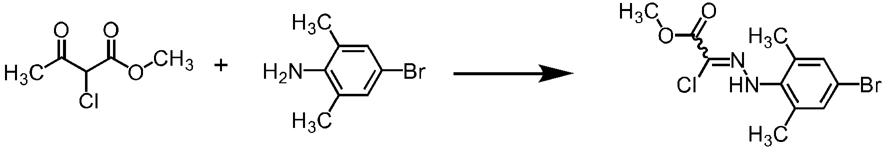

To a mixture of 4-bromo-2,6-dimethylaniline (45 g) and ethanol (72 mL) were added water (54 mL) and concentrated hydrochloric acid (47.3 mL) at room temperature. The reaction mixture was cooled to -10°C or below, and then thereto was added dropwise and slowly an aqueous solution (54 mL) of sodium nitrite (17.1 g) with the reaction temperature maintained at 0°C or below. The mixture was stirred at the same temperature for 30 minutes, and then thereto were added methyl 2-chloro-3-oxobutanoate (27.1 mL) and an aqueous solution (315 mL) of sodium acetate (55.4 g). The mixture was stirred at room temperature for 2 hours. The reaction mixture was extracted with ethyl acetate, and then washed with saturated brine. The obtained organic layer was dried over anhydrous magnesium sulfate, and then solvent was removed under reduced pressure to give a crude product of the title compound.

### Step 3-2: methyl 1-(4-bromo-2,6-dimethylphenyl)-4-cyano-1H-pyrazole-3-carboxylate

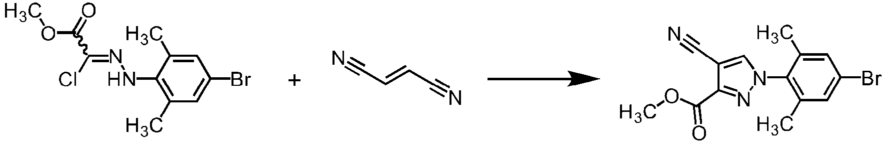

To a mixture of the crude product of methyl 2-{2-(4-bromo-2,6-dimethylphenyl)hydrazinylidene}-2-chloroacetate and chloroform (575 mL) were added fumaronitrile (20.2 g) and triethylamine (36.1 mL), and the mixture was stirred at 80°C for 4 hours. To the reaction mixture was added water, and then the mixture was extracted with chloroform twice. The obtained organic layer was washed with saturated brine, and then thereto were added anhydrous magnesium sulfate and silica gel (200 g). The resultant was stirred at room temperature for 1 hour and filtered through a basic silica gel column (Fuji Silysia Chemical Ltd., 200 g) (eluent solvent (s): ethyl acetate), and then solvent was removed under reduced pressure. The resulted solid was stirred with a mixed solution of ethyl acetate (50 mL) and hexane (50 mL), and then solid was collected through filtration. The resulted solid was washed with a mixed solution of ethyl acetate (50 mL) and hexane (50 mL) to give the title compound (38.9 g).
¹H-NMR (DMSO-D₆) δ: 9.00 (1H, s), 7.57 (2H, s), 3.90 (3H, s), 1.96 (6H, s).

### Step 3-3: 1-(4-bromo-2,6-dimethylphenyl)-4-cyano-1H-pyrazole-3-carboxylic acid

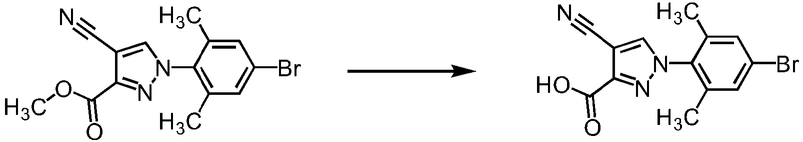

To a mixture of methyl 1-(4-bromo-2,6-dimethylphenyl)-4-cyano-1*H*-pyrazole-3-carboxylate (36 g) and methanol (360 mL) was added 2M aqueous sodium hydroxide solution (108 mL), and the mixture was stirred at room temperature for 2 hours. The reaction mixture was neutralized with 2 M hydrochloric acid, and then stirred at room temperature for 30 minutes. To the reaction mixture was added water (200 mL), and the mixture was stirred for additional 2 hours. The resulted solid was collected through filtration, and then washed sequentially with water and hexane to give the title compound (33.8 g).
¹H-NMR (DMSO-D₆) δ: 13.85 (1H, br s), 8.95 (1H, s), 7.56 (2H, s), 1.97 (6H, s).

### Step 3-4: 3-amino-1-(4-bromo-2,6-dimethylphenyl)-1H-pyrazole-4-carbonitrile

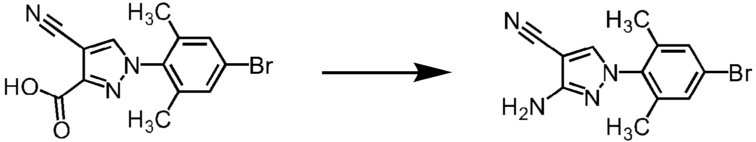

To a mixture of 1-(4-bromo-2,6-dimethylphenyl)-4-cyano-1H-pyrazole-3-carboxylic acid (33.8 g), triethylamine (29.4 mL), and tert-butanol (507 mL) was added diphenylphosphoryl azide (34.0 mL) under a nitrogen atmosphere, and then the mixture was stirred at 90°C for 8 hours. Then, solvent was removed under reduced pressure. To a mixture of the residue and chloroform (200 mL) was added trifluoroacetic acid (24.4 mL), and the mixture was stirred at room temperature overnight. Solvent was removed under reduced pressure, and then the residue was purified by column chromatography (eluent solvent(s): 1 vol% to 40 vol% ethyl acetate/hexane) to give the title compound (30.7 g).
¹H-NMR (DMSO-D₆) δ: 8.31 (1H, s), 7.46 (2H, s), 5.76 (2H, s), 2.01 (6H, s).
LC-MS (MH+): 291.

### Step 3-5: 3-amino-1-(4-bromo-2,6-dimethylphenyl)-1H-pyrazole-4-carboxamide

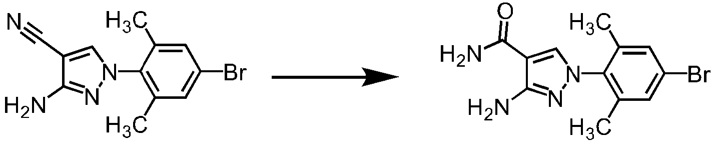

To a mixture of 3-amino-1-(4-bromo-2,6-dimethylphenyl)-1*H*-pyrazole-4-carbonitrile (30.7 g), ethanol (184 mL), and dimethylsulfoxide (46 mL) were added sodium hydroxide (12.7 g) and 30% hydrogen peroxide solution (43.1 mL) under ice-bath cooling. The reaction mixture was stirred at room temperature for 1 hour, and then thereto was added 10% aqueous sodium sulfite solution. The mixture was acidified with 6 M hydrochloric acid, and then extracted with ethyl acetate. The obtained organic layer was washed with saturated brine and dried over anhydrous magnesium sulfate, and then solvent was removed under reduced pressure. The residue was purified by column chromatography (eluent solvent(s): 30 vol% to 100 vol% ethyl acetate/hexane) to give the title compound (8.11 g).
¹H-NMR (DMSO-D₆) δ: 7.97 (1H, s), 7.45 (2H, s), 7.30 (1H, br s), 6.91 (1H, br s), 5.53 (2H, br s), 2.02 (6H, s).

### Step 3-6: 2-(4-bromo-2,6-dimethylphenyl)-6-{(1RS,2SR)-2-fluorocyclopropyl}-2,5-dihydro-4H-pyrazolo[3,4-d]pyrimidin-4-one

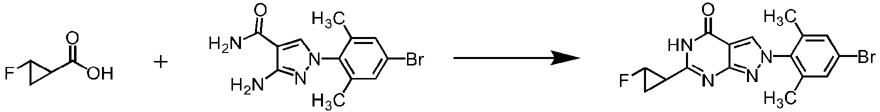

To a mixture of *trans*-2-fluorocyclopropane-1-carboxylic acid (252 mg) and *N,N*-dimethylformamide (3.0 mL) were added 1-[bis(dimethylamino)methylene]-1H-1,2,3-triazolo[4,5-b]pyridinium-3-oxide hexafluorophosphate (922 mg) and *N,N-*diisopropylethylamine (627 mg) under an argon atmosphere, and the mixture was stirred at room temperature for 5 minutes. To the reaction mixture was added 3-amino-1-(4-bromo-2,6-dimethylphenyl)-1H-pyrazole-4-carboxamide (300 mg), and the mixture was stirred at 50°C for 3 hours. Then, the mixture was let cool to room temperature. To the reaction mixture was added ethyl acetate, and the mixture was washed with water twice. Then, the mixture was dried over anhydrous magnesium sulfate, and solvent was removed under reduced pressure. To the residue were added ethanol (4.8 mL), water (4.8 mL), and 1,8-diazabicyclo[5.4.0]undec-7-ene (1.17 mL), and then the mixture was stirred at 90°C for 24 hours. Then, to the mixture was added 1,8-diazabicyclo[5.4.0]undec-7-ene (0.5 mL). The mixture was stirred at 100°C for 14 hours. The reaction mixture was let cool, and then thereto was added ethyl acetate. The mixture was washed sequentially with water and saturated brine. The obtained organic layer was dried over anhydrous magnesium sulfate, and solvent was removed under reduced pressure. The residue was purified by column chromatography (eluent solvent(s): 33 vol% to 50 vol% ethyl acetate/hexane) to give the title compound (237 mg). ¹H-NMR (CDCl₃) δ: 9.91 (1H, br s), 8.07 (1H, s), 7.34 (2H, s), 5.19-4.98 (1H, m), 2.31-2.16 (1H, m), 2.02 (6H, s), 1.83-1.64 (2H, m).

### Step 3-7: 2-[4-(1-ethoxyvinyl)-2,6-dimethylphenyl]-6-{(1RS,2SR)-2-fluorocyclopropyl}-2,5-dihydro-4H-pyrazolo[3,4-d]pyrimidin-4-one

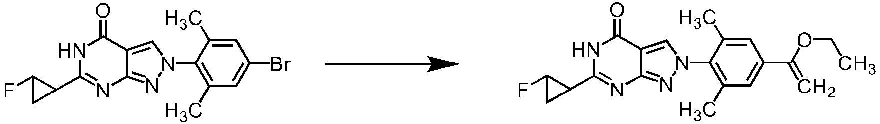

A mixture of 2-(4-bromo-2,6-dimethylphenyl)-6-{(1*RS*,2*SR*)-2-fluorocyclopropyl}-2,5-dihydro-4H-pyrazolo[3,4-d]pyrimidin-4-one (237 mg), tributyl(1-ethoxyvinyl)stannane (340 mg), bis(triphenylphosphine)palladium (II) dichloride (44.1 mg), and toluene (4.7 mL) was stirred at 120°C for 1 hour under an argon atmosphere. The reaction mixture was let cool, and then purified by column chromatography (eluent solvent(s): 20 vol% to 50 vol% ethyl acetate/hexane) to give the title compound (149 mg).
¹H-NMR (CDCl₃) δ: 10.46 (1H, br s), 8.08 (1H, s), 7.43 (2H, s), 5.20-5.00 (1H, m), 4.68 (1H, d, J = 2.8 Hz), 4.27 (1H, d, J = 2.8 Hz), 3.94 (2H, q, J = 7.0 Hz), 2.36-2.24 (1H, m), 2.05 (6H, s), 1.83-1.63 (2H, m), 1.45 (3H, t, J = 7.0 Hz).

### Step 3-8: 2-(4-acetyl-2,6-dimethylphenyl)-6-{(1RS,2SR)-2-fluorocyclopropyl}-2,5-dihydro-4H-pyrazolo[3,4-d]pyrimidin-4-one

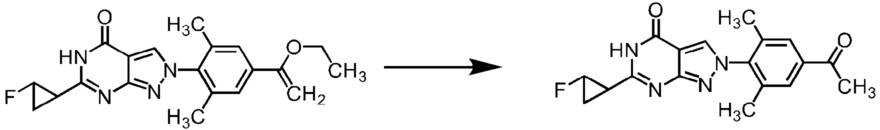

A mixture of 2-[4-(1-ethoxyvinyl)-2,6-dimethylphenyl]-6-{(1RS,2SR)-2-fluorocyclopropyl}-2,5-dihydro-4H-pyrazolo[3,4-d]pyrimidin-4-one (149 mg), 2M hydrochloric acid (1.0 mL), and tetrahydrofuran (2.0 mL) was stirred at 50°C for 1 hour under an argon atmosphere. The reaction mixture was let cool, and thereto was added ethyl acetate. The mixture was washed with saturated brine. The obtained organic layer was dried over anhydrous magnesium sulfate, and then solvent was removed under reduced pressure to give the title compound (138 mg).
¹H-NMR (CDCl₃) δ: 10.22 (1H, br s), 8.11 (1H, s), 7.76 (2H, s), 5.20-4.99 (1H, m), 2.64 (3H, s), 2.34-2.23 (1H, m), 2.12 (6H, s), 1.84-1.64 (2H, m).

### Step 3-9: 2-[4-(1,1-difluoroethyl)-2,6-dimethylphenyl]-6-[(1RS,2SR)-2-fluorocyclopropyl]-2,5-dihydro-4H-pyrazolo[3,4-d]pyrimidin-4-one (Example 142)

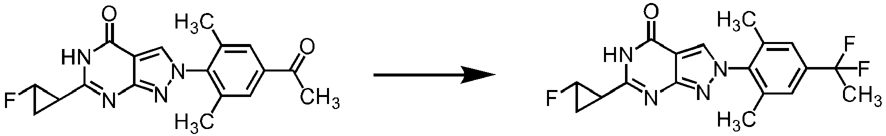

A mixture of 2-(4-acetyl-2,6-dimethylphenyl)-6-{(1RS,2SR)-2-fluorocyclopropyl}-2,5-dihydro-4H-pyrazolo[3,4-d]pyrimidin-4-one (110 mg) and bis(2-methoxyethyl)aminosulfur trifluoride (1.1 mL) was stirred at 70°C for 4 hours under an argon atmosphere. The reaction mixture was added dropwise and slowly to saturated aqueous sodium hydrogen carbonate solution stirred under ice cooling, and then the mixture was extracted with ethyl acetate. The obtained organic layer was washed with saturated brine and dried over anhydrous magnesium sulfate, and then solvent was removed under reduced pressure. The residue was purified by column chromatography (eluent solvent(s): 20 vol% to 50 vol% ethyl acetate/hexane) to give the title compound (89 mg). ¹H-NMR (CDCl₃) δ: 10.14 (br s, 1H), 8.10 (s, 1H), 7.32 (s, 2H), 5.20-5.16 (m, 0.5H), 5.04-5.00 (m, 0.5H), 2.33-2.22 (m, 1H), 2.08 (s, 6H), 1.95 (t, 3H, J = 18.3 Hz), 1.84-1.63 (m, 2H).
LC-MS (MH+): 363.

### Step 3-10: 2-[4-(1,1-difluoroethyl)-2,6-dimethylphenyl]-6-[(1R,2S)-2-fluorocyclopropyl]-2,5-dihydro-4H-pyrazolo[3,4-d]pyrimidin-4-one (Example 147), and 2-[4-(1,1-difluoroethyl)-2,6-dimethylphenyl]-6-[(1S,2R)-2-fluorocyclopropyl]-2,5-dihydro-4H-pyrazolo[3,4-d]pyrimidin-4-one (Example 146)

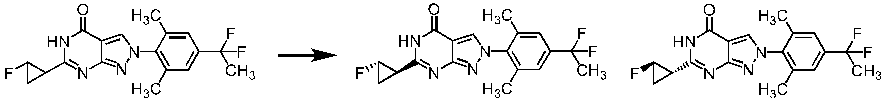

2-[4-(1,1-Difluoroethyl)-2,6-dimethylphenyl]-6-[(1RS,2SR)-2-fluorocyclopropyl]-2,5-dihydro-4H-pyrazolo[3,4-d]pyrimidin-4-one (89 mg) was optically resolved by column chromatography with a chiral column {apparatus: Japan Analytical Industry; automatic recycling preparative HPLC LaboACE LC-7080; column: Daicel CHIRALPAK IH, 20 mm (I.D.) x 250 mm (L), 5 µm; guard column: Daicel CHIRALPAK IH, 10 mm (I.D.) x 20 mm (L), 5 µm; column temperature: 30°C; flow rate of mobile phase: 20 mL/min; mixing ratio of mobile phase: isocratic, hexane/ethanol = 80/20} to give 2-[4-(1,1-difluoroethyl)-2,6-dimethylphenyl]-6-[(1R,2S)-2-fluorocyclopropyl]-2,5-dihydro-4H-pyrazolo[3,4-d]pyrimidin-4-one (Example 147 compound, 38 mg) for the first peak fraction (24.2 to 32.0 min), and 2-[4-(1,1-difluoroethyl)-2,6-dimethylphenyl]-6-[(1S,2R)-2-fluorocyclopropyl]-2,5-dihydro-4H-pyrazolo[3,4-d]pyrimidin-4-one (Example 146 compound, 38 mg) for the second peak fraction (34.3 to 44.0 min). The absolute configuration of Example 147 compound was determined by X-ray crystallography.
[2-[4-(1,1-difluoroethyl)-2,6-dimethylphenyl]-6-[(1R,2S)-2-fluorocyclopropyl]-2,5-dihydro-4H-pyrazolo[3,4-d]pyrimidin-4-one:Example 147]
¹H-NMR (CDCl₃) δ: 10.47 (br s, 1H), 8.10 (s, 1H), 7.32 (s, 2H), 5.21-5.00 (m, 1H), 2.37-2.24 (m, 1H), 2.09 (s, 6H), 1.95 (t, 3H, J = 18.1 Hz), 1.83-1.65 (m, 2H).
LC-MS (MH+): 363.
[2-[4-(1,1-difluoroethyl)-2,6-dimethylphenyl]-6-[(1S,2R)-2-fluorocyclopropyl]-2,5-dihydro-4H-pyrazolo[3,4-d]pyrimidin-4-one : Example 146]
¹H-NMR (CDCl₃) δ: 10.21 (br s, 1H), 8.10 (s, 1H), 7.32 (s, 2H), 5.20-5.00 (m, 1H), 2.34-2.23 (m, 1H), 2.08 (s, 6H), 1.95 (t, 3H, J = 18.1 Hz), 1.83-1.65 (m, 2H).
LC-MS (MH+): 363.

### [Preparation example 4] : Synthesis of (R)-2-{4-(1,1-difluoroethyl)-2,6-dimethylphenyl}-6-(1-hydroxyethyl)-2,5-dihydro-4H-pyrazolo[3,4-d]pyrimidin-4-one (Example 154), and (S)-2-{4-(1,1-difluoroethyl)-2,6-dimethylphenyl}-6-(1-hydroxyethyl)-2,5-dihydro-4H-pyrazolo[3,4-d]pyrimidin-4-one (Example 155)

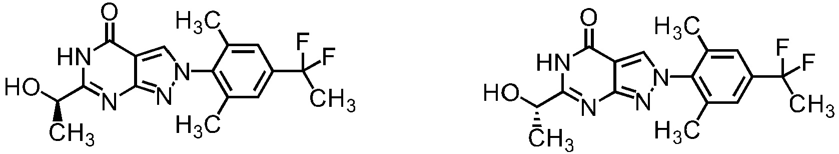

### Step 4-1: 1-(4-bromo-3,5-dimethylphenyl)ethan-1-ol

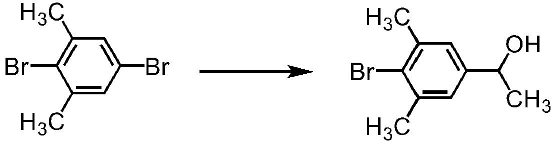

To a mixture of under an argon atmosphere 2,5-dibromo-1,3-dimethylbenzene (6.0 g) and tetrahydrofuran (120 mL) was added dropwise 1.56 M solution of n-butyllithium in hexane (13.8 mL) at -78°C over 5 minutes. The mixture was stirred at the same temperature for 15 minutes, and thereto was added acetaldehyde (3.0 g). Then, the mixture was stirred for 15 minutes under ice-bath cooling. To the reaction mixture was added saturated aqueous ammonium chloride solution, and then the mixture was extracted with a mixed solution of ethyl acetate/hexane. The obtained organic layer was washed with saturated brine and dried over anhydrous magnesium sulfate, and then solvent was removed under reduced pressure to give a crude product of the title compound (7.4 g).

### Step 4-2: 1-(4-bromo-3,5-dimethylphenyl)ethan-1-one

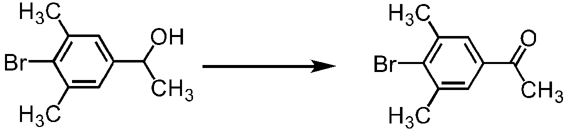

To a mixture of the crude product of 1-(4-bromo-3,5-dimethylphenyl)ethan-1-ol (3.7 g) and dichloromethane (30 mL) was added slowly Dess-Martin reagent (5.8 g) under water-bath cooling, and the mixture was stirred at room temperature for 30 minutes. To the reaction mixture was added isopropanol (1.0 mL), and then the mixture was purified by column chromatography (eluent solvent(s): 10 vol% ethyl acetate/hexane) to give the title compound (2.1 g).
¹H-NMR (CDCl₃) δ: 7.64 (2H, s), 2.57 (3H, s), 2.47 (6H, s).

### Step 4-3: 2-bromo-5-(1,1-difluoroethyl)-1,3-dimethylbenzene

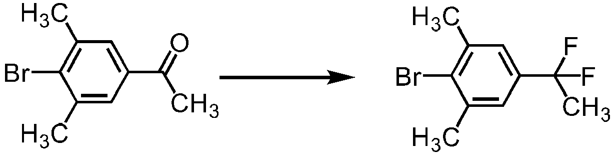

A mixture of 1-(4-bromo-3,5-dimethylphenyl)ethan-1-one (3.2 g), synthesized in a similar manner to Step 4-2, and bis(2-methoxyethyl)aminosulfur trifluoride (12.5 g) was stirred at 85°C for 2 hours under an argon atmosphere. The reaction mixture was added dropwse to saturated aqueous sodium hydrogen carbonate solution stirred under ice cooling, and then the mixture was extracted with a mixed solution of hexane/ethyl acetate. The obtained organic layer was washed with saturated brine and dried over anhydrous magnesium sulfate, and then solvent was removed under reduced pressure. The residue was purified by silica gelchromatography (eluent solvent(s): 0 vol% to 10 vol% ethyl acetate/hexane) to give the title compound (1.6 g).
¹H-NMR (CDCl₃) δ: 7.20 (2H, s), 2.45 (6H, s), 1.89 (3H, t, J = 18.1 Hz).

### Step 4-4: di-tert-butyl 1-{4-(1,1-difluoroethyl)-2,6-dimethylphenyl}hydrazine-1,2-dicarboxylate

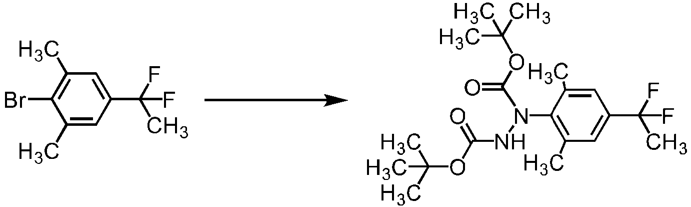

To a mixture of 2-bromo-5-(1,1-difluoroethyl)-1,3-dimethylbenzene (750 mg) and tetrahydrofuran (15 mL) was added dropwise 1.56 M solution of n-butyllithium in hexane (2.3 mL) under an argon atmosphere at -78°C, and the mixture was stirred at the same temperature for 10 minutes. Then, thereto was added di-tert-butyl azodicarboxylate (1.0 g). The mixture was stirred at the same temperature for 15 minutes, and thereto was added acetic acid (0.3 mL). Then, the mixture was diluted with ethyl acetate. The reaction mixture was washed with saturated brine, and then dried over anhydrous magnesium sulfate. Solvent was removed under reduced pressure. The residue was purified by column chromatography (eluent solvent(s): 5 vol% to 20 vol% ethyl acetate/hexane) to give the title compound (854 mg).
¹H-NMR (CDCl₃) δ: 7.19 (2H, d, J = 4.6 Hz), 6.59 (1H, br s), 2.38 (6H, s), 1.95-1.82 (3H, m), 1.49-1.37 (18H, br m).

### Step 4-5: {4-(1,1-difluoroethyl)-2,6-dimethylphenyl}hydrazine hydrochloride

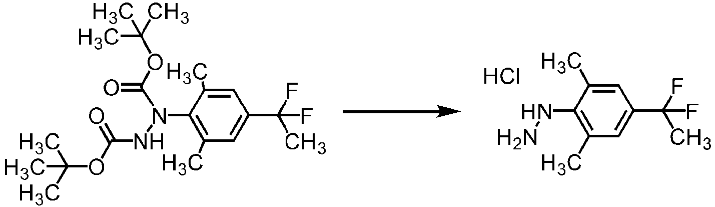

A mixture of di-tert-butyl 1-{4-(1,1-difluoroethyl)-2,6-dimethylphenyl}hydrazine-1,2-dicarboxylate (854 mg) and 4 M solution of hydrogen chloride in ethyl acetate (17.1 mL) was stirred at room temperature for 1.5 hours. Solvent was removed under reduced pressure, and then the resultant was dried at room temperature for 1 hour under reduced pressure to give the title compound (497 mg).
¹H-NMR (DMSO-D₆) δ: 9.60 (3H, s), 7.30 (2H, s), 6.85 (1H, br s), 2.41 (6H, s), 1.93 (3H, t, J = 18.8 Hz).

### Step 4-6: 2,4,6-trichloro-5-(dimethoxymethyl)pyrimidine

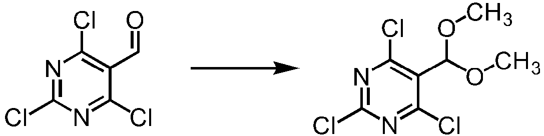

To 2,4,6-trichloropyrimidine-5-carbaldehyde (165 g) in toluene (990 mL) were added trimethyl orthoformate (500 mL) and sulfuric acid (1.0 mL) under a nitrogen atmosphere, and the mixture was stirred at room temperature for 1.5 hours. To the reaction mixture was added basic silica gel (Fuji Silysia Chemical Ltd., 330 g), and the mixture was stirred for 1.5 hours. Then, the added silica gel was collected by filtration. The silica gel was washed with ethyl acetate (1.3 L), and then solvent was removed under reduced pressure to give the title compound (177 g).
¹H-NMR (CDCl₃) δ: 5.68 (1H, s), 3.49 (6H, s).

### Step 4-7: 2,4-dichloro-6-[2-{4-(1,1-difluoroethyl)-2,6-dimethylphenyl}hydrazinyl]-5-(dimethoxymethyl)pyrimidine

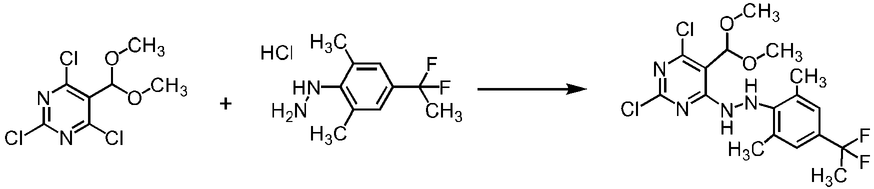

To a mixture of 2,4,6-trichloro-5-(dimethoxymethyl) pyrimidine (541 mg), triethylamine (0.88 mL), and methanol (9.9 mL) was added {4-(1,1-difluoroethyl)-2,6-dimethylphenyl}hydrazine hydrochloride (497 mg) at 0°C, and the mixture was stirred at room temperature for 1 hour. To the reaction mixture was added ethyl acetate, and the mixture was stirred. Then, the resulted solid was collected by filtration, and then solvent was removed under reduced pressure to give a crude product of the title compound (885 mg).
¹H-NMR (CDCl₃) δ: 8.35 (1H, d, J = 4.6 Hz), 7.12 (2H, s), 6.28 (1H, d, J = 4.9 Hz), 5.58 (1H, s), 3.48 (6H, s), 2.50 (6H, s), 1.88 (3H, t, J = 18.1 Hz).

### Step 4-8: 4,6-dichloro-2-{4-(1,1-difluoroethyl)-2,6-dimethylphenyl}-2H-pyrazolo[3,4-d]pyrimidine

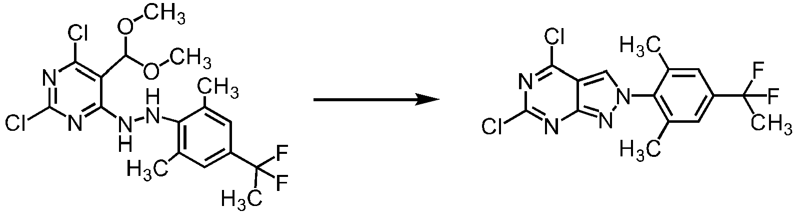

To a mixture of the crude product of 2,4-dichloro-6-[2-{4-(1,1-difluoroethyl)-2,6-dimethylphenyl}hydrazinyl]-5-(dimethoxymethyl)pyrimidine (885 mg) and toluene (8.9 mL) was added trifluoroacetic acid (0.40 mL), and then the mixture was stirred at room temperature for 1 hour. The reaction mixture was ice-cooled, and thereto was added 2M aqueous potassium phosphate solution (4.0 mL). Then, the mixture was extracted with ethyl acetate. The obtained organic layer was washed with saturated brine and dried over anhydrous magnesium sulfate, and then solvent was removed under reduced pressure. The residue was purified by column chromatography (eluent solvent(s): 5 vol% to 33 vol% ethyl acetate/hexane) to give the title compound (441 mg).
¹H-NMR (CDCl₃) δ: 8.21 (1H, s), 7.37 (2H, s), 2.07 (6H, s), 1.95 (3H, t, J = 18.1 Hz).

### Step 4-9: 4-(benzyloxy)-6-chloro-2-{4-(1,1-difluoroethyl)-2,6-dimethylphenyl}-2H-pyrazolo[3,4-d]pyrimidine

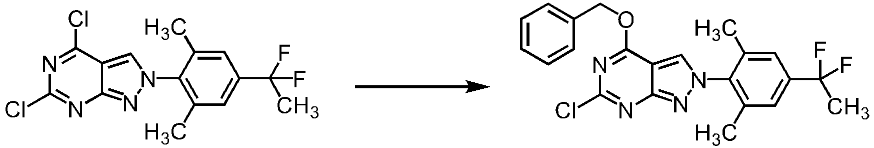

To a mixture of sodium hydride (60% in oil, 52 mg) and tetrahydrofuran (8.8 mL) was added benzyl alcohol (0.15 mL) under an argon atmosphere, and then the mixture was stirred at 50°C for 30 minutes. The reaction mixture was ice-cooled, and then thereto was added 4,6-dichloro-2-{4-(1,1-difluoroethyl)-2,6-dimethylphenyl}-2H-pyrazolo[3,4-d]pyrimidine (440 mg). Then, the mixture was stirred at the same temperature for 30 minutes. To the reaction mixture was added 2 M hydrochloric acid (0.75 mL), adn then the mixture was extracted with ethyl acetate. The obtained organic layer was washed with saturated brine and dried over anhydrous magnesium sulfate, and then solvent was removed under reduced pressure. The resulted solid was stirred with a mixed solution of hexane/ethyl acetate (v/v = 4/1) and then collected by filtration to give the title compound (470 mg) .
¹H-NMR (CDCl₃) δ: 8.04 (1H, s), 7.54 (2H, d, J = 6.5 Hz), 7.46-7.39 (3H, m), 7.32 (2H, s), 5.66 (2H, s), 2.04 (6H, s), 1.93 (3H, t, J = 18.1 Hz).

### Step 4-10: 1-[4-(benzyloxy)-2-{4-(1,1-difluoroethyl)-2,6-dimethylphenyl}-2H-pyrazolo[3,4-d]pyrimidine-6-yl]ethan-1-one

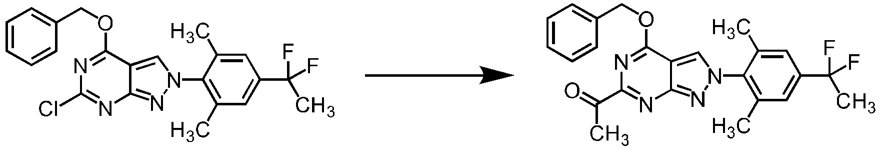

A mixture of 4-(benzyloxy)-6-chloro-2-{4-(1,1-difluoroethyl)-2,6-dimethylphenyl}-2H-pyrazolo[3,4-d]pyrimidine (200 mg), tributyl(1-ethoxyvinyl)tin (253 mg), bis(triphenylphosphine)palladium (II) dichloride (33 mg), and toluene (4.0 mL) was stirred at 100°C for 2 hours under an argon atmosphere. The reaction mixture was let cool to room temperature, and then thereto was added 2 M hydrochloric acid (4.0 mL). Then, the mixture was stirred at 50°C for 1.5 hours. The reaction mixture was let cool to room temperature, and then extracted with ethyl acetate. The obtained organic layer was dried over anhydrous magnesium sulfate, and then solvent was removed under reduced pressure. The residue was purified by column chromatography (eluent solvent(s): 10 vol% to 50 vol% ethyl acetate/hexane) to give a crude product of the title compound (85 mg).
¹H-NMR (DMSO-D₆) δ: 9.06 (1H, d, J = 10.9 Hz), 7.62 (2H, t, J = 4.3 Hz), 7.53 (2H, s), 7.45-7.36 (3H, m), 5.73 (2H, s), 2.74 (3H, s), 2.07-1.98 (9H, m).

### Step 4-11: 6-acetyl-2-{4-(1,1-difluoroethyl)-2,6-dimethylphenyl}-2,5-dihydro-4H-pyrazolo[3,4-d]pyrimidin-4-one

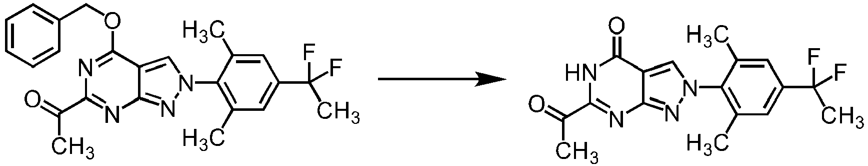

A mixture of the crude product of 1-[4-(benzyloxy)-2-{4-(1,1-difluoroethyl)-2,6-dimethylphenyl}-2H-pyrazolo[3,4-d]pyrimidine-6-yl]ethan-1-one (85 mg) and formic acid (0.85 mL) was stirred at 80°C for 1 hour. The reaction mixture was cooled to room temperature, and then solvent was removed under reduced pressure. To the residue were added ethyl acetate, water, and saturated aqueous sodium hydrogen carbonate solution, and the mixture was separated. Then, the resulted aqueous layer was extracted with ethyl acetate. All organic layers were combined and dried over anhydrous magnesium sulfate, and then solvent was removed under reduced pressure to give the title compound (68 mg).
¹H-NMR (DMSO-D₆) δ: 11.80 (1H, s), 8.91 (1H, s), 7.52 (2H, s), 2.63 (3H, s), 2.05-1.99 (9H, m).

### Step 4-12: (RS)-2-{4-(1,1-difluoroethyl)-2,6-dimethylphenyl}-6-(1-hydroxyethyl)-2,5-dihydro-4H-pyrazolo[3,4-d]pyrimidin-4-one (Example 144)

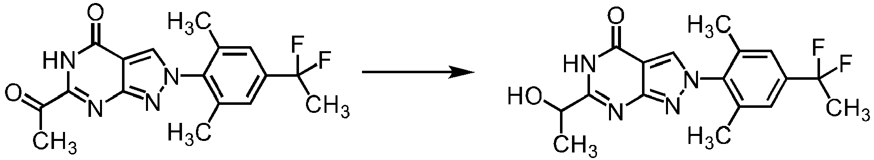

To a mixture of 6-acetyl-2-{4-(1,1-difluoroethyl)-2,6-dimethylphenyl}-2,5-dihydro-4H-pyrazolo[3,4-d]pyrimidin-4-one (142 mg), synthesized in a similar manner to Step 4-11, and methanol (2.8 mL) was added sodium borohydride (47 mg) under ice cooling, and the mixture was stirred at the same temperature for 30 minutes. Then, solvent was removed under reduced pressure. To the residue were added ethyl acetate, water, and saturated aqueous ammonium chloride solution, and the mixture was separated. Then, the resulted aqueous layer was extracted with ethyl acetate. All organic layers were combined and dried over anhydrous magnesium sulfate, and then solvent was removed under reduced pressure. The residue was purified by reverse-phase column chromatography (eluent solvent(s): 10 vol% to 100 vol% acetonitrile/water) to give the title compound (87 mg).
¹H-NMR (DMSO-D₆) δ: 11.32 (1H, s), 8.75 (1H, s), 7.49 (2H, s), 5.65 (1H, s), 4.58 (1H, dd, J = 13.6, 6.2 Hz), 2.01 (9H, dd, J = 21.4, 16.5 Hz), 1.43 (3H, d, J = 6.5 Hz).
LC-MS (MH+): 349.

### Step 4-13: (R)-2-(4-(1,1-difluoroethyl)-2,6-dimethylphenyl)-6-(1-hydroxyethyl)-2,5-dihydro-4H-pyrazolo[3,4-d]pyrimidin-4-one (Example 154), and (S)-2-(4-(1,1-difluoroethyl)-2,6-dimethylphenyl)-6-(1-hydroxyethyl)-2,5-dihydro-4H-pyrazolo[3,4-d]pyrimidin-4-one (Example 155)

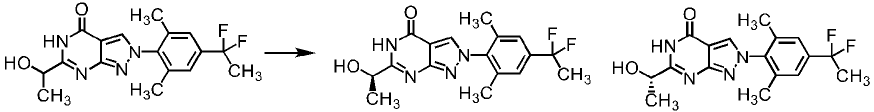

(RS)-2-(4-(1,1-difluoroethyl)-2,6-dimethylphenyl)-6-(1-hydroxyethyl)-2,5-dihydro-4H-pyrazolo[3,4-d]pyrimidin-4-one (85 mg) was optically resolved by supercritical fluid chromatography {apparatus: Waters SFC Prep 15 System; column: Daicel CHIRALPAK IH/SFC, 10 mm (I.D.) x 250 mm (L), 5 µm; column temperature: 40°C; flow rate of mobile phase: 15 mL/min; mixing ratio of mobile phase: isocratic, carbon dioxide/methanol = 92/8} to give (R)-2-(4-(1,1-difluoroethyl)-2,6-dimethylphenyl)-6-(1-hydroxyethyl)-2,5-dihydro-4H-pyrazolo[3,4-d]pyrimidin-4-one (Example 154 compound, 33.4 mg) for the first peak fraction (8.0 to 9.5 min), and (S)-2-(4-(1,1-difluoroethyl)-2,6-dimethylphenyl)-6-(1-hydroxyethyl)-2,5-dihydro-4H-pyrazolo[3,4-d]pyrimidin-4-one (35.6 mg) for the second peak fraction (11.2 to 13.3 min). The absolute configuration of Example 154 compound was determined by X-ray crystallography.
[(R)-2-{4-(1,1-difluoroethyl)-2,6-dimethylphenyl}-6-(1-hydroxyethyl)-2,5-dihydro-4H-pyrazolo[3,4-d]pyrimidin-4-one: Example 154]
¹H-NMR (DMSO-D₆) δ: 11.35 (1H, s), 8.76 (1H, s), 7.49 (2H, s), 5.64 (1H, d, J = 5.1 Hz), 4.61-4.55 (1H, m), 2.04-1.98 (9H, m), 1.44 (3H, d, J = 6.5 Hz). LC-MS (MH+): 349.
[(S)-2-{4-(1,1-difluoroethyl)-2,6-dimethylphenyl}-6-(1-hydroxyethyl)-2,5-dihydro-4H-pyrazolo[3,4-d]pyrimidin-4-one: Example 155]
¹H-NMR (DMSO-D₆) δ: 11.37 (1H, s), 8.74 (1H, s), 7.49 (2H, s), 5.67 (1H, s), 4.58 (1H, q, J = 6.6 Hz), 2.04-1.98 (9H, m), 1.43 (3H, d, J = 6.5 Hz).
LC-MS (MH+): 349.

### [Preparation example 5]: Synthesis of (R)-2-{4-(difluoromethoxy)-2,6-dimethylphenyl}-6-(1-hydroxyethyl)-2,5-dihydro-4H-pyrazolo[3,4-d]pyrimidin-4-one (Example 165), and (S)-2-{4-(difluoromethoxy)-2,6-dimethylphenyl}-6-(1-hydroxyethyl)-2,5-dihydro-4H-pyrazolo[3,4-d]pyrimidin-4-one (Example 166)

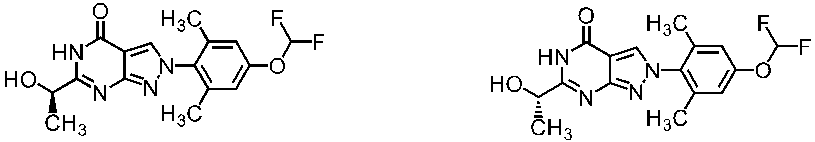

### Step 5-1: 5-(difluoromethoxy)-1,3-dimethyl-2-nitrobenzene

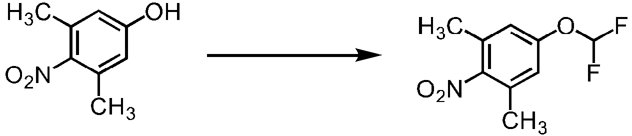

To a mixture of 3,5-dimethyl-4-nitrophenol (20 g) and acetonitrile (120 mL) were added 8 M aqueous potassium hydroxide solution (59.8 mL) and diethyl (bromodifluoromethyl)phosphonate (38.3 mL) at -18°C. Then, the mixture was stirred at the same temperature for 30 minutes, and then warmed to room temperature, followed by stirring for additional 30 minutes. To the reaction mixture was added concentrated hydrochloric acid (20 mL) at 0°C, and then solvent was removed under reduced pressure. The residue was extracted with ethyl acetate and dried over anhydrous sodium sulfate, and then solvent was removed under reduced pressure. The residue was purified by column chromatography (eluent solvent(s): 0 vol% to 11 vol% ethyl acetate/hexane) to give the title compound (23.1 g).
¹H-NMR (CDCl₃) δ: 6.88 (2H, s), 6.52 (1H, t, J = 73.1 Hz), 2.33 (6H, s).

### Step 5-2: 4-(difluoromethoxy)-2,6-dimethylaniline hydrochloride

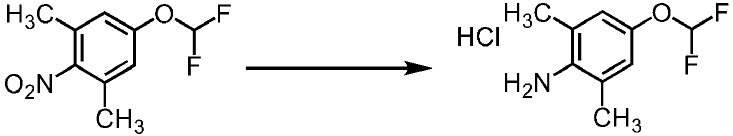

To a mixture of 5-(difluoromethoxy)-1,3-dimethyl-2-nitrobenzene (23.1 g) and ethanol (130 mL) was added 10% palladium carbon (566 mg), and the mixture was stirred at room temperature overnight under a hydrogen atmosphere (at an ordinary pressure). The palladium catalyst was filtered off through Celite, and then solvent was removed under reduced pressure. To the residue were added 4 M solution of hydrogen chloride in ethyl acetate (35 mL) and a mixed solution of hexane/ethyl acetate (v/v = 3/1, 100 mL) under ice cooling. The resulted solid was collected by filtration, and then washed with a mixed solution of hexane/ethyl acetate (v/v = 3/1) to give the title compound (19.9 g).
¹H-NMR (DMSO-D₆) δ: 7.14 (1H, t, J = 74.3 Hz), 6.93 (2H, s), 2.32 (6H, s).
LC-MS (MH+): 188.

### Step 5-3: {4-(difluoromethoxy)-2,6-dimethylphenyl}hydrazine hydrochloride

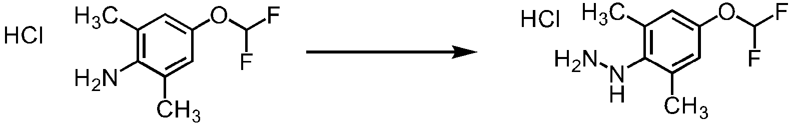

To a mixture of 4-(difluoromethoxy)-2,6-dimethylaniline hydrochloride (19.5 g) and 6 M hydrochloric acid (98 mL) was added concentrated hydrochloric acid (59 mL), and then the mixture was cooled to -10°C. Thereto was added dropwise and slowly an aqueous solution (19.5 mL) of sodium nitrite (6.3 g) at the same temperature over 10 minutes. Then, the mixture was stirred for additional 2 hours. To the reaction mixture was added a mixture of tin (II) chloride 2 hydrate (29.5 g) and concentrated hydrochloric acid (33 mL) at the same temperature, and then the mixture was stirred for 1.5 hours while being let warm to room temperature. The resulted solid was collected by filtration, and then washed sequentially with 2 M hydrochloric acid and diisopropylether to give the title compound (10 g).
¹H-NMR (DMSO-D₆) δ: 9.61 (3H, s), 7.20 (1H, t, J = 74.4 Hz), 6.93 (2H, s), 6.77 (1H, br s), 2.39 (6H, s).

### Step 5-4: 2,4-dichloro-6-[2-{4-(difluoromethoxy)-2,6-dimethylphenyl}hydrazinyl]-5-(dimethoxymethyl)pyrimidine

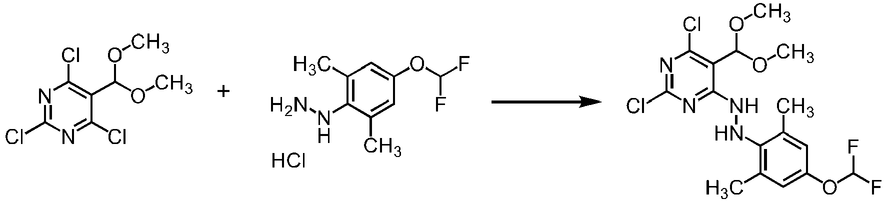

To a mixture of {4-(difluoromethoxy)-2,6-dimethylphenyl}hydrazine hydrochloride (7.9 g), 2,4,6-trichloro-5-(dimethoxymethyl)pyrimidine (10 g), synthesized in a similar manner to Preparation method 4 Step 4-6, and methanol (100 mL) was added triethylamine (16.2 mL) at 0°C. The reaction mixture was stirred at room temperature for 1 hour, and then solvent was removed under reduced pressure. To the residue was added ethyl acetate, and the resulted salt was filtered off. Then, solvent was removed under reduced pressure to give a crude product of the title compound.

### Step 5-5: 4,6-dichloro-2-{4-(difluoromethoxy)-2,6-dimethylphenyl}-2H-pyrazolo[3,4-d]pyrimidine

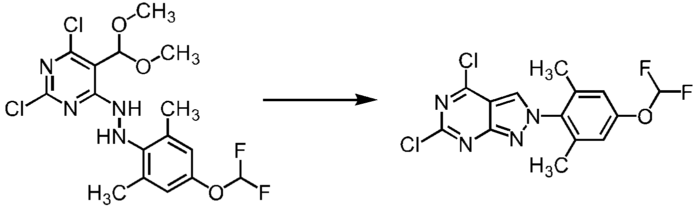

To a mixture of the crude product of 2,4-dichloro-6-[2-{4-(difluoromethoxy)-2,6-dimethylphenyl}hydrazinyl]-5-(dimethoxymethyl)pyrimidine and toluene (278 mL) was added trifluoroacetic acid (12.7 mL), and the mixture was stirred at room temperature for 1 hour. The reaction mixture was let stand overnight, and then solvent was removed under reduced pressure. The residue was neutralized by addition of saturated aqueous sodium hydrogen carbonate solution, and then extracted with ethyl acetate. The obtained organic layer was dried over anhydrous sodium sulfate, and then solvent was removed under reduced pressure to give a crude product of the title compound.

### Step 5-6: 6-chloro-2-{4-(difluoromethoxy)-2,6-dimethylphenyl}-2,5-dihydro-4H-pyrazolo[3,4-d]pyrimidin-4-one

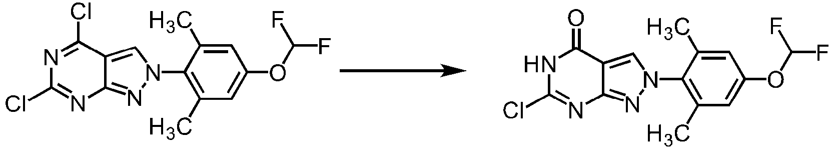

To a mixture of the crude product of 4,6-dichloro-2-{4-(difluoromethoxy)-2,6-dimethylphenyl}-2H-pyrazolo[3,4-d]pyrimidine and tetrahydrofuran (118 mL) was added 2 M aqueous sodium hydroxide solution (32.9 mL), and the mixture was stirred at room temperature for 1 hour. The reaction mixture was neutralized with 2 M hydrochloric acid, and then extracted with ethyl acetate. The obtained organic layer was dried over anhydrous sodium sulfate, and then solvent was removed under reduced pressure. The residue was purified by column chromatography (eluent solvent(s): 25 vol% to 55 vol% ethyl acetate/hexane). The resulted solid was stirred with a mixed solution of diisopropylether/ethyl acetate (v/v =2/1), and then collected by filtration to give the title compound (4.83 g).
¹H-NMR (DMSO-D₆) δ: 12.88 (1H, s), 8.81 (1H, s), 7.32 (1H, t, J = 73.9 Hz), 7.12 (2H, s), 1.98 (6H, s).

### Step 5-7: 2-{4-(difluoromethoxy)-2,6-dimethylphenyl}-6-(1-ethoxyvinyl)-2,5-dihydro-4H-pyrazolo[3,4-d]pyrimidin-4-one

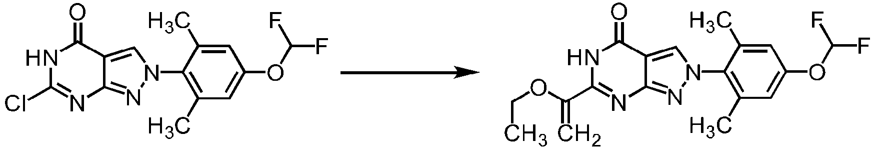

A mixture of 6-chloro-2-{4-(difluoromethoxy)-2,6-dimethylphenyl}-2,5-dihydro-4H-pyrazolo[3,4-d]pyrimidin-4-one (300 mg), tributyl(1-ethoxyvinyl)tin (477 mg), bis(triphenylphosphine)palladium (II) dichloride (62 mg), and toluene (6.0 mL) was stirred at 100°C for 1.5 hours under an argon atmosphere. The reaction mixture was cooled to room temperature and separated after addition of water, and then the resulted aqueous layer was extracted with ethyl acetate. All organic layers were combined and dried over anhydrous magnesium sulfate, and then solvent was removed under reduced pressure to give a crude product of the title compound (331 mg).
LC-MS (MH+): 377.

### Step 5-8: 6-acetyl-2-{4-(difluoromethoxy)-2,6-dimethylphenyl}-2,5-dihydro-4H-pyrazolo[3,4-d]pyrimidin-4-one

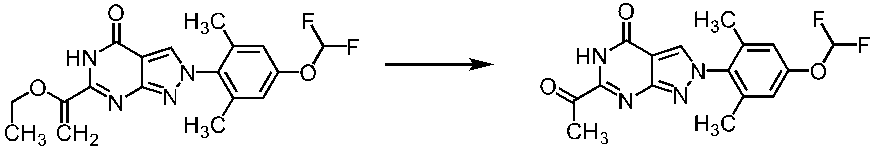

To a mixture of the crude product of 2-{4-(difluoromethoxy)-2,6-dimethylphenyl}-6-(1-ethoxyvinyl)-2,5-dihydro-4H-pyrazolo[3,4-d]pyrimidin-4-one (332 mg) and tetrahydrofuran (5.0 mL) was added 2M hydrochloric acid (5.0 mL), and the mixture was stirred at room temperature for 30 minutes, followed by stirring at 50°C for 1.5 hours. The reaction mixture was cooled to room temperature, and then separated after addition of ethyl acetate, water, and saturated aqueous sodium hydrogen carbonate solution. Then, the resulted aqueous layer was extracted with ethyl acetate. All organic layers were combined and dried over anhydrous magnesium sulfate, and solvent was removed under reduced pressure. The residue was purified by column chromatography (eluent solvent(s): 30 vol% to 80 vol% ethyl acetate/hexane) to give the title compound (271 mg).
¹H-NMR (DMSO-D₆) δ: 11.79 (1H, s), 8.88 (1H, s), 7.33 (1H, t, J = 73.8 Hz), 7.15 (2H, s), 2.63 (3H, s), 1.99 (6H, s).

### Step 5-9: (RS)-2-{4-(difluoromethoxy)-2,6-dimethylphenyl}-6-(1-hydroxyethyl)-2,5-dihydro-4H-pyrazolo[3,4-d]pyrimidin-4-one (Example 141)

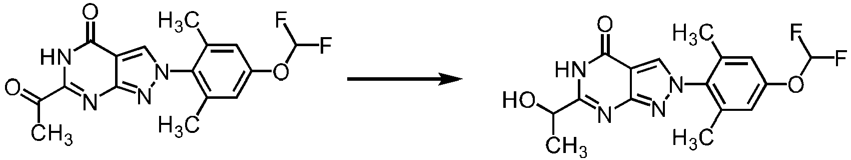

To a mixture of 6-acetyl-2-{4-(difluoromethoxy)-2,6-dimethylphenyl}-2,5-dihydro-4H-pyrazolo[3,4-d]pyrimidin-4-one (271 mg), methanol (5.4 mL), and tetrahydrofuran (2.7 mL) was added sodium borohydride (59 mg) at 0°C, and the mixture was stirred at the same temperature for 45 minutes. Solvent was removed under reduced pressure, and then the residue was separated after addition of ethyl acetate, water, and saturated aqueous ammonium chloride solution. Then, the resulted aqueous layer was extracted with ethyl acetate. All organic layers were combined and dried over anhydrous magnesium sulfate, and then solvent was removed under reduced pressure. The residue was purified by column chromatography (eluent solvent (s): 60 vol% to 100 vol% ethyl acetate/hexane, followed by 0 vol% to 10 vol% methanol/ethyl acetate) to give the title compound (98 mg).
¹H-NMR (DMSO-D₆) δ: 11.33 (1H, s), 8.72 (1H, d, J = 0.5 Hz), 7.32 (1H, t, J = 73.9 Hz), 7.12 (2H, s), 5.64 (1H, s), 4.58 (1H, d, J = 5.5 Hz), 1.98 (6H, s), 1.43 (3H, d, J = 6.5 Hz). LC-MS (MH+): 351.

### Step 5-10: (R)-2-(4-(difluoromethoxy)-2,6-dimethylphenyl)-6-(1-hydroxyethyl)-2,5-dihydro-4H-pyrazolo[3,4-d]pyrimidin-4-one (Example 165), and (S)-2-(4-(difluoromethoxy)-2,6-dimethylphenyl)-6-(1-hydroxyethyl)-2,5-dihydro-4H-pyrazolo[3,4-d]pyrimidin-4-one (Example 166)

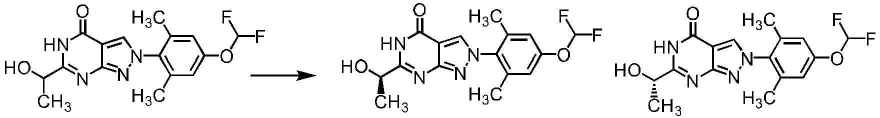

(RS)-2-(4-(Difluoromethoxy)-2,6-dimethylphenyl)-6-(1-hydroxyethyl)-2,5-dihydro-4H-pyrazolo[3,4-d]pyrimidin-4-one (98 mg) was optically resolved by column chromatography with a chiral column {apparatus: Japan Analytical Industry; automatic recycling preparative HPLC LaboACE LC-7080; column: Daicel CHIRALPAK IJ, 20 mm (I.D.) x 250 mm (L), 5 µm; column temperature: room temperature; flow rate of mobile phase: 15 mL/min; mixing ratio of mobile phase: isocratic, water/acetonitrile = 70/30} to give (R)-2-{4-(difluoromethoxy)-2,6-dimethylphenyl}-6-(1-hydroxyethyl)-2,5-dihydro-4H-pyrazolo[3,4-d]pyrimidin-4-one (Example 165 compound, 42.9 mg) for the first peak fraction (23.2 to 28.5 min), and (S)-2-{4-(difluoromethoxy)-2,6-dimethylphenyl}-6-(1-hydroxyethyl)-2,5-dihydro-4H-pyrazolo[3,4-d]pyrimidin-4-one (Example 166 compound, 42.6 mg) for the second peak fraction (28.9 to 34.5 min). The absolute configuration of Example 165 compound was determined by X-ray crystallography. [(R)-2-{4-(difluoromethoxy)-2,6-dimethylphenyl}-6-(1-hydroxyethyl)-2,5-dihydro-4H-pyrazolo[3,4-d]pyrimidin-4-one: Example 165]
¹H-NMR (DMSO-D₆) δ: 11.30 (1H, s), 8.71 (1H, s), 7.32 (1H, t, J = 73.9 Hz), 7.12 (2H, s), 5.61 (1H, s), 4.57 (1H, q, J = 6.6 Hz), 1.98 (6H, s), 1.43 (3H, d, J = 6.7 Hz).
LC-MS (MH+): 351.
[(S)-2-{4-(difluoromethoxy)-2,6-dimethylphenyl}-6-(1-hydroxyethyl)-2,5-dihydro-4H-pyrazolo[3,4-d]pyrimidin-4-one: Example 166]
¹H-NMR (DMSO-D₆) δ: 11.31 (1H, br s), 8.71 (1H, s), 7.32 (1H, t, J = 73.9 Hz), 7.12 (2H, s), 5.65 (1H, br s), 4.57 (1H, q, J = 6.5 Hz), 1.98 (6H, s), 1.43 (3H, d, J = 6.7 Hz).
LC-MS (MH+): 351.

### [Preparation example 6]: Synthesis of 2-(4-cyclopropyl-2,6-dimethylphenyl)-6-(1,4-dioxepan-5-yl)-2,5-dihydro-4H-pyrazolo[3,4-d]pyrimidin-4-one (Example 163, racemate) and each optically active substance thereof (Example 178, 179)

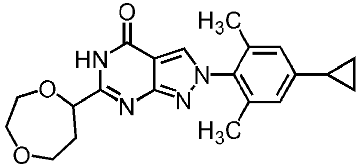

### Step 6-1: methyl 2-{2-(4-bromo-2,6-dimethylphenyl) hydrazinylidene}-2-chloroacetate

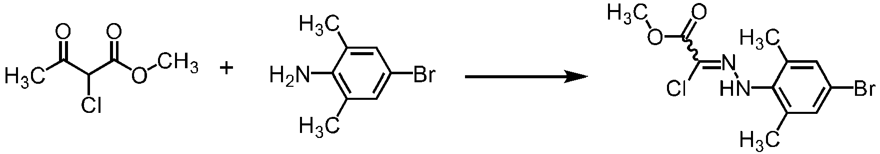

To a mixture of 4-bromo-2,6-dimethylaniline (45 g), ethanol (72 mL), and water (25 mL) was added concentrated hydrochloric acid (47 mL), and then the mixture was cooled to -10°C. To the reaction mixture was added dropwise and slowly an aqueous solution (54 mL) of sodium nitrite (17.1 g) over 30 minutes at the same temperature, and then the mixture was stirred for additional 30 minutes. To the reaction mixture was added dropwise an aqueous solution (315 mL) of methyl 2-chloro-3-oxobutanoate (27 mL) and sodium acetate (55.4 g) at the same temperature. The reaction mixture was stirred for additional 2 hours, and then the resulted aqueous layer was extracted with ethyl acetate. The obtained organic layer was dried over anhydrous magnesium sulfate, and then solvent was removed under reduced pressure to give a crude product of the title compound.

### Step 6-2: methyl 1-(4-bromo-2,6-dimethylphenyl)-4-cyano-1H-pyrazole-3-carboxylate

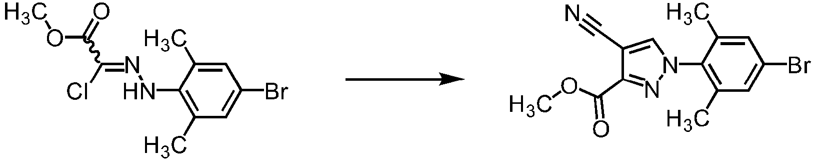

To a mixture of the crude product of methyl 2-{2-(4-bromo-2,6-dimethylphenyl)hydrazinylidene}-2-chloroacetate and chloroform (580 mL) were added triethylamine (36 mL) and fumaronitrile (20 g) under an argon atmosphere. The reaction mixture was heated to 80°C and stirred for 40 minutes. The reaction mixture was cooled to room temperature, and then thereto was added silica gel (250 g). Then, the mixture was stirred for 30 minutes. The added silica gel was collected by filtration, and the silica gel was washed with ethyl acetate. Solvent was removed under reduced pressure, and then the resulted solid was washed with ethyl acetate to give the title compound (36.7 g).
¹H-NMR (DMSO-D₆) δ: 9.01 (1H, s), 7.57 (2H, s), 3.90 (3H, s), 1.96 (6H, s).

### Step 6-3: 1-(4-bromo-2,6-dimethylphenyl)-4-cyano-1H-pyrazole-3-carboxylic acid

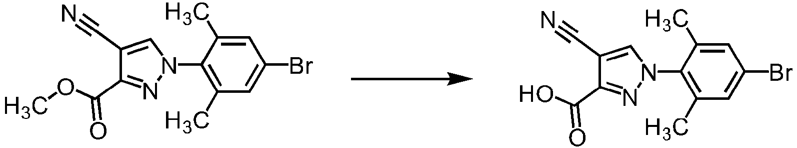

To a mixture of methyl 1-(4-bromo-2,6-dimethylphenyl)-4-cyano-1H-pyrazole-3-carboxylate (36 g) and methanol (360 mL) was added 2 M aqueous sodium hydroxide solution (110 mL), and the mixture was stirred at room temperature for 2.5 hours. The reaction mixture was neutralized with 6 M hydrochloric acid, and then the resulted solid was collected by filtration. To the resulted solid were added 1M hydrochloric acid, saturated brine, and ethyl acetate, and the mixture was separated. The resulted aqueous layer was extracted with ethyl acetate. All organic layers were mixed and dried over anhydrous magnesium sulfate, and then solvent was removed under reduced pressure to give the desired product 1. A mother solution upon the filtration of the solid was extracted with ethyl acetate, and the resulted organic layer was dried over anhydrous magnesium sulfate. Solvent was removed under reduced pressure to give the desired product 2. The desired products 1 and 2 were combined and stirred in a mixed solution of ethyl acetate/diisopropylether/hexane, and then the resulted solid was collected by filtration to give the title compound (30 g).
¹H-NMR (DMSO-D₆) δ: 13.86 (1H, s), 8.95 (1H, s), 7.56 (2H, s), 1.97 (6H, s).

### Step 6-4: 3-amino-1-(4-bromo-2,6-dimethylphenyl)-1H-pyrazole-4-carbonitrile

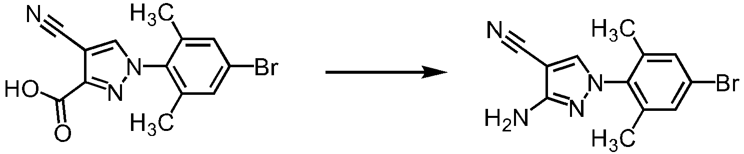

To a mixture of 1-(4-bromo-2,6-dimethylphenyl)-4-cyano-1H-pyrazole-3-carboxylic acid (30 g) and tert-butanol (450 mL) were added triethylamine (26 mL) and diphenylphosphoryl azide (30 mL) under an argon atmosphere, and the mixture was stirred at 90°C for 3 hours. The reaction mixture was cooled to room temperature, and then solvent was removed under reduced pressure. The residue was diluted with chloroform (180 mL), and then thereto was added trifluoroacetic acid (22 mL). The mixture was stirred at 80°C for 1 hour. The reaction mixture was cooled to 0°C and neutralized with saturated aqueous sodium hydrogen carbonate solution, and then extracted with ethyl acetate. The organic layer was dried over anhydrous sodium sulfate, and solvent was removed under reduced pressure. The resulted crude product was purified by column chromatography (eluent solvent(s): 1 vol% to 40 vol% ethyl acetate/hexane). The resulted solid was washed with ethyl acetate/diisopropylether to give the title compound (11 g).
¹H-NMR (DMSO-D₆) δ: 8.31 (1H, s), 7.46 (2H, s), 5.76 (2H, s), 2.01 (6H, s).

### Step 6-5: 3-amino-1-(4-bromo-2,6-dimethylphenyl)-1H-pyrazole-4-carboxamide

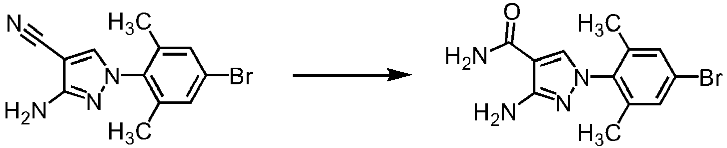

To a mixture of 3-amino-1-(4-bromo-2,6-dimethylphenyl)-1H-pyrazole-4-carbonitrile (11 g), ethanol (66 mL), and dimethylsulfoxide (17 mL) were added sodium hydroxide (4.5 g) and 30% hydrogen peroxide solution (15 mL) at 0°C under an argon atmosphere. The mixture was stirred at room temperature for 40 minutes, and then thereto were added saturated aqueous sodium sulfite solution and concentrated hydrochloric acid. The reaction mixture was extracted with ethyl acetate and dried over anhydrous magnesium sulfate, and then solvent was removed under reduced pressure. The resulted crude product was washed with ethyl acetate/diisopropylether to give the title compound (10 g). ¹H-NMR (DMSO-D₆) δ: 7.97 (1H, s), 7.45 (2H, s), 7.31 (1H, br s), 6.91 (1H, br s), 5.53 (2H, s), 2.02 (6H, s).

### Step 6-6: dimethyl 2,2'-[1,2-ethanediylbis(oxy)]diacetate

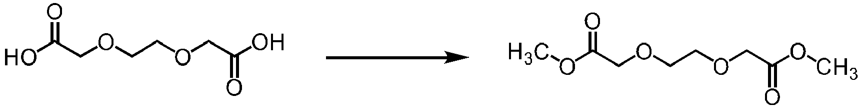

To a mixture of 2,2'-[1,2-ethanediylbis(oxy)]diacetic acid (10 g) and methanol (50 mL) was added sulfuric acid (0.15 mL) at room temperature under an argon atmosphere. The reaction mixture was stirred at 80°C for 24 hours. The reaction mixture was cooled to room temperature, and then thereto were added saturated aqueous sodium hydrogen carbonate solution and ethyl acetate. The resulted organic layer was separated, and then the resulted aqueous layer was extracted with ethyl acetate. All organic layers were combined and dried over anhydrous magnesium sulfate, and then solvent was removed under reduced pressure. The resulted crude product was purified by column chromatography (eluent solvent(s): 29 vol% to 50 vol% hexane/ethyl acetate) to give the title compound (9.5 g).
¹H-NMR (DMSO-D₆) δ: 4.14 (4H, s), 3.65 (6H, s), 3.61 (4H, s).

### Step 6-7: methyl 6-oxo-1,4-dioxepane-5-carboxylate

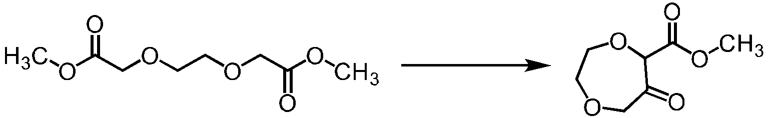

To a mixture of sodium tert-butoxide (9.8 g) and tetrahydrofuran (150 mL) was added dropwise a mixture of dimethyl 2,2'-[1,2-ethanediylbis(oxy)]diacetate (9.5 g) and tetrahydrofuran (150 mL) at 100°C over 1 hour under an argon atmosphere. The mixture was stirred at the same temperature for 3 hours, and then stirred overnight after being cooled to room temperature. To the reaction mixture was added acetic acid (6.6 mL), and then solvent was removed under reduced pressure. To the residue were added water and ethyl acetate, and the mixture was separated. Then, the resulted aqueous layer was extracted with ethyl acetate. All organic layers were combined and dried over anhydrous magnesium sulfate, and then solvent was removed under reduced pressure. The resulted crude product was purified by column chromatography (eluent solvent(s): 5 vol% to 60 vol% hexane/ethyl acetate) to give the title compound (3.2 g). ¹H-NMR (DMSO-D₆) δ: 5.10 (1H, s), 4.35 (1H, d, J = 17.6 Hz), 4.25 (1H, d, J = 17.8 Hz), 4.10-4.00 (2H, m), 3.92-3.85 (1H, m), 3.72-3.65 (4H, m).

### Step 6-8: methyl 6-[{(trifluoromethyl)sulfonyl}oxy]-2,3-dihydro-5H-1,4-dioxepine7-carboxylate

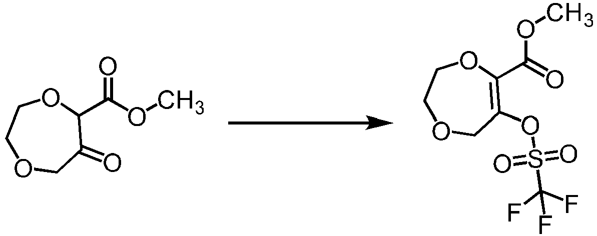

To a mixture of methyl 6-oxo-1,4-dioxepane-5-carboxylate (3.0 g) and deuterated chloroform (35 mL) were added N,N-diisopropylethylamine (7.1 mL) and trifluoromethanesulfonic anhydride (3.4 mL) at 0°C under an argon atmosphere, and the mixture was stirred at the same temperature for 1 hour. To the reaction mixture were added saturated aqueous sodium hydrogen carbonate solution, water, and ethyl acetate. Then, the mixture was separated, and then the resulted aqueous layer was extracted with ethyl acetate. All organic layers were combined and dried over anhydrous magnesium sulfate, and then solvent was removed under reduced pressure. The resulted crude product was purified by column chromatography (eluent solvent(s): 0 vol% to 30 vol% hexane/ethyl acetate) to give the title compound (4.8 g).
¹H-NMR (DMSO-D₆) δ: 4.49 (2H, s), 4.21-4.18 (2H, m), 3.89-3.87 (2H, m), 3.77 (3H, s).

### Step 6-9: methyl 1,4-dioxepane-5-carboxylate

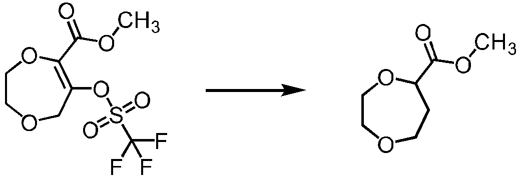

To a mixture of methyl 6-[{(trifluoromethyl)sulfonyl}oxy]-2,3-dihydro-5H-1,4-dioxepine7-carboxylate (4.8 g) and methanol (71 mL) was added nickel (II) chloride (0.6 g) at 0°C. To the reaction mixture was added sodium borohydride (2.1 g) in 4 divided portions at the same temperature, and then the mixture was warmed to room temperature and stirred for additional 1 hour. To the reaction mixture were added saturated aqueous ammonium chloride solution, water, and ethyl acetate. The resulted organic layer was separated, and then the resulted aqueous layer was extracted with ethyl acetate. All organic layers were combined and dried over anhydrous magnesium sulfate, and then solvent was removed under reduced pressure. The resulted crude product was purified by column chromatography (eluent solvent(s): 5 vol% to 60 vol% hexane/ethyl acetate) to give the title compound (1.7 g).
¹H-NMR (DMSO-D₆) δ: 4.36 (1H, dd, J = 9.7, 5.4 Hz), 3.87-3.84 (1H, m), 3.71-3.64 (8H, m), 2.27-2.19 (1H, m), 2.07-1.97 (1H, m).

### Step 6-10: 2-(4-bromo-2,6-dimethylphenyl)-6-(1,4-dioxepan-5-yl)-2,5-dihydro-4H-pyrazolo[3,4-d]pyrimidin-4-one

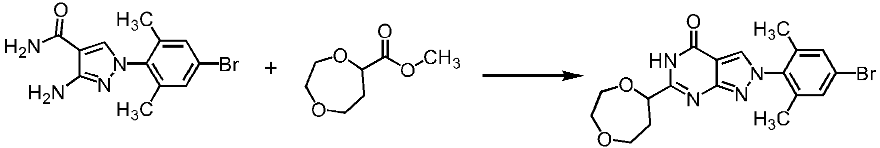

A mixture of 3-amino-1-(4-bromo-2,6-dimethylphenyl)-1H-pyrazole-4-carboxamide (600 mg), methyl 1,4-dioxepane-5-carboxylate (370 mg), sodium methoxide (5M methanol solution, 1.6 mL), and methanol (3.6 mL) was stirred at 120°C for 2 hours under microwave irradiation. The reaction mixture was cooled to room temperature, and then thereto were added 1M hydrochloric acid, water, and ethyl acetate. The resulted organic layer was separated, and then the resulted aqueous layer was extracted with ethyl acetate. All organic layers were combined and dried over anhydrous magnesium sulfate, and solvent was removed under reduced pressure. The resulted crude product was purified by column chromatography (eluent solvent(s): 20 vol% to 100 vol% hexane/ethyl acetate) to give the title compound (380 mg).
¹H-NMR (DMSO-D₆) δ: 11.65 (1H, s), 8.75 (1H, s), 7.54 (2H, s), 4.69 (1H, dd, J = 9.5, 4.9 Hz), 4.02-3.96 (1H, m), 3.78-3.68 (5H, m), 2.43-2.39 (1H, m), 2.33-2.29 (1H, m), 1.96 (6H, s).

### Step 6-11: (RS)-2-(4-cyclopropyl-2,6-dimethylphenyl)-6-(1,4-dioxepan-5-yl)-2,5-dihydro-4H-pyrazolo[3,4-d]pyrimidin-4-one (Example 163)

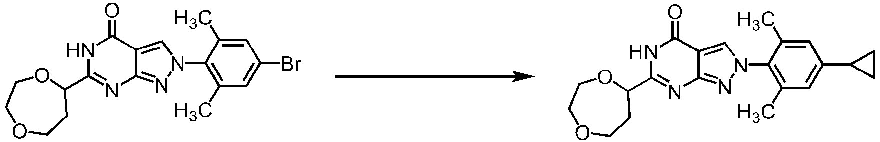

To a mixture of 2-(4-bromo-2,6-dimethylphenyl)-6-(1,4-dioxepan-5-yl)-2,5-dihydro-4H-pyrazolo[3,4-d]pyrimidin-4-one (155 mg), cyclopropylboronic acid (95 mg), [1,1'-bis(di-tert-butylphosphino)ferrocene]palladium (II) dichloride (24 mg), and toluene (3.0 mL) was added 2M aqueous tripotassium phosphate solution (0.55 mL) under an argon atmosphere, and the mixture was stirred at 100°C for 2 hours. The reaction mixture was cooled to room temperature, and then thereto was added water. The resulted organic layer was separated, and then the resulted aqueous layer was extracted with ethyl acetate. All organic layers were combined and dried over anhydrous magnesium sulfate, and solvent was removed under reduced pressure. The resulted crude product was purified by column chromatography (eluent solvent(s): 20 vol% to 80 vol% hexane/ethyl acetate), followed by purification by reverse-phase column chromatography (eluent solvent(s): 20 vol% to 100 vol% acetonitrile/water) to give the title compound (96 mg).
¹H-NMR (DMSO-D₆) δ: 11.61 (1H, s), 8.67 (1H, s), 6.96 (2H, s), 4.69 (1H, dd, J = 9.4, 5.0 Hz), 4.01-3.98 (1H, m), 3.87-3.69 (5H, m), 2.45-2.40 (1H, m), 2.32-2.29 (1H, m), 1.97-1.91 (7H, m), 1.01-0.96 (2H, m), 0.74 (2H, dt, J = 8.3, 3.2 Hz).
LC-MS (MH+): 381.

### Step 6-12: Each optically active substance of 2-(4-cyclopropyl-2,6-dimethylphenyl)-6-(1,4-dioxepan-5-yl)-2,5-dihydro-4H-pyrazolo[3,4-d]pyrimidin-4-one (Example 178, 179)

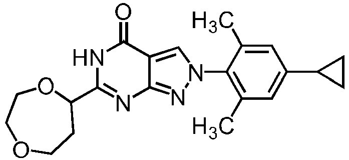

(RS)-2-(4-Cyclopropyl-2,6-dimethylphenyl)-6-(1,4-dioxepan-5-yl)-2,5-dihydro-4H-pyrazolo[3,4-d]pyrimidin-4-one (97 mg) was optically resolved by supercritical fluid chromatography {apparatus: Waters SFC Prep15 System;column: Daicel CHIRALPAK IG/SFC, 10 mm (I.D.) x 250 mm (L), 5 µm; column temperature: 40°C; mobile phaseflow rate: 15 mL/min; mixing ratio of mobile phase: isocratic, carbon dioxide/methanol = 60/40} to give Example 178 compound (37 mg) for the first peak fraction (6.4 to 7.7 min), and Example 179 compound (40 mg) for the second peak fraction (9.2 to 11.5 min).

### [Example 178]

¹H-NMR (DMSO-D₆) δ: 11.61 (1H, s), 8.66 (1H, s), 6.96 (2H, s), 4.68 (1H, dd, J = 9.2, 4.8 Hz), 4.01-3.98 (1H, m), 3.83-3.73 (5H, m), 2.38-2.32 (2H, m), 1.95-1.92 (7H, m), 0.98 (2H, td, J = 7.1, 4.7 Hz), 0.76-0.72 (2H, m).
LC-MS (MH+): 381.

### [Example 179]

¹H-NMR (DMSO-D₆) δ: 11.62 (1H, s), 8.66 (1H, s), 6.96 (2H, s), 4.68 (1H, dd, *J =* 9.4, 4.6 Hz), 4.00 (1H, t, *J =* 8.1 Hz), 3.84-3.69 (5H, m), 2.43-2.29 (2H, m), 1.94-1.91 (7H, m), 0.98 (2H, dd, *J* = 13.2, 5.1 Hz), 0.73 (2H, q, *J* = 4.8 Hz).
LC-MS (MH+): 381.

### [Preparation example 7]: Synthesis of 2-(4-bromo-2,6-dimethylphenyl)-4-methoxy-6-methyl-2H-pyrazolo[3,4-d]pyrimidine (Example 210)

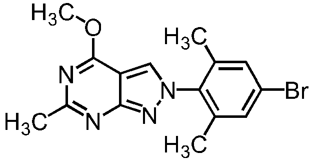

### Step 7-1: 2-(4-bromo-2,6-dimethylphenyl)-4-chloro-6-methyl-2H-pyrazolo[3,4-d]pyrimidine

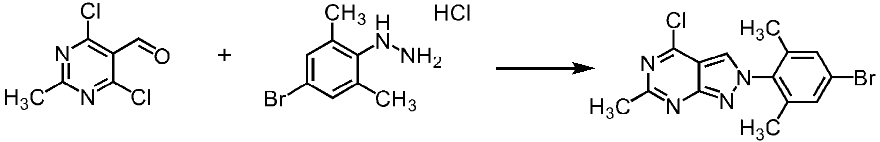

To a mixture of (4-bromo-2,6-dimethylphenyl)hydrazine hydrochloride (200 mg), triethylamine (0.44 mL), tetrahydrofuran (1.5 mL), and water (0.75 mL) was added 4,6-dichloro-2-methylpyrimidine-5-carbaldehyde (150 mg) at 0°C under an argon atmosphere, and then the mixture was stirred at room temperature for 3 hours. To the reaction mixture was added water, and then the mixture was extracted with ethyl acetate. The obtained organic layer was washed with saturated brine and dried over anhydrous magnesium sulfate, and then solvent was removed under reduced pressure. The residue was purified by column chromatography (eluent solvent(s): 10 vol% to 70 vol% ethyl acetate/hexane) to give the title compound (100 mg).
¹H-NMR (CDCl₃) δ: 8.11 (1H, s), 7.39 (2H, s), 2.84 (3H, s), 2.01 (6H, s).
LC-MS (MH+): 353.

### Step 7-2: 2-(4-bromo-2,6-dimethylphenyl)-4-methoxy-6-methyl-2H-pyrazolo[3,4-d]pyrimidine

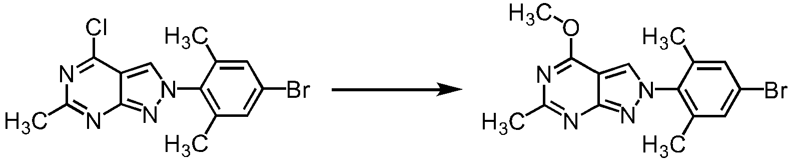

To a mixture of 2-(4-bromo-2,6-dimethylphenyl)-4-chloro-6-methyl-2H-pyrazolo[3,4-d]pyrimidine (100 mg) and methanol (1.5 mL) was added 5 M solution of sodium methoxide in methanol (0.28 mL) under an argon atmosphere, and the mixture aws stirred at room temperature for 1.5 hours. To the reaction mixture was added saturated aqueous ammonium chloride solution, and then the mixture was extracted with ethyl acetate. The obtained organic layer was washed with saturated brine and dried over anhydrous magnesium sulfate, and then solvent was removed under reduced pressure. The residue was purified by column chromatography (eluent solvent(s): 10 vol% to 80 vol% ethyl acetate/hexane) to give the title compound (15 mg).
¹H-NMR (DMSO-D₆) δ: 8.82 (1H, s), 7.56 (2H, s), 4.09 (3H, s), 2.58 (3H, s), 1.93 (6H, s).
LC-MS (MH+): 347.

Example compounds in addition to those described above were obtained in a similar manner to the above Preparation methods and Preparation examples, or if necessary, by known methods. The structure and physical property data of each Example compound are shown in the following tables.

| Example No. | Structure | Comments | ¹H-NMR (400 MHz) | MS (M+H) |
|---|---|---|---|---|
| 1 | 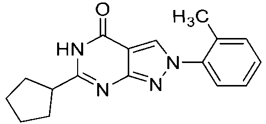 | | ¹H-NMR (DMSO-D₆) δ: 11.67 (1H, br s), 8.74 (1H, s), 7.45-7.35 (4H, m), 3.06-2.98 (1H, m), 2.21 (3H, s), 2.01-1.93 (2H, m), 1.88-1.84 (2H, m), 1.76-1.71 (2H, m), 1.62-1.58 (2H, m). | 295 |
| 2 | 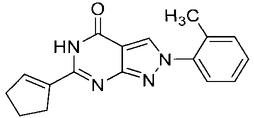 | | ¹H-NMR (DMSO-D₆) δ: 11.64 (1H, br s), 8.78 (1H, s), 7.45-7.38 (4H, m), 7.07-7.07 (1H, m), 2.75-2.72 (2H, m), 2.58-2.53 (2H, m), 2.22 (3H, s), 1.94-1.89 (2H, m). | 293 |
| 3 | 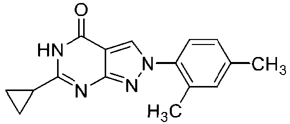 | | ¹H-NMR (CDCl₃) δ: 10.43 (br s, 1H), 8.19 (s, 1H), 7.27 (d, 1H, J = 7.6 Hz), 7.15 (s, 1H), 7.11 (d, 1H, J = 7.6 Hz), 2.39 (s, 3H), 2.26 (s, 3H), 1.89-1.81 (m, 1H), 1.41-1.36 (m, 2H), 1.14-1.08 (m, 2H). | 281 |
| 4 | 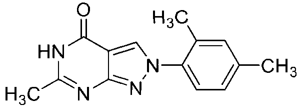 | | ¹H-NMR (DMSO-D₆) δ: 11.71 (1H, br s), 8.68 (1H, s), 7.31 (1H, d, J = 8.1 Hz), 7.23 (1H, s), 7.17 (1H, d, J = 8.1 Hz), 2.34 (3H, s), 2.31 (3H, s), 2.15 (3H, s) . | 255 |
| 5 | 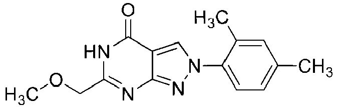 | | ¹H-NMR (DMSO-D₆) δ: 11.73 (1H, s), 8.76 (1H, s), 7.34 (1H, d, J = 7.9 Hz), 7.25 (1H, s), 7.19 (1H, d, J = 8.1 Hz), 4.32 (2H, s), 3.37 (3H, s), 2.36 (3H, s), 2.17 (3H, s) . | 285 |
| 6 | 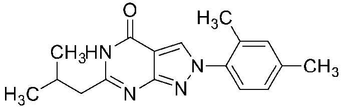 | | ¹H-NMR (DMSO-D₆) δ: 11.69 (1H, s), 8.70 (1H, s), 7.34 (1H, d, J = 8.1 Hz), 7.25 (1H, s), 7.18 (1H, d, J = 8.1 Hz), 2.46 (2H, d, J = 7.2 Hz), 2.36 (3H, s), 2.23-2.13 (1H, m), 2.18 (3H, s), 0.94 (6H, d, J = 6.7 Hz). | 297 |
| 7 | 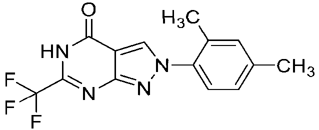 | | ¹H-NMR (DMSO-D₆) δ: 13.18 (1H, br s), 8.94 (1H, s), 7.36 (1H, d, J = 7.5 Hz), 7.28 (1H, s), 7.21 (1H, d, J = 8.2 Hz), 2.37 (3H, s), 2.18 (3H, s). | 309 |
| 8 | 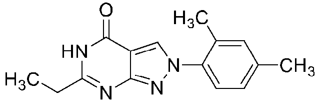 | | ¹H-NMR (DMSO-D₆) δ: 11.70 (1H, s), 8.70 (1H, s), 7.32 (1H, d, J = 8.1 Hz), 7.25 (1H, s), 7.18 (1H, d, J = 7.9 Hz), 2.61 (2H, q, J = 7.6 Hz), 2.36 (3H, s), 2.17 (3H, s), 1.23 (3H, t, J = 7.5 Hz). | 269 |
| 9 | 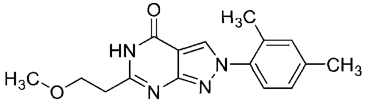 | | ¹H-NMR (DMSO-D₆) δ: 11.73 (1H, s), 8.68 (1H, s), 7.32 (1H, d, J = 7.9 Hz), 7.24 (1H, s), 7.18 (1H, d, J = 8.1 Hz), 3.74 (2H, t, J = 6.6 Hz), 3.26 (3H, s), 2.84 (2H, t, J = 6.5 Hz), 2.36 (3H, s), 2.17 (3H, s) . | 299 |
| 10 | 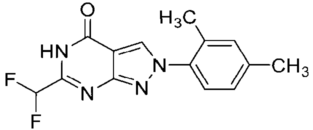 | | ¹H-NMR (DMSO-D₆) δ: 12.60 (1H, br s), 8.87 (1H, d, J = 1.8 Hz), 7.34 (1H, d, J = 8.1 Hz), 7.25 (1H, s), 7.19 (1H, d, J = 8.3 Hz), 6.78 (1H, t, J = 53.2 Hz), 2.35 (3H, s), 2.16 (3H, s). | 291 |
| 11 | 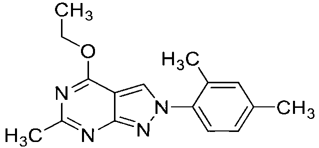 | | ¹H-NMR (DMSO-D₆) δ: 8.79 (1H, s), 7.37(1H, d, J = 8.4 Hz), 7.27 (1H, s), 7.20 (1H, d, J = 8.4 Hz), 4.59 (2H, q, J = 7.6 Hz), 2.56 (3H, s), 2.37 (3H, s), 1.99 (3H, s) . | 283 |
| 12 | 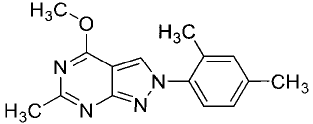 | | ¹H-NMR (DMSO-D₆) δ: 8.82 (1H, s), 7.37 (1H, d, J = 8.4 Hz), 7.27 (1H, s), 7.21 (1H, d, J = 8.4 Hz), 4.09 (3H, s), 2.57 (3H, s), 2.38 (3H, s), 2.15 (3H, s) . | 269 |
| 13 | 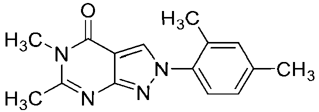 | | ¹H-NMR (DMSO-D₆) δ: 8.74 (1H, s), 7.33 (1H, d, J = 7.9 Hz), 7.25 (1H, s), 7.18 (1H, d, J = 8.1 Hz), 3.48 (3H, s), 2.56 (3H, s), 2.36 (3H, s), 2.16 (3H, s) . | 269 |
| 14 | 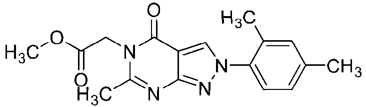 | | ¹H-NMR (DMSO-D₆) δ: 8.82 (1H, s), 7.35 (1H, d, J = 8.2 Hz), 7.26 (1H, s), 7.20 (1H, d, J = 8.2 Hz), 4.91 (2H, s), 3.74 (3H, s), 2.37 (3H, s), 2.18 (3H, s) . | 327 |
| 15 | 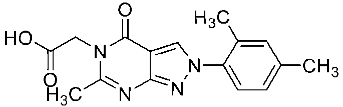 | | ¹H-NMR (DMSO-D₆) δ: 13.29 (1H, s), 8.81 (1H, s), 7.35 (1H, d, J = 8.2 Hz), 7.26 (1H, s), 7.20 (1H, d, J = 8.2 Hz), 4.81 (2H, s), 2.37 (3H, s), 2.18 (3H, s) . | 313 |
| 16 | 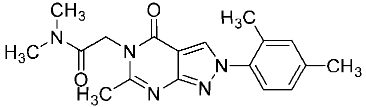 | | ¹H-NMR (DMSO-D₆) δ: 8.78 (1H, s), 7.35 (1H, d, J = 8.2 Hz), 7.26 (1H, s), 7.20 (1H, d, J = 8.2 Hz), 4.98 (2H, s), 3.13 (3H, s), 2.89 (3H, s), 2.42 (3H, s), 2.37 (3H, s), 2.19 (3H, s) . | 340 |
| 17 | 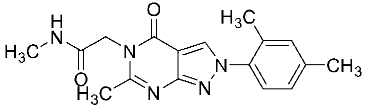 | | ¹H-NMR (DMSO-D₆) δ: 8.78 (1H, s), 8.20-8.18 (1H, m), 7.34 (1H, d, J = 8.2 Hz), 7.26 (1H, s), 7.20 (1H, d, J = 8.2 Hz), 4.69 (2H, s), 2.65 (3H, d, J = 4.5 Hz), 2.47 (3H, s), 2.37 (3H, s), 2.19 (3H, s). | 326 |
| 18 | 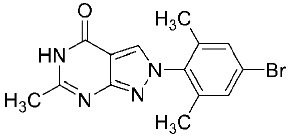 | | ¹H-NMR (DMSO-D₆) δ: 11.76 (1H, br s), 8.67 (1H, s), 7.52 (2H, s), 2.31 (3H, s), 1.94 (6H, s). | 334 |
| 19 | 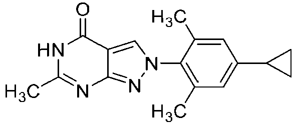 | | ¹H-NMR (DMSO-D₆) δ: 11.72 (1H, br s), 8.59 (1H, s), 6.93 (2H, s), 2.31 (3H, s), 1.93-1.90 (7H, m), 0.98-0.95 (2H, m), 0.72-0.70 (2H, m). | 295 |
| 20 | 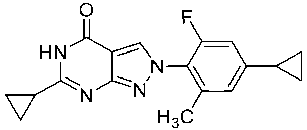 | | ¹H-NMR (DMSO-D₆) δ: 12.02 (1H, br s), 8.70 (1H, s), 7.01-6.99 (2H, m), 2.03 (3H, s), 2.00-1.93 (2H, m), 1.08-0.99 (6H, m), 0.78-0.76 (2H, m). | 325 |
| 21 | 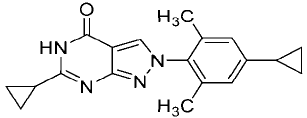 | | ¹H-NMR (DMSO-D₆) δ: 12.00 (1H, s), 8.58 (1H, br s), 6.94 (2H, s), 1.95-1.91 (8H, m), 1.08-1.07 (2H, m), 1.01-0.98 (4H, m), 0.74-0.72 (2H, m). | 321 |
| 22 | 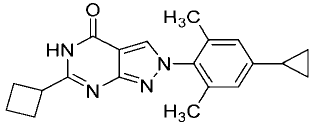 | | ¹H-NMR (DMSO-D₆) δ: 11.60 (1H, s), 8.60 (1H, s), 6.96 (2H, s), 3.53-3.44 (1H, m), 2.42-2.33 (2H, m), 2.27-2.21 (2H, m), 1.99-1.94 (7H, m), 1.43-1.39 (2H, m), 1.02-0.95 (2H, m), 0.75-0.72 (2H, m). | 335 |
| 23 | 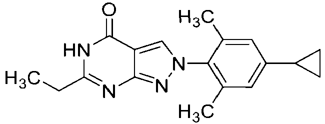 | | ¹H-NMR (DMSO-D₆) δ: 11.70 (1H, s), 8.61 (1H, s), 6.95 (2H, s), 2.61 (2H, q, J = 7.6 Hz), 1.95-1.92 (7H, m), 1.24 (3H, t, J = 7.5 Hz), 1.01-0.96 (2H, m), 0.74-0.72 (2H, m). | 309 |
| 24 | 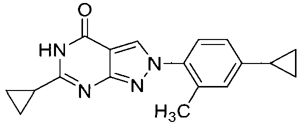 | | ¹H-NMR (DMSO-D₆) δ: 11.97 (1H, br s), 8.65 (1H, s), 7.27 (1H, d, J = 8.1 Hz), 7.11 (1H, d, J = 1.8 Hz), 7.04 (1H, dd, J = 8.1, 1.8 Hz), 2.14 (3H, s), 1.96-1.94 (2H, m), 1.07-0.96 (6H, m), 0.73-0.71 (2H, m). | 307 |
| 25 | 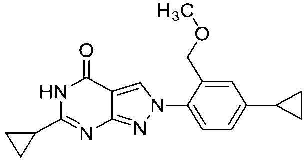 | | ¹H-NMR (CDCl₃) δ: 9.83 (br s, 1H), 8.38 (s, 1H), 7.39 (d, 1H, J = 8.2 Hz), 7.29 (d, 1H, J = 2.2 Hz), 7.11 (dd, 1H, J = 8.2, 2.2 Hz), 4.39 (s, 2H), 3.36 (s, 3H), 2.01-1.93 (m, 1H), 1.83-1.76 (m, 1H), 1.40-1.35 (m, 2H), 1.14-1.09 (m, 2H), 1.07-1.01 (m, 2H), 0.79-0.75 (m, 2H) . | 337 |
| 26 | 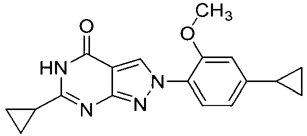 | | ¹H-NMR (CDCl₃) δ: 9.72 (br s, 1H), 8.77 (s, 1H), 7.86 (d, 1H, J = 8.3 Hz), 6.80 (d, 1H, J = 1.7 Hz), 6.76 (dd, 1H, J = 8.3, 1.7 Hz), 3.92 (s, 3H), 1.98-1.90 (m, 1H), 1.81-1.73 (m, 1H), 1.41-1.35 (m, 2H), 1.13-1.07 (m, 2H), 1.06-1.00 (m, 2H), 0.78-0.73 (m, 2H). | 323 |
| 27 | 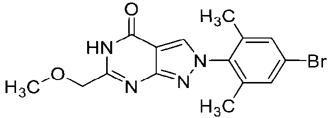 | | ¹H-NMR (DMSO-D₆) δ: 11.79 (1H, s), 8.76 (1H, s), 7.54 (2H, s), 4.32 (2H, s), 3.38 (3H, s), 1.96 (6H, s) . | 363 |
| 28 | 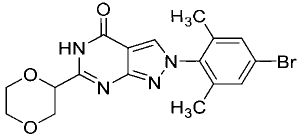 | racemate | ¹H-NMR (DMSO-D₆) δ: 11.68 (1H, s), 8.77 (1H, s), 7.54 (2H, s), 4.53 (1H, dd, J = 9.7, 3.0 Hz), 4.00 (1H, dd, J = 11.6, 2.6 Hz), 3.90-3.87 (1H, m), 3.77-3.73 (3H, m), 3.62-3.59 (1H, m), 1.96 (6H, s). | 405 |
| 29 | 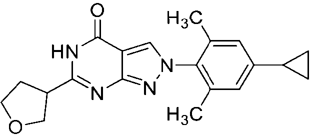 | racemate | ¹H-NMR (DMSO-D₆) δ: 11.81 (1H, s), 8.63 (1H, s), 6.95 (2H, s), 4.03-4.00 (1H, m), 3.91-3.85 (2H, m), 3.80-3.74 (1H, m), 3.41-3.40 (1H, m), 2.32-2.21 (2H, m), 1.95-1.90 (7H, m), 1.01-0.96 (2H, m), 0.75-0.71 (2H, m). | 351 |
| 30 | 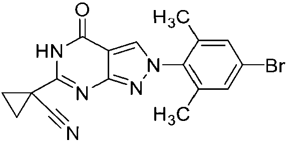 | | ¹H-NMR (DMSO-D₆) δ: 11.86 (1H, s), 8.76 (1H, s), 7.55 (2H, s), 1.96 (6H, s), 1.91-1.88 (2H, m), 1.78-1.76 (2H, m) . | 384 |
| 31 | 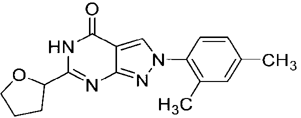 | racemate | ¹H-NMR (DMSO-D₆) δ: 11.49 (1H, s), 8.76 (1H, s), 7.33 (1H, d, J = 8.0 Hz), 7.25 (1H, s), 7.19 (1H, d, J = 8.1 Hz), 4.75 (1H, dd, J = 7.4, 6.4 Hz), 4.03 (1H, dd, J = 14.7, 7.1 Hz), 3.84-3.82 (1H, m), 2.36 (3H, s), 2.27-2.11 (5H, m), 2.00-1.91 (2H, m). | 311 |
| 32 | 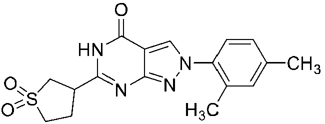 | racemate | ¹H-NMR (DMSO-D₆) δ: 11.91 (1H, s), 8.78 (1H, s), 7.33 (1H, d, J = 8.0 Hz), 7.26 (1H, s), 7.19 (1H, d, J = 8.3 Hz), 3.67 (1H, dd, J = 15.5, 8.6 Hz), 3.53-3.45 (2H, m), 3.38-3.34 (1H, m), 3.20-3.13 (1H, m), 2.58-2.55 (1H, m), 2.39-2.35 (4H, m), 2.17 (3H, s) . | 359 |
| 33 | 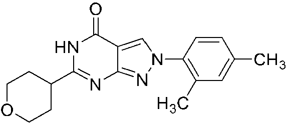 | | ¹H-NMR (DMSO-D₆) δ: 11.71 (1H, s), 8.73 (1H, s), 7.32 (1H, d, J = 8.1 Hz), 7.25 (1H, s), 7.18 (1H, d, J = 7.7 Hz), 3.97-3.94 (2H, m), 3.40-3.36 (2H, m), 2.86-2.82 (1H, m), 2.36 (3H, s), 2.18 (3H, s), 1.86-1.74 (4H, m). | 325 |
| 34 | 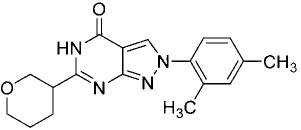 | racemate | ¹H-NMR (DMSO-D₆) δ: 11.74 (1H, s), 8.73 (1H, s), 7.32 (1H, d, J = 8.0 Hz), 7.25 (1H, s), 7.18 (1H, d, J = 7.7 Hz), 4.05-4.03 (1H, m), 3.87-3.84 (1H, br m), 3.52 (1H, t, J = 10.8 Hz), 3.37-3.32 (1H, m), 2.87-2.81 (1H, m), 2.36 (3H, s), 2.17 (3H, s), 2.09-2.05 (1H, m), 1.85-1.80 (1H, m), 1.70-1.57 (2H, m). | 325 |
| 35 | 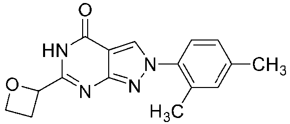 | racemate | ¹H-NMR (DMSO-D₆) δ: 11.78 (1H, s), 8.79 (1H, s), 7.35 (1H, d, J = 8.0 Hz), 7.26 (1H, s), 7.20 (1H, d, J = 8.0 Hz), 5.53 (1H, t, J = 7.5 Hz), 4.68 (2H, t, J = 7.7 Hz), 2.94 (2H, q, J = 7.7 Hz), 2.37 (3H, s), 2.18 (3H, s). | 297 |
| 36 | 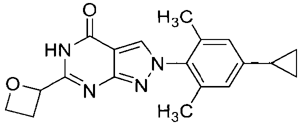 | racemate | ¹H-NMR (DMSO-D₆) δ: 11.78 (1H, s), 8.69 (1H, s), 6.96 (2H, s), 5.53 (1H, dd, J = 8.2, 6.9 Hz), 4.68 (2H, t, J = 7.7 Hz), 2.96-2.94 (2H, m), 1.96-1.93 (7H, m), 1.00-0.98 (2H, m), 0.74-0.73 (2H, m). | 337 |
| 37 | 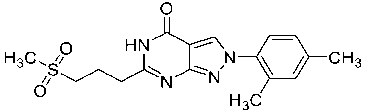 | | ¹H-NMR (DMSO-D₆) δ: 11.77 (1H, s), 8.73 (1H, s), 7.33 (1H, d, J = 8.0 Hz), 7.25 (1H, s), 7.19 (1H, d, J = 8.0 Hz), 3.25-3.21 (2H, m), 2.99 (3H, s), 2.76 (2H, t, J = 7.4 Hz), 2.36 (3H, s), 2.20-2.13 (5H, m) . | 361 |
| 38 | 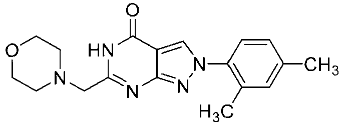 | | ¹H-NMR (DMSO-D₆) δ: 11.45 (1H, s), 8.75 (1H, s), 7.33 (1H, d, J = 8.0 Hz), 7.25 (1H, s), 7.19 (1H, d, J = 8.0 Hz), 3.61 (4H, t, J = 4.6 Hz), 3.43 (2H, s), 2.36 (3H, s), 2.17 (3H, s) . | 340 |
| 39 | 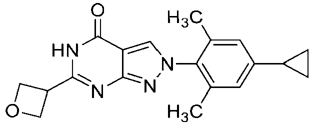 | | ¹H-NMR (DMSO-D₆) δ: 11.73 (1H, s), 8.66 (1H, s), 6.96 (2H, s), 4.87 (2H, t, J = 6.2 Hz), 4.82-4.80 (2H, m), 4.25-4.21 (1H, m), 1.96-1.91 (7H, m), 1.00-0.98 (2H, m), 0.75-0.73 (2H, m) . | 337 |
| 40 | 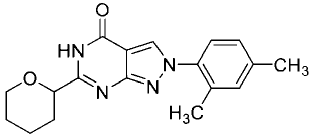 | racemate | ¹H-NMR (DMSO-D₆) δ: 11.39 (1H, s), 8.76 (1H, s), 7.34 (1H, d, J = 8.0 Hz), 7.25 (1H, s), 7.19 (1H, d, J = 8.0 Hz), 4.30 (1H, dd, J = 10.8, 2.4 Hz), 4.00-3.99 (1H, m), 3.57-3.51 (1H, m), 2.36 (3H, s), 2.17 (3H, s), 1.88-1.77 (3H, m), 1.61-1.56 (3H, m) . | 325 |
| 41 | 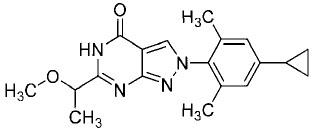 | racemate | ¹H-NMR (DMSO-D₆) δ: 11.64 (1H, s), 8.67 (1H, s), 6.96 (2H, s), 4.26 (1H, q, J = 6.6 Hz), 3.28 (3H, s), 1.96-1.92 (7H, m), 1.44 (3H, d, J = 6.6 Hz), 1.00-0.97 (2H, m), 0.75-0.72 (2H, m) . | 339 |
| 42 | 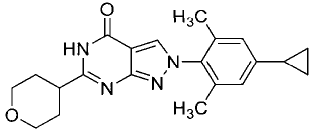 | | ¹H-NMR (DMSO-D₆) δ: 11.72 (1H, s), 8.62 (1H, d, J = 1.2 Hz), 6.95 (2H, s), 3.96-3.94 (2H, m), 3.39-3.36 (2H, m), 2.87-2.80 (1H, m), 1.97-1.91 (7H, m), 1.83-1.78 (4H, m), 0.99-0.97 (2H, m), 0.74-0.72 (2H, m). | 365 |
| 43 | 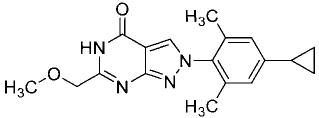 | | ¹H-NMR (DMSO-D₆) δ: 11.73 (1H, s), 8.67 (1H, s), 6.96 (2H, s), 4.32 (2H, s), 3.38 (3H, s), 1.97-1.91 (7H, m), 1.00-0.97 (2H, m), 0.74-0.72 (2H, m) . | 325 |
| 44 | 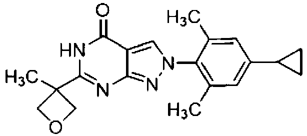 | | ¹H-NMR (DMSO-D₆) δ: 6.96 (2H, s), 4.99 (2H, d, J = 5.9 Hz), 4.43 (2H, d, J = 6.1 Hz), 1.98-1.91 (7H, m), 1.68 (2H, s), 1.00-0.97 (2H, m), 0.75-0.73 (2H, m) . | 351 |
| 45 | 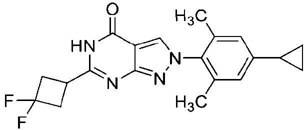 | | ¹H-NMR (DMSO-D₆) δ: 11.86 (1H, s), 8.65 (1H, s), 6.96 (2H, s), 3.38-3.36 (1H, m), 3.07-3.01 (2H, m), 2.96-2.88 (2H, m), 1.97-1.93 (7H, m), 1.01-0.96 (2H, m), 0.75-0.73 (2H, m). | 371 |
| 46 | 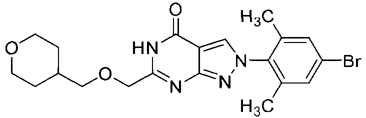 | | ¹H-NMR (DMSO-D₆) δ: 11.74 (1H, s), 8.75 (1H, s), 7.54 (2H, s), 4.36 (2H, s), 3.87-3.80 (2H, m), 3.39 (2H, d, J = 6.5 Hz), 3.31-3.24 (2H, m), 1.96 (6H, s), 1.90-1.79 (1H, m), 1.64-1.56 (2H, m), 1.32-1.09 (2H, m) . | 447 |
| 47 | 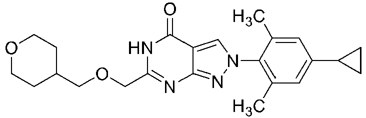 | | ¹H-NMR (DMSO-D₆) δ: 11.70 (1H, s), 8.67 (1H, s), 6.96 (2H, s), 4.36 (2H, s), 3.86-3.80 (2H, m), 3.39 (2H, d, J = 6.5 Hz), 3.30-3.23 (2H, m), 1.91 (6H, s), 1.89-1.80 (1H, m), 1.64-1.56 (2H, m), 1.32-1.09 (3H, m), 1.02-0.96 (2H, m), 0.76-0.71 (2H, m) . | 409 |
| 48 | 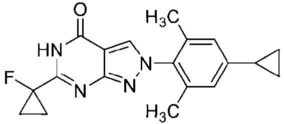 | | ¹H-NMR (DMSO-D₆) δ: 12.05 (1H, s), 8.68 (1H, s), 6.95 (2H, s), 1.95-1.92 (7H, m), 1.56-1.48 (4H, m), 0.99-0.97 (2H, m), 0.74-0.72 (2H, m). | 339 |
| 49 | 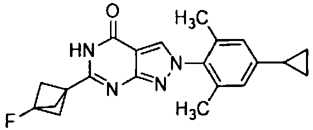 | | ¹H-NMR (DMSO-D₆) δ: 11.88 (1H, s), 8.65 (1H, s), 6.95 (2H, s), 2.53-2.51 (6H, m), 1.96-1.90 (7H, m), 0.99-0.97 (2H, m), 0.74-0.72 (2H, m) . | 365 |
| 50 | 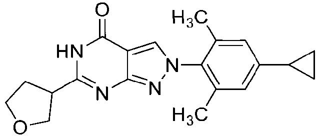 | optically active substance (enantiomer of Example 51) | ¹H-NMR (DMSO-D₆) δ: 11.80 (1H, s), 8.63 (1H, s), 6.95 (2H, s), 4.02 (1H, t, J = 8.0 Hz), 3.91-3.85 (2H, m), 3.79-3.75 (1H, m), 3.44-3.37 (1H, m), 2.35-2.18 (2H, m), 1.96-1.93 (7H, m), 0.99-0.97 (2H, m), 0.74-0.72 (2H, m) . | 351 |
| 51 | 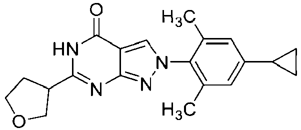 | optically active substance (enantiomer of Example 50) | ¹H-NMR (DMSO-D₆) δ: 11.80 (1H, s), 8.63 (1H, s), 6.95 (2H, s), 4.02 (1H, t, J = 8.1 Hz), 3.89-3.86 (2H, m), 3.79-3.75 (1H, m), 3.44-3.37 (1H, m), 2.29-2.24 (2H, m), 1.97-1.91 (7H, m), 0.99-0.97 (2H, m), 0.74-0.72 (2H, m). | 351 |
| 52 | 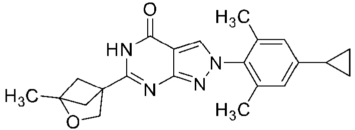 | | ¹H-NMR (DMSO-D₆) δ: 11.78 (1H, s), 8.65 (1H, s), 6.96 (2H, s), 3.96 (2H, s), 2.23 (2H, dd, J = 4.3, 1.5 Hz), 1.96-1.91 (7H, m), 1.86 (2H, dd, J = 4.5, 1.7 Hz), 1.40 (3H, s), 0.99-0.97 (2H, m), 0.74-0.72 (2H, m). | 377 |
| 53 | 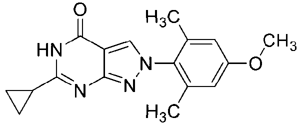 | | ¹H-NMR (DMSO-D₆) δ: 8.56 (1H, s), 6.81 (2H, s), 3.79 (3H, s), 1.93-1.91 (7H, m), 1.08-1.07 (2H, m), 1.03-1.01 (2H, m) . | 311 |
| 54 | 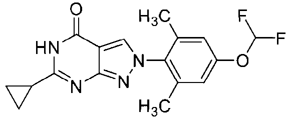 | | ¹H-NMR (DMSO-D₆) δ: 12.02 (1H, s), 8.64 (1H, s), 7.31 (1H, t, J = 73.8 Hz), 7.10 (2H, s), 1.98-1.97 (7H, m), 1.09-1.00 (4H, m) . | 347 |
| 55 | 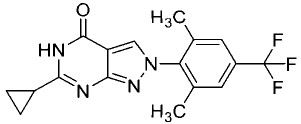 | | ¹H-NMR (DMSO-D₆) δ: 12.04 (1H, br s), 8.71 (1H, s), 7.67 (2H, s), 2.03 (6H, s), 1.97-1.95 (1H, m), 1.08-1.07 (2H, m), 1.03-1.00 (2H, m) . | 349 |
| 56 | 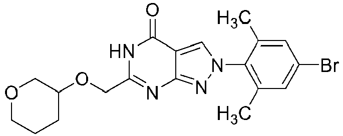 | racemate | ¹H-NMR (DMSO-D₆) δ: 11.71 (1H, s), 8.75 (1H, s), 7.54 (2H, s), 4.44-4.40 (2H, m), 3.81-3.78 (1H, m), 3.62-3.51 (2H, m), 3.42-3.32 (2H, m), 1.96 (6H, s), 1.73-1.71 (1H, m), 1.58-1.54 (1H, m), 1.47-1.40 (1H, m) . | 433 |
| 57 | 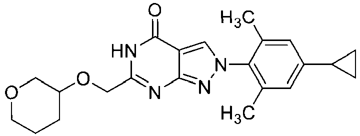 | racemate | ¹H-NMR (DMSO-D₆) δ: 11.66 (1H, s), 8.66 (1H, s), 6.96 (2H, s), 4.43-4.39 (2H, m), 3.81-3.78 (1H, m), 3.63-3.57 (1H, m), 3.55-3.51 (1H, m), 3.41-3.35 (2H, m), 1.97-1.91 (8H, m), 1.73-1.71 (1H, m), 1.58-1.53 (1H, m), 1.47-1.41 (1H, m), 1.01-0.96 (2H, m), 0.74-0.72 (2H, m) . | 395 |
| 58 | 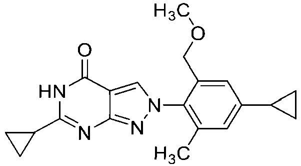 | | ¹H-NMR (CDCl₃) δ: 9.72 (br s, 1H), 8.11 (s, 1H), 7.09-7.07 (m, 1H), 6.97-6.95 (m, 1H), 4.07 (br s, 2H), 3.24 (s, 3H), 2.02 (s, 3H), 1.97-1.89 (m, 1H), 1.83-1.76 (m, 1H), 1.40-1.35 (m, 2H), 1.14-1.08 (m, 2H), 1.05-0.99 (m, 2H), 0.79-0.74 (m, 2H). | 351 |
| 59 | 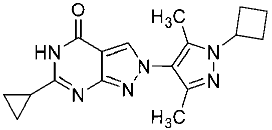 | | ¹H-NMR (DMSO-D₆) δ: 11.96 (1H, s), 8.58 (1H, s), 4.86-4.77 (1H, m), 2.56-2.51 (2H, m), 2.33 (2H, br s), 2.16 (3H, s), 2.12 (3H, s), 1.96-1.94 (1H, br m), 1.81-1.79 (2H, br m), 1.07-1.00 (4H, m) . | 325 |
| 60 | 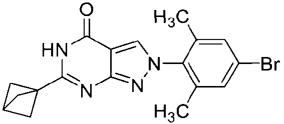 | | ¹H-NMR (DMSO-D₆) δ: 11.74 (1H, s), 8.70 (1H, s), 7.53 (2H, s), 2.19 (6H, s), 1.95 (6H, s). | 385 |
| 61 | 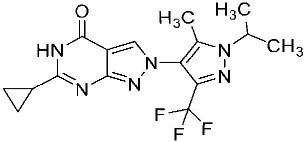 | | 1H-NMR (DMSO-D₆) δ: 12.05 (1H, s), 8.67 (1H, s), 4.76-4.70 (1H, m), 2.23 (3H, s), 1.97 (1H, t, J = 8.3Hz), 1.45 (6H, d, J = 6.5 Hz), 1.09-1.01 (4H, m) . | 367 |
| 62 | 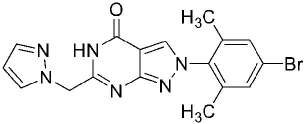 | | ¹H-NMR (DMSO-D₆) δ: 12.06 (1H, br s), 8.74 (1H, s), 7.87 (1H, d, J = 2.3 Hz), 7.51 (2H, s), 7.47 (1H, dd, J = 1.8, 0.7 Hz), 6.29 (1H, t, J = 2.1 Hz), 5.29 (2H, s), 1.92 (6H, s). | 399 |
| 63 | 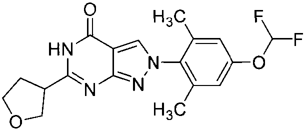 | racemate | ¹H-NMR (DMSO-D₆) δ: 8.67 (1H, s), 7.32 (1H, t, J = 73.9 Hz), 7.11 (2H, s), 4.03-4.01 (1H, m), 3.91-3.85 (2H, m), 3.78-3.76 (1H, m), 3.41-3.39 (1H, m), 2.34-2.24 (2H, m), 1.98 (6H, s). | 377 |
| 64 | 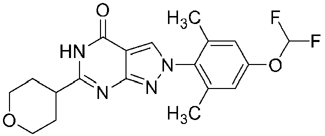 | | ¹H-NMR (DMSO-D₆) δ: 11.74 (1H, s), 8.68 (1H, s), 7.32 (1H, t, J = 73.9 Hz), 7.11 (2H, s), 3.96-3.94 (2H, m), 3.40-3.35 (2H, m), 2.87-2.81 (1H, m), 1.98 (6H, s), 1.82-1.79 (4H, br m). | 391 |
| 65 | 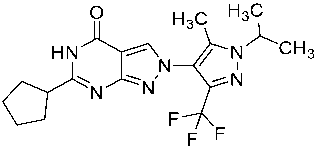 | | ¹H-NMR (DMSO-D₆) δ: 11.73 (1H, s), 8.70 (1H, s), 4.77-4.71 (1H, m), 3.06-2.98 (1H, m), 2.24 (3H, s), 2.00-1.95 (2H, m), 1.89-1.84 (2H, m), 1.76-1.71 (2H, m), 1.62-1.59 (2H, m), 1.45 (6H, d, J = 6.7 Hz) . | 395 |
| 66 | 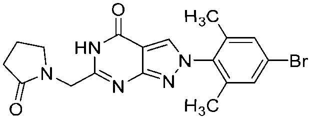 | | ¹H-NMR (DMSO-D₆) δ: 11.84 (1H, br s), 8.71 (1H, s), 7.52 (2H, s), 4.34 (2H, s), 3.52 (2H, t, J = 7.2 Hz), 2.28 (2H, t, J = 8.3 Hz), 1.99 (2H, t, J = 8.0 Hz), 1.94 (6H, s) . | 416 |
| 67 | 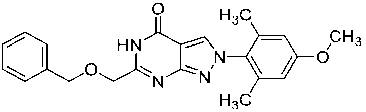 | | ¹H-NMR (DMSO-D₆) δ: 11.79 (1H, s), 8.65 (1H, s), 7.42-7.28 (5H, m), 6.83 (2H, s), 4.64 (2H, s), 4.44 (2H, s), 3.80 (3H, s), 1.93 (6H, s). | 391 |
| 68 | 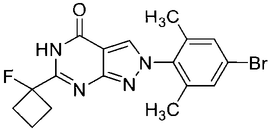 | | ¹H-NMR (DMSO-D₆) δ: 12.00 (1H, s), 8.77 (1H, s), 7.55 (2H, s), 2.83-2.77 (2H, m), 1.98-1.88 (7H, m), 1.70-1.66 (1H, m) . | 391 |
| 69 | 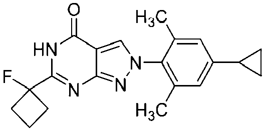 | | ¹H-NMR (CDCl₃) δ: 11.99 (1H, s), 8.71 (1H, s), 6.97 (2H, s), 2.84-2.76 (2H, m), 2.54-2.50 (2H, m), 1.99-1.91 (8H, m), 1.71-1.66 (1H, m), 1.00-0.98 (2H, m), 0.75-0.73 (2H, m) . | 353 |
| 70 | 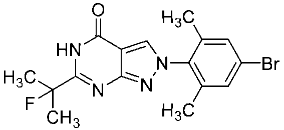 | | ¹H-NMR (DMSO-D₆) δ: 11.82 (1H, br s), 8.76 (1H, s), 7.53 (2H, s), 1.95 (6H, s), 1.71 (6H, d, J = 22.0 Hz). | 379 |
| 71 | 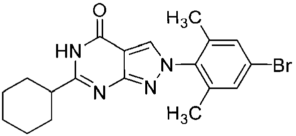 | | ¹H-NMR (DMSO-D₆) δ: 11.67 (1H, s), 8.68 (1H, s), 7.53 (2H, s), 2.59-2.56 (1H, m), 1.95-1.92 (8H, m), 1.81-1.78 (2H, br m), 1.70-1.67 (1H, br m), 1.58-1.52 (2H, m), 1.32-1.20 (3H, br m). | 401 |
| 72 | 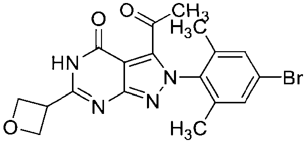 | | ¹H-NMR (DMSO-D₆) δ: 12.19 (1H, s), 7.49 (2H, s), 4.89-4.81 (4H, m), 4.30-4.25 (1H, m), 2.89 (3H, s), 1.85 (6H, s). | 417 |
| 73 | 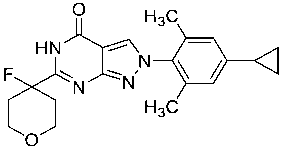 | | ¹H-NMR (DMSO-D₆) δ: 11.89 (1H, s), 8.71 (1H, s), 6.96 (2H, s), 3.90-3.87 (2H, m), 3.67-3.65 (2H, br m), 2.33-2.16 (2H, m), 2.08 (2H, t, J = 11.9 Hz), 1.97-1.94 (7H, m), 1.00-0.97 (2H, m), 0.75-0.73 (2H, m) . | 383 |
| 74 | 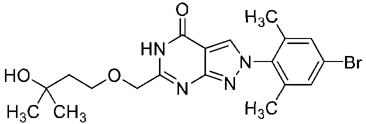 | | ¹H-NMR (DMSO-D₆) δ: 11.73 (1H, br s), 8.73 (1H, s), 7.54 (2H, s), 4.35 (2H, s), 4.32 (1H, br s), 3.65 (2H, t, J = 7.0 Hz), 1.96 (6H, s), 1.70 (2H, t, J = 7.0 Hz), 1.11 (6H, s). | 435 |
| 75 | 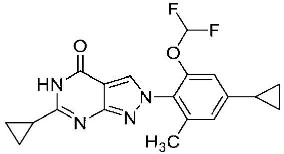 | | ¹H-NMR (CDCl₃) δ: 9.89 (br s, 1H), 8.11 (s, 1H), 6.89 (s, 1H), 6.85 (s, 1H), 6.35 (t, 1H, J = 73.5 Hz), 2.10 (s, 3H), 1.97-1.89 (m, 1H), 1.84-1.77 (m, 1H), 1.40-1.34 (m, 2H), 1.14-1.04 (m, 4H), 0.79-0.74 (m, 2H). | 373 |
| 76 | 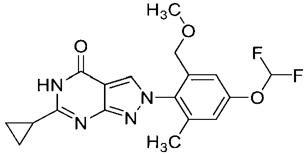 | | ¹H-NMR (CDCl₃) δ: 9.99 (br s, 1H), 8.12 (s, 1H), 7.19 (d, 1H, J = 2.1 Hz), 7.04 (d, 1H, J = 2.1 Hz), 6.59 (t, 1H, J = 73.3 Hz), 4.11 (br s, 2H), 3.28 (s, 3H), 2.07 (s, 3H), 1.87-1.79 (m, 1H), 1.42-1.36 (m, 2H), 1.16-1.09 (m, 2H) . | 377 |
| 77 | 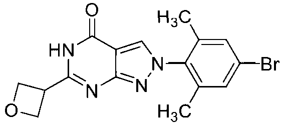 | | ¹H-NMR (DMSO-D₆) δ: 11.78 (1H, s), 8.73 (1H, s), 7.55 (2H, s), 4.88-4.79 (4H, m), 4.27-4.20 (1H, m), 1.98 (6H, s) . | 375 |
| 78 | 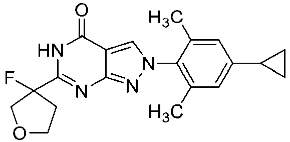 | optically active substance (enantiomer of Example 79) | ¹H-NMR (DMSO-D₆) δ: 12.07 (1H, s), 8.72 (1H, s), 6.96 (2H, s), 4.28-3.97 (4H, m), 2.80-2.54 (2H, m), 1.97-1.92 (7H, m), 1.00-0.97 (2H, m), 0.75-0.72 (2H, m) . | 369 |
| 79 | 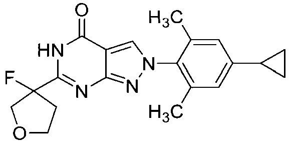 | optically active substance (enantiomer of Example 78) | ¹H-NMR (DMSO-D₆) δ: 12.07 (1H, s), 8.72 (1H, s), 6.96 (2H, s), 4.28-3.97 (4H, m), 2.72-2.61 (2H, m), 1.96-1.91 (7H, m), 1.02-0.96 (2H, m), 0.76-0.72 (2H, m) . | 369 |
| 80 | 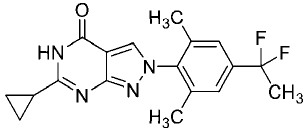 | | ¹H-NMR (DMSO-D₆) δ: 12.03 (1H, s), 8.68 (1H, s), 7.47 (2H, s), 2.03-1.97 (10H, m), 1.10-1.08 (2H, m), 1.03-1.01 (2H, m). | 345 |
| 81 | 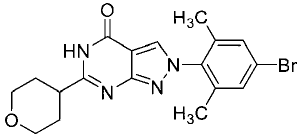 | | ¹H-NMR (DMSO-D₆) δ: 11.75 (1H, s), 8.70 (1H, s), 7.54 (2H, s), 3.96-3.95 (2H, m), 3.39-3.36 (2H, m), 2.87-2.81 (1H, m), 1.96 (6H, s), 1.83-1.78 (4H, m) . | 403 |
| 82 | 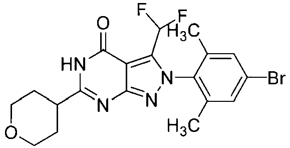 | | ¹H-NMR (DMSO-D₆) δ: 12.14 (1H, s), 7.56 (2H, s), 7.30 (1H, t, J = 51.9 Hz), 3.97-3.94 (2H, m), 3.41-3.35 (2H, m), 2.87-2.85 (1H, m), 1.91 (6H, s), 1.85-1.80 (4H, m). | 453 |
| 83 | 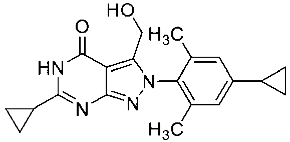 | | ¹H-NMR (DMSO-D₆) δ: 12.04 (1H, s), 6.92 (2H, s), 4.46 (2H, d, J = 4.9 Hz), 1.98-1.90 (2H, m), 1.84 (6H, s), 1.09-1.05 (2H, m), 1.01-0.97 (4H, m), 0.75-0.72 (2H, m) . (1 peak lost (OH)) | 351 |
| 84 | 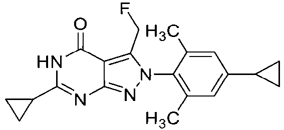 | | ¹H-NMR (DMSO-D₆) δ: 12.21 (1H, s), 6.97 (2H, s), 5.36 (2H, d, J = 48.3 Hz), 1.98-1.93 (2H, m), 1.83 (6H, s), 1.10-1.09 (2H, m), 1.02-0.99 (4H, m), 0.77-0.74 (2H, m) . | 353 |
| 85 | 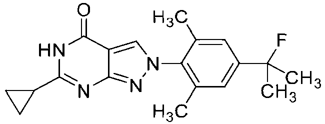 | | ¹H-NMR (DMSO-D₆) δ: 12.01 (1H, s), 8.64 (1H, s), 7.29 (2H, s), 1.98-1.96 (7H, m), 1.68 (6H, d, J = 22.2 Hz), 1.09-1.07 (2H, m), 1.02-1.01 (2H, m) . | 341 |
| 86 | 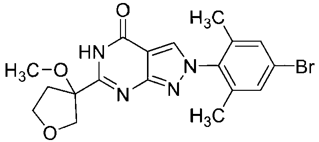 | racemate | ¹H-NMR (DMSO-D₆) δ: 11.71 (1H, s), 8.76 (1H, s), 7.54 (2H, s), 4.11 (1H, d, J = 9.7 Hz), 4.01 (1H, d, J = 9.7 Hz), 3.92-3.86 (2H, m), 3.13 (3H, s), 2.59-2.54 (1H, m), 2.40-2.36 (1H, m), 1.97 (6H, s). | 419 |
| 87 | 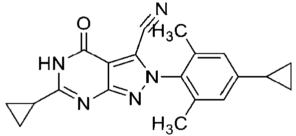 | | ¹H-NMR (DMSO-D₆) δ: 12.61 (1H, s), 7.03 (2H, s), 2.03-1.90 (8H, m), 1.13-0.99 (6H, m), 0.78 (2H, dt, J = 8.6, 3.2 Hz) . | 346 |
| 88 | 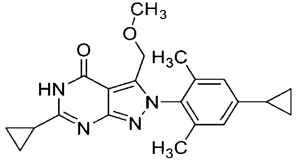 | | ¹H-NMR (DMSO-D₆) δ: 12.04 (1H, s), 6.94 (2H, s), 4.39 (2H, s), 3.15 (3H, s), 1.99-1.90 (2H, m), 1.82 (6H, s), 1.09-1.07 (2H, m), 1.02-0.96 (4H, m), 0.76-0.72 (2H, m) . | 365 |
| 89 | 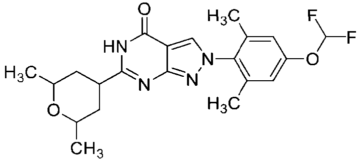 | 2RS, 6SR, 4(RS and SR) two diastereomeric mixture | ¹H-NMR (DMSO-D₆) δ: 11.71 (1H, s), 8.68 (1H, s), 7.32 (1H, t, J = 73.9 Hz), 7.11 (2H, s), 3.54-3.46 (2H, m), 2.92-2.87 (1H, m), 1.97 (6H, s), 1.89-1.87 (2H, m), 1.43-1.34 (2H, m), 1.14 (6H, d, J = 6.2 Hz). | 419 |
| 90 | 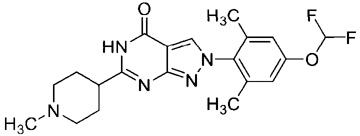 | | ¹H-NMR (DMSO-D₆) δ: 11.71 (1H, s), 8.66 (1H, s), 7.32 (1H, t, J = 73.9 Hz), 7.11 (2H, s), 2.87-2.85 (2H, m), 2.18 (3H, s), 1.97 (6H, s), 1.87-1.81 (7H, m) . | 404 |
| 91 | 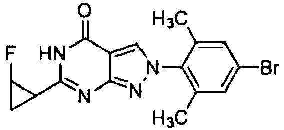 | cis-isomer, racemate | ¹H-NMR (CDCl₃) δ: 9.26 (br s, 1H), 8.08 (s, 1H), 7.35 (s, 2H), 5.10-5.04 (m, 0.5H), 4.94-4.86 (m, 0.5H), 2.25-2.16 (m, 1H), 2.10-1.97 (m, 1H), 2.03 (s, 6H), 1.48-1.36 (m, 1H). | 377 |
| 92 | 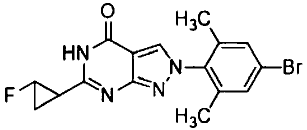 | trans-isomer, racemate | ¹H-NMR (CDCl₃) δ: 10.43 (br s, 1H), 8.08 (s, 1H), 7.35 (s, 2H), 5.19-5.15 (m, 0.5H), 5.03-4.99 (m, 0.5H), 2.35-2.24 (m, 1H), 2.02 (s, 6H), 1.83-1.63 (m, 2H) . | 377 |
| 93 | 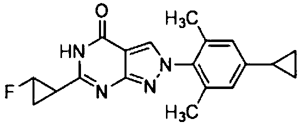 | trans-isomer, racemate | ¹H-NMR (CDCl₃) δ: 10.45 (br s, 1H), 8.07 (s, 1H), 6.85 (s, 2H), 5.20-5.15 (m, 0.5H), 5.04-4.99 (m, 0.5H) 2.36-2.25 (m, 1H), 1.99 (s, 6H),1.93-1.84 (m, 1H), 1.82-1.62 (m, 2H), 1.04-0.97 (m, 2H), 0.76-0.71 (m, 2H) . | 339 |
| 94 | 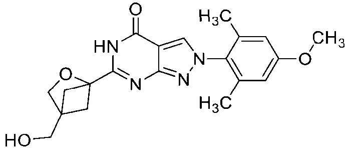 | | ¹H-NMR (DMSO-D₆) δ: 11.57 (1H, s), 8.66 (1H, s), 6.83 (2H, s), 4.77 (1H, t, J = 5.4 Hz), 3.80 (3H, s), 3.73 (4H, d, J = 5.8 Hz), 2.06 (2H, dd, J = 4.4, 1.6 Hz), 1.93 (6H, s), 1.91 (2H, dd, J = 4.5, 1.7 Hz) . | 383 |
| 95 | 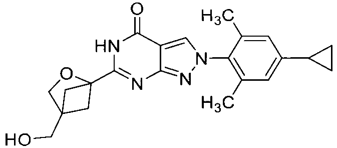 | | ¹H-NMR (DMSO-D₆) δ: 11.57 (1H, s), 8.67 (1H, s), 6.96 (2H, s), 4.77 (1H, t, J = 5.5 Hz), 3.73 (4H, d, J = 5.5 Hz), 2.06 (2H, d, J = 5.3 Hz), 1.96-1.92 (9H, m), 1.00-0.97 (2H, m), 0.76-0.73 (2H, m). | 393 |
| 96 | 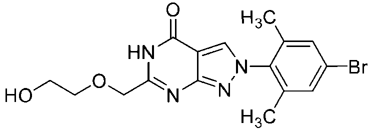 | | ¹H-NMR (DMSO-D₆) δ: 8.75 (1H, s), 7.54 (2H, s), 4.44 (2H, s), 3.60-3.57 (4H, m), 1.96 (6H, s). | 393 |
| 97 | 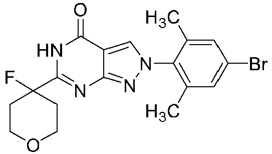 | | ¹H-NMR (DMSO-D₆) δ: 11.94 (1H, s), 8.78 (1H, s), 7.55 (2H, s), 3.90-3.87 (2H, m), 3.67-3.65 (2H, m), 2.33-2.05 (4H, m), 1.97 (6H, s). | 421 |
| 98 | 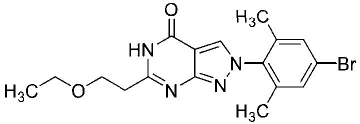 | | ¹H-NMR (DMSO-D₆) δ: 11.75 (1H, s), 8.70 (1H, s), 7.54 (2H, s), 3.79 (2H, t, J = 6.7 Hz), 3.47 (2H, q, J = 7.0 Hz), 2.85 (2H, t, J = 6.7 Hz), 1.96 (6H, s), 1.10 (3H, t, J = 7.1 Hz). | 391 |
| 99 | 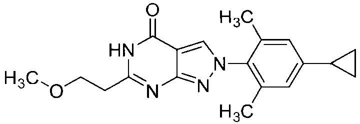 | | ¹H-NMR (DMSO-D₆) δ: 11.73 (1H, s), 8.62 (1H, s), 6.95 (2H, s), 3.75 (2H, t, J = 6.4 Hz), 3.26 (3H, s), 2.84 (2H, t, J = 6.7 Hz), 1.99-1.91 (7H, m), 1.01-0.96 (2H, m), 0.74-0.72 (2H, m) . | 339 |
| 100 | 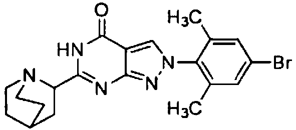 | racemate | ¹H-NMR (DMSO-D₆) δ: 8.77 (1H, s), 7.54 (2H, s), 5.51 (1H, s), 2.52 (1H, s), 2.19-1.23 (17H, m) . | 428 |
| 101 | 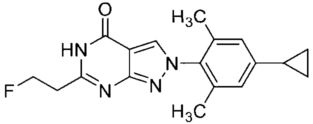 | | ¹H-NMR (DMSO-D₆) δ: 11.85 (1H, s), 8.64 (1H, s), 6.96 (2H, s), 4.87 (2H, dt, J = 47.0, 6.1 Hz), 3.04 (2H, dt, J = 25.0, 6.0 Hz), 1.96-1.92 (7H, m), 0.99-0.97 (2H, m), 0.74-0.72 (2H, m) . | 327 |
| 102 | 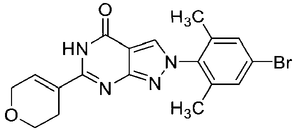 | | ¹H-NMR (DMSO-D₆) δ: 11.80 (1H, s), 8.70 (1H, s), 7.53 (2H, s), 6.53 (1H, dd, J = 6.4, 2.0 Hz), 4.91 (1H, dd, J = 6.2, 3.8 Hz), 4.17-4.12 (1H, m), 4.03-3.98 (1H, m), 3.44-3.44 (1H, m), 2.24-2.21 (1H, m), 2.08-2.02 (1H, m), 1.96 (6H, s). | 401 |
| 103 | 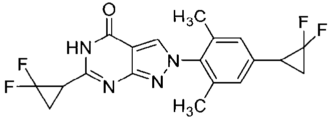 | stereoisomeric mixture | ¹H-NMR (CDCl₃) δ: 10.17 (br s, 1H), 8.12 (s, 1H), 7.04 (s, 2H), 2.84-2.71 (m, 2H), 2.63-2.53 (m, 1H), 2.04 (s, 6H), 2.00-1.82 (m, 2H), 1.72-1.62 (m, 1H). | 393 |
| 104 | 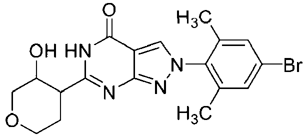 | racemate, not determined for relative configuration (single diastereomer) | ¹H-NMR (DMSO-D₆) δ: 8.70 (1H, s), 7.54 (2H, s), 3.89-3.82 (3H, m), 3.28-3.24 (1H, m), 3.04-2.98 (1H, m), 2.72-2.66 (1H, m), 1.97 (7H, s), 1.86-1.81 (2H, m) . | 419 |
| 105 | 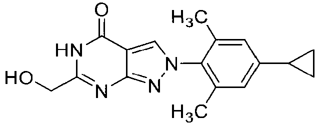 | | ¹H-NMR (DMSO-D₆) δ: 11.44 (1H, s), 8.65 (1H, s), 6.96 (2H, s), 5.57 (1H, s), 4.37 (2H, s), 1.96-1.93 (7H, m), 1.00-0.97 (2H, m), 0.74-0.72 (2H, m). | 311 |
| 106 | 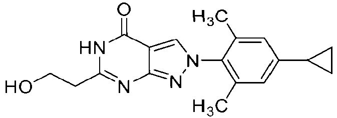 | | ¹H-NMR (DMSO-D₆) δ: 11.66 (1H, s), 8.60 (1H, s), 6.95 (2H, s), 3.82 (2H, t, J = 6.5 Hz), 2.75 (2H, t, J = 6.5 Hz), 1.95-1.92 (7H, m), 1.00-0.97 (2H, m), 0.74-0.72 (2H, m). (1 peak lost (OH)). | 325 |
| 107 | 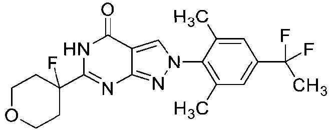 | | ¹H-NMR (DMSO-D₆) δ: 11.94 (1H, s), 8.81 (1H, s), 7.50 (2H, s), 3.90-3.87 (2H, m), 3.66 (2H, td, J = 11.7, 2.1 Hz), 2.34-2.17 (2H, m), 2.10 (2H, d, J = 12.5 Hz), 2.04-1.99 (9H, m) . | 407 |
| 108 | 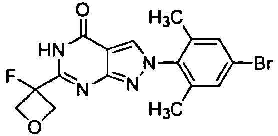 | | ¹H-NMR (DMSO-D₆) δ: 12.25 (1H, s), 8.81 (1H, s), 7.56 (2H, s), 5.15 (2H, ddd, J = 22.9, 8.4, 1.3 Hz), 4.86 (2H, ddd, J = 22.0, 8.6, 1.3 Hz), 1.98 (6H, s). | 393 |
| 109 | 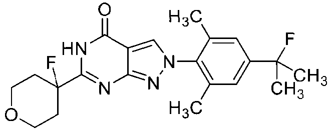 | | ¹H-NMR (DMSO-D₆) δ: 11.92 (1H, s), 8.77 (1H, s), 7.32 (2H, s), 3.88 (2H, dd, J = 11.4, 3.4 Hz), 3.66 (2H, dd, J = 11.8, 9.9 Hz), 2.34-2.17 (2H, m), 2.09 (2H, t, J = 12.0 Hz), 1.99 (6H, s), 1.69 (6H, d, J = 22.2 Hz). | 403 |
| 110 | 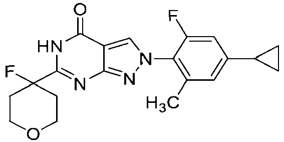 | | ¹H-NMR (DMSO-D₆) δ: 11.91 (1H, s), 8.83 (1H, s), 7.03-7.02 (2H, m), 3.88-3.85 (2H, m), 3.69-3.62 (2H, m), 2.32-1.91 (8H, m), 1.03-1.01 (2H, m), 0.80-0.77 (2H, m) . | 387 |
| 111 | 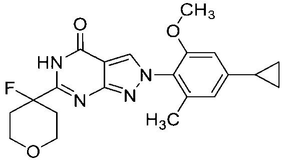 | | ¹H-NMR (DMSO-D₆) δ: 11.79 (1H, br s), 8.58 (1H, s), 6.76 (1H, d, J = 1.6 Hz), 6.69 (1H, d, J = 1.6 Hz), 3.88-3.85 (2H, m), 3.69-3.62 (5H, m), 2.32-1.93 (8H, m), 1.03-0.96 (2H, m), 0.79-0.77 (2H, m). | 399 |
| 112 | 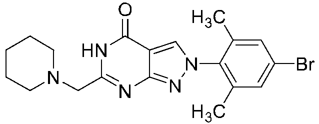 | | ¹H-NMR (DMSO-D₆) δ: 11.26 (1H, br s), 8.71 (1H, s), 7.52 (2H, s), 3.38 (2H, s), 2.46-2.45 (4H, m), 1.95 (6H, s), 1.55-1.49 (4H, m), 1.38-1.35 (2H, m) . | 416 |
| 113 | 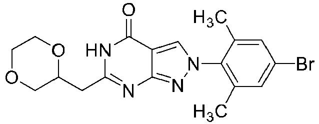 | racemate | ¹H-NMR (DMSO-D₆) δ: 11.77 (br s, 1H), 8.71 (s, 1H), 7.54 (s, 2H), 3.85-3.79 (m, 1H), 3.75-3.44 (m, 4H), 3.28 (t, 2H, J = 10.9 Hz), 2.70 (t, 2H, J = 6.4 Hz), 1.96 (s, 6H). | 419 |
| 114 | 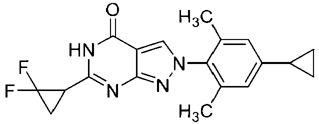 | racemate | ¹H-NMR (CDCl₃) δ: 10.15 (br s, 1H), 8.10 (s, 1H), 6.85 (s, 2H), 2.83-2.75 (m, 1H), 2.63-2.53 (m, 1H), 2.00 (s, 6H), 1.98-1.84 (m, 2H), 1.05-0.98 (m, 2H), 0.77-0.71 (m, 2H) . | 357 |
| 115 | 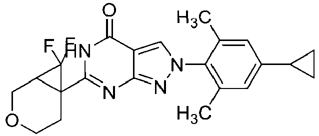 | racemate | ¹H-NMR (DMSO-D₆) δ: 12.21 (1H, s), 8.69 (1H, s), 6.96 (2H, s), 4.00-3.96 (2H, m), 3.56-3.50 (1H, m), 3.34-3.32 (1H, m), 2.61-2.59 (1H, m), 2.37-2.29 (1H, m), 2.02-2.00 (1H, m), 1.95-1.92 (7H, m), 1.00-0.97 (2H, m), 0.74-0.72 (2H, m). | 413 |
| 116 | 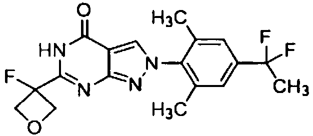 | | ¹H-NMR (DMSO-D₆) δ: 12.26 (1H, s), 8.86 (1H, s), 7.51 (2H, s), 5.16 (2H, dd, J = 23.4, 9.0 Hz), 4.87 (2H, dd, J = 22.0, 8.6 Hz), 2.05-1.99 (9H, m) . | 379 |
| 117 | 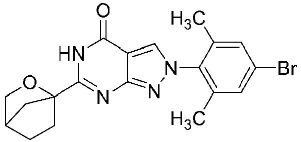 | racemate | ¹H-NMR (DMSO-D₆) δ: 11.37 (1H, s), 8.75 (1H, s), 7.54 (2H, s), 3.95-3.93 (1H, m), 3.68 (1H, d, J = 6.5 Hz), 2.65 (1H, s), 2.25 (1H, td, J = 12.7, 4.6 Hz), 2.07 (1H, d, J = 9.5 Hz), 1.97 (6H, s), 1.90-1.80 (3H, m), 1.63-1.60 (1H, m) . | 415 |
| 118 | 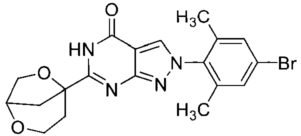 | racemate | ¹H-NMR (DMSO-D₆) δ: 11.51 (1H, s), 8.75 (1H, s), 7.54 (2H, s), 4.58 (1H, s), 4.22 (1H, dd, J = 9.5, 3.0 Hz), 4.18 (1H, d, J = 9.5 Hz), 3.90-3.87 (2H, m), 2.22-2.12 (2H, m), 2.01 (1H, d, J = 11.1 Hz), 1.97 (6H, s), 1.84 (1H, d, J = 14.1 Hz) . | 431 |
| 119 | 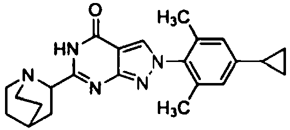 | racemate | ¹H-NMR (DMSO-D₆) δ: 10.91 (1H, br s), 8.64 (1H, s), 6.94 (2H, s), 3.90-3.87 (1H, m), 3.31-3.28 (6H, m), 3.08-2.88 (2H, m), 2.25-2.22 (1H, m), 1.96-1.46 (9H, m), 0.98-0.96 (2H, m), 0.73-0.71 (2H, m). | 390 |
| 120 | 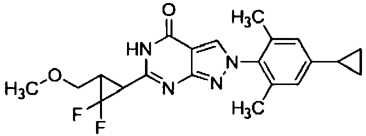 | trans-isomer, racemate | ¹H-NMR (CDCl₃) δ: 9.59 (br s, 1H), 8.08 (s, 1H), 6.85 (s, 2H), 3.74-3.62 (m, 2H), 3.41 (s, 3H), 3.12-3.02 (m, 1H), 2.65-2.59 (m, 1H), 2.00 (s, 6H), 1.93-1.85 (m, 1H), 1.04-0.98 (m, 2H), 0.76-0.71 (m, 2H) . | 401 |
| 121 | 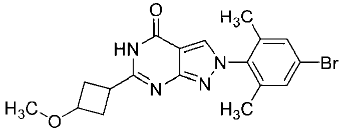 | not determined for relative configuration (diastereomer of Example 122) | ¹H-NMR (DMSO-D₆) δ: 11.71 (1H, s), 8.69 (1H, s), 7.54 (2H, s), 4.07-4.04 (1H, m), 3.46-3.38 (1H, m), 3.16 (3H, s), 2.60-2.58 (2H, m), 2.27-2.20 (2H, m), 1.97 (6H, s). | 403 |
| 122 | 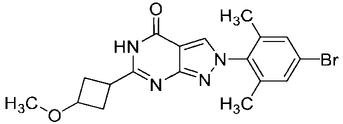 | not determined for relative configuration (diastereomer of Example 121) | ¹H-NMR (DMSO-D₆) δ: 11.73 (1H, s), 8.69 (1H, s), 7.54 (2H, s), 3.89-3.82 (1H, m), 3.16 (3H, s), 3.03-2.94 (1H, m), 2.57-2.55 (2H, m), 2.23-2.19 (2H, m), 1.97 (6H, s). | 403 |
| 123 | 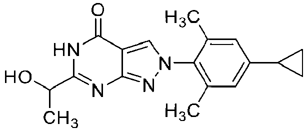 | racemate | ¹H-NMR (DMSO-D₆) δ: 11.30 (1H, s), 8.65 (1H, s), 6.96 (2H, s), 5.65 (1H, s), 4.57 (1H, q, J = 6.6 Hz), 1.94 (7H, dt, J = 10.9, 5.3 Hz), 1.43 (3H, d, J = 6.7 Hz), 0.98 (2H, ddd, J = 9.5, 5.3, 3.1 Hz), 0.73 (2H, dt, J = 8.6, 3.2 Hz) . | 325 |
| 124 | 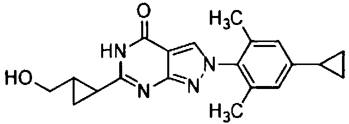 | trans-isomer, racemate | ¹H-NMR (CDCl₃) δ: 11.04 (br s, 1H), 8.10 (s, 1H), 6.84 (s, 2H), 4.00-3.92 (m, 1H), 3.39-3.30 (m, 1H), 2.83 (br s, 1H), 1.99 (s, 6H), 1.94-1.84 (m, 3H), 1.79-1.72 (m, 1H), 1.09-0.97 (m, 3H), 0.76-0.70 (m, 2H). | 351 |
| 125 | 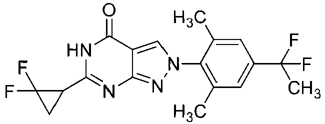 | racemate | ¹H-NMR (CDCl₃) δ: 9.55 (br s, 1H), 8.12 (s, 1H), 7.33 (s, 2H), 2.80-2.71 (m, 1H), 2.63-2.51 (m, 1H), 2.09 (s, 6H), 2.01-1.90 (m, 1H), 1.95 (t, 3H, J = 18.5 Hz). | 381 |
| 126 | 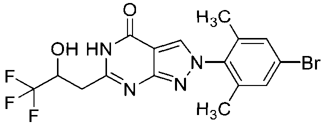 | racemate | ¹H-NMR (DMSO-D₆) δ: 11.88 (1H, br s), 8.73 (1H, s), 7.53 (2H, s), 6.54 (1H, d, J = 8.1 Hz), 4.66-4.63 (1H, m), 3.02-2.99 (1H, m), 2.84-2.81 (1H, m), 1.95 (6H, s). | 431 |
| 127 | 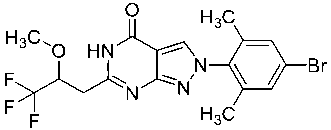 | racemate | ¹H-NMR (DMSO-D₆) δ: 11.92 (1H, br s), 8.74 (1H, s), 7.53 (2H, s), 4.59-4.56 (1H, m), 3.48 (3H, s), 3.07-3.03 (1H, m), 2.94-2.90 (1H, m), 1.95 (6H, s). | 445 |
| 128 | 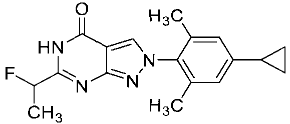 | racemate | ¹H-NMR (DMSO-D₆) δ: 12.04 (1H, s), 8.65 (1H, s), 6.96 (2H, s), 5.51 (1H, dq, J = 47.4, 6.5 Hz), 1.95-1.92 (7H, m), 1.65 (3H, dd, J = 24.5, 6.5 Hz), 0.98 (2H, ddd, J = 9.5, 5.3, 3.1 Hz), 0.74 (2H, dt, J = 8.6, 3.2 Hz) . | 327 |
| 129 | 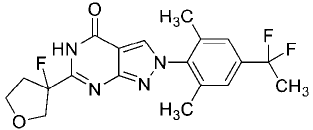 | optically active substance (enantiomer of Example 130) | ¹H-NMR (DMSO-D₆) δ: 12.13 (1H, s), 8.84 (1H, s), 7.50 (2H, s), 4.26-4.16 (2H, m), 4.03-4.00 (2H, m), 2.76-2.68 (1H, m), 2.59-2.49 (1H, m), 2.04-1.99 (9H, m) . | 393 |
| 130 | 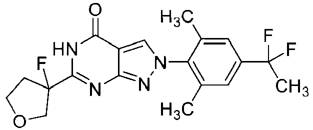 | optically active substance (enantiomer of Example 129) | ¹H-NMR (DMSO-D₆) δ: 12.13 (1H, s), 8.83 (1H, s), 7.50 (2H, s), 4.26-4.16 (2H, m), 4.06-3.98 (2H, m), 2.76-2.68 (1H, m), 2.58-2.49 (1H, m), 2.04-1.99 (9H, m). | 393 |
| 131 | 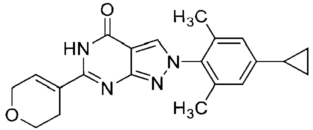 | | ¹H-NMR (DMSO-D₆) δ: 11.60 (lH, s), 8.64 (1H, s), 6.99 (1H, s), 6.96 (2H, s), 4.27-4.27 (2H, m), 3.79 (2H, t, J = 5.4 Hz), 2.54 (2H, br s), 1.96-1.92 (7H, m), 1.01-0.96 (2H, m), 0.75-0.73 (2H, m) . | 363 |
| 132 | 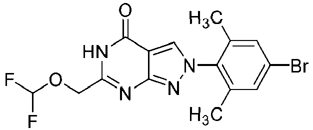 | | ¹H-NMR (DMSO-D₆) δ: 12.03 (1H, br s), 8.77 (1H, s), 7.53 (2H, s), 6.84 (1H, t, J = 75.0 Hz), 4.79 (2H, s), 1.95 (6H, s) . | 399 |
| 133 | 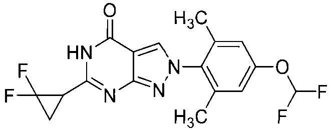 | racemate | ¹H-NMR (CDCl₃) δ: 10.29 (br s, 1H), 8.12 (s, 1H), 6.95 (s, 2H), 6.56 (t, 1H, J = 73.3 Hz), 2.85-2.75 (m, 1H), 2.65-2.54 (m, 1H), 2.06 (s, 6H), 2.01-1.89 (m, 1H) . | 383 |
| 134 | 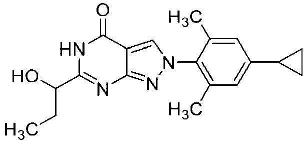 | racemate | ¹H-NMR (DMSO-D₆) δ: 11.27 (1H, s), 8.65 (1H, d, J = 0.5 Hz), 6.96 (2H, s), 5.59 (1H, s), 4.33 (1H, t, J = 6.5 Hz), 1.97-1.92 (7H, m), 1.84-1.74 (2H, m), 1.00-0.97 (2H, m), 0.92 (3H, t, J = 7.4 Hz), 0.74-0.72 (2H, m) . | 339 |
| 135 | 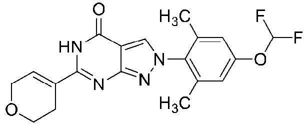 | | ¹H-NMR (DMSO-D₆) δ: 11.63 (1H, s), 8.73 (1H, s), 7.32 (1H, t, J = 73.8 Hz), 7.12 (2H, s), 7.01-7.00 (1H, br m), 4.28-4.27 (2H, m), 3.79 (2H, t, J = 5.4 Hz), 2.54 (2H, br s), 1.99 (6H, s) . | 389 |
| 136 | 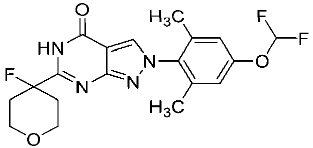 | | ¹H-NMR (DMSO-D₆) δ: 11.93 (1H, s), 8.79 (1H, s), 7.33 (1H, t, J = 73.8 Hz), 7.12 (2H, s), 3.90-3.87 (2H, br m), 3.67-3.65 (2H, br m), 2.28-2.21 (2H, m), 2.10-2.07 (2H, m), 1.98 (6H, s) . | 409 |
| 137 | 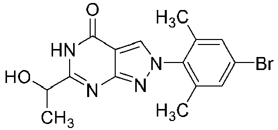 | racemate | ¹H-NMR (DMSO-D₆) δ: 11.34 (1H, s), 8.73 (1H, s), 7.54 (2H, s), 5.64 (1H, d, J = 5.5 Hz), 4.61-4.55 (1H, m), 1.96 (6H, s), 1.43 (3H, d, J = 6.5 Hz). | 363 |
| 138 | 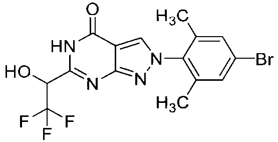 | racemate | ¹H-NMR (DMSO-D₆) δ: 11.92 (1H, s), 8.83 (1H, s), 7.55 (2H, s), 7.39 (1H, d, J = 6.7 Hz), 5.11 (1H, t, J = 5.9 Hz), 1.97 (6H, s). | 417 |
| 139 | 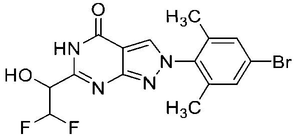 | racemate | ¹H-NMR (DMSO-D₆) δ: 11.80 (1H, s), 8.80 (1H, s), 7.55 (2H, s), 6.77 (1H, s), 6.33 (1H, td, J = 54.4, 4.8 Hz), 4.70 (1H, dt, J = 14.6, 5.5 Hz), 1.97 (6H, s). | 399 |
| 140 | 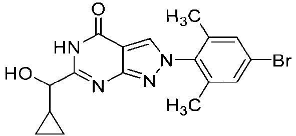 | racemate | ¹H-NMR (DMSO-D₆) δ: 11.40 (1H, s), 8.74 (1H, s), 7.54 (2H, s), 5.67 (1H, s), 3.85 (1H, d, J = 7.9 Hz), 1.97 (6H, s), 1.33 (1H, dd, J = 12.8, 5.2 Hz), 0.48-0.43 (4H, m). | 389 |
| 141 | 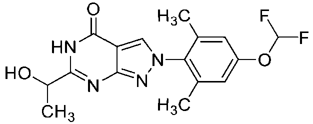 | racemate | ¹H-NMR (DMSO-D₆) δ: 11.33 (1H, s), 8.72 (1H, s), 7.32 (1H, t, J = 73.8 Hz), 7.12 (2H, s), 5.64 (1H, d, J = 5.3 Hz), 4.58 (1H, t, J = 6.1 Hz), 1.98 (6H, s), 1.43 (3H, d, J = 6.7 Hz). | 351 |
| 142 | 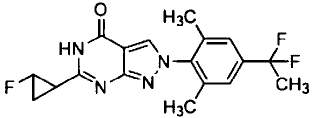 | trans-isomer, racemate | ¹H-NMR (CDCl₃) δ: 10.14 (br s, 1H), 8.10 (s, 1H), 7.32 (s, 2H), 5.20-5.16 (m, 0.5H), 5.04-5.00 (m, 0.5H), 2.33-2.22 (m, 1H), 2.08 (s, 6H), 1.95 (t, 3H, J = 18.3 Hz), 1.84-1.63 (m, 2H). | 363 |
| 143 | 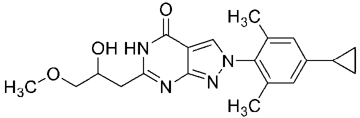 | racemate | ¹H-NMR (CDCl₃) δ: 9.73 (br s, 1H), 8.07 (s, 1H), 6.85 (s, 2H), 4.37-4.29 (m, 1H), 3.56-3.50 (m, 1H), 3.46-3.39 (m, 1H), 3.42 (s, 3H), 2.98-2.84 (m, 2H), 2.00 (s, 6H), 1.93-1.83 (m, 1H), 1.04-0.97 (m, 2H), 0.76-0.70 (m, 2H).(-OH) | 369 |
| 144 | 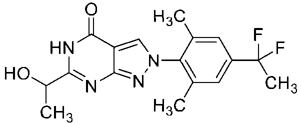 | racemate | ¹H-NMR (DMSO-D₆) δ: 11.32 (1H, s), 8.75 (1H, s), 7.49 (2H, s), 5.65 (1H, s), 4.58 (1H, dd, J = 13.6, 6.2 Hz), 2.01 (9H, dd, J = 21.4, 16.5 Hz), 1.43 (3H, d, J = 6.5 Hz) . | 349 |
| 145 | 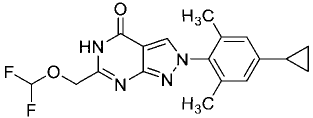 | | ¹H-NMR (DMSO-D₆) δ: 11.99 (1H, br s), 8.69 (1H, s), 6.96-6.72 (3H, m), 4.78 (2H, s), 1.97-1.90 (7H, m), 0.99-0.96 (2H, m), 0.73-0.71 (2H, m) . | 361 |
| 146 | 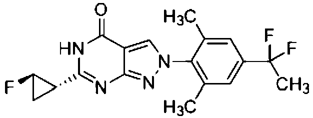 | optically active substance (1S, 2R) | ¹H-NMR (CDCl₃) δ: 10.21 (br s, 1H), 8.10 (s, 1H), 7.32 (s, 2H), 5.20-5.16 (m, 0.5H), 5.04-5.00 (m, 0.5H), 2.34-2.23 (m, 1H), 2.08 (s, 6H), 1.95 (t, 3H, J = 18.1 Hz), 1.83-1.65 (m, 2H). | 363 |
| 147 | 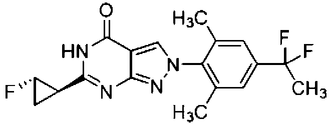 | optically active substance (1R, 2S) | ¹H-NMR (CDCl₃) δ: 10.47 (br s, 1H), 8.10 (s, 1H), 7.32 (s, 2H), 5.21-5.16 (m, 0.5H), 5.04-5.00 (m, 0.5H), 2.37-2.24 (m, 1H), 2.09 (s, 6H), 1.95 (t, 3H, J = 18.1 Hz), 1.83-1.65 (m, 2H). | 363 |
| 148 | 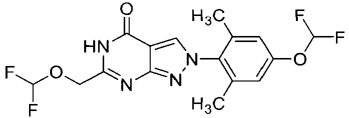 | | ¹H-NMR (DMSO-D₆) δ: 12.03 (1H, br s), 8.76 (1H, s), 7.31 (1H, t, J = 73.9 Hz), 7.11 (2H, s), 6.84 (1H, t, J = 75.0 Hz), 4.79 (2H, s), 1.96 (6H, s). | 387 |
| 149 | 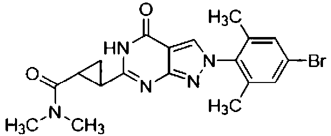 | trans-isomer, racemate | ¹H-NMR (CDCl₃) δ: 10.67 (br s, 1H), 8.06 (s, 1H), 7.34 (s, 2H), 3.25 (s, 3H), 3.09 (s, 3H), 2.82-2.76 (m, 1H), 2.75-2.68 (m, 1H), 2.02 (s, 6H), 1.81-1.75 (m, 1H), 1.63-1.56 (m, 1H). | 430 |
| 150 | 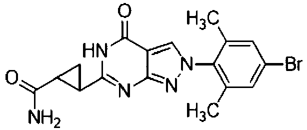 | trans-isomer, racemate | ¹H-NMR (DMSO-D₆) δ: 12.11 (br s, 1H), 8.69 (s, 1H), 7.75 (br s, 1H), 7.53 (s, 2H), 7.04 (br s, 1H), 2.36-2.30 (m, 2H), 1.95 (s, 6H), 1.46-1.39 (m, 1H), 1.38-1.32 (m, 1H). | 402 |
| 151 | 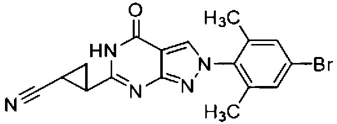 | trans-isomer, racemate | ¹H-NMR (CDCl₃) δ: 11.35 (br s, 1H), 8.14 (s, 1H), 7.36 (s, 2H), 2.61-2.55 (m, 1H), 2.43-2.37 (m, 1H), 2.03 (s, 6H), 2.01-1.93 (m, 1H), 1.77-1.71 (m, 1H). | 384 |
| 152 | 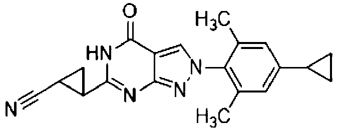 | trans-isomer, racemate | ¹H-NMR (CDCl₃) δ: 11.14 (br s, 1H), 8.12 (s, 1H), 6.86 (s, 2H), 2.59-2.53 (m, 1H), 2.44-2.38 (m, 1H), 2.03-1.85 (m, 8H), 1.76-1.69 (m, 1H), 1.05-0.98 (m, 2H), 0.76-0.71 (m, 2H). | 346 |
| 153 | 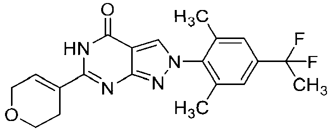 | | ¹H-NMR (DMSO-D₆) δ: 11.65 (1H, s), 8.76 (1H, s), 7.49 (2H, s), 7.01 (1H, s), 4.28-4.27 (2H, m), 3.79 (2H, t, J = 5.5 Hz), 2.54 (2H, s), 2.06-1.97 (9H, m) . | 387 |
| 154 | 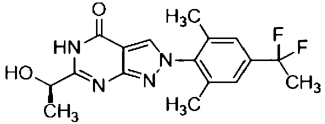 | optically active substance (R) | ¹H-NMR (DMSO-D₆) δ: 11.35 (1H, s), 8.76 (1H, s), 7.49 (2H, s), 5.64 (1H, d, J = 5.1 Hz), 4.61-4.55 (1H, m), 2.04-1.98 (9H, m), 1.44 (3H, d, J = 6.5 Hz). | 349 |
| 155 | 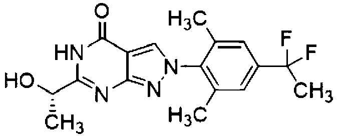 | optically active substance (S) | ¹H-NMR (DMSO-D₆) δ: 11.37 (1H, s), 8.74 (1H, s), 7.49 (2H, s), 5.67 (1H, s), 4.58 (1H, q, J = 6.6 Hz), 2.04-1.98 (9H, m), 1.43 (3H, d, J = 6.5 Hz) . | 349 |
| 156 | 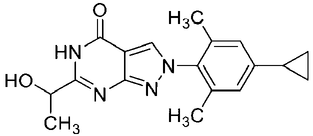 | optically active substance (enantiomer of Example 157) | ¹H-NMR (DMSO-D₆) δ: 11.23 (1H, s), 8.63 (1H, s), 6.96 (2H, s), 5.70 (1H, s), 4.57 (1H, q, J = 6.6 Hz), 1.97-1.91 (7H, m), 1.43 (3H, d, J = 6.5 Hz), 0.98 (2H, ddd, J = 9.5, 5.2, 3.1 Hz), 0.73 (2H, dt, J = 8.5, 3.2 Hz). | 325 |
| 157 | 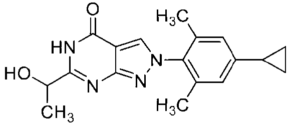 | optically active substance (enantiomer of Example 156) | ¹H-NMR (DMSO-D₆) δ: 11.26 (1H, s), 8.64 (1H, s), 6.96 (2H, s), 5.60 (1H, s), 4.57 (1H, q, J = 6.6 Hz), 1.94 (7H, dt, J = 10.8, 5.2 Hz), 1.43 (3H, d, J = 6.7 Hz), 0.98 (2H, ddd, J = 9.7, 5.3, 3.1 Hz), 0.73 (2H, dt, J = 8.5, 3.2 Hz). | 325 |
| 158 | 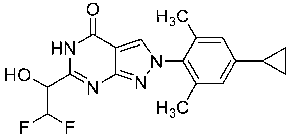 | racemate | ¹H-NMR (DMSO-D₆) δ: 11.71 (1H, s), 8.71 (1H, s), 6.96 (2H, s), 6.33 (1H, td, J = 55.0, 4.3 Hz), 4.68 (1H, td, J = 10.3, 4.3 Hz), 1.96-1.91 (7H, m), 0.99 (2H, ddd, J = 9.5, 5.3, 3.1 Hz), 0.74 (2H, dt, J = 8.6, 3.2 Hz) (1 peak lost (OH)). | 361 |
| 159 | 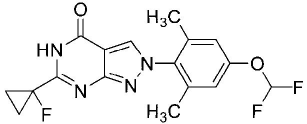 | | ¹H-NMR (DMSO-D₆) δ: 12.09 (1H, s), 8.76 (1H, s), 7.32 (1H, t, J = 73.9 Hz), 7.11 (2H, s), 1.97 (6H, s), 1.58-1.48 (4H, m) . | 365 |
| 160 | 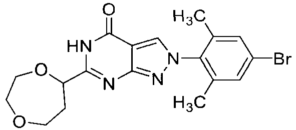 | racemate | ¹H-NMR (DMSO-D₆) δ: 11.65 (1H, s), 8.75 (1H, s), 7.54 (2H, s), 4.69 (1H, dd, J = 9.2, 4.9 Hz), 3.99 (1H, dt, J = 11.7, 2.8 Hz), 3.87-3.69 (5H, m), 2.43-2.39 (1H, m), 2.33-2.29 (1H, m), 1.96 (6H, s). | 419 |
| 161 | 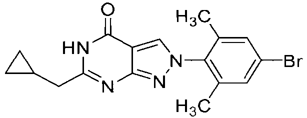 | | ¹H-NMR (DMSO-D₆) δ: 11.72 (1H, br s), 8.68 (1H, s), 7.52 (2H, s), 2.52-2.49 (2H, m), 1.95 (6H, s), 1.19-1.16 (1H, m), 0.51-0.46 (2H, m), 0.27-0.25 (2H, m) . | 373 |
| 162 | 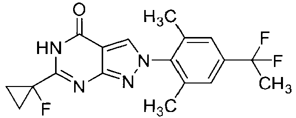 | | ¹H-NMR (DMSO-D₆) δ: 12.10 (1H, s), 8.80 (1H, s), 7.49 (2H, s), 2.06-1.96 (9H, m), 1.56-1.51 (4H, m) . | 363 |
| 163 | 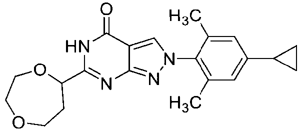 | racemate | ¹H-NMR (DMSO-D₆) δ: 11.60 (1H, s), 8.67 (1H, s), 6.96 (2H, s), 4.69 (1H, dd, J = 9.1, 4.7 Hz), 4.00-3.97 (1H, m), 3.84-3.69 (5H, m), 2.46-2.38 (1H, m), 2.33-2.29 (1H, m), 1.95-1.92 (7H, m), 1.00-0.97 (2H, m), 0.75-0.72 (2H, m) . | 381 |
| 164 | 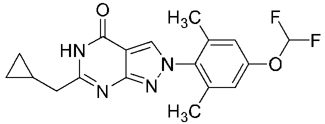 | | ¹H-NMR (DMSO-D₆) δ: 11.71 (1H, br s), 8.67 (1H, s), 7.30 (1H, t, J = 73.9 Hz), 7.10 (2H, s), 2.46-2.44 (2H, m), 1.96 (6H, s), 1.19-1.16 (1H, m), 0.50-0.47 (2H, m), 0.28-0.24 (2H, m). | 361 |
| 165 | 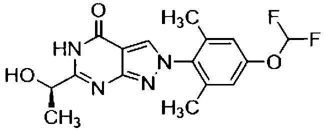 | optically active substance (R) | ¹H-NMR (DMSO-D₆) δ: 11.30 (1H, s), 8.71 (1H, s), 7.32 (1H, t, J = 73.9 Hz), 7.12 (2H, s), 5.61 (1H, s), 4.57 (1H, q, J = 6.6 Hz), 1.98 (6H, s), 1.43 (3H, d, J = 6.7 Hz) . | 351 |
| 166 | 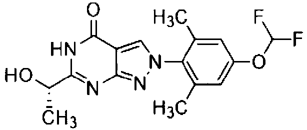 | optically active substance (S) | ¹H-NMR (DMSO-D₆) δ: 11.31 (1H, br s), 8.71 (1H, s), 7.32 (1H, t, J = 73.9 Hz), 7.12 (2H, s), 5.65 (1H, br s), 4.57 (1H, q, J = 6.5 Hz), 1.98 (6H, s), 1.43 (3H, d, J = 6.7 Hz) . | 351 |
| 167 | 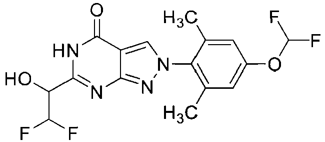 | racemate | ¹H-NMR (DMSO-D₆) δ: 11.80 (1H, br s), 8.79 (1H, s), 7.32 (1H, t, J = 73.8 Hz), 7.13 (2H, s), 6.77 (1H, br s), 6.33 (1H, td, J = 55.0, 4.4 Hz), 4.70 (1H, dt, J = 10.1, 4.6 Hz), 1.98 (6H, s). | 387 |
| 168 | 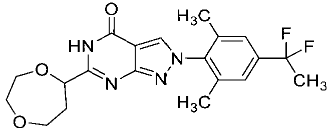 | racemate | ¹H-NMR (DMSO-D₆) δ: 11.66 (1H, s), 8.78 (1H, s), 7.49 (2H, s), 4.70 (1H, dd, J = 9.5, 4.9 Hz), 4.02-3.97 (1H, m), 3.88-3.69 (5H, m), 2.43-2.41 (1H, m), 2.33-2.29 (1H, m), 2.01 (9H, dd, J = 21.6, 16.3 Hz). | 405 |
| 169 | 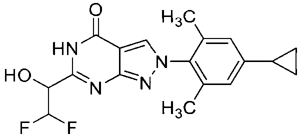 | optically active substance (enantiomer of Example 170) | ¹H-NMR (DMSO-D₆) δ: 11.76 (1H, br s), 8.72 (1H, d, J = 3.7 Hz), 6.96 (2H, s), 6.76 (1H, br s), 6.33 (1H, dt, J = 75.0, 27.5 Hz), 4.69 (1H, td, J = 9.9, 4.9 Hz), 1.97-1.90 (7H, m), 0.99 (2H, ddd, J = 9.5, 5.3, 3.0 Hz), 0.74 (2H, dt, J = 8.6, 3.2 Hz) . | 361 |
| 170 | 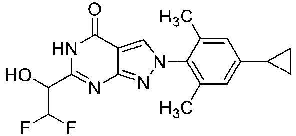 | optically active substance (enantiomer of Example 169) | ¹H-NMR (DMSO-D₆) δ: 11.74 (1H, br s), 8.70 (1H, s), 6.95 (2H, s), 6.75 (1H, br s), 6.31 (1H, dt, J = 74.9, 27.5 Hz), 4.67 (1H, dt, J = 14.3, 5.5 Hz), 1.96-1.90 (7H, m), 0.97 (2H, ddd, J = 9.5, 5.3, 3.0 Hz), 0.72 (2H, dt, J = 8.5, 3.2 Hz). | 361 |
| 171 | 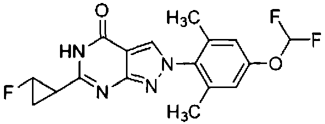 | trans-isomer, racemate | ¹H-NMR (CDCl₃) δ: 10.31 (br s, 1H), 8.09 (s, 1H), 6.94 (s, 2H), 6.56 (t, 1H, J = 73.4 Hz), 5.20-5.15 (m, 0.5H), 5.04-4.99 (m, 0.5H), 2.34-2.24 (m, 1H), 2.05 (s, 6H), 1.83-1.64 (m, 2H) . | 365 |
| 172 | 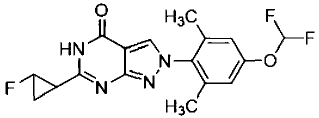 | trans-isomer, optically active substance (enantiomer of Example 173) | ¹H-NMR (CDCl₃) δ: 10.53 (br s, 1H), 8.10 (s, 1H), 6.94 (s, 2H), 6.56 (t, 1H, J = 73.4 Hz), 5.20-5.15 (m, 0.5H), 5.04-5.00 (m, 0.5H), 2.36-2.26 (m, 1H), 2.05 (s, 6H), 1.83-1.65 (m, 2H) . | 365 |
| 173 | 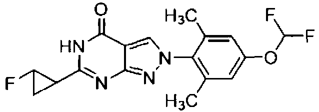 | trans-isomer, optically active substance (enantiomer of Example 172) | ¹H-NMR (CDCl₃) δ: 10.68 (br s, 1H), 8.10 (s, 1H), 6.94 (s, 2H), 6.56 (t, 1H, J = 73.4 Hz), 5.21-5.16 (m, 0.5H), 5.05-5.00 (m, 0.5H), 2.39-2.27 (m, 1H), 2.05 (s, 6H), 1.83-1.64 (m, 2H). | 365 |
| 174 | 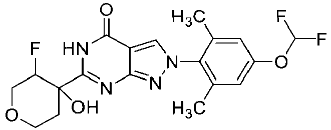 | racemate, not determined for relative configuration (single diastereomer) | ¹H-NMR (DMSO-D₆) δ: 11.53 (1H, br s), 8.76 (1H, s), 7.33 (1H, t, J = 73.8 Hz), 7.13 (2H, s), 6.27 (1H, br s), 4.89 (1H, d, J = 45.9 Hz), 3.98-3.66 (5H, m), 1.98 (6H, s), 1.79 (1H, d, J = 14.0 Hz) . | 425 |
| 175 | 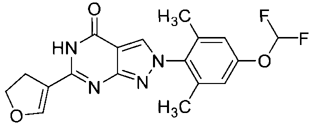 | | ¹H-NMR (DMSO-D₆) δ: 11.58 (1H, s), 8.65 (1H, s), 7.84-7.84 (1H, m), 7.32 (1H, t, J = 73.8 Hz), 7.11 (2H, s), 4.53 (2H, t, J = 9.8 Hz), 2.98 (2H, td, J = 9.7, 1.4 Hz), 1.99 (6H, s). | 375 |
| 176 | 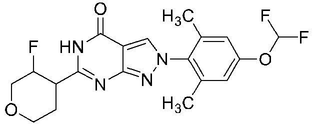 | racemate, not determined for relative configuration (diastereomer of Example 177) | ¹H-NMR (DMSO-D₆) δ: 11.96 (1H, s), 8.73 (1H, s), 7.33 (1H, t, J = 73.8 Hz), 7.12 (2H, s), 5.02-4.96 (1H, m), 4.15-4.13 (1H, m), 3.89-3.87 (1H, br m), 3.39-3.36 (2H, m), 3.09-3.06 (1H, m), 2.08-2.04 (1H, br m), 1.98 (6H, s), 1.87-1.81 (1H, br m) . | 409 |
| 177 | 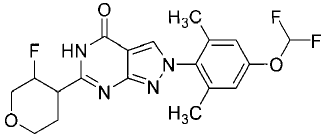 | racemate, not determined for relative configuration (diastereomer of Example 176) | ¹H-NMR (DMSO-D₆) δ: 11.85 (1H, s), 8.71 (1H, s), 7.33 (1H, t, J = 73.8 Hz), 7.12 (2H, s), 5.10 (1H, d, J = 48.5 Hz), 4.07-4.00 (2H, m), 3.60-3.44 (2H, m), 3.19-3.10 (1H, m), 2.31-2.27 (1H, m), 1.98 (6H, s), 1.76-1.73 (1H, br m) . | 409 |
| 178 | 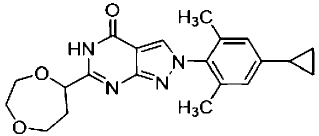 | optically active substance (enantiomer of Example 179) | ¹H-NMR (DMSO-D₆) δ: 11.61 (1H, s), 8.66 (1H, s), 6.96 (2H, s), 4.68 (1H, dd, J = 9.2, 4.8 Hz), 4.01-3.98 (1H, m), 3.83-3.73 (5H, m), 2.38-2.32 (2H, m), 1.95-1.92 (7H, m), 0.98 (2H, td, J = 7.1, 4.7 Hz), 0.76-0.72 (2H, m) . | 381 |
| 179 | 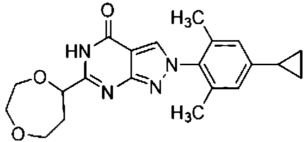 | optically active substance (enantiomer of Example 178) | ¹H-NMR (DMSO-D₆) δ: 11.62 (1H, s), 8.66 (1H, s), 6.96 (2H, s), 4.68 (1H, dd, J = 9.4, 4.6 Hz), 4.00 (1H, t, J = 8.1 Hz), 3.84-3.69 (5H, m), 2.43-2.29 (2H, m), 1.94-1.91 (7H, m), 0.98 (2H, dd, J = 13.2, 5.1 Hz), 0.73 (2H, q, J = 4.8 Hz). | 381 |
| 180 | 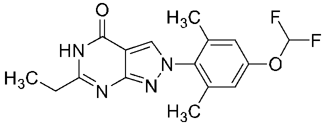 | | ¹H-NMR (DMSO-D₆) δ: 11.74 (1H, s), 8.67 (1H, s), 7.32 (1H, t, J = 73.9 Hz), 7.11 (2H, s), 2.61 (2H, q, J = 7.5 Hz), 1.97 (6H, s), 1.24 (3H, t, J = 7.5 Hz) . | 335 |
| 181 | 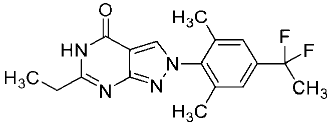 | | ¹H-NMR (DMSO-D₆) δ: 11.76 (1H, s), 8.72 (1H, s), 7.48 (2H, s), 2.62 (2H, q, J = 7.4 Hz), 2.02 (6H, s), 2.01 (3H, t, J = 18.9 Hz), 1.24 (3H, t, J = 7.5 Hz) . | 333 |
| 182 | 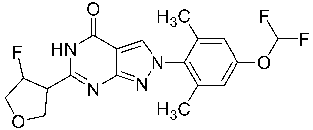 | racemate, not determined for relative configuration (diastereomer of Example 183) | ¹H-NMR (DMSO-D₆) δ: 12.02 (1H, s), 8.73 (1H, s), 7.32 (1H, t, J = 73.8 Hz), 7.12 (2H, s), 5.78-5.64 (1H, m), 4.27 (1H, t, J = 8.2 Hz), 4.09-3.90 (3H, m), 3.68-3.61 (1H, m), 1.97 (6H, s). | 395 |
| 183 | 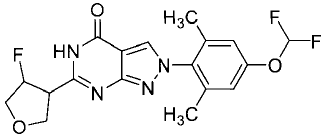 | racemate, not determined for relative configuration (diastereomer of Example 182) | ¹H-NMR (DMSO-D₆) δ: 12.06 (1H, s), 8.74 (1H, s), 7.32 (1H, t, J = 73.8 Hz), 7.12 (2H, s), 5.80-5.66 (1H, m), 4.41 (1H, t, J = 9.1 Hz), 4.09-4.04 (4H, m), 3.71-3.63 (1H, m), 1.98 (6H, s) . | 395 |
| 184 | 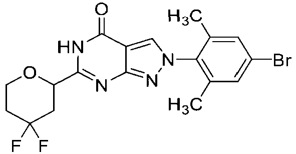 | racemate | ¹H-NMR (DMSO-D₆) δ: 11.85 (1H, s), 8.78 (1H, s), 7.55 (2H, s), 4.49-4.47 (1H, m), 4.15 (1H, d, J = 13.9 Hz), 3.64 (1H, td, J = 11.3, 4.0 Hz), 2.42-2.35 (2H, m), 2.11-2.08 (2H, m), 1.96 (6H, s). | 439 |
| 185 | 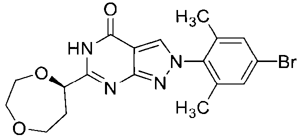 | optically active substance (R) | ¹H-NMR (DMSO-D₆) δ: 11.66 (1H, s), 8.75 (1H, s), 7.54 (2H, s), 4.69 (1H, dd, J = 9.7, 4.8 Hz), 4.01-3.98 (1H, m), 3.84-3.69 (5H, m), 2.45-2.37 (1H, m), 2.32-2.30 (1H, m), 1.96 (6H, s). | 419 |
| 186 | 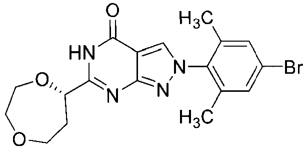 | optically active substance (S) | ¹H-NMR (DMSO-D₆) δ: 11.66 (1H, s), 8.75 (1H, s), 7.54 (2H, s), 4.69 (1H, dd, J = 9.2, 4.8 Hz), 4.02-3.96 (1H, m), 3.86-3.69 (5H, m), 2.42-2.38 (1H, m), 2.33-2.29 (1H, m), 1.96 (6H, s). | 419 |
| 187 | 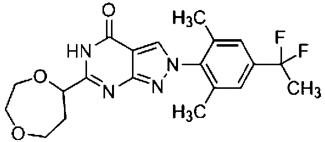 | optically active substance (enantiomer of Example 188) | ¹H-NMR (DMSO-D₆) δ: 11.66 (1H, s), 8.77 (1H, s), 7.49 (2H, s), 4.70 (1H, dd, J = 9.5, 4.9 Hz), 4.02-3.97 (1H, m), 3.87-3.69 (5H, m), 2.47-2.38 (1H, m), 2.32-2.30 (1H, m), 2.02 (6H, s), 2.01 (3H, t, J = 18.8 Hz) . | 405 |
| 188 | 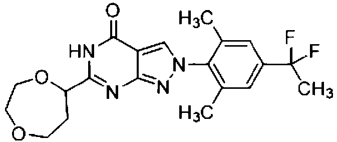 | optically active substance (enantiomer of Example 187) | ¹H-NMR (DMSO-D₆) δ: 11.67 (1H, s), 8.78 (1H, s), 7.49 (2H, s), 4.70 (1H, dd, J = 9.4, 5.0 Hz), 4.02-3.97 (1H, m), 3.86-3.69 (5H, m), 2.47-2.38 (1H, m), 2.32-2.30 (1H, m), 2.02 (6H, s), 2.01 (3H, t, J = 18.8 Hz) . | 405 |
| 189 | 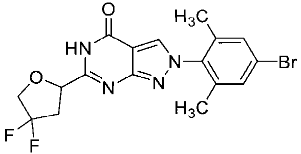 | racemate | ¹H-NMR (DMSO-D₆) δ: 11.99 (1H, s), 8.79 (1H, s), 7.55 (2H, s), 5.13 (1H, t, J = 7.3 Hz), 4.26-4.16 (1H, m), 4.09-4.04 (1H, m), 3.08-2.94 (1H, m), 2.87-2.81 (1H, m), 1.97 (6H, s). | 425 |
| 190 | 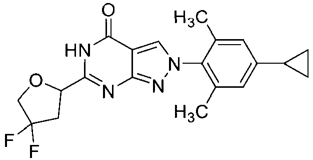 | racemate | ¹H-NMR (DMSO-D₆) δ: 11.94 (1H, s), 8.71 (1H, s), 6.96 (2H, s), 5.12 (1H, t, J = 7.5 Hz), 4.26-4.16 (1H, m), 4.08-4.04 (1H, m), 3.04-2.98 (1H, m), 2.89-2.78 (1H, m), 1.97-1.92 (7H, m), 1.00-0.98 (2H, m), 0.74-0.73 (2H, m). | 387 |
| 191 | 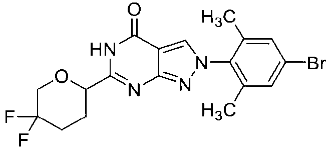 | racemate | ¹H-NMR (DMSO-D₆) δ: 11.80 (1H, s), 8.76 (1H, s), 7.54 (2H, s), 4.52 (1H, t, J = 6.2 Hz), 4.05-3.99 (1H, m), 3.76 (1H, dt, J = 22.4, 8.9 Hz), 2.32-2.14 (4H, m), 1.96 (6H, s). | 439 |
| 192 | 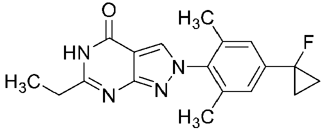 | | ¹H-NMR (DMSO-D₆) δ: 11.73 (1H, s), 8.66 (1H, s), 7.17 (2H, s), 2.62 (2H, q, J = 7.6 Hz), 1.97 (6H, s), 1.52 (2H, ddd, J = 19.3, 8.0, 5.9 Hz), 1.24-1.22 (5H, m) . | 327 |
| 193 | 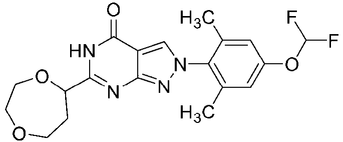 | optically active substance (enantiomer of Example 194) | ¹H-NMR (DMSO-D₆) δ: 11.64 (1H, s), 8.73 (1H, s), 7.32 (1H, t, J = 73.8 Hz), 7.12 (2H, s), 4.69 (1H, dd, J = 9.4, 4.9 Hz), 4.02-3.97 (1H, m), 3.81-3.73 (5H, m), 2.47-2.38 (1H, m), 2.33-2.30 (1H, m), 1.98 (6H, s). | 407 |
| 194 | 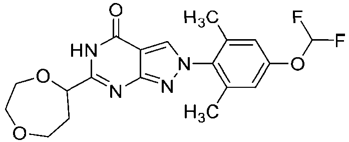 | optically active substance (enantiomer of Example 193) | ¹H-NMR (DMSO-D₆) δ: 11.64 (1H, s), 8.72 (1H, s), 7.32 (1H, t, J = 73.8 Hz), 7.12 (2H, s), 4.69 (1H, dd, J = 9.4, 4.8 Hz), 4.02-3.97 (1H, m), 3.87-3.69 (5H, m), 2.47-2.38 (1H, m), 2.34-2.28 (1H, m), 1.98 (6H, s). | 407 |
| 195 | 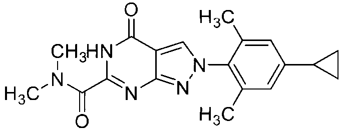 | | ¹H-NMR (CDCl₃) δ: 10.00 (1H, br s), 8.14 (1H, s), 6.86 (2H, s), 3.71 (3H, s), 3.20 (3H, s), 2.00 (6H, s), 1.94-1.86 (1H, m), 1.05-0.99 (2H, m), 0.76-0.71 (2H, m) . | 352 |
| 196 | 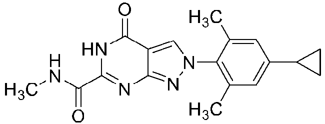 | | ¹H-NMR (CDCl₃) δ: 9.93 (1H, br s), 8.14 (1H, s), 7.88-7.79 (1H, m), 6.87 (2H, s), 3.05 (3H, d, J = 5.1 Hz), 2.00 (6H, s), 1.94-1.86 (1H, m), 1.05-0.99 (2H, m), 0.77-0.72 (2H, m) . | 338 |
| 197 | 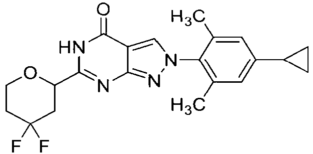 | racemate | ¹H-NMR (DMSO-D₆) δ: 11.79 (1H, s), 8.69 (1H, s), 6.96 (2H, s), 4.48-4.47 (1H, m), 4.16-4.15 (1H, m), 3.64 (1H, td, J = 11.3, 3.9 Hz), 2.42-2.34 (2H, m), 2.10-2.06 (2H, m), 1.96-1.92 (7H, m), 1.00-0.97 (2H, m), 0.75-0.72 (2H, m) . | 401 |
| 198 | 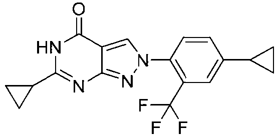 | | ¹H-NMR (DMSO-D₆) δ: 12.04 (1H, s), 8.71 (1H, s), 7.66 (1H, s), 7.58-7.50 (2H, m), 2.21-2.15 (1H, m), 1.99-1.91 (1H, m), 1.12-0.99 (6H, m), 0.87-0.85 (2H, m) . | 361 |
| 199 | 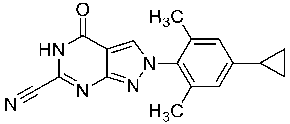 | | ¹H-NMR (CDCl₃) δ: 10.44 (1H, br s), 8.21 (1H, s), 6.88 (2H, s), 1.99 (6H, s), 1.94-1.87 (1H, m), 1.06-1.00 (2H, m), 0.77-0.72 (2H, m) . | 306 |
| 200 | 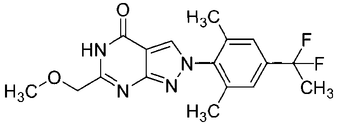 | | ¹H-NMR (CDCl₃) δ: 9.24 (1H, br s), 8.11 (1H, s), 7.32 (2H, s), 4.52 (2H, s), 3.55 (3H, s), 2.09 (6H, s), 1.94 (3H, t, J = 18.1 Hz) . | 349 |
| 201 | 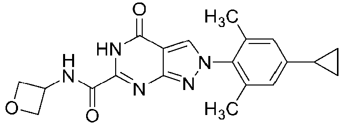 | | ¹H-NMR (CDCl₃) δ: 9.83 (1H, br s), 8.38 (1H, d, J = 8.1 Hz), 8.15 (1H, s), 6.88 (2H, s), 5.29-5.17 (1H, m), 5.00 (2H, t, J = 7.0 Hz), 4.66 (2H, t, J = 7.0 Hz), 2.01 (6H, s), 1.95-1.87 (1H, m), 1.06-1.00 (2H, m), 0.78-0.72 (2H, m) . | 380 |
| 202 | 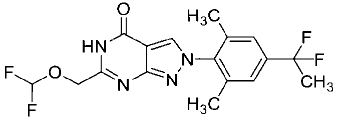 | | ¹H-NMR (DMSO-D₆) δ: 12.05 (1H, s), 8.80 (1H, s), 7.49 (2H, s), 6.86 (1H, t, J = 75.1 Hz), 4.81 (2H, s), 2.02-1.96 (9H, m) . | 385 |
| 203 | 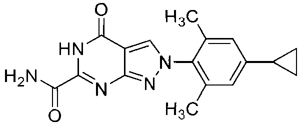 | | ¹H-NMR (CDCl₃) δ: 10.02 (1H, br s), 8.16 (1H, s), 7.71 (1H, br s), 6.88 (2H, s), 5.91 (1H, br s), 2.01 (6H, s), 1.95-1.86 (1H, m), 1.06-0.99 (2H, m), 0.78-0.72 (2H, m) . | 324 |
| 204 | 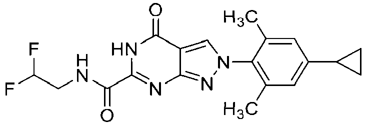 | | ¹H-NMR (CDCl₃) δ: 9.85 (1H, br s), 8.16 (1H, s), 8.13-8.06 (1H, m), 6.88 (2H, s), 6.12-5.78 (1H, m), 3.93-3.79 (2H, m), 2.00 (6H, s), 1.95-1.86 (1H, m), 1.06-1.00 (2H, m), 0.78-0.72 (2H, m) . | 388 |
| 205 | 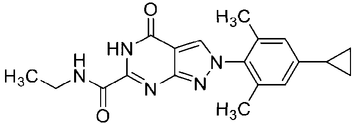 | | ¹H-NMR (CDCl₃) δ: 9.94 (1H, br s), 8.14 (1H, s), 7.88-7.80 (1H, m), 6.87 (2H, s), 3.56-3.47 (2H, m), 2.00 (6H, s), 1.95-1.86 (1H, m), 1.28 (3H, t, J = 7.5 Hz), 1.07-0.99 (2H, m), 0.78-0.72 (2H, m) . | 352 |
| 206 | 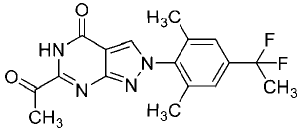 | | ¹H-NMR (CDCl₃) δ: 9.65 (1H, br s), 8.20 (1H, s), 7.35 (2H, s), 2.80 (3H, s), 2.10 (6H, s), 1.96 (3H, t, J = 18.2 Hz). | 347 |
| 207 | 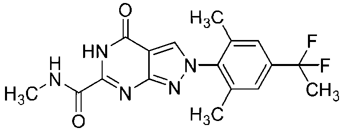 | | ¹H-NMR (DMSO-D₆) δ: 11.67 (1H, br s), 9.15-9.10 (1H, m), 8.90 (1H, s), 7.51 (2H, s), 2.81 (3H, d, J = 4.8 Hz), 2.04 (6H, s), 2.01 (3H, t, J = 18.4 Hz) . | 362 |
| 208 | 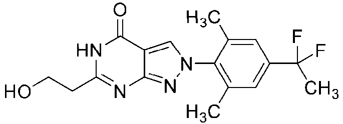 | | ¹H-NMR (DMSO-D₆) δ: 8.72 (1H, s), 7.49 (2H, s), 4.77 (1H, br s), 3.83 (2H, t, J = 6.5 Hz), 2.76 (2H, t, J = 6.5 Hz), 2.09 (1H, s), 2.01 (9H, dd, J = 21.3, 16.4 Hz). | 349 |
| 209 | 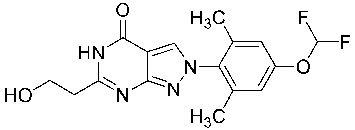 | | ¹H-NMR (DMSO-D₆) δ: 11.71 (1H, br s), 8.69 (1H, s), 7.51-7.11 (3H, m), 4.84-4.75 (1H, m), 3.88-3.80 (2H, m), 2.78-2.74 (2H, m), 1.98 (6H, s). | 351 |
| 210 | 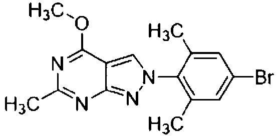 | | ¹H-NMR (DMSO-D₆) δ: 8.82 (1H, s), 7.56 (2H, s), 4.09 (3H, s), 2.58 (3H, s), 1.93 (6H, s). | 347 |
| 211 | 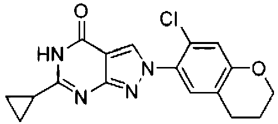 | | ¹H-NMR (DMSO-D₆) δ: 12.03 (1H, br s), 8.70 (1H, s), 7.39 (1H, s), 7.07 (1H, s), 4.22 (2H, t, J = 5.1 Hz), 2.77 (2H, t, J = 6.2 Hz), 1.96-1.94 (3H, m), 1.09-0.99 (4H, m) . | 343 |

### Test example 1: Evaluation of NLRP3 inflammasome inhibitory activity

The NLRP3 inflammasome inhibitory activity of test compounds were evaluated on the basis of the inhibitory activity of the IL-1β production in THP1-Null cells (Product Number: thp-null, InvivoGen). Cells were maintained for culture in RPMI-1640 media containing 10% (v/v) fetal bovine serum, 25 mmol/L HEPES, 100 U/mL penicillin, 100 µg/mL streptomycin, 100 µg/mL normocin, and 200 µg/mL hygromycin B (set at 37°C, 5% CO₂/95% air) .

Cells were suspended with media for assay containing 0.5 µmol/L PMA (RPMI-1640 media containing 10% (v/v) fetal bovine serum, 100 U/mL penicillin, and 100 µg/mL streptomycin), and the suspended cells were seeded on Corning (registered trademark) 384-well Flat Clear Bottom Black Polystyrene TC-treated Microplates (25,000 cells/25 µL/well), followed by incubation (set at 37°C, 5% CO₂/95% air) overnight. The supernatant of the culture was removed, and thereto was added media for assay (25 µL/well) containing 1 µg/mL Lipopolysaccharides (Product Number: L2654, Sigma-Aldrich (registered trademark)). Then, the culture was further incubated for 3 hours (set at 37°C, 5% CO₂/95% air) . The supernatant of the culture was removed. Then, a vehicle solution prepared from Opti-MEM (trademark) medium (Product Number: 31985-070, Invitrogen) was added to blank-setting wells and control-setting wells (20 µL/well), followed by incubation for 15 minutes (set at 37°C, 5% CO₂/95% air). A solution containing a test compound (20 µL/well) was added to test compound-setting wells. Further, Opti-MEM (trademark) medium containing Nigericin (Product Number: N7143, Sigma-Aldrich (registered trademark)) was added to the control-setting wells and test compound-setting wells (5 µL/well), followed by incubation for 1.5 hours (set at 37°C, 5% CO₂/95% air). The final concentration of Nigericin was adjusted to be 7.5 µmol/L. 5 µL/well of Opti-MEM (trademark) medium was added to the blank-setting wells. The supernatant of the culture was cryonically stored (set at -20°C) until measurement of IL-1β.

The amount of IL-1β in the culture supernatant was quantitated with AlphaLISA (registered trademark) Human IL-1β Detection Kit (Product Number: AL220C, Perkin Elmer). Fluorescence intensity was measured with a microplate reader EnSpier (Model number: 2300-00J, Perkin Elmer) or EnSight (Model number: HH34000000, Perkin Elmer) according to procedure manuals attached thereto. Inhibition rates of the test compound-setting wells were calculated on the basis of 100% for the blank-setting wells and 0% for the control-setting wells. IC₅₀ values (i.e., 50% inhibitory concentrations) of the test compounds were calculated by logistic regression analysis. The result of each Example compound is shown in the following table.

| **Example** No. | **IC₅₀ (µM)** |
|---|---|
| 1 | 2.3 |
| 2 | 1.3 |
| 3 | 0.24 |
| 4 | 0.54 |
| 5 | 0.58 |
| 6 | 0.43 |
| 7 | 44% inhibition at 30 µM |
| 8 | 0.23 |
| 9 | 1.1 |
| 10 | 13 |
| 11 | 43% inhibition at 24 µM |
| 12 | 26% inhibition at 24 µM |
| 13 | 3% inhibition at 24 µM |
| 14 | -13% inhibition |
| | at 2.4 µM |
| 15 | -3% inhibition at 2.4 µM |
| 16 | -14% inhibition at 2.4 µM |
| 17 | -15% inhibition at 2.4 µM |
| 18 | 0.22 |
| 19 | 0.17 |
| 20 | 0.013 |
| 21 | 0.012 |
| 22 | 0.012 |
| 23 | 0.024 |
| 24 | 0.047 |
| 25 | 0.044 |
| 26 | 0.076 |
| 27 | 0.029 |
| 28 | 0.19 |
| 29 | 0.009 |
| 30 | 0.28 |
| 31 | 0.79 |
| 32 | 0.27 |
| 33 | 0.068 |
| 34 | 0.2 |
| 35 | 2.1 |
| 36 | 0.15 |
| 37 | 1.1 |
| 38 | 40% inhibition at 2.4 µM |
| 39 | 0.016 |
| 40 | 0.43 |
| 41 | 0.093 |
| 42 | 0.004 |
| 43 | 0.042 |
| 44 | 0.047 |
| 45 | 0.007 |
| 46 | 0.14 |
| 47 | 0.18 |
| 48 | 0.023 |
| 49 | 0.024 |
| 50 | 0.005 |
| 51 | 0.024 |
| 52 | 0.04 |
| 53 | 0.14 |
| 54 | 0.033 |
| 55 | 0.031 |
| 56 | 0.35 |
| 57 | 0.34 |
| 58 | 0.036 |
| 59 | 1.7 |
| 60 | 0.06 |
| 61 | 0.46 |
| 62 | 0.23 |
| 63 | 0.045 |
| 64 | 0.022 |
| 65 | 0.3 |
| 66 | 0.43 |
| 67 | 0.78 |
| 68 | 0.076 |
| 69 | 0.067 |
| 70 | 0.13 |
| 71 | 0.011 |
| 72 | 0.057 |
| 73 | 0.021 |
| 74 | 0.19 |
| 75 | 0.011 |
| 76 | 0.082 |
| 77 | 0.014 |
| 78 | 0.033 |
| 79 | 0.075 |
| 80 | 0.029 |
| 81 | 0.004 |
| 82 | 0.016 |
| 83 | 0.024 |
| 84 | 0.015 |
| 85 | 0.029 |
| 86 | 0.35 |
| 87 | 0.011 |
| 88 | 0.018 |
| 89 | 0.45 |
| 90 | 0.068 |
| 91 | 0.008 |
| 92 | 0.007 |
| 93 | 0.008 |
| 94 | 0.22 |
| 95 | 0.042 |
| 96 | 0.27 |
| 97 | 0.024 |
| 98 | 0.098 |
| 99 | 0.043 |
| 100 | 0.054 |
| 101 | 0.024 |
| 102 | 0.008 |
| 103 | 0.064 |
| 104 | 0.015 |
| 105 | 0.013 |
| 106 | 0.028 |
| 107 | 0.015 |
| 108 | 0.049 |
| 109 | 0.017 |
| 110 | 0.019 |
| 111 | 0.015 |
| 112 | 0.14 |
| 113 | 0.14 |
| 114 | 0.017 |
| 115 | 0.43 |
| 116 | 0.28 |
| 117 | 0.24 |
| 118 | 0.18 |
| 119 | 0.031 |
| 120 | 0.04 |
| 121 | 0.038 |
| 122 | 0.061 |
| 123 | 0.023 |
| 124 | 0.008 |
| 125 | 0.024 |
| 126 | 0.024 |
| 127 | 0.073 |
| 128 | 0.044 |
| 129 | 0.05 |
| 130 | 0.24 |
| 131 | 0.005 |
| 132 | 0.024 |
| 133 | 0.041 |
| 134 | 0.024 |
| 135 | 0.033 |
| 136 | 0.049 |
| 137 | 0.024 |
| 138 | 0.12 |
| 139 | 0.036 |
| 140 | 0.046 |
| 141 | 0.061 |
| 142 | 0.017 |
| 143 | 0.086 |
| 144 | 0.058 |
| 145 | 0.013 |
| 146 | 0.015 |
| 147 | 0.024 |
| 148 | 0.061 |
| 149 | 0.022 |
| 150 | 0.018 |
| 151 | 0.017 |
| 152 | 0.012 |
| 153 | 0.017 |
| 154 | 0.025 |
| 155 | 0.61 |
| 156 | 0.013 |
| 157 | 0.34 |
| 158 | 0.026 |
| 159 | 0.057 |
| 160 | 0.037 |
| 161 | 0.01 |
| 162 | 0.057 |
| 163 | 0.023 |
| 164 | 0.067 |
| 165 | 0.075 |
| 166 | 1.3 |
| 167 | 0.23 |
| 168 | 0.074 |
| 169 | 0.026 |
| 170 | 0.51 |
| 171 | 0.024 |
| 172 | 0.008 |
| 173 | 0.015 |
| 174 | 0.03 |
| 175 | 54% inhibition at 0.002 µM |
| 176 | 0.075 |
| 177 | 0.024 |
| 178 | 0.011 |
| 179 | 0.051 |
| 180 | 0.02 |
| 181 | 0.018 |
| 182 | 0.008 |
| 183 | 0.035 |
| 184 | 0.051 |
| 185 | 0.023 |
| 186 | 0.087 |
| 187 | 0.067 |
| 188 | 0.19 |
| 189 | 0.071 |
| 190 | 0.041 |
| 191 | 0.072 |
| 192 | 0.02 |
| 193 | 0.027 |
| 194 | 0.1 |
| 195 | 0.18 |
| 196 | 0.022 |
| 197 | 0.036 |
| 198 | 0.011 |
| 199 | 0.24 |
| 200 | 0.075 |
| 201 | 0.74 |
| 202 | 0.04 |
| 203 | 0.29 |
| 204 | 0.05 |
| 205 | 0.011 |
| 206 | 0.36 |
| 207 | 0.27 |
| 208 | 0.066 |
| 209 | 0.025 |
| 210 | 9% inhibition at 24 µM |
| 211 | 0.027 |

Formulation examples of the present invention include the following formulations, but are not intended to be limited thereto.

### Formulation Example 1: Preparation of a capsule

| | | |
|---|---|---|
| (1) | A compound of Example 1 | 30 mg |
| (2) | Microcrystalline cellulose | 10 mg |
| (3) | Lactose | 19 mg |
| (4) | Magnesium stearate | 1 mg |

Ingredients (1), (2), (3), and (4) are mixed to be filled in a gelatin capsule.

### Formulation Example 2: Preparation of a tablet

| | | |
|---|---|---|
| (1) | A compound of Example 1 | 10 g |
| (2) | Lactose | 50 g |
| (3) | Cornstarch | 15 g |
| (4) | Carmellose calcium | 44 g |
| (5) | Magnesium stearate | 1 g |

The total amounts of Ingredients (1), (2), and (3) and 30 g of Ingredient (4) are combined with water, dried in vacuo, and then granulated. The resulted granules are mixed with 14 g of Ingredient (4) and 1 g of Ingredient (5), and tableted with a tabletting machine. In this manner, 1,000 tablets of which each tablet comprises 10 mg of the compound of Example 1 are obtained.

### INDUSTRIAL APPLICABILITY

A compound of Formula [I], or a pharmaceutically acceptable salt thereof, has an NLRP3 inflammasome inhibitory activity, and thus is expected to be useful for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and Neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

## Claims

1. A compound of Formula [I]: 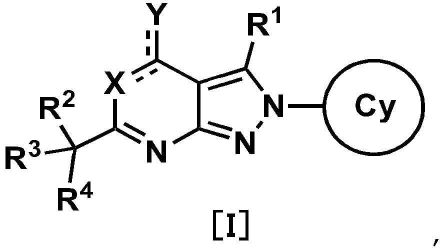 or a pharmaceutically acceptable salt thereof, wherein the partial structure: 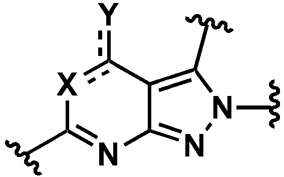 **is:**
(1) a structure of the formula: 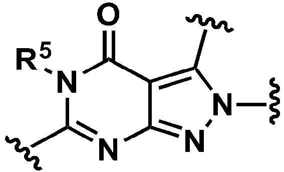 wherein R⁵ is hydrogen or C₁₋₄ alkyl, wherein the alkyl may be optionally substituted with:
(a) carboxy,
(b) -CO-C₁₋₄ alkoxy, or
(c) -CO-NR⁶R⁷, wherein R⁶ and R⁷ are each independently hydrogen or C₁₋₄ alkyl, or
(2) a structure of the formula: 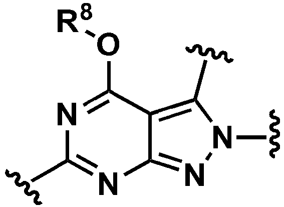 wherein R⁸ is C₁₋₄ alkyl;
a cyclic group Cy is:
(1) a group of the formula: 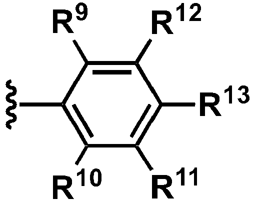 wherein R⁹ and R¹⁰ are each independently
(a) hydrogen,
(b) C₁₋₄ alkyl, wherein the alkyl may be optionally substituted with C₁₋₄ alkoxy,
(c) C₁₋₄ alkoxy,
(d) halogen,
(e) C₁₋₄ haloalkyl, or
(f) -O-C₁₋₄ haloalkyl,
R¹¹ and R¹² are each independently
(a) hydrogen,
(b) C₁₋₄ alkyl, or
(c) C₁₋₄ haloalkyl,
R¹³ is
(a) hydrogen,
(b) C₁₋₄ alkyl,
(c) C₁₋₄ alkoxy,
(d) halogen,
(e) C₁₋₆ haloalkyl,
(f) -O-C₁₋₄ haloalkyl, or
(g) C₃₋₆ cycloalkyl, wherein the cycloalkyl may be optionally substituted with 1 or 2 halogen atoms, or
R¹³ may be combined together with R¹¹ or R¹² and the carbon atom to which it is attached to form:
(a) C₅₋₆ cycloalkene, or
(b) 5- to 7-membered heterocycloalkene comprising 1 or 2 oxygen atoms, or
(2) a group of the formula: 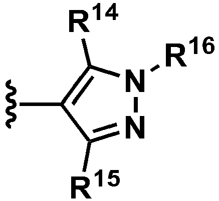 wherein R¹⁴ and R¹⁵ are each independently C₁₋₄ alkyl or C₁₋₄ haloalkyl,
R¹⁶ is C₁₋₆ alkyl or C₃₋₆ cycloalkyl,
R¹ is:
(1) hydrogen,
(2) cyano,
(3) C₁₋₄ alkyl, wherein the alkyl may be optionally substituted with hydroxy or C₁₋₄ alkoxy,
(4) C₁₋₄ haloalkyl, or
(5) -CO-C₁₋₄ alkyl,
R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
(a) hydroxy,
(b) C₁₋₄ alkoxy, and
(c) -SO₂-C₁₋₄ alkyl,
(4) C₁₋₆ alkoxy, wherein the alkoxy may be optionally substituted with:
(a) hydroxy,
(b) phenyl, or
(c) 4- to 6-membered heterocycloalkyl comprising 1 or 2 heteroatoms independently selected from the group consisting of nitrogen and oxygen atoms,
(5) halogen,
(6) C₁₋₄ haloalkyl, wherein the haloalkyl may be optionally substituted with hydroxy or C₁₋₄ alkoxy,
(7) -O-C₁₋₄ haloalkyl,
(8) -OR¹⁷, wherein R¹⁷ is 4- to 6-membered heterocycloalkyl comprising 1 or 2 heteroatoms independently selected from the group consisting of nitrogen and oxygen atoms,
(9) C₃₋₆ cycloalkyl,
(10) 4- to 6-membered heterocycloalkyl comprising 1 or 2 heteroatoms independently selected from the group consisting of nitrogen and oxygen atoms, wherein the heterocycloalkyl may be optionally substituted with oxo, or
(11) a group of the formula: 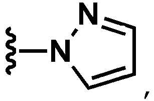 or
R², R³, and R⁴ may be combined together with the carbon atom to which they are attached and the -CR²R³R⁴ group may form:
(a) cyano,
(b) C₃₋₆ cycloalkyl, wherein the cycloalkyl may be optionally substituted with 1 to 3 substituents independently selected from the group consisting of:
(1) cyano,
(2) C₁₋₄ alkyl, wherein the alkyl may be optionally substituted with hydroxy or C₁₋₄ alkoxy,
(3) C₁₋₄ alkoxy,
(4) halogen, and
(5) -CO-NR¹⁸R¹⁹, wherein R¹⁸ and R¹⁹ are each independently hydrogen or C₁₋₄ alkyl,
(c) 4- to 7-membered heterocycloalkyl comprising 1 or 2 heteroatoms independently selected from the group consisting of nitrogen, oxygen, and sulfur atom, wherein the heterocycloalkyl may be optionally substituted with 1 or 2 substituents independently selected from the group consisting of:
(1) hydroxy,
(2) C₁₋₄ alkyl,
(3) C₁₋₄ alkoxy, and
(4) halogen, or
any one of the ring-constituting atoms of the heterocycloalkyl may be optionally substituted with 1 or 2 oxo,
(d) 7- to 9-membered saturated fused heterocyclic group comprising 1 or 2 heteroatoms independently selected from the group consisting of nitrogen and oxygen atoms, wherein the fused heterocyclic group may be optionally substituted with 1 or 2 halogen atoms,
(e) 5- to 8-membered bridged cycloalkyl, wherein the bridged cycloalkyl may be optionally substituted with halogen,
(f) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
(g) C₅₋₆ cycloalkenyl,
(h) 5- or 6-membered heterocycloalkenyl comprising 1 or 2 heteroatoms independently selected from the group consisting of nitrogen and oxygen atoms, or
(i) a group of the formula: 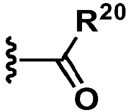 wherein R²⁰ is:
(1) C₁₋₄ alkyl, or
(2) -NR²¹R²², wherein R²¹ and R²² are each independently
(a) hydrogen,
(b) C₁₋₄ alkyl,
(c) C₁₋₄ haloalkyl, or
(d) 4- to 6-membered heterocycloalkyl comprising 1 or 2 heteroatoms independently selected from the group consisting of nitrogen and oxygen atoms.

2. The compound according to claim 1, or a pharmaceutically acceptable salt thereof, wherein the partial structure: 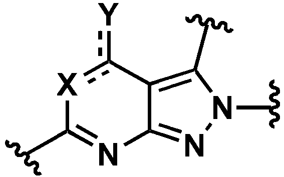 is
(1) a structure of the formula: 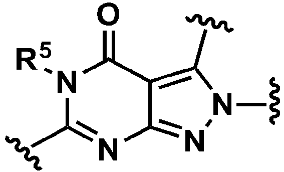 wherein R⁵ is the same as defined in claim 1.

3. The compound according to claim 1 or 2, or a pharmaceutically acceptable salt thereof, wherein R¹ is hydrogen.

4. The compound according to any one of claims 1 to 3, or a pharmaceutically acceptable salt thereof, wherein R⁵ is hydrogen.

5. The compound according to any one of claims 1 to 4, or a pharmaceutically acceptable salt thereof, wherein the cyclic group Cy is:
(1) a group of the formula: 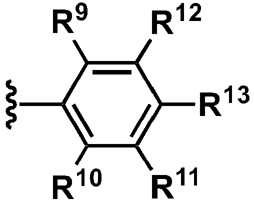 wherein R⁹, R¹⁰, R¹¹, R¹², and R¹³ are the same as defined in claim 1.

6. The compound according to any one of claims 1 to 5, or a pharmaceutically acceptable salt thereof, represented by Formula [II]: 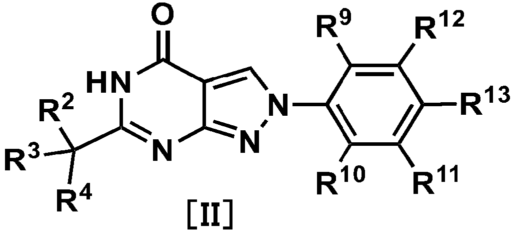 wherein R², R³, R⁴, R⁹, R¹⁰, R¹¹, R¹², and R¹³ are the same as defined in claim 1.

7. The compound according to any one of claims 1 to 6, or a pharmaceutically acceptable salt thereof, wherein R¹¹ and R¹² are hydrogen.

8. The compound according to any one of claims 1 to 7, or a pharmaceutically acceptable salt thereof, represented by Formula [III]: 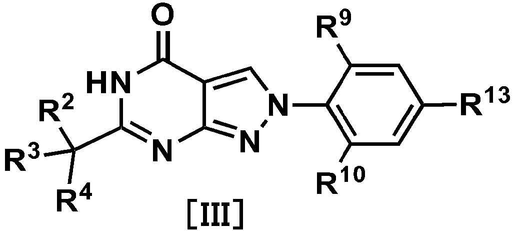 wherein R², R³, R⁴, R⁹, R¹⁰, and R¹³ are the same as defined in claim 1.

9. The compound according to any one of claims 1 to 8, or a pharmaceutically acceptable salt thereof, wherein at least one of R⁹ and R¹⁰ is:
(1) C₁₋₄ alkyl, wherein the alkyl may be optionally substituted with C₁₋₄ alkoxy,
(2) C₁₋₄ alkoxy,
(3) halogen,
(4) C₁₋₄ haloalkyl, or
(5) -O-C₁₋₄ haloalkyl.

10. The compound according to any one of claims 1 to 9, or a pharmaceutically acceptable salt thereof, wherein R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
(a) hydroxy,
(b) C₁₋₄ alkoxy, and
(c) -SO₂-C₁₋₄ alkyl,
(4) C₁₋₆ alkoxy, wherein the alkoxy may be optionally substituted with
(a) hydroxy,
(b) phenyl, or
(c) 4- to 6-membered heterocycloalkyl comprising 1 or 2 heteroatoms independently selected from the group consisting of nitrogen and oxygen atoms,
(5) C₁₋₄ haloalkyl, wherein the haloalkyl may be optionally substituted with hydroxy or C₁₋₄ alkoxy,
(6) -O-C₁₋₄ haloalkyl, or
(7) C₃₋₆ cycloalkyl, or
R², R³, and R⁴ may be combined together with the carbon atom to which they are attached, and the -CR²R³R⁴ group may form:
(a) C₃₋₆ cycloalkyl, wherein the cycloalkyl may be optionally substituted with 1 to 3 substituents independently selected from the group consisting of:
(1) cyano,
(2) C₁₋₄ alkyl, wherein the alkyl may be optionally substituted with hydroxy or C₁₋₄ alkoxy,
(3) C₁₋₄ alkoxy,
(4) halogen, and
(5) -CO-NR¹⁸R¹⁹, wherein R¹⁸ and R¹⁹ are each independently hydrogen or C₁₋₄ alkyl,
(b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
(1) hydroxy,
(2) C₁₋₄ alkyl,
(3) C₁₋₄ alkoxy, and
(4) halogen, or
any one of the ring-constituting atoms of the heterocycloalkyl may be optionally substituted with 1 or 2 oxo groups,
(c) 5- to 8-membered bridged cycloalkyl, wherein the bridged cycloalkyl may be optionally substituted with halogen,
(d) 5- to 8-membered bridged heterocycloalkyl comprising 1 or 2 heteroatoms independently selected from the group consisting of nitrogen and oxygen atoms, wherein the bridged heterocycloalkyl may be optionally substituted with C₁₋₄ alkyl optionally substituted with hydroxy,
(e) 5- or 6-membered heterocycloalkenyl comprising 1 or 2 heteroatoms independently selected from the group consisting of nitrogen and oxygen atoms, or
(f) a group of the formula: 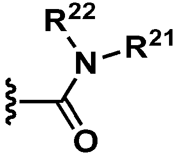 wherein R²¹ and R²² are each independently
(1) hydrogen,
(2) C₁₋₄ alkyl,
(3) C₁₋₄ haloalkyl, or
(4) 4- to 6-membered heterocycloalkyl comprising 1 or 2 heteroatoms independently selected from the group consisting of nitrogen and oxygen atoms.

11. The compound according to any one of claims 1 to 10, or a pharmaceutically acceptable salt thereof, wherein R², R³, and R⁴ are each independently
(1) hydrogen,
(2) hydroxy,
(3) C₁₋₆ alkyl, wherein the alkyl may be optionally substituted with 1 or 2 substituents independently selected from the group consisting of:
(a) hydroxy,
(b) C₁₋₄ alkoxy, and
(c) -SO₂-C₁₋₄ alkyl,
(4) C₁₋₆ alkoxy, wherein the alkoxy may be optionally substituted with:
(a) hydroxy,
(b) phenyl, or
(c) 4- to 6-membered heterocycloalkyl comprising 1 or 2 heteroatoms independently selected from the group consisting of nitrogen and oxygen atoms, or
(5) C₁₋₄ haloalkyl, wherein the haloalkyl may be optionally substituted with hydroxy or C₁₋₄ alkoxy, or
R², R³, and R⁴ may be combined together with the carbon atom to which they are attached, and the -CR²R³R⁴ group may form:
(a) C₃₋₆ cycloalkyl, wherein the cycloalkyl may be optionally substituted with 1 to 3 substituents independently selected from the group consisting of:
(1) cyano,
(2) C₁₋₄ alkyl, wherein the alkyl may be optionally substituted with hydroxy or C₁₋₄ alkoxy,
(3) C₁₋₄ alkoxy,
(4) halogen, and
(5) -CO-NR¹⁸R¹⁹, wherein R¹⁸ and R¹⁹ are each independently hydrogen or C₁₋₄ alkyl,
(b) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of:
(1) hydroxy,
(2) C₁₋₄ alkyl,
(3) C₁₋₄ alkoxy, and
(4) halogen, or
any one of the ring-constituting atoms of the heterocycloalkyl may be optionally substituted with 1 or 2 oxo groups, or
(c) a group of the formula: 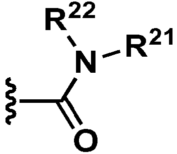 wherein R²¹ and R²² are each independently
(1) hydrogen,
(2) C₁₋₄ alkyl,
(3) C₁₋₄ haloalkyl, or
(4) 4- to 6-membered heterocycloalkyl comprising 1 or 2 heteroatoms independently selected from the group consisting of nitrogen and oxygen atoms.

12. The compound according to claim 1, or a pharmaceutically acceptable salt thereof, selected from the group consisting of the following formulae: 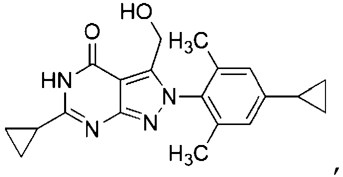 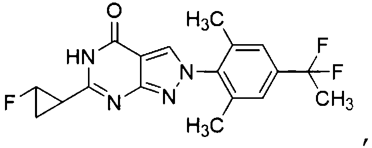 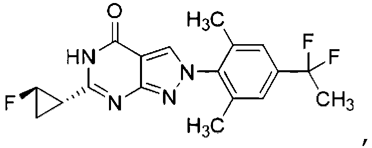 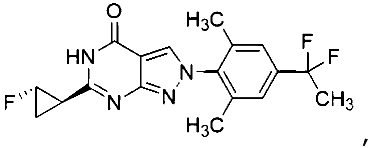 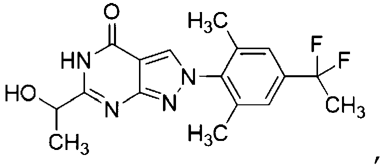 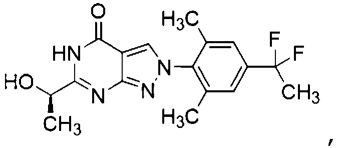 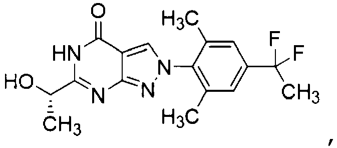 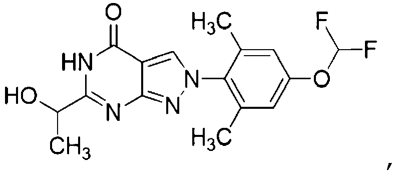 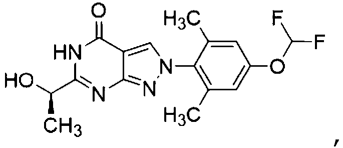 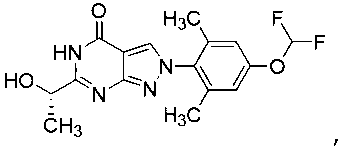 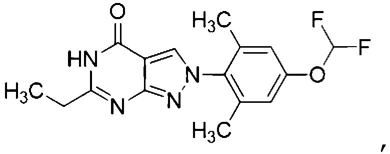 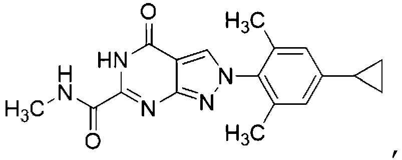 and

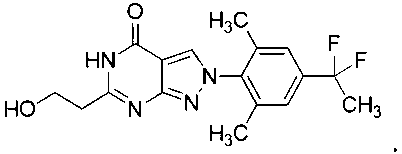

13. The compound according to claim 1, or a pharmaceutically acceptable salt thereof, selected from the group consisting of the following formulae: 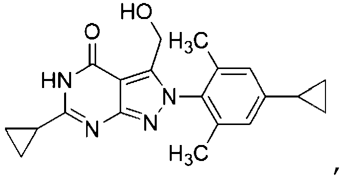 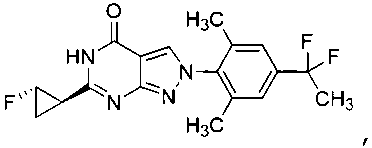 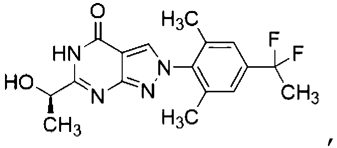 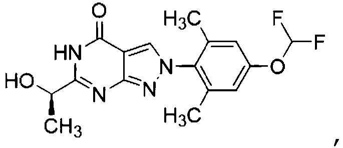 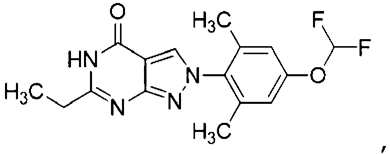 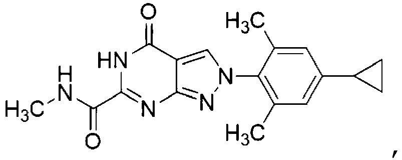 and

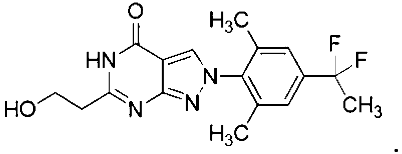

14. A pharmaceutical composition comprising a compound according to any one of claims 1 to 13, or a pharmaceutically acceptable salt thereof, and a pharmaceutically acceptable carrier.

15. An NLRP3 inflammasome inhibitor comprising a compound according to any one of claims 1 to 13, or a pharmaceutically acceptable salt thereof.

16. A medicament for treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, amyotrophic lateral sclerosis, and traumatic brain injury, comprising a compound according to any one of claims 1 to 13, or a pharmaceutically acceptable salt thereof.

17. The medicament according to claim 16, wherein the inflammatory bowel disease is ulcerative colitis or Crohn's disease.

18. The medicament according to claim 16, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

19. A method of inhibiting NLRP3 inflammasome, comprising administering a therapeutically effective amount of a compound according to any one of claims 1 to 13, or a pharmaceutically acceptable salt thereof, to a mammal.

20. A method of treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, amyotrophic lateral sclerosis, and traumatic brain injury, comprising administering a therapeutically effective amount of a compound according to any one of claims 1 to 13, or a pharmaceutically acceptable salt thereof, to a mammal.

21. The method according to claim 20, wherein the inflammatory bowel disease is ulcerative colitis or Crohn's disease.

22. The method according to claim 20, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

23. Use of a compound according to any one of claims 1 to 13, or a pharmaceutically acceptable salt thereof, in the manufacture of an NLRP3 inflammasome inhibitor.

24. Use of a compound according to any one of claims 1 to 13, or a pharmaceutically acceptable salt thereof, in the manufacture of a medicament for treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, amyotrophic lateral sclerosis, and traumatic brain injury.

25. The use according to claim 24, wherein the inflammatory bowel disease is ulcerative colitis or Crohn's disease.

26. The use according to claim 24, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

27. A compound according to any one of claims 1 to 13, or a pharmaceutically acceptable salt thereof, for use in inhibiting NLRP3 inflammasome.

28. A compound according to any one of claims 1 to 13, or a pharmaceutically acceptable salt thereof, for use in treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, amyotrophic lateral sclerosis, and traumatic brain injury.

29. The compound according to claim 28, or a pharmaceutically acceptable salt thereof, for use, wherein the inflammatory bowel disease is ulcerative colitis or Crohn's disease.

30. The compound according to claim 28, or a pharmaceutically acceptable salt thereof, for use, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.
